# EUROPEAN PATENT SPECIFICATION

(11) **EP 2 630 256 B1**
(45) Date of publication and mention of the grant of the patent: **05.08.2015**
(21) Application number: 11774032.4
(22) Date of filing: 20.10.2011
(51) Int. Cl.: C12Q 1/68, G01N 33/543

(54) **USE OF SPECIFIC GENES FOR THE PROGNOSIS OF LUNG CANCER AND THE CORRESPONDING PROGNOSIS METHOD**
VERWENDUNG SPEZIFISCHER GENE FÜR DIE PROGNOSE VON LUNGENKREBS UND ENTSPRECHENDES PROGNOSEVERFAHREN
UTILISATION DE GÈNES SPÉCIFIQUES POUR PRONOSTIQUER LE CANCER DU POUMON ET MÉTHODE PRONOSTIQUE CORRESPONDANTE

(30) Priority: 20.10.2010 EP 10306142
(43) Date of publication of application: 28.08.2013
(73) Proprietor: Université Joseph Fourier, 38041 Grenoble Cedex 09 (FR)
(72) Inventor: PISON-ROUSSEAUX, Sophie, F-38410 Saint Martin D'uriage (FR); KHOCHBIN, Saadi, F-38240 Meylan (FR)
(74) Representative: Grosset-Fournier, Chantal Catherine
(86) International application number: PCT/EP2011/068375
(87) International publication number: WO 2012/052524

(56) References cited:
- EP-A1- 2 107 127
- WO-A1-2010/065944
- WO-A2-2006/029176
- WO-A2-2008/082730
- US-A1- 2009 047 689
- GURE ALI O ET AL: "Cancer-testis genes are coordinately expressed and are markers of poor outcome in non-small cell lung cancer.", CLINICAL CANCER RESEARCH : AN OFFICIAL JOURNAL OF THE AMERICAN ASSOCIATION FOR CANCER RESEARCH 15 NOV 2005 LNKD- PUBMED:16299236, vol. 11, no. 22, 15 November 2005 (2005-11-15), pages 8055-8062, XP002621272, ISSN: 1078-0432 cited in the application
- LUCAS S ET AL: "MAGE-B5, MAGE-B6, MAGE-C2, and MAGE-C3: Four new members of the MAGE family with tumor-specific expression", INTERNATIONAL JOURNAL OF CANCER, JOHN WILEY & SONS, INC, UNITED STATES, SWITZERLAND, GERMANY, vol. 87, no. 1, 1 July 2000 (2000-07-01), pages 55-60, XP002471717, ISSN: 0020-7136, DOI: DOI:10.1002/1097-0215(20000701)87:1<55::AI D-IJC8>3.0.CO;2-J
- CHEN YAO-TSENG ET AL: "Identification of CT46/HORMAD1, an immunogenic cancer/testis antigen encoding a putative meiosis-related protein", CANCER IMMUNITY, ACADEMY OF CANCER IMMUNOLOGY, CH, vol. 5, 7 July 2005 (2005-07-07), page 9, XP002380625, ISSN: 1424-9634
- KWIATKOWSKI D J ET AL: "Molecular pathologic substaging in 244 stage I non-small-cell lung cancer patients: Clinical implications", JOURNAL OF CLINICAL ONCOLOGY, AMERICAN SOCIETY OF CLINICAL ONCOLOGY, US, vol. 16, no. 7, 1 January 1998 (1998-01-01), pages 2468-2477, XP009096353, ISSN: 0732-183X

## Description

The present invention relates to the use of specific genes for the prognosis of lung cancer and the corresponding prognosis method.

Lung cancer is a disease of uncontrolled cell growth in tissues of the lung. This growth may lead to metastasis, which is the invasion of adjacent tissues and infiltration beyond the lungs. The vast majority of primary lung cancers are carcinomas of the lung, derived from epithelial cells. Lung cancer, the most common cause of cancer-related death in men and women, is responsible for 1,3 million deaths worldwide annually, as of 2004. The most common symptoms are shortness of breath, coughing (including coughing up blood), and weight loss.

Due to the high prevalence of this type of tumors, there is a need to efficiently diagnose lung cancer. Moreover, it is important to propose a prognosis method that allows the pathologist to determine, when a patient is afflicted by lung tumors, the survival rate during a short and a long period, and consequently to propose an adapted therapy.

Presently, several clinical and pathological parameters help defining the prognosis, including histological subtypes, TNM stages (tumour size, presence of tumour cells in lymph nodes, presence of distant metastasis).

Classically, prognosis and diagnosis methods intend to detect the variation of expression of genes between the sample from a patient and a healthy control sample. However, with these methods, false positive results are frequent and indistinguishable from real positive samples.

Cancer Testis (CT) genes are genes that are expressed in testis cells, but not expressed in somatic non pathologic cells. In cancers, CT genes are deregulated and are expressed ectopically in somatic cells. They appear as good candidate for cancer diagnosis.

Some works have intended to identify a "general" strategy for diagnosing lung cancer, by detecting cancer testis gene expression.

For instance, European application EP 2 107 127 A discloses genes with cancer/testis-restricted expression whose expression is indicative of the progression of cancer, e.g. lung cancer. The final list of 20 preferred genes comprises MGC27016.

The international application WO 2009/121878 discloses the use of a minimal group of CT genes for identifying any somatic or ovarian cancer. However, even if specific genes, or combinations of genes, can be used for diagnosing cancer, there is no indication that these genes can be used to establish a reliable prognosis during a short or a long period.
Recently, Gure et al. (Gure at al. Clin Cancer reaserch, 2005, 11(22) p:8055-8061) have proposed that cancer testis genes are coordinatly expressed in non-small cell lung cancers, and are markers of poor outcome. The study suggests that X-linked CT genes can be associated with worse prognosis, either by their expression, or by their increased expression.
Therefore, there is a need to provide prognosis marker that can give specific evolution of tumoral progression, and patient survival, for instance by using CT genes.
One aim of the invention is to a simple, rapid, easy-to-use and effective method for giving a prognosis of lung cancer.
Another aim of the invention is to provide a general prognosis method of lung tumor. Another aim of the invention is to provide a kit for diagnosing lung cancer.

The present invention relates to the use of
- at least 13 genes chosen among a set of 28 genes comprising or consisting of the nucleic acid sequences SEQ ID NO 70 to 97,
- or complementary sequences of said least 13 genes chosen among a set of 28 genes comprising or consisting of the nucleic acid sequences SEQ ID NO 70 to 97,
- or sequences having at least 80% homology with said genes,
- or proteins coded by said at least 13 genes chosen among a set of 28 genes comprising or consisting of said nucleic acid sequences,
said at least 13 genes being such that
- 12 genes comprise or consist of the nucleic acid sequences SEQ ID NO: 70 to 81, and
- at least one gene belongs to a subset of 16 genes comprising or consisting of the nucleic acid sequences SEQ ID NO: 82-97,
for carrying out a method for identifying at least 66% of patients having a survival rate of at most about 20% 30 months after the diagnosis of their lung cancer, among a population of patients afflicted by lung cancer having an estimated survival rate of at least 30% 30 months after the diagnosis of their lung cancer based on the diagnosis of said lung cancer according to histopathological criteria,
said method comprising a step of measuring, in a biological sample of said patients, the expression of said at least 13 genes, or said complementary sequences of said at least 13 genes, or said sequences having at least 80% homology with said genes or said proteins coded by said at least 13 genes.

The present disclosure also described the use of
- at least 2 genes chosen among a set of 23 genes comprising or consisting of the nucleic acid sequences SEQ ID NO 1 to 23,
- or fragments of said genes,
- or complementary sequences of said genes,
- or sequences having at least 80% homology with said genes or fragment thereof,
- or protein coded by said genes,
- or fragments of said proteins,
- or antibodies directed against said proteins,
said at least 2 genes being such that
- at least one gene belongs to a first set A of 7 genes comprising or consisting of the nucleic acid sequences SEQ ID NO: 1-7
- at least one gene belongs to a second set B of 16 genes comprising or consisting of the nucleic acid sequences SEQ ID NO: 8-23
for the implementation of a prognosis method, preferably *in vitro,* of the survival rate of a patient afflicted by a lung cancer, said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one set A or B is expressed, the patient survival rate during a period of time from 30 to 120 months after the diagnosis of said lung cancer is about from about 3% to about 70%.

The invention also relates to an in vitro method for identifying patients afflicted by a lung cancer having a survival rate of at most about 20% 30 months after the diagnosis of their lung cancer, among a population of patients afflicted by lung cancer having an estimated survival rate of at least 30% 30 months after the diagnosis of their lung cancer based on the diagnosis of said lung cancer according to histopathological criteria,
said method allowing the identification of at least 66% of patients afflicted by a lung cancer having a survival rate of at most about 20% 30 months after the diagnosis of their lung cancer,
said method comprising
* a step of measuring, in a biological sample of said patients, the expression of
   - at least 13 genes chosen among a set of 28 genes comprising or consisting of the nucleic acid sequences SEQ ID NO 70 to 97,
   - or complementary sequences of said genes
said at least 13 genes being such that
   - 12 genes comprise or consist of the nucleic acid sequences SEQ ID NO: 70 to 81, and
   - at least one gene belongs to a subset of 16 genes comprising or consisting of the nucleic acid sequences SEQ ID NO 82-97, and
*a step of identifying biological samples expressing said at least 13 genes.

Preferred embodiments of the invention are as defined in the claims.

The present invention is based on the unexpected observation made by the Inventors that the expression of at least 13 genes of a group of 28 determined genes is sufficient to determine the survival rate of a patient afflicted by a lung cancer.

In other words, and as explained and exemplified hereafter, according to the invention the determination of the gene expression status on a ON/OFF basis of at least 13 genes chosen among a set of 28 -genes allows to estimate at 30 months, after the diagnosis of a lung cancer, the probability of survival of an individual.

The diagnosis method proposed by the Inventors can also be carried out by detecting the expression of proteins expressed by at least 13 genes above mentioned, chosen among proteins coded by said 28 genes, also on a absence/presence basis.

A key aspect of the invention is the concept of ON/OFF for gene expression. The concept of ON/OFF expression can be extended to presence/absence of the proteins and antibodies detecting these proteins. This specific approach has the advantage of simplifying the analyses and making them independent of complex statistical tests to measure variations in expression levels applied to the majority of the existing tests.

The ON/OFF status of gene expression is established by determination of a threshold of gene expression allowing them to decide on the ON/OFF status of a gene such that:
- if a gene is expressed at a level lower than the threshold, the gene is considered as not expressed or weakly expressed (defined as OFF), and
- if a gene is expressed at a level upper to the threshold, the gene is considered as being expressed (defined as ON).

According to the invention, the prognosis is carried out as described hereafter:
The Inventors have identified 23 genes comprising or being constituted by the nucleic acid sequences SEQ ID NO 1 to 23, as being cancer testis genes (CT genes) that can be used to carry out a prognosis method also described herein.

The above 23 genes have been identified as being liable to be "expressed" (form here by, "expressed" refers to the ON status and "not expressed" to the OFF status) in lung cancer cells, but not in healthy samples. In other words, the above 23 genes are such as
- they are not expressed, or weakly expressed in healthy lung cells, and
- they maybe expressed in lung tumor cells.

The difference between the absence of expression, or weak expression, and the expression determines its ON/OFF status, which is a key step of the invention.

Indeed, the Inventors have identified that the ON status of the above 23 genes is a key step to determine the prognosis of lung cancer.

On microarrays, the expression level of the above mentioned genes is determined by the fact that a threshold of expression has been identified by the Inventors allowing to determine expression (ON) and non-expression (OFF) of said genes. The threshold determination is detailed hereafter, in the Example section.

For the microarrays, the threshold enabling to determine the expression status of a gene (ON versus OFF) is calculated by using the signal mean value and distribution obtained from transcriptomic data (in the same technology) with the corresponding probes in a large number of somatic tissues (which do not express the genes).

A similar strategy enables determining a threshold for the presence/absence of the encoded proteins or antibodies. For each protein or antibody, the mean value and distribution of the signal intensities obtained in an appropriate number of control somatic tissues serves as a basis for calculating the threshold.

By "not expressed" it is defined in the invention the fact that the transcription of a gene is either not carried out, or is not detectable by common techniques known in the art, such as Quantitative RT-PCR, Northern blot or when microarrays data are considered.

By "weakly expressed", it is defined in the invention that a gene is expressed at a low level, meaning that the values are within the range of those measured for healthy tissue samples by Q-RT-PCR and by Northern blots or below the threshold when microarray data are considered. These values are considered as false-positive expressions, due to probe cross hybridization for instance. All the expression falling in these categories are considered as "OFF"

By "expressed", the invention defined that the transcript of a gene is detectable by the above known techniques while it is not detectable in healthy tissues or determined as being above the threshold when microarrays data are considered.

The 23 genes also described herein have been classified by the Inventors in two sets:
- a first set of 7 genes,
- and a second set of 16 genes.

The first set, also called set A, of 7 genes consists of the genes comprising or constituted by the nucleic acid sequences SEQ ID NO: 1, SEQ ID NO: 2, SEQ ID NO: 3, SEQ ID NO: 4, SEQ ID NO: 5, SEQ ID NO: 6 and SEQ ID NO: 7. The second set, also called set B, of 16 genes consists of the genes comprising or constituted by the nucleic acid sequences SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, and SEQ ID NO: 23.

The inventors have also defined subsets of each of the above sets A and B as follows: Set A is divided into 4 subsets A1, A5, A6 and A7, said subset being such that :
- subset A1 consists of the genes comprising or being constituted by the nucleic acid sequences SEQ ID NO: 1 and SEQ ID NO: 2,
- subset A5 consists of the gene comprising or being constituted by the nucleic acid sequence SEQ ID NO: 3,
- subset A6 consists of the genes comprising or being constituted by the nucleic acid sequence SEQ ID NO: 4, and SEQ ID NO: 5, and
- subset A7 consists of the genes comprising or being constituted by the nucleic acid sequences SEQ ID NO: 6 and SEQ ID NO: 7

Set A can also be divided into the following subsets:
- subset A1 consists of the genes comprising or being constituted by the nucleic acid sequences SEQ ID NO: 1 and SEQ ID NO: 2,
- subset A2 consists of the genes comprising or being constituted by the nucleic acid sequences SEQ ID NO: 1, SEQ ID NO: 2 and SEQ ID NO: 3,
- subset A3 consists of the genes comprising or being constituted by the nucleic acid sequences SEQ ID NO: 1, SEQ ID NO: 2, SEQ ID NO: 3, SEQ ID NO: 4 and SEQ ID NO: 5, and
- subset A4 consists of the genes comprising or being constituted by the nucleic acid sequences SEQ ID NO: 1, SEQ ID NO: 2, SEQ ID NO: 3, SEQ ID NO: 4, SEQ ID NO: 5, SEQ ID NO: 6 and SEQ ID NO: 7.

Set B is divided into 3 subsets B1, B4 and B5, said subset being such that:
- subset B1 consists of the genes comprising or being constituted by the nucleic acid sequences SEQ ID NO: 8, SEQ ID NO: 9, SEQ ID NO: 10, SEQ ID NO: 11, SEQ ID NO: 12, SEQ ID NO: 13, SEQ ID NO: 14 and SEQ ID NO: 15,
- subset B4 consists of the genes comprising or being constituted by the nucleic acid sequences SEQ ID NO: 16, SEQ ID NO: 17, SEQ ID NO: 18, SEQ ID NO: 19, SEQ ID NO: 20 and SEQ ID NO: 21, and
- subset B5 consists of the genes comprising or being constituted by the nucleic acid sequences SEQ ID NO: 22, and SEQ ID NO: 23.

Set B can also be divided into the following subsets:
- subset B1 consists of the genes comprising or being constituted by the nucleic acid sequences SEQ ID NO: 8, SEQ ID NO: 9, SEQ ID NO: 10, SEQ ID NO: 11, SEQ ID NO: 12, SEQ ID NO: 13, SEQ ID NO: 14 and SEQ ID NO: 15,
- subset B2 consists of the genes comprising or being constituted by the nucleic acid sequences SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20 and SEQ ID NO: 21, and
- subset B3 consists of the genes comprising or being constituted by the nucleic acid sequences SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20 and SEQ ID NO: 21, SEQ ID NO: 22, and SEQ ID NO: 23.

Thus, the prognosis method described herein is such that, when determining the expression status of 2 genes on a ON/OFF basis, belonging to the set of 23 genes comprising or consisting of nucleic acid sequences SEQ ID NO : 1 to 23,
- if none of said at least 2 genes are expressed, the patient survival rate at 30 months, 60 months or 120 months following the diagnosis is from about 59% to about 78% or more, and
- if at least one of said at least two genes is expressed, according to the defined expression threshold, the patient survival rate at 30 months, 60 months or 120 months following the diagnosis is from about 3% to about 70%.

Accordingly, the above mentioned at least 2 genes are such that:
- at least one of said at least two genes belongs to a first set A of 7 genes comprising or consisting of nucleic acid sequences SEQ ID NO : 1 to 7, and
- at least one of said at least two genes belongs to a first set B of 16 genes comprising or consisting of nucleic acid sequences SEQ ID NO : 8 to 23.

Therefore, the prognosis method described herein can be carried out as by measuring at least the expression of the following 112 couples of genes: SEQ ID NO: 1 + SEQ ID NO: 8, SEQ ID NO: 1 + SEQ ID NO: 9, SEQ ID NO: 1 + SEQ ID NO: 10, SEQ ID NO: 1 + SEQ ID NO: 11, SEQ ID NO: 1 + SEQ ID NO: 12, SEQ ID NO: 1 + SEQ ID NO: 13, SEQ ID NO: 1 + SEQ ID NO: 14, SEQ ID NO: 1 + SEQ ID NO: 15, SEQ ID NO: 1 + SEQ ID NO: 16, SEQ ID NO: 1 + SEQ ID NO: 17, SEQ ID NO: 1 + SEQ ID NO: 18, SEQ ID NO: 1 + SEQ ID NO: 19, SEQ ID NO: 1 + SEQ ID NO: 20, SEQ ID NO: 1 + SEQ ID NO: 21, SEQ ID NO: 1 + SEQ ID NO: 22, SEQ ID NO: 1 + SEQ ID NO: 23, SEQ ID NO: 2 + SEQ ID NO: 8, SEQ ID NO: 2 + SEQ ID NO: 9, SEQ ID NO: 2 + SEQ ID NO: 10, SEQ ID NO: 2 + SEQ ID NO: 11, SEQ ID NO: 2 + SEQ ID NO: 12, SEQ ID NO: 2 + SEQ ID NO: 13, SEQ ID NO: 2 + SEQ ID NO: 14, SEQ ID NO: 2 + SEQ ID NO: 15, SEQ ID NO: 2 + SEQ ID NO: 16, SEQ ID NO: 2 + SEQ ID NO: 17, SEQ ID NO: 2 + SEQ ID NO: 18, SEQ ID NO: 2 + SEQ ID NO: 19, SEQ ID NO: 2 + SEQ ID NO: 20, SEQ ID NO: 2 + SEQ ID NO: 21, SEQ ID NO: 2 + SEQ ID NO: 22, SEQ ID NO: 2 + SEQ ID NO: 23, SEQ ID NO: 3 + SEQ ID NO: 8, SEQ ID NO: 3 + SEQ ID NO: 9, SEQ ID NO: 3 + SEQ ID NO: 10, SEQ ID NO: 3 + SEQ ID NO: 11, SEQ ID NO: 3 + SEQ ID NO: 12, SEQ ID NO: 3 + SEQ ID NO: 13, SEQ ID NO: 3 + SEQ ID NO: 14, SEQ ID NO: 3 + SEQ ID NO: 15, SEQ ID NO: 3 + SEQ ID NO: 16, SEQ ID NO: 3 + SEQ ID NO: 17, SEQ ID NO: 3 + SEQ ID NO: 18, SEQ ID NO: 3 + SEQ ID NO: 19, SEQ ID NO: 3 + SEQ ID NO: 20, SEQ ID NO: 3 + SEQ ID NO: 21, SEQ ID NO: 3 + SEQ ID NO: 22, SEQ ID NO: 3 + SEQ ID NO: 23, SEQ ID NO: 4 + SEQ ID NO: 8, SEQ ID NO: 4 + SEQ ID NO: 9, SEQ ID NO: 4 + SEQ ID NO: 10, SEQ ID NO: 4 + SEQ ID NO: 11, SEQ ID NO: 4 + SEQ ID NO: 12, SEQ ID NO: 4 + SEQ ID NO: 13, SEQ ID NO: 4 + SEQ ID NO: 14, SEQ ID NO: 4 + SEQ ID NO: 15, SEQ ID NO: 4 + SEQ ID NO: 16, SEQ ID NO: 4 + SEQ ID NO: 17, SEQ ID NO: 4 + SEQ ID NO: 18, SEQ ID NO: 4 + SEQ ID NO: 19, SEQ ID NO: 4 + SEQ ID NO: 20, SEQ ID NO: 4 + SEQ ID NO: 21, SEQ ID NO: 4 + SEQ ID NO: 22, SEQ ID NO: 4 + SEQ ID NO: 23, SEQ ID NO: 5 + SEQ ID NO: 8, SEQ ID NO: 5 + SEQ ID NO: 9, SEQ ID NO: 5 + SEQ ID NO: 10, SEQ ID NO: 5 + SEQ ID NO: 11, SEQ ID NO: 5 + SEQ ID NO: 12, SEQ ID NO: 5 + SEQ ID NO: 13, SEQ ID NO: 5 + SEQ ID NO: 14, SEQ ID NO: 5 + SEQ ID NO: 15, SEQ ID NO: 5 + SEQ ID NO: 16, SEQ ID NO: 5 + SEQ ID NO: 17, SEQ ID NO: 5 + SEQ ID NO: 18, SEQ NO: 5 + SEQ ID NO: 19, SEQ ID NO: 5 + SEQ ID NO: 20, SEQ ID NO: 5 + SEQ ID NO: 21, SEQ ID NO: 5 + SEQ ID NO: 22, SEQ ID NO: 5 + SEQ ID NO: 23, SEQ ID NO: 6 + SEQ ID NO: 8, SEQ ID NO: 6 + SEQ ID NO: 9, SEQ ID NO: 6 + SEQ ID NO: 10, SEQ ID NO: 6 + SEQ ID NO: 11, SEQ ID NO: 6 + SEQ ID NO: 12, SEQ ID NO: 6 + SEQ ID NO: 13, SEQ ID NO: 6 + SEQ ID NO: 14, SEQ ID NO: 6 + SEQ ID NO: 15, SEQ ID NO: 6 + SEQ ID NO: 16, SEQ ID NO: 6 + SEQ ID NO: 17, SEQ ID NO: 6 + SEQ ID NO: 18, SEQ ID NO: 6 + SEQ ID NO: 19, SEQ ID NO: 6 + SEQ ID NO: 20, SEQ ID NO: 6 + SEQ ID NO: 21, SEQ ID NO: 6 + SEQ ID NO: 22, SEQ ID NO: 6 + SEQ ID NO: 23, SEQ ID NO: 7 + SEQ ID NO: 8, SEQ ID NO: 7 + SEQ ID NO: 9, SEQ ID NO: 7 + SEQ ID NO: 10, SEQ ID NO: 7 + SEQ ID NO: 11, SEQ ID NO: 7 + SEQ ID NO: 12, SEQ ID NO: 7 + SEQ ID NO: 13, SEQ ID NO: 7 + SEQ ID NO: 14, SEQ ID NO: 7 + SEQ ID NO: 15, SEQ ID NO: 7 + SEQ ID NO: 16, SEQ ID NO: 7 + SEQ ID NO: 17, SEQ ID NO: 7 + SEQ ID NO: 18, SEQ ID NO: 7 + SEQ ID NO: 19, SEQ ID NO: 7 + SEQ ID NO: 20, SEQ ID NO: 7 + SEQ ID NO: 21, SEQ ID NO: 7 + SEQ ID NO: 22 and SEQ ID NO: 7 + SEQ ID NO: 23.

For instance, the prognosis method described herein can be carried out by determining the expression status of the above couple SEQ ID NO: 1 + SEQ ID NO: 8, wherein
- if neither SEQ ID NO: 1 nor SEQ ID NO : 8 is expressed, the patient survival rate at 30 months, 60 months or 120 months following the diagnosis is from about 59% to about 78% or more, and
- if either SEQ ID NO: 1 or SEQ ID NO: 8 or both SEQ ID NO: 1+ SEQ ID NO: 8 is(are) expressed, the patient survival rate at 30 months, 60 months or 120 months following the diagnosis is from about 3% to about 70%.

According to the invention, the terms "about X%" means that the percentage of survival proposed for the prognosis method have to be considered with a standard deviation corresponding to individual variability. This standard deviation is about 5%.

By "at least 2 genes/proteins/antibodies chosen among a set of 23 genes/proteins/antibodies", it is defined in the invention : 2 genes/proteins/antibodies, or 3 genes/proteins/antibodies, or 4 genes/proteins/antibodies, or 5 genes/proteins/antibodies, or 6 genes/proteins/antibodies, or 7 genes/proteins/antibodies, or 8 genes/proteins/antibodies, or 9 genes/proteins/antibodies, or 10 genes/proteins/antibodies, or 11 genes/proteins/antibodies, or 12 genes/proteins/antibodies, or 13 genes/proteins/antibodies, or 14 genes/proteins/antibodies, or 15 genes/proteins/antibodies, or 16 genes/proteins/antibodies, or 17 genes/proteins/antibodies, or 18 genes/proteins/antibodies, or 19 genes/proteins/antibodies, or 20 genes/proteins/antibodies, or 21 genes/proteins/antibodies, or 22 genes/proteins/antibodies, or 23 genes/proteins/antibodies.

To summarise, the subject-matter also described herein relates to the use of
- at least 2 genes chosen among a set of 23 genes comprising or consisting of the nucleic acid sequences SEQ ID NO 1 to 23,
- or fragments of said least 2 genes chosen among a set of 23 genes comprising or consisting of the nucleic acid sequences SEQ ID NO 1 to 23,
- or complementary sequences of said least 2 genes chosen among a set of 23 genes comprising or consisting of the nucleic acid sequences SEQ ID NO 1 to 23,
- or sequences having at least 80% homology with said genes or fragment thereof,
   - or proteins coded by said least 2 genes chosen among a set of 23 genes comprising or consisting of the nucleic acid sequences SEQ ID NO 1 to 23, said proteins comprising or consisting in amino acid sequences SEQ ID NO 24 to 46
   - or fragments of said proteins comprising or consisting in amino acid sequences SEQ ID NO 24 to 46,
   - or antibodies directed against said proteins comprising or consisting in amino acid sequences SEQ ID NO 24 to 46,
said
◆ at least 2 genes being such that
   - at least one gene belongs to a first set A of 7 genes comprising or consisting of the nucleic acid sequences SEQ ID NO: 1-7
   - at least one gene belongs to a second set B of 16 genes comprising or consisting of the nucleic acid sequences SEQ ID NO: 8-23,
◆ at least 2 proteins being such that
   - at least one protein belongs to a first set AP of 7 proteins comprising or consisting of the amino acid sequences SEQ ID NO: 24-30,
   - at least one protein belongs to a second set BP of 16 proteins comprising or consisting of the amino acid sequences SEQ ID NO: 31-46,
◆ at least 2 antibodies directed against said 2 proteins being such that
   - at least one antibody specifically recognises one protein that belongs to a first set AP of 7 proteins comprising or consisting of the amino acid sequences SEQ ID NO: 24-30,
   - at least one antibody specifically recognises one protein that belongs to a second set BP of 16 proteins comprising or consisting of the amino acid sequences SEQ ID NO: 31-46,
for the implementation of a prognosis method, preferably *in vitro,* of the survival rate of a patient afflicted by a lung cancer, said prognosis being such that:
either
▪ if none of the 23 genes of said set is expressed, the patient survival rate during a period of time from 30 to 120 months after the diagnosis of said lung cancer is from about 59% to about 78% or more, and
▪ if at least one gene of at least one set A or B is expressed, the patient survival rate during a period of time from 30 to 120 months after the diagnosis of said lung cancer is about from about 3% to about 70%,
or
▪ if none of the 23 proteins of said set is expressed, the patient survival rate during a period of time from 30 to 120 months after the diagnosis of said lung cancer is from about 59% to about 78% or more, and
▪ if at least one protein of at least one set AP or BP is expressed, the patient survival rate during a period of time from 30 to 120 months after the diagnosis of said lung cancer is about from about 3% to about 70%,
or
▪ if none of the antibodies directed against said 23 proteins of said set is expressed, the patient survival rate during a period of time from 30 to 120 months after the diagnosis of said lung cancer is from about 59% to about 78% or more, and
▪ if at least one antibody directed against one protein of at least one set AP or BP is expressed, the patient survival rate during a period of time from 30 to 120 months after the diagnosis of said lung cancer is about from about 3% to about 70%,
wherein said gene, protein or antibody is determined as being expressed when:
- either it is expressed in a sample of a patient afflicted by a lung cancer but not in a control sample of an healthy individual,
- or it is expressed above a threshold corresponding to a background signal observed in a series of reference healthy tissues for each detection method, and
said gene, protein or antibody is determined as being not expressed when:
- it is neither expressed in a sample of a patient afflicted by a lung cancer nor in a control sample of an healthy individual,
- or it is expressed in a sample of a patient afflicted by a lung cancer at a level substantially equal or inferior to the level in a control sample of an healthy individual.

According to the invention, "a control sample of an healthy individual" corresponds to a somatic tissue in which the CT gene is not expressed or weakly expressed as defined above, said somatic tissue originating from a person not afflicted by cancer.

In the invention "is expressed at a level above a threshold corresponding to a background signal observed in a series of reference healthy tissues for each detection method" means that the threshold, which corresponds to the key step of the invention, is determined by measuring the background signal in negative control samples of healthy tissues, in which there is no expression of CT genes.

This background signal depends upon the method used to carry out the invention. However, it is easy for a skilled person to measure such threshold whatever the method used, i.e.:
- if the number of control samples is significantly statistically representative, e.g. at least 30 independent control samples, and the background signal of these control sample follows a normal distribution (Gaussian distribution), the threshold is determined by the mean + 2 standard deviations of the background signal measured in the control samples,
- If the number of control sample is not statistically representative, e.g. less than 30 independent control samples, or if the background signal of these control sample does not follow a normal distribution, the threshold is determined as being the maximal value of the background signal measured in the control samples.

According to the present disclosure, the gene comprising or consisting of the nucleic acid sequence SEQ ID NO : 1 codes for the protein comprising or consisting of the amino acid sequence SEQ ID NO : 24, the gene comprising or consisting of the nucleic acid sequence SEQ ID NO : 2 codes for the protein comprising or consisting of the amino acid sequence SEQ ID NO : 25, the gene comprising or consisting of the nucleic acid sequence SEQ ID NO : 3 codes for the protein comprising or consisting of the amino acid sequence SEQ ID NO : 26, the gene comprising or consisting of the nucleic acid sequence SEQ ID NO : 4 codes for the protein comprising or consisting of the amino acid sequence SEQ ID NO : 27, the gene comprising or consisting of the nucleic acid sequence SEQ ID NO : 5 codes for the protein comprising or consisting of the amino acid sequence SEQ ID NO : 28, the gene comprising or consisting of the nucleic acid sequence SEQ ID NO : 6 codes for the protein comprising or consisting of the amino acid sequence SEQ ID NO : 29, the gene comprising or consisting of the nucleic acid sequence SEQ ID NO : 7 codes for the protein comprising or consisting of the amino acid sequence SEQ ID NO : 30, the gene comprising or consisting of the nucleic acid sequence SEQ ID NO : 8 codes for the protein comprising or consisting of the amino acid sequence SEQ ID NO : 31, the gene comprising or consisting of the nucleic acid sequence SEQ ID NO : 9 codes for the protein comprising or consisting of the amino acid sequence SEQ ID NO : 32, the gene comprising or consisting of the nucleic acid sequence SEQ ID NO : 10 codes for the protein comprising or consisting of the amino acid sequence SEQ ID NO : 33, the gene comprising or consisting of the nucleic acid sequence SEQ ID NO : 11 codes for the protein comprising or consisting of the amino acid sequence SEQ ID NO : 34, the gene comprising or consisting of the nucleic acid sequence SEQ ID NO : 12 codes for the protein comprising or consisting of the amino acid sequence SEQ ID NO : 35, the gene comprising or consisting of the nucleic acid sequence SEQ ID NO : 13 codes for the protein comprising or consisting of the amino acid sequence SEQ ID NO : 36, the gene comprising or consisting of the nucleic acid sequence SEQ ID NO : 14 codes for the protein comprising or consisting of the amino acid sequence SEQ ID NO : 37, the gene comprising or consisting of the nucleic acid sequence SEQ ID NO : 15 codes for the protein comprising or consisting of the amino acid sequence SEQ ID NO : 38, the gene comprising or consisting of the nucleic acid sequence SEQ ID NO : 16 codes for the protein comprising or consisting of the amino acid sequence SEQ ID NO : 39, the gene comprising or consisting of the nucleic acid sequence SEQ ID NO : 17 codes for the protein comprising or consisting of the amino acid sequence SEQ ID NO : 40, the gene comprising or consisting of the nucleic acid sequence SEQ ID NO : 18 codes for the protein comprising or consisting of the amino acid sequence SEQ ID NO : 41, the gene comprising or consisting of the nucleic acid sequence SEQ ID NO : 19 codes for the protein comprising or consisting of the amino acid sequence SEQ ID NO : 42, the gene comprising or consisting of the nucleic acid sequence SEQ ID NO : 20 codes for the protein comprising or consisting of the amino acid sequence SEQ ID NO : 43, the gene comprising or consisting of the nucleic acid sequence SEQ ID NO : 21 codes for the protein comprising or consisting of the amino acid sequence SEQ ID NO : 44, the gene comprising or consisting of the nucleic acid sequence SEQ ID NO : 22 codes for the protein comprising or consisting of the amino acid sequence SEQ ID NO : 45 and the gene comprising or consisting of the nucleic acid sequence SEQ ID NO : 23 codes for the protein comprising or consisting of the amino acid sequence SEQ ID NO : 46.

The 23 genes according to the present disclosure code for 23 proteins. The 23 proteins have been classified by the Inventors in two sets:
- a first set of 7 proteins,
- and a second set of 16 proteins.

The first set, also called set AP, of 7 proteins consists of the proteins comprising or constituted by the amino acid sequences SEQ ID NO: 24, SEQ ID NO: 25, SEQ ID NO: 26, SEQ ID NO: 27, SEQ ID NO: 28, SEQ ID NO: 29 and SEQ ID NO: 30.

The second set, also called set BP, of 16 proteins consists of the proteins comprising or constituted by the amino acid sequences SEQ ID NO: 31, SEQ ID NO: 32, SEQ ID NO: 33, SEQ ID NO: 34, SEQ ID NO: 35, SEQ ID NO: 36, SEQ ID NO: 37, SEQ NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, and SEQ ID NO: 46.

The inventors have also defined subsets of each of the above sets AP and BP as follows:
Set AP is divided into 4 subsets AP1, AP5 A6 and AP7, said subset being such that:
   - subset AP1 consists of the proteins comprising or being constituted by the amino acid sequences SEQ ID NO: 24 and SEQ ID NO: 25,
   - subset AP5 consists of the protein comprising or being constituted by the amino acid sequence SEQ ID NO: 26,
   - subset AP6 consists of the proteins comprising or being constituted by the amino acid sequences SEQ ID NO: 27 and SEQ ID NO: 28, and
   - subset AP7 consists of the proteins comprising or being constituted by the amino acid sequences SEQ ID NO: 29 and SEQ ID NO: 30.
Set AP can also be divided into the following subsets :
   - subset AP1 consists of the proteins comprising or being constituted by the amino acid sequences SEQ ID NO: 24 and SEQ ID NO: 25,
   - subset AP2 consists of the proteins comprising or being constituted by the amino acid sequences SEQ ID NO: 24, SEQ ID NO: 25 and SEQ ID NO: 26,
   - subset AP3 consists of the proteins comprising or being constituted by the amino acid sequences SEQ ID NO: 24, SEQ ID NO: 25, SEQ ID NO: 26, SEQ ID NO: 27 and SEQ ID NO: 28, and
   - subset AP4 consists of the proteins comprising or being constituted by the amino acid sequences SEQ ID NO: 24, SEQ ID NO: 25, SEQ ID NO: 26, SEQ ID NO: 27, SEQ ID NO: 28, SEQ ID NO: 29 and SEQ ID NO: 30.
Set BP is divided into 3 subsets BP1, BP4 and BP5, said subset being such that:
   - subset BP1 consists of the proteins comprising or being constituted by the amino acid sequences SEQ ID NO: 31, SEQ ID NO: 32, SEQ ID NO: 33, SEQ ID NO: 34, SEQ ID NO: 35, SEQ ID NO: 36, SEQ ID NO: 37 and SEQ ID NO: 38,
   - subset BP4 consists of the proteins comprising or being constituted by the amino acid sequences SEQ ID NO: 39, SEQ ID NO: 40, SEQ ID NO: 41, SEQ ID NO: 42, SEQ ID NO: 43 and SEQ ID NO: 44, and
   - subset BP5 consists of the proteins comprising or being constituted by the amino and sequences SEQ ID NO: 45, and SEQ ID NO: 46.
Set BP can also be divided into the following subsets :
   - subset BP1 consists of the proteins comprising or being constituted by the amino acid sequences SEQ ID NO: 31, SEQ ID NO: 32, SEQ ID NO: 33, SEQ ID NO: 34, SEQ ID NO: 35, SEQ ID NO: 36, SEQ ID NO: 37 and SEQ ID NO: 38,
   - subset BP2 consists of the proteins comprising or being constituted by the amino acid sequences SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43 and SEQ ID NO: 44, and
   - subset BP3 consists of the proteins comprising or being constituted by the amino acid sequences SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, and SEQ ID NO: 46.

The present disclosure also describes the use of
- at least 2 genes chosen among a set of 23 genes comprising or consisting of the nucleic acid sequences SEQ ID NO 1 to 23,
- or fragments of said genes,
- or complementary sequences of said genes,
- or sequences having at least 80% homology with said genes or fragment thereof,
- or proteins coded by said genes,
- or fragments of said proteins,
- or antibodies directed against said proteins,
said at least 2 genes being such that
- at least one gene belongs to a subset A1 of a first set A of 7 genes comprising or consisting of the nucleic acid sequences SEQ ID NO: 1-7, said subset A1 comprising or consisting of nucleic acid sequences SEQ ID NO : 1 or 2
- at least one gene belongs to a subset B1 of a second set B of 16 genes comprising or consisting of the nucleic acid sequences SEQ ID NO: 8-23, said subset B1 comprising or consisting of nucleic acid sequences SEQ ID NO: 8 to 15
for the implementation of a prognosis method, preferably *in vitro,* of the survival rate of a patient afflicted by a lung cancer, said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one of the subset A1 or B1 is expressed, the patient survival rate during a period of time from 30 to 120 months after the diagnosis of said lung cancer is about from about 3% to about 70%.

**The** prognosis method is carried out by using at least one of the following couples of genes:
SEQ ID NO: 1 + SEQ ID NO: 8, SEQ ID NO: 1 + SEQ ID NO: 9, SEQ ID NO: 1 + SEQ ID NO: 10, SEQ ID NO: 1 + SEQ ID NO: 11, SEQ ID NO: 1 + SEQ ID NO: 12, SEQ ID NO: 1 + SEQ ID NO: 13, SEQ ID NO: 1 + SEQ ID NO: 14, SEQ ID NO: 1 + SEQ ID NO: 15, SEQ ID NO: 2 + SEQ ID NO: 8, SEQ ID NO: 2 + SEQ ID NO: 9, SEQ ID NO: 2 + SEQ ID NO: 10, SEQ ID NO: 2 + SEQ ID NO: 11, SEQ ID NO: 2 + SEQ ID NO: 12, SEQ ID NO: 2 + SEQ ID NO: 13, SEQ ID NO: 2 + SEQ ID NO: 14 and SEQ ID NO: 2 + SEQ ID NO: 15.

The above 16 couples are sufficient to define a significant prognosis over 120 month of lung cancer.

Any other supplementary genes belonging to the group of 23 genes according to the present disclosure can be used in order to affine the prognosis according to the present disclosure.

The present disclosure also describes the use of
- at least 2 genes chosen among a set of 23 genes comprising or consisting of the nucleic acid sequences SEQ ID NO 1 to 23,
- or fragments of said genes,
- or complementary sequences of said genes,
- or sequences having at least 80% homology with said genes or fragment thereof,
- or proteins coded by said genes,
- or fragments of said proteins,
- or antibodies directed against said proteins,
said at least 2 genes being such that
- at least one gene belongs to a subset A2 of a first set A of 7 genes comprising or consisting of the nucleic acid sequences SEQ ID NO: 1-7, said subset A1 comprising or consisting of nucleic acid sequences SEQ ID NO : 1 to 3
- at least one gene belongs to a subset B2 of a second set B of 16 genes comprising or consisting of the nucleic acid sequences SEQ ID NO: 8-23, said subset B1 comprising or consisting of nucleic acid sequences SEQ ID NO : 8 to 21
for the implementation of a prognosis method, preferably *in vitro,* of the survival rate of a patient afflicted by a lung cancer, said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one of the subset A2 or B2 is expressed, the patient survival rate during a period of time from 30 to 120 months after the diagnosis of said lung cancer is about from about 3% to about 70%.

The prognosis method is carried out by using at least one of the following couples of genes:
SEQ ID NO: 1 + SEQ ID NO: 8, SEQ ID NO: 1 + SEQ ID NO: 9, SEQ ID NO: 1 + SEQ ID NO: 10, SEQ ID NO: 1 + SEQ ID NO: 11, SEQ ID NO: 1 + SEQ ID NO: 12, SEQ ID NO: 1 + SEQ ID NO: 13, SEQ ID NO: 1 + SEQ ID NO: 14, SEQ ID NO: 1 + SEQ ID NO: 15, SEQ ID NO: 1 + SEQ ID NO: 16, SEQ ID NO: 1 + SEQ ID NO: 17, SEQ ID NO: 1 + SEQ ID NO: 18, SEQ ID NO: 1 + SEQ ID NO: 19, SEQ ID NO: 1 + SEQ ID NO: 20, SEQ ID NO: 1 + SEQ ID NO: 21, SEQ ID NO: 2 + SEQ ID NO: 8, SEQ ID NO: 2 + SEQ ID NO: 9, SEQ ID NO: 2 + SEQ ID NO: 10, SEQ ID NO: 2 + SEQ ID NO: 11, SEQ ID NO: 2 + SEQ ID NO: 12, SEQ ID NO: 2 + SEQ ID NO: 13, SEQ ID NO: 2 + SEQ ID NO: 14, SEQ ID NO: 2 + SEQ ID NO: 15, SEQ ID NO: 2 + SEQ ID NO: 16, SEQ ID NO: 2 + SEQ ID NO: 17, SEQ ID NO: 2 + SEQ ID NO: 18, SEQ ID NO: 2 + SEQ ID NO: 19, SEQ ID NO: 2 + SEQ ID NO: 20, SEQ ID NO: 2 + SEQ ID NO: 21, SEQ ID NO: 3 + SEQ ID NO: 8, SEQ ID NO: 3 + SEQ ID NO: 9, SEQ ID NO: 3 + SEQ ID NO: 10, SEQ NO: 3 + SEQ ID NO: 11, SEQ ID NO: 3 + SEQ ID NO: 12, SEQ ID NO: 3 + SEQ ID NO: 13, SEQ ID NO: 3 + SEQ ID NO: 14, SEQ ID NO: 3 + SEQ ID NO: 15, SEQ ID NO: 3 + SEQ ID NO: 16, SEQ ID NO: 3 + SEQ ID NO: 17, SEQ ID NO: 3 + SEQ ID NO: 18, SEQ ID NO: 3 + SEQ ID NO: 19 and SEQ ID NO: 3 + SEQ ID NO: 20.

The present disclosure also describes the use of
- at least 2 genes chosen among a set of 23 genes comprising or consisting of the nucleic acid sequences SEQ ID NO 1 to 23,
- or fragments of said genes,
- or complementary sequences of said genes,
- or sequences having at least 80% homology with said genes or fragment thereof,
- or proteins coded by said genes,
- or fragments of said proteins,
- or antibodies directed against said proteins,
said at least 2 genes being such that
- at least one gene belongs to a subset A3 of a first set A of 7 genes comprising or consisting of the nucleic acid sequences SEQ ID NO: 1-7, said subset A1 comprising or consisting of nucleic acid sequences SEQ ID NO : 1 to 5
- at least one gene belongs to a subset B2 of a second set B of 16 genes comprising or consisting of the nucleic acid sequences SEQ ID NO: 8-23, said subset B1 comprising or consisting of nucleic acid sequences SEQ ID NO: 8 to 21
for the implementation of a prognosis method, preferably *in vitro,* of the survival rate of a patient afflicted by a lung cancer, said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one of the subset A3 or B2 is expressed, the patient survival rate during a period of time from 30 to 120 months after the diagnosis of said lung cancer is about from about 3% to about 70%.

The prognosis method is carried out by using at least one of the following couples of genes:
SEQ ID NO: 1 + SEQ ID NO: 8, SEQ ID NO: 1 + SEQ ID NO: 9, SEQ ID NO: 1 + SEQ ID NO: 10, SEQ ID NO: 1 + SEQ ID NO: 11, SEQ ID NO: 1 + SEQ ID NO: 12, SEQ ID NO: 1 + SEQ ID NO: 13, SEQ ID NO: 1 + SEQ ID NO: 14, SEQ ID NO: 1 + SEQ ID NO: 15, SEQ ID NO: 1 + SEQ ID NO: 16, SEQ ID NO: 1 + SEQ ID NO: 17, SEQ ID NO: 1 + SEQ ID NO: 18, SEQ ID NO: 1 + SEQ ID NO: 19, SEQ ID NO: 1 + SEQ ID NO: 20, SEQ ID NO: 1 + SEQ ID NO: 21, SEQ ID NO: 1 + SEQ ID NO: 22, SEQ ID NO: 1 + SEQ ID NO: 23, SEQ ID NO: 2 + SEQ ID NO: 8, SEQ ID NO: 2 + SEQ ID NO: 9, SEQ ID NO: 2 + SEQ ID NO: 10, SEQ ID NO: 2 + SEQ ID NO: 11, SEQ ID NO: 2 + SEQ ID NO: 12, SEQ ID NO: 2 + SEQ ID NO: 13, SEQ ID NO: 2 + SEQ ID NO: 14, SEQ ID NO: 2 + SEQ ID NO: 15, SEQ ID NO: 2 + SEQ ID NO: 16, SEQ ID NO: 2 + SEQ ID NO: 17, SEQ ID NO: 2 + SEQ ID NO: 18, SEQ ID NO: 2 + SEQ ID NO: 19, SEQ ID NO: 2 + SEQ ID NO: 20, SEQ ID NO: 2 + SEQ ID NO: 21, SEQ ID NO: 2 + SEQ ID NO: 22, SEQ ID NO: 2 + SEQ ID NO: 23, SEQ ID NO: 3 + SEQ ID NO: 8, SEQ ID NO: 3 + SEQ ID NO: 9, SEQ ID NO: 3 + SEQ ID NO: 10, SEQ ID NO: 3 + SEQ ID NO: 11, SEQ ID NO: 3 + SEQ ID NO: 12, SEQ ID NO: 3 + SEQ ID NO: 13, SEQ ID NO: 3 + SEQ ID NO: 14, SEQ ID NO: 3 + SEQ ID NO: 15, SEQ ID NO: 3 + SEQ ID NO: 16, SEQ ID NO: 3 + SEQ ID NO: 17, SEQ ID NO: 3 + SEQ ID NO: 18, SEQ ID NO: 3 + SEQ ID NO: 19, SEQ ID NO: 3 + SEQ ID NO: 20, SEQ ID NO: 3 + SEQ ID NO: 21, SEQ ID NO: 3 + SEQ ID NO: 22 and SEQ ID NO: 3 + SEQ ID NO: 23.

The present disclosure also describes the use of
- at least 2 proteins chosen among a set of 23 proteins coded by said 23 genes, said 23 proteins comprising or consisting of SEQ ID NO : 24 to 46,
- or fragments of said proteins,
- or antibodies directed against said proteins,
said at least 2 proteins being such that
- at least one protein belongs to a subset AP1 of a first set AP of 7 proteins comprising or consisting of the amino acid sequences SEQ ID NO: 24-30, said subset AP1 comprising or consisting of amino acid sequences SEQ ID NO : 24 or 25
- at least one protein belongs to a subset BP1 of a second set BP of 16 proteins comprising or consisting of the amino acid sequences SEQ ID NO: 31-46, said subset BP1 comprising or consisting of nucleic acid sequences SEQ ID NO : 31 to 38
for the implementation of a prognosis method, preferably *in vitro,* of the survival rate of a patient afflicted by a lung cancer, said prognosis being such that:
▪ if none of the 23 proteins of said set is expressed, the patient survival rate during a period of time from 30 to 120 months after the diagnosis of said lung cancer is from about 59% to about 78% or more, and
▪ if at least one protein of at least one of the subset AP1 or BP1 is expressed, the patient survival rate during a period of time from 30 to 120 months after the diagnosis of said lung cancer is about from about 3% to about 70%.

The prognosis method is carried out by using at least one of the following couples of proteins:
SEQ ID NO: 24 + SEQ ID NO: 31, SEQ ID NO: 24 + SEQ ID NO: 32, SEQ ID NO: 24 + SEQ ID NO: 33, SEQ ID NO: 24 + SEQ ID NO: 34, SEQ ID NO: 24 + SEQ ID NO: 35, SEQ ID NO: 24 + SEQ ID NO: 36, SEQ ID NO: 24 + SEQ ID NO: 37, SEQ ID NO: 24 + SEQ ID NO: 38, SEQ ID NO: 25 + SEQ ID NO: 31, SEQ ID NO: 25 + SEQ ID NO: 32, SEQ ID NO: 25 + SEQ ID NO: 33, SEQ ID NO: 25 + SEQ ID NO: 34, SEQ ID NO: 25 + SEQ ID NO: 35, SEQ ID NO: 25 + SEQ ID NO: 36, SEQ ID NO: 25 + SEQ ID NO: 37 and SEQ ID NO: 25 + SEQ ID NO: 38.

The above 16 couples are sufficient to defined a significant prognosis over 120 month of lung cancer.

The present disclosure. also describes the use of
- at least 2 proteins chosen among a set of 23 proteins coded by said 23 genes, said 23 proteins comprising or consisting of SEQ ID NO : 24 to 46,
- or fragments of said proteins,
- or antibodies directed against said proteins,
said at least 2 proteins being such that
- at least one protein belongs to a subset AP2 of a first set AP of 7 proteins comprising or consisting of the amino acid sequences SEQ ID NO: 24-30, said subset AP2 comprising or consisting of amino acid sequences SEQ ID NO : 24 or 26
- at least one protein belongs to a subset BP2 of a second set BP of 16 proteins comprising or consisting of the amino acid sequences SEQ ID NO: 31-46, said subset BP2 comprising or consisting of nucleic acid sequences SEQ ID NO : 31 to 44
for the implementation of a prognosis method, preferably *in vitro,* of the survival rate of a patient afflicted by a lung cancer, said prognosis being such that:
▪ if none of the 23 proteins of said set is expressed, the patient survival rate during a period of time from 30 to 120 months after the diagnosis of said lung cancer is from about 59% to about 78% or more, and
▪ if at least one protein of at least one of the subset AP2 or BP2 is expressed, the patient survival rate during a period of time from 30 to 120 months after the diagnosis of said lung cancer is about from about 3% to about 70%.

The present disclosure also describes the use of
- at least 2 proteins chosen among a set of 23 proteins coded by said 23 genes, said 23 proteins comprising or consisting of SEQ ID NO : 24 to 46,
- or fragments of said proteins,
- or antibodies directed against said proteins,
said at least 2 proteins being such that
- at least one protein belongs to a subset AP3 of a first set AP of 7 proteins comprising or consisting of the amino acid sequences SEQ ID NO: 24-30, said subset AP1 comprising or consisting of amino acid sequences SEQ ID NO : 24 or 28
- at least one protein belongs to a subset BP2 of a second set BP of 16 proteins comprising or consisting of the amino acid sequences SEQ ID NO: 31-46, said subset B1 comprising or consisting of nucleic acid sequences SEQ ID NO : 31 to 44
for the implementation of a prognosis method, preferably *in vitro,* of the survival rate of a patient afflicted by a lung cancer, said prognosis being such that:
▪ if none of the 23 proteins of said set is expressed, the patient survival rate during a period of time from 30 to 120 months after the diagnosis of said lung cancer is from about 59% to about 78% or more, and
▪ if at least one protein of at least one of the subset AP3 or BP2 is expressed, the patient survival rate during a period of time from 30 to 120 months after the diagnosis of said lung cancer is about from about 3% to about 70%.

The present disclosure also describes the use of
- at least 2 genes chosen among a set of 23 genes comprising or consisting of the nucleic acid sequences SEQ ID NO 1 to 23,
- or fragments of said genes,
- or complementary sequences of said genes,
- or sequences having at least 80% homology with said genes or fragment thereof,
- or proteins coded by said genes,
- or fragments of said proteins,
- or antibodies directed against said proteins,
said at least 2 genes being such that
- at least one gene belongs to a subset A5 of a first set A of 7 genes comprising or consisting of the nucleic acid sequences SEQ ID NO: 1-7, said subset A1 comprising or consisting of nucleic acid sequences SEQ ID NO : 3,
- at least one gene belongs to a subset B4 of a second set B of 16 genes comprising or consisting of the nucleic acid sequences SEQ ID NO: 8-23, said subset B1 comprising or consisting of nucleic acid sequences SEQ ID NO : 16 to 21,
for the implementation of a prognosis method, preferably *in vitro,* of the survival rate of a patient afflicted by a lung cancer, said prognosis being such that:
▪ if none of the 23 genes of said set is expressed, the patient survival rate during a period of time from 30 to 120 months after the diagnosis of said lung cancer is from about 59% to about 78% or more, and
▪ if at least one gene of at least one of the subset A5 or B4 is expressed, the patient survival rate during a period of time from 30 to 120 months after the diagnosis of said lung cancer is about from about 3% to about 70%.

The present disclosure also describes the use of
- at least 2 proteins chosen among a set of 23 proteins coded by said 23 genes, said 23 proteins comprising or consisting of SEQ ID NO : 24 to 46,
- or fragments of said proteins,
- or antibodies directed against said proteins,
said at least 2 proteins being such that
- at least one protein belongs to a subset AP5 of a first set AP of 7 proteins comprising or consisting of the amino acid sequences SEQ ID NO: 24-30, said subset AP5 comprising or consisting of amino acid sequences SEQ ID NO: 26
- at least one protein belongs to a subset BP4 of a second set BP of 16 proteins comprising or consisting of the amino acid sequences SEQ ID NO: 31-46, said subset BP4 comprising or consisting of nucleic acid sequences SEQ ID NO : 39 to 44
for the implementation of a prognosis method, preferably *in vitro,* of the survival rate of a patient afflicted by a lung cancer, said prognosis being such that:
▪ if none of the 23 proteins of said set is expressed, the patient survival rate during a period of time from 30 to 120 months after the diagnosis of said lung cancer is from about 59% to about 78% or more, and
▪ if at least one protein of at least one of the subset AP5 or BP4 is expressed, the patient survival rate during a period of time from 30 to 120 months after the diagnosis of said lung cancer is about from about 3% to about 70%.

The present disclosure also describes the use as mentioned above, wherein said prognosis method is such that:
▪ if none of the 23 genes, or proteins, or antibodies, of said set is expressed, the patient survival rate during a period of time from 30 to 120 months after the diagnosis of said lung cancer is from about 59% to about 78% or more,
▪ if none of the genes or proteins of the set B or BP, or of the subsets B 1, BP1, B2, or BP2, or antibodies, is expressed and at least one gene or protein, or antibody, of the set A or AP, or of the subset A1, AP1, A2, AP2, A3 or AP3 is expressed, the patient survival rate during a period of time from 30 to 120 months after the diagnosis of said lung cancer is from about 27% to about 70%, and
▪ if at least one gene, or protein, of the set B or BP, or of the subsets B1, BP1, B2 or BP2 or antibody, is expressed, the patient survival rate during a period of time from 30 to 120 months after the diagnosis of said lung cancer is from about 3% to about 55%.

The present disclosure also describes the use as mentioned above, wherein said prognosis method is such that:
▪ if none of the 23 genes, of said set is expressed, the patient survival rate during a period of time from 30 to 120 months after the diagnosis of said lung cancer is from about 59% to about 78% or more,
▪ if none of the genes of the set B, or of the subsets B1 or B2 is expressed and at least one gene of the set A or of the subset A1, A2, or A3 is expressed, the survival rate during a period of time from 30 to 120 months after the diagnosis of said lung cancer is from about 27% to about 70%, and
▪ if at least one gene of the set B or of the subsets B1, or B2 is expressed, survival rate during a period of time from 30 to 120 months after the diagnosis of said lung cancer is from about 3% to about 55%.

The present disclosure also describes the use as mentioned above, wherein said prognosis method is such that:
▪ if none of the 23 proteins, of said set is expressed, the patient survival rate during a period of time from 30 to 120 months after the diagnosis of said lung cancer is from about 59% to about 78% or more,
▪ if none of the proteins, of the BP, or of the subsets BPlor BP2, is expressed and at least one protein, of the set AP, or of the subset AP1, AP2, or AP3 is expressed, the patient survival rate during a period of time from 30 to 120 months after the diagnosis of said lung cancer is from about 27% to about 70%, and
▪ if at least one protein, of the set BP, or of the subsets BP1 or BP2 is expressed, the survival rate during a period of time from 30 to 120 months after the diagnosis of said lung cancer is from about 3% to about 55%.

The present disclosure also describes the above defined use, wherein said prognosis method is such that:
▪ if none of the 23 genes or proteins, or antibodies, of said set is expressed, the patient survival rate during a period of time from 30 to 120 months after the diagnosis of said lung cancer is from about 59% to about 78% or more,
▪ if
   o none of the genes or proteins of the set B or BP, or of the subset B1 BP1, B2 or BP2, or antibodies, is expressed and at least 3 genes or proteins of the set A or AP, or of the subset A1 AP1, A2, AP2, A3 or AP3, or antibodies, is expressed, or
   o at least 1 gene or protein of the set B or BP, or of the subset B1, BP1, B2 or BP2, or antibody, is expressed and from none to 2 genes or proteins of the set A or AP, or of the subset A1, AP1, A2, AP2, A3 or AP3, or antibodies, is expressed, or
   o at least 2 genes or proteins of the set B or BP, or of the subset B1, BP1, B2 or BP2, or antibodies, is expressed and no gene or protein, of the set A or AP, or of the subset A1, AP1, A2, AP2, A3 or AP3, or antibodies, is expressed,
the patient survival rate during a period of time from 30 to 120 months after the diagnosis of said lung cancer is from about 27% to about 55%, and
▪ if
   ∘ one gene or protein, of the genes or proteins of the set B or BP, or of the subset B1, BP1, B2 or BP2, or antibodies, is expressed and at least 3 genes or proteins of the set A or AP, or of the subset A1, AP1, A2, AP2, A3 or AP3, or antibodies, are expressed, or
   o at least 2 genes or proteins of the set B or BP, or of the subset B1, BP1, B2 or BP2, or antibodies, are expressed and at least 1 gene or protein of the set A or AP, or of the subset A1, AP1, A2, AP2, A3 or AP3, or antibodies, are expressed,
the patient survival rate during a period of time from 30 to 120 months after the diagnosis of said lung cancer is from about 3% to about 13%.

In the present disclosure, "from none to 2" corresponds to none, or one or two.

By analogy, in the present disclosure, "from none to X", X varying from 3 to 23, corresponds to none, or one, or two, or three, or four, or five, or six, or seven... or twenty three.

The present disclosure also describes the above defined use, wherein said prognosis method is such that :
▪ if none of the 23 genes of said set is expressed, the patient survival rate during a period of time from 30 to 120 months after the diagnosis of said lung cancer is from about 59% to about 78% or more,
▪ if
   o none of the genes of the set B or of the subset B1 or B2, is expressed and at least 3 genes of the set A, or of the subset A1, A2 or A3 is expressed, or
   o at least 1 gene of the set B, or of the subset B1 or B2, is expressed and from none to 2 genes of the set A or of the subset A1, A2, or A3, is expressed, or
   o at least 2 genes of the set B or of the subset B1 or B2 is expressed and no gene of the set A or of the subset A1, A2, or A3, is expressed,
the patient survival rate during a period of time from 30 to 120 months after the diagnosis of said lung cancer is from about 27% to about 55%, and
▪ if
   ∘ one gene of the genes of the set B or of the subset B1 or B2, is expressed and at least 3 genes of the set A or of the subset A1, A2, or A3, are expressed, or
   o at least 2 genes of the set B or of the subset B1, or B2 are expressed and at least 1 gene of the set A or of the subset A1, A2, or A3, are expressed,
the patient survival rate during a period of time from 30 to 120 months after the diagnosis of said lung cancer is from about 3% to about 13%.

The present disclosure also describes the above defined use, wherein said prognosis method is such that:
▪ if none of the 23 proteins, of said set is expressed, the patient survival rate during a period of time from 30 to 120 months after the diagnosis of said lung cancer is from about 59% to about 78% or more,
▪ if
   o none of the proteins of the set BP, or of the subset BP1 or BP2, is expressed and at least 3 proteins, of the set AP, or of the subset AP1, AP2 or AP3, is expressed, or
   o at least 1 protein of the set BP, or of the subset BP1 or BP2, is expressed and from none to 2 proteins, of the set AP, or of the subset AP1, AP2 or AP3, is expressed, or
   o at least 2 proteins of the set BP, or of the subset BP1 or BP2, is expressed and no protein of the set AP, or of the subset AP1, AP2, or AP3, is expressed,
the patient survival rate during a period of time from 30 to 120 months after the diagnosis of said lung cancer is from about 27% to about 55%, and
▪ if
   o one protein of the proteins of the set BP, or of the subset BP1 or BP2, is expressed and at least 3 proteins of the set AP, or of the subset AP 1, AP2 or AP3, are expressed, or
   o at least 2 proteins of the set BP, or of the subset BP 1 or BP2, are expressed and at least 1 protein of the set AP, or of the subset AP1, AP2 or AP3, are expressed,
the patient survival rate during a period of time from 30 to 120 months after the diagnosis of said lung cancer is from about 3% to about 13%.

The present disclosure also describes the use as defined above, wherein said prognosis method is such that:
▪ if none of the 23 genes or proteins of said set, or antibodies, is expressed the patient survival rate during a period of time from 30 to 120 months after the diagnosis of said lung cancer is from about 59% to about 78% or more,
▪ if none of the genes or proteins of the set B or BP, or of the subsets B1, BP1, B2 or BP2, or antibodies, is expressed and one or two genes or proteins of the set A or AP, or of the subsets A1, AP1, A2, AP2, A3 or AP3, or antibodies, is expressed, the patient survival rate during a period of time from 30 to 120 months after the diagnosis of said lung cancer is from about 38% to about 70%,
▪ if
   o none of the genes or proteins of the set B or BP, or of the subsets B1, BP1, B2 or BP2, or antibodies, is expressed and at least 3 genes or proteins of the first set A or AP or of the subsets A1, AP1, A2, AP2, A3 or AP3, or antibodies, is expressed, or
   o at least 1 gene or protein of the set B or BP, or of the subsets B1, BP1, B2 or BP2, or antibody, is expressed and from none to 2 genes or proteins of the set A or AP, or of the subsets A1, AP1, A2, AP2, A3 or AP3, or antibodies, is expressed, or
   o at least 2 genes or proteins of the set B or BP, or of the subsets B1, BP1, B2 or BP2, or antibodies, is expressed and no gene or protein of the set A or AP, or of the subsets A1, AP1, A2, AP2, A3 or AP3, or antibody, is expressed,
the patient survival rate during a period of time from 30 to 120 months after the diagnosis of said lung cancer is from about 27% to about 55%, and
▪ if
   ∘ one gene or protein of the genes or proteins, or antibodies, of the set B or BP, or of the subsets B1, BP1, B2 or BP2, or antibody, is expressed and at least 3 genes or proteins of the set A or AP, or of the subsets A1, AP1, A2, AP2, A3 or AP3, or antibodies, are expressed, or
   o at least 2 genes or proteins of the set B or BP, or of the subsets B1, BP1, B2 or BP2, or antibodies, are expressed and at least 1 gene or protein of the set A or AP, or of the subsets A1, AP1, A2, AP2, A3 or AP3, or antibody, is expressed
the patient survival rate during a period of time from 30 to 120 months after the diagnosis of said lung cancer is from about 3% to about 13%.

The present disclosure also describes the use as defined above, wherein said prognosis method is such that :
▪ if none of the 23 genes of said set is expressed, the patient survival rate during a period of time from 30 to 120 months after the diagnosis of said lung cancer is from about 59% to about 78% or more,
▪ if none of the genes of the set B or of the subsets B1 or B2, is expressed and one or two genes of the set A or of the subsets A1, A2 or A3, is expressed, the patient survival rate is from about 38% to about 70%,
▪ if
   o none of the genes of the set B or of the subsets B1 or B2, is expressed and at least 3 genes of the first set is expressed, or
   o at least 1 gene of the set B or of the subsets B1 or B2, is expressed and from none to 2 genes of the set A or of the subsets A1, A2 or A3, is expressed, or
   o at least 2 genes of the set B or of the subsets B1 or B2, is expressed and no gene of the set A or of the subsets A1, A2 or A3, is expressed,
the patient survival rate during a period of time from 30 to 120 months after the diagnosis of said lung cancer is from about 27% to about 55%, and
▪ if
   ∘ one gene of the genes of the set B or of the subsets B1 or B2, is expressed and at least 3 genes of the set A or of the subsets A1, A2 or A3, are expressed, or
   o at least 2 genes of the set B or of the subsets B1 or B2, are expressed and at least 1 gene of the set A or of the subsets A1, A2 or A3, are expressed
the patient survival rate during a period of time from 30 to 120 months after the diagnosis of said lung cancer is from about 3% to about 13%.

The present disclosure also describes the use as defined above, wherein said prognosis method is such that:
▪ if none of the 23 proteins of said set is expressed, the patient survival rate during a period of time from 30 to 120 months after the diagnosis of said lung cancer is from about 59% to about 78% or more,
▪ if none of the proteins of the set BP or of the subsets BP1 or BP2, is expressed and one or two proteins of the set AP or of the subsets AP1, AP2 or AP3, is expressed, the patient survival rate during a period of time from 30 to 120 months after the diagnosis of said lung cancer is from about 38% to about 70%,
▪ if
   o none of the proteins of the set BP or of the subsets BP1 or BP2, is expressed and at least 3 proteins of the first set is expressed, or
   o at least 1 protein of the set BP or of the subsets BP1 or BP2, is expressed and from none to 2 proteins of the set AP or of the subsets AP1, AP2 or AP3, is expressed, or
   o at least 2 proteins of the set BP or of the subsets BP1 or BP2, is expressed and no protein of the set AP or of the subsets AP1, AP2 or AP3, is expressed,
the patient survival rate during a period of time from 30 to 120 months after the diagnosis of said lung cancer is from about 27% to about 55%, and
▪ if
   ∘ one protein of the proteins of the set BP or of the subsets BP1 or BP2, is expressed and at least 3 proteins of the set AP or of the subsets AP1, AP2 or AP3, are expressed, or
   o at least 2 proteins of the set BP or of the subsets BP1 or BP2, are expressed and at least 1 protein of the set AP or of the subsets AP1, AP2 or AP3, are expressed
the patient survival rate during a period of time from 30 to 120 months after the diagnosis of said lung cancer is from about 3% to about 13%. '

The present disclosure also describes a prognosis method previously defined, wherein the, step of measuring is carried out by using a technique chosen among the set consisting of:
- Quantitative PCR**,**
- DNA CHIP, and
- Northern blot.

The present disclosure also describes a prognosis method as previously defined, wherein the step of measuring is carried out by using nucleic acid molecules consisting of from 15 to 100 nucleotides molecules being complementary to said at least 2 genes.

The present disclosure also describes a prognosis method as defined above, wherein the step of measuring is carried out by DNA CHIP using
- at least one nucleic acid probe comprising or being constituted by the nucleic acid sequences SEQ ID NO: 47 to SEQ ID NO: 53, and
- at least one nucleic acid probe comprising or being constituted by the nucleic acid sequences SEQ ID NO: 54 to SEQ ID NO: 69.

The present disclosure also describes the method as defined above, wherein the step of measuring is carried out by DNA CHIP using at least 2, preferably at least 3 nucleic acid probes as defined above.

The present disclosure also describes the above method wherein the nucleic acid probes comprising or being constituted by the nucleic acid sequences SEQ ID NO: 47 to 69 are used, together.

In the present disclosure the correspondence between the genes and the nucleic acid probes are as follows: SEQ ID NO:1 is able to be detected by the nucleic acid probe comprising or being constituted by the nucleic acid sequence SEQ ID NO: 47, SEQ ID NO:2 is able to be detected by the nucleic acid probe comprising or being constituted by the nucleic acid sequence SEQ ID NO: 48, SEQ ID NO:3 is able to be detected by the nucleic acid probe comprising or being constituted by the nucleic acid sequence SEQ ID NO: 49, SEQ ID NO:4 is able to be detected by the nucleic acid probe comprising or being constituted by the nucleic acid sequence SEQ ID NO: 50, SEQ ID NO:5 is able to be detected by the nucleic acid probe comprising or being constituted by the nucleic acid sequence SEQ ID NO: 51, SEQ ID NO:6 is able to be detected by the nucleic acid probe comprising or being constituted by the nucleic acid sequence SEQ ID NO: 52, SEQ ID NO:7 is able to be detected by the nucleic acid probe comprising or being constituted by the nucleic acid sequence SEQ ID NO: 53, SEQ ID NO:8 is able to be detected by the nucleic acid probe comprising or being constituted by the nucleic acid sequence SEQ ID NO: 54, SEQ ID NO:9 is able to be detected by the nucleic acid probe comprising or being constituted by the nucleic acid sequence SEQ ID NO: 55, SEQ ID NO:10 is able to be detected by the nucleic acid probe comprising or being constituted by the nucleic acid sequence SEQ ID NO: 56, SEQ ID NO:11 is able to be detected by the nucleic acid probe comprising or being constituted by the nucleic acid sequence SEQ ID NO: 57, SEQ ID NO:12 is able to be detected by the nucleic acid probe comprising or being constituted by the nucleic acid sequence SEQ ID NO: 58, SEQ ID NO:13 is able to be detected by the nucleic acid probe comprising or being constituted by the nucleic acid sequence SEQ ID NO: 59, SEQ ID NO:14 is able to be detected by the nucleic acid probe comprising or being constituted by the nucleic acid sequence SEQ ID NO: 60, SEQ ID NO: 15 is able to be detected by the nucleic acid probe comprising or being constituted by the nucleic acid sequence SEQ ID NO: 61, SEQ ID NO:16 is able to be detected by the nucleic acid probe comprising or being constituted by the nucleic acid sequence SEQ ID NO: 62, SEQ ID NO: 17 is able to be detected by the nucleic acid probe comprising or being constituted by the nucleic acid sequence SEQ ID NO: 63, SEQ ID NO:18 is able to be detected by the nucleic acid probe comprising or being constituted by the nucleic acid sequence SEQ ID NO: 64, SEQ ID NO: 19 is able to be detected by the nucleic acid probe comprising or being constituted by the nucleic acid sequence SEQ ID NO: 65, SEQ ID NO:20 is able to be detected by the nucleic acid probe comprising or being constituted by the nucleic acid sequence SEQ ID NO: 66, SEQ ID NO:21 is able to be detected by the nucleic acid probe comprising or being constituted by the nucleic acid sequence SEQ ID NO: 67, SEQ ID NO:22 is able to be detected by the nucleic acid probe comprising or being constituted by the nucleic acid sequence SEQ ID NO: 68, SEQ ID NO:23 is able to be detected by the nucleic acid probe comprising or being constituted by the nucleic acid sequence SEQ ID NO: 69.

The present disclosure also describes a prognosis method, preferably *in vitro,* of the survival rate of a patient afflicted by a lung tumour, from a biological sample containing said lung tumor, at a time from 30 to 120 months after the diagnosis of said lung cancer, said method comprising a step of measuring, in said biological sample, the expression of
- at least 2 proteins chosen among a set of 23 proteins coded by 23 genes comprising or consisting of the nucleic acid sequences SEQ ID NO 1 to 23,
- or fragments of said proteins,
said at least 2 proteins being such that
- at least one protein belongs to a first set AP of 7 proteins, each 7 proteins being coded by one of 7 genes comprising or consisting of the nucleic acid sequences SEQ ID NO: 1-7
- at least one protein belongs to a second set BP of 16 proteins, each 16 proteins being coded by one of 16 genes comprising or consisting of the nucleic acid sequences SEQ ID NO: 8-23
said prognosis method being such that
▪ if none of the 23 proteins is expressed, the patient survival rate is from about 59% to about 78%, or more, and
▪ if at least one protein of said set AP or BP is expressed, the patient survival rate from about 3% to about 70%.

In one another advantageous embodiment, the present disclosure relates to a prognosis method,
wherein the step of measuring is carried out by using a technique chosen among the set consisting of:
- western Blot,
- ELISA,
- Immunofluorescence, and
- Immunohistochemistry.

In one another advantageous embodiment, the present disclosure relates to a prognosis method,
wherein the step of measuring is carried out by using antibodies directed against said at least 2 proteins coded by said at least two genes.

In one another advantageous embodiment, the present disclosure relates to a prognosis method,
further comprising a step of comparison of said measured expression to the expression in at least one control sample.

According to the present disclosure the above mentioned gene, proteins or antibody expression can be compared with the expression level of the same genes, proteins and antibodies measured in
- a control sample corresponding to a sample originating from an healthy individual, and/or
- a positive sample corresponding to a sample of an individual expressing the above mentioned gene, protein, or antibodies.

The present disclosure also concerns a kit comprising a DNA CHIP comprising at least the nucleic acid probes comprising or being constituted by the nucleic acid sequences SEQ ID NO: 47 to 69.

The present disclosure also relates to the use of
- at least 13 genes chosen among a set of 28 genes comprising or consisting of the nucleic acid sequences SEQ ID NO 70 to 97,
- or fragments of said least 13 genes chosen among a set of 28 genes comprising or consisting of the nucleic acid sequences SEQ ID NO 70 to 97,
- or complementary sequences of said least 13 genes chosen among a set of 28 genes comprising or consisting of the nucleic acid sequences SEQ ID NO 70 to 97,
- or sequences having at least 80% homology with said genes or fragment thereof,
   - or proteins coded by said least 13 genes chosen among a set of 28 genes comprising or consisting of the nucleic acid sequences
   - or fragments of said proteins,
   - or antibodies directed against said proteins,
   said at least 13 genes being such that
   - 12 genes comprise or consist of the nucleic acid sequences SEQ ID NO: 70 to 81, and
   - at least one gene belongs to a subset of 16 genes comprising or consisting of the nucleic acid sequences SEQ ID NO: 82-97,
for carrying out a method for identifying at least 66% of patients of those having a survival rate of at most about 20% at 30 months, among a population of patients afflicted by lung cancer having an estimated survival rate of about at least about 30% at 30 months based on the diagnosis of said lung cancer according to histopathological criteria.

Lung cancer is one of the most frequent cancer in humans and is the most frequent cause of mortality by cancer in human.

Classically, when diagnosed, lung tumors are classified according to the TNM classification (tumor size, node positivity and metastasis) by clinicians and into histopathological subtypes by histopathologists. TNM corresponds to the clinical criteria according to the invention.

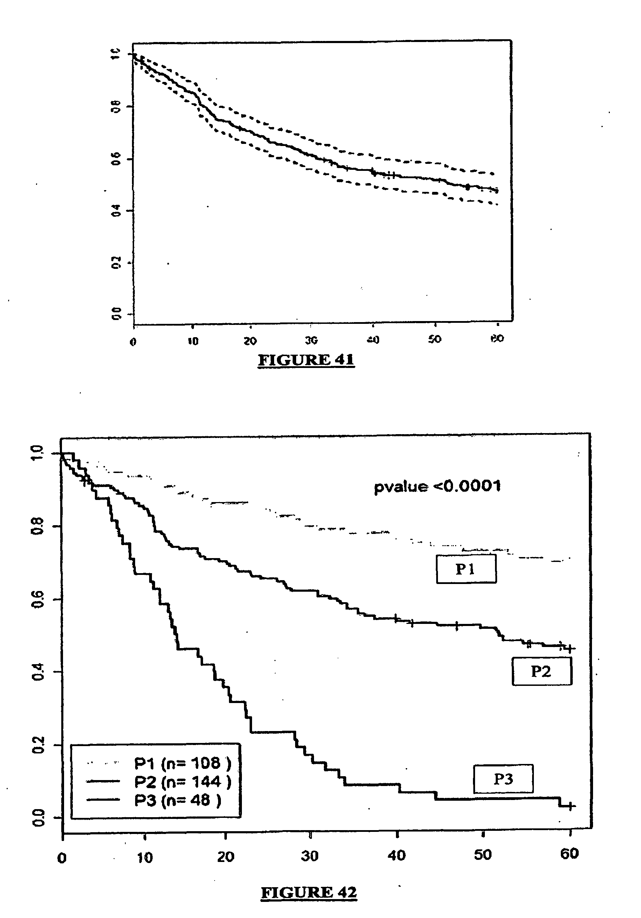

Based on the TNM analysis, it is possible to establish for a patient a survival probability, in percent, at 30months, 60 months and 120 months from the diagnosis. At about 60 months, patients afflicted by lung tumors have a survival rate of about 50% (shown in Figure 41).

In the invention, terms "survival rate" can be uniformally replaced by "survival probability" or "survival estimate"

The present invention is based on the unexpected observation made by the Inventors that the expression of at least 13 genes of a group of 28 determined genes is sufficient to discriminate at least 2/3 of the patients having a very poor prognosis, i.e. a survival rate very low, said patients being non identified by the clinical or histopatological criteria.

According to the invention as explained and exemplified hereafter, the determination of the gene expression status on a ON/OFF basis of at least 13 genes chosen among a set of 28 genes as defined above.

A key aspect of the invention is the concept of ON/OFF for gene expression. The concept of ON/OFF expression can be extended to presence/absence of the proteins and antibodies detecting these proteins. This specific approach has the advantage of simplifying the analyses and making them independent of complex statistical tests to measure variations in expression levels applied to the majority of the existing tests.

The ON/OFF status of gene expression is established by determination of a threshold of gene expression allowing them to decide on the ON/OFF status of a gene such that:
- if a gene is expressed at a level lower than the threshold, the gene is considered as not expressed (defined as OFF), and
- if a gene is expressed at a level upper to the threshold, the gene is considered as being expressed (defined as ON).

The above 28 genes have been identified as being liable to be "expressed" (form here by, "expressed" refers to the ON status and "not expressed" to the OFF status) in lung cancer cells, but not in healthy samples. In other words, the above 28 genes are such as
- they are not expressed, in healthy lung cells, and
- they maybe expressed in lung tumor cells.

The difference between the absence of expression and the expression determines its ON/OFF status, which is a key step of the invention.

Indeed, the Inventors have identified that the ON status of the above 28 genes is a key step to determine the prognosis of lung cancer.

On microarrays, the expression level of the above mentioned genes is determined by the fact that a threshold of expression has been identified by the Inventors allowing to determine expression (ON) and non-expression (OFF) of said genes. The threshold determination is detailed hereafter, in the Example section.

For the microarrays, the threshold enabling to determine the expression status of a gene (ON versus OFF) is calculated by using the signal mean value and distribution obtained from transcriptomic data (in the same technology) with the corresponding probes in a large number of somatic tissues (which do not express the genes).

A similar strategy enables determining a threshold for the presence/absence of the encoded proteins or antibodies. For each protein or antibody, the mean value and distribution of the signal intensities obtained in an appropriate number of control somatic tissues serves as a basis for calculating the threshold.

By "not expressed" it is defined in the invention the fact that the transcription of a gene is either not carried out, or is not detectable by common techniques known in the art, such as Quantitative RT-PCR, Northern blot or when microarrays data are considered.

By "expressed", the invention defined that the transcript of a gene is detectable by the above known techniques while it is not detectable in healthy tissues or determined as being above the threshold when microarrays data are considered.

In the invention, terms "carrying out" and "implementation" are used uniformly.

The subgroup of 13 determined genes chosen among a group of 28 determined genes identified by the Inventors allows the identification of at least 2/3, or 66%, of patients having a prognosis to be alive 30 months after the diagnosis of their lung tumor of about at most 20%. Said patients with the above bad/poor prognosis are not detected by the histopathological methods, such as the TNM method.

Actually, the subgroup of 13 determined genes chosen among a group of 28 determined genes identified by the Inventors allows to separate 3 distinct populations: P1 and P2 populations: patients having a survival rate of about at least 20% after 30 months from the diagnosis of their lung tumors, and

P3 population: patients having a survival rate of about at most 20% after 30 months from the diagnosis of their lung tumors.

This is illustrated in figure 42.

The patients of the P3 population have to be identified in order to treat them very rapidly, when possible, in view of the agressivity of their tumors, and to inform them that they have a very poor prognosis.

The above explanation applies when lung tumors are analysed independently from any further status.

The method according to the invention, the use as mentioned above also applies when tumors are detected at early stage (in the invention called "T1N0") or at late stage (in the invention called "T+N+").

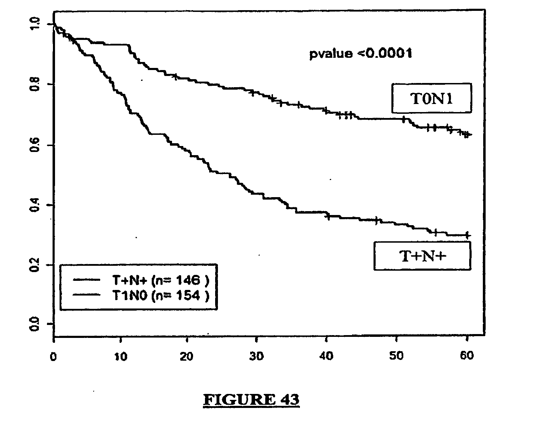

According to the TNM classification, the survival rate over the months are represented in figure 43.

When applying the method as described above, in each of the T1N0 and T+N+, 3 polulations can be defined: i.e. the above mentioned P1, P2 and P3 populations.

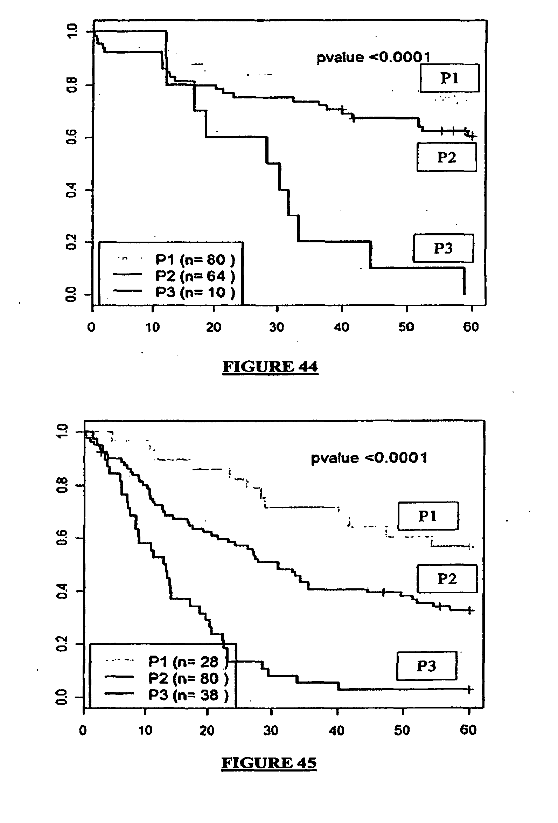

The repartition of P1, P2 and P3 populations is represented in figure 44 for T1N0 and in figure 45 for T+N+.

The same applies when tumors are identified according to their histological status, chosen among SQC (squamous cell cancer), LCNE (Large Cell Neuroendocrine tumour) and BAS (basaloid tumour).

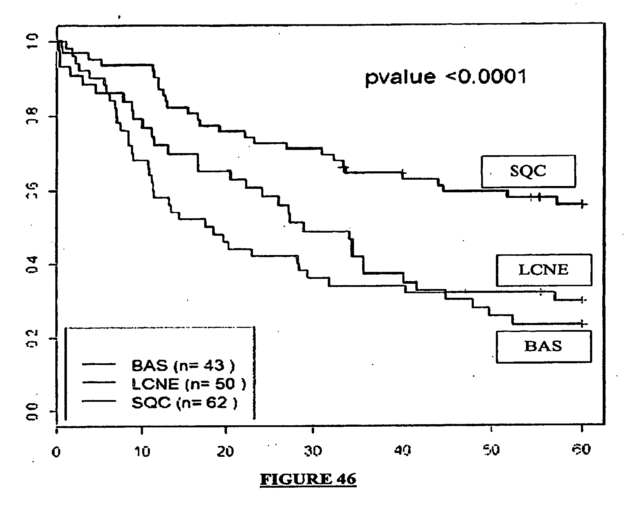

According to the histopathological classification, the survival rates over the months are represented in figure 46.

When applying the method as decribed above, in each of the BAS, SQC or LCNE tumors, 3 populations can be defined: i.e. the above mentioned P1, P2 and P3 populations.

The repartition of P1, P2 and P3 populations is represented in figure 47 for BAS, in figure 48 for SQC and in figure 49 for LCNE.

According to the invention, it is sufficient to use at least 13 genes of the group of 28 genes comprising or consisting of SEQ ID NO: 70 to 97, to identify at least 66% of patients having a poor prognosis as defined above, said 13 genes being such that:
- 12 of these 13 genes comprise or consist of the nucleic acid sequences SEQ ID NO: 70-81, and
- at least one gene chosen among the genes comprising or consisting of the nucleic acid sequences SEQ ID NO: 82-97.

In the invention, it is possible to use, at least 13 genes as mentioned above, or fragments of said at least 13 genes, or complementary sequences of said at least 13 genes or fragments thereof. For the purpose of the invention, the term "gene" refers to the transcriptional product of the genes, also called cDNA, or to the genomic counterpart.

The invention also relates to the use of at least 13 proteins, said proteins being coded by said at least 13 genes.

The correspondence between the genes and the proteins of the invention is as follows:

| **Gene name** | **DNA: SEQ ID NO** | **Protein: SEQ ID NO:** |
|---|---|---|
| MAGEB6 | 70 | 98 |
| TPTE/TPTE2 | 71 | 99 |
| RBM46 | 72 | 100 |
| HIST1H3A; HIST1H3C | 73 | 101 |
| CPA5 | 74 | 102 |
| RFX4 | 75 | 103 |
| TUBA3C/TUBA3D | 76 | 104 |
| KIAA1257 | 77 | 105 |
| ARHGEF40 | 78 | 106 |
| TKTL2 | 79 | 107 |
| CCDC83 | 80 | 108 |
| DPEP3 | 81 | 109 |
| C10orf82 | 82 | 110 |
| C12orf37 | 83 | -- |
| PIWIL1 | 84 | 111 |
| ROPN1 | 85 | 112 |
| NBPF4/NBPF6 | 86 | 113 |
| LOC220115 | 87 | 114 |
| BTG4 | 88 | 115 |
| ISM2 | 89 | 116 |
| OR7E156P | 90 | -- |
| EBI3 | 91 | 117 |
| LGALS14 | 92 | 118 |
| LOC441601 | 93 | -- |
| VCY/VCY1B | 94 | 119 |
| FLJ43944 | 95 | 120 |
| IGFBP1 | 96 | 121 |
| CCDC38 | 97 | 122 |

There is, in the invention, 16 minimal sets of 13 genes that can be used to identify said at least 66% of patients belonging to the P3 population:
- SEQ ID NO: 70, 71, 72, 73, 74, 75, 76, 77, 78, 79, 80 and 81, plus SEQ ID NO: 82,
- SEQ ID NO: 70, 71, 72, 73, 74, 75, 76, 77, 78, 79, 80 and 81, plus SEQ ID NO: 83,
- SEQ ID NO: 70, 71, 72, 73, 74, 75, 76, 77, 78, 79, 80 and 81, plus SEQ ID NO: 84,
- SEQ ID NO: 70, 71, 72, 73, 74, 75, 76, 77, 78, 79, 80 and 81, plus SEQ ID NO: 85,
- SEQ ID NO: 70, 71, 72, 73, 74, 75, 76, 77, 78, 79, 80 and 81, plus SEQ ID NO: 86,
- SEQ ID NO: 70, 71, 72, 73, 74, 75, 76, 77, 78, 79, 80 and 81, plus SEQ ID NO: 87,
- SEQ ID NO: 70, 71, 72, 73, 74, 75, 76, 77, 78, 79, 80 and 81, plus SEQ ID NO: 88,
- SEQ ID NO: 70, 71, 72, 73, 74, 75, 76, 77, 78, 79, 80 and 81, plus SEQ ID NO: 89,
- SEQ ID NO: 70, 71, 72, 73, 74, 75, 76, 77, 78, 79, 80 and 81, plus SEQ ID NO: 90,
- SEQ ID NO: 70, 71, 72, 73, 74, 75, 76, 77, 78, 79, 80 and 81, plus SEQ ID NO: 91,
- SEQ ID NO: 70, 71, 72, 73, 74, 75, 76, 77, 78, 79, 80 and 81, plus SEQ ID NO: 92,
- SEQ ID NO: 70, 71, 72, 73, 74, 75, 76, 77, 78, 79, 80 and 81, plus SEQ ID NO: 93,
- SEQ ID NO: 70, 71, 72, 73, 74, 75, 76, 77, 78, 79, 80 and 81, plus SEQ ID NO: 94,
- SEQ ID NO: 70, 71, 72, 73, 74, 75, 76, 77, 78, 79, 80 and 81, plus SEQ ID NO: 95,
- SEQ ID NO: 70, 71, 72, 73, 74, 75, 76, 77, 78, 79, 80 and 81, plus SEQ ID NO: 96,
- SEQ ID NO: 70, 71, 72, 73, 74, 75, 76, 77, 78, 79, 80 and 81, plus SEQ ID NO: 97,

According to the invention, "the use of at least 13 genes of the group of 28 genes" means that 13, 14, 15, 16, 17, 18, 19, 20, 21, 22, 23, 24, 25, 26, 27 or 28 genes are used.

When at least 14 genes are considered, or more, the skilled person could easily combine SEQ ID NO: 70, 71, 72, 73, 74, 75, 76, 77, 78, 79, 80 and 81 plus two genes from those of the list SEQ ID NO: 82, 83, 84, 85, 86, 87, 88, 89, 90, 91, 92, 93, 94, 95, 96 and 97.

In one advantageous embodiment, the invention relates to the use of at least 13 proteins coded by said least 13 genes chosen among a set of 25 proteins
- or fragments of said proteins,
- or antibodies directed against said proteins,
said at least 13 proteins genes being such that
- 12 proteins comprise or consist of the nucleic acid sequences SEQ ID NO: 98 to 110, and
- at least one protein belongs to a subset of 13 genes comprising or consisting of the nucleic acid sequences SEQ ID NO: 111-122,
for carrying out a method for identifying at least 66% of patients of those having a survival rate of at most about 20% at 30 months, among a population of patients afflicted by lung cancer having an estimated survival rate of at least about 30% at 30 months based on the diagnosis of said lung cancer according to histopathological criteria.

Advantageously, using the above 13 gene, i.e. SEQ ID NO: 70-81 + anyone of gene chosen among SEQ ID NO: 82-97, allows identification from about 66% to about 70% of patients of those having a survival rate of at most about 20% at 30 months

In one advantageous embodiment, the invention relates to the use defined above, of
- at least 13 genes chosen among a set of 28 genes comprising or consisting of the nucleic acid sequences SEQ ID NO 70 to 97,
- or fragments of said least 13 genes chosen among a set of 28 genes comprising or consisting of the nucleic acid sequences SEQ ID NO 70 to 97,
- or complementary sequences of said least 13 genes chosen among a set of 28 genes comprising or consisting of the nucleic acid sequences SEQ ID NO 70 to 97,
- or sequences having at least 80% homology with said genes or fragments thereof,
said at least 13 genes being such that
- 12 genes comprise or consist of the nucleic acid sequences SEQ ID NO: 70 to 81, and
- at least one gene belongs to a subset of 16 genes comprising or consisting of the nucleic acid sequences SEQ ID NO: 82-97,
for carrying out a method for identifying at least 66% of patients of those having a survival rate of at most about 20% at 30 months, among a population of patients afflicted by lung cancer having an estimated survival rate of at least about 30% at 30 months based on the diagnosis of said lung cancer according to histopathological criteria.

In the invention, all the percentages are expressed as "about X%". This means that said percent are the X value ± (plus or minus) the variation of 5% of the value X. Thus "about 66% percent" encompass the interval 66-5%≤ 66 ≤ 66+5%, i.e from 62,7% to 69,3%.

From this explanation, the skilled person knows how to correctly determine the advantageous intervals of the invention.

In another advantageous embodiment, the invention relates to the use defined above, wherein said at least one gene belongs to a subset of 16 genes comprising or consisting of the nucleic acid sequences SEQ ID NO: 82-97, is at least the gene comprising or consisting of the nucleic acid sequences SEQ ID NO: 82,
for carrying out a method for identifying at least 70% of patients having a survival rate of at most about 20% at 30 months.

In another advantageous embodiment, the invention relates to the use defined above, of at least 18 genes
said at least 18 genes being such that
- 12 genes comprise or consist of the nucleic acid sequences SEQ ID NO: 70 to 81, and
- at least 6 genes belongs to a subset of 16 genes comprising or consisting of the nucleic acid sequences SEQ ID NO: 82-97, said at least 6 genes comprising or consisting of the nucleic acid sequences SEQ ID NO:82-85 and 87-88,
for carrying out a method for identifying at least 83% of patients of those having a survival rate of at most about 20% at 30 months.

In another advantageous embodiment, the invention relates to the use defined above, of at least 21 genes,
said at least 21 genes being such that
- 12 genes comprise or consist of the nucleic acid sequences SEQ ID NO: 70 to 81, and
- at least 9 gene belongs to a subset of 16 genes comprising or consisting of the nucleic acid sequences SEQ ID NO: 82-97, said at least 9 genes preferably comprising or consisting of the nucleic acid sequences SEQ ID NO: 82-88 and 91-92,
for carrying out a method for identifying at least 89% of patients having a survival rate of at most about 20% at 30 months.

In another advantageous embodiment, the invention relates to the use defined above, of at least 26 genes
said at least 26 genes being such that
- 12 genes comprise or consist of the nucleic acid sequences SEQ ID NO: 70 to 81, and
- at least 14 gene belongs to a subset of 16 genes comprising or consisting of the nucleic acid sequences SEQ ID NO: 82-97, said at least 14 genes preferably comprising or consisting of the nucleic acid sequences SEQ ID NO: 82-92 and 93-96,
for carrying out a method for identifying at least 97% of patients of those having a survival rate of at most about 20% at 30 months.

In another advantageous embodiment, the invention relates to the use defined above, of at least 27 genes
said at least 26 genes being such that
- 12 genes comprise or consist of the nucleic acid sequences SEQ ID NO: 70 to 81, and
- at least 15 gene belongs to a subset of 16 genes comprising or consisting of the nucleic acid sequences SEQ ID NO: 82-97
for carrying out a method for identifying at least 97% of patients of those having a survival rate of at most about 20% at 30 months.

In another advantageous embodiment, the invention relates to the use defined above, of 28 genes comprising or consisting of the nucleic acid sequences SEQ ID NO: 70-97 for carrying out a method for identifying 100% of patients of those having a survival rate of at most about 20% at 30 months.

Hereafter are indicated the advantageous groups of genes according to the invention, along with the percentage of patients belonging to the P3 group they allow to detect.

| **GENES** | **%** |
|---|---|
| 13genes = SEQ ID NO : 70-81 + SEQ ID NO : 82 | 70,8% |
| 13genes = SEQ ID NO : 70-81 + SEQ ID NO : 83 | 66,7% |
| 13genes = SEQ ID NO : 70-81 + SEQ ID NO : 84 | 64,6% |
| 13genes = SEQ ID NO : 70-81 + SEQ ID NO : 85 | 66,7% |
| 13genes = SEQ ID NO : 70-81 + SEQ ID NO : 86 | 64,6% |
| 13genes = SEQ ID NO : 70-81 + SEQ ID NO : 87 | 66,7% |
| 13genes = SEQ ID NO : 70-81 + SEQ ID NO : 88 | 66,7% |
| 13genes = SEQ ID NO : 70-81 + SEQ ID NO : 89 | 66,7% |
| 13genes = SEQ ID NO : 70-81 + SEQ ID NO : 90 | 66,7% |
| 13genes = SEQ ID NO : 70-81 + SEQ ID NO : 91 | 66,7% |
| 13genes = SEQ ID NO : 70-81 + SEQ ID NO : 92 | 66,7% |
| 13genes = SEQ ID NO : 70-81 + SEQ ID NO : 93 | 64,6% |
| 13genes = SEQ ID NO : 70-81 + SEQ ID NO : 94 | 64,6% |
| 13genes = SEQ ID NO : 70-81 + SEQ ID NO : 95 | 66,7% |
| 13genes = SEQ ID NO : 70-81 + SEQ ID NO : 96 | 66,7% |
| 13genes = SEQ ID NO : 70-81 + SEQ ID NO : 97 | 66,7% |
| 14 genes: SEQ ID NO : 70-83 | 72,9% |
| 15 genes: SEQ ID NO : 70-84 | 72,9% |
| 16 genes: SEQ ID NO : 70-85 | 75,0% |
| 17 genes: SEQ ID NO : 70-86 | 75,0% |
| 18 genes: SEQ ID NO : 70-87 | 81,3% |
| 19 genes: SEQ ID NO: 70-88 | 87,5% |
| 20 genes: SEQ ID NO : 70-89 | 87,5% |
| 21 genes: SEQ ID NO : 70-90 | 87,5% |
| 22 genes: SEQ ID NO : 70-91 | 89,6% |
| 23 genes: SEQ ID NO : 70-92 | 93,8% |
| 24 genes: SEQ ID NO : 70-93 | 93,8% |
| 25 genes: SEQ ID NO : 70-94 | 95,8% |
| 26 genes: SEQ ID NO : 70-95 | 97,9% |
| 27 genes: SEQ ID NO : 70-96 | 100,0% |
| All 28 genes SEQ ID NO :70-97 | 100,0% |
| 13genes = SEQ ID NO : 70-81 + SEQ ID NO : 82 | 70,8% |
| 14genes = SEQ ID NO : 70-81 + SEQ ID NO : 82 + SEQ ID NO : 83 | 72,9% |
| 14genes = SEQ ID NO : 70-81 + SEQ ID NO : 82 + SEQ ID NO : 84 | 70,8% |
| 14genes = SEQ ID NO : 70-81 + SEQ ID NO : 82 + SEQ ID NO : 85 | 72,9% |
| 14genes = SEQ ID NO : 70-81 + SEQ ID NO : 82 + SEQ ID NO : 86 | 70,8% |
| 14genes = SEQ ID NO : 70-81 + SEQ ID NO : 82 + SEQ ID NO : 87 | 72,9% |
| 14genes = SEQ ID NO : 70-81 + SEQ ID NO : 82 + SEQ ID NO : 88 | 72,9% |
| 14genes = SEQ ID NO : 70-81 + SEQ ID NO : 82 + SEQ ID NO : 89 | 72,9% |
| 14genes = SEQ ID NO : 70-81 + SEQ ID NO : 82 + SEQ ID NO : 90 | 72,9% |
| 14genes = SEQ ID NO : 70-81 + SEQ ID NO : 82 + SEQ ID NO : 91 | 72,9% |
| 14genes = SEQ ID NO : 70-81 + SEQ ID NO : 82 + SEQ ID NO : 92 | 72,9% |
| 14genes = SEQ ID NO : 70-81 + SEQ ID NO : 82 + SEQ ID NO : 93 | 70,8% |
| 14genes = SEQ ID NO : 70-81 + SEQ ID NO : 82 + SEQ ID NO : 94 | 70,8% |
| 14genes = SEQ ID NO : 70-81 + SEQ ID NO : 82 + SEQ ID NO : 95 | 72,9% |
| 14genes = SEQ ID NO : 70-81 + SEQ ID NO : 82 + SEQ ID NO : 96 | 72,9% |
| 14genes = SEQ ID NO : 70-81 + SEQ ID NO : 82 + SEQ ID NO : 97 | 72,9% |
| 14genes = SEQ ID NO : 70-83 | 72,9% |
| 15genes = SEQ ID NO : 70-83 + SEQ ID NO 84 | 72,9% |
| 15genes = SEQ ID NO : 70-83 + SEQ ID NO : 85 | 75,0% |
| 15genes = SEQ ID NO : 70-83 + SEQ ID NO : 86 | 72,9% |
| 15genes = SEQ ID NO : 70-83 + SEQ ID NO : 87 | 75,0% |
| 15genes = SEQ ID NO : 70-83 + SEQ ID NO : 88 | 77,1% |
| 15genes = SEQ ID NO : 70-83 + SEQ ID NO : 89 | 75,0% |
| 15genes = SEQ ID NO : 70-83 + SEQ ID NO : 90 | 75,0% |
| 15genes = SEQ ID NO : 70-83 + SEQ ID NO : 91 | 75,0% |
| 15genes = SEQ ID NO : 70-83 + SEQ ID NO : 92 | 75,0% |
| 15genes = SEQ ID NO : 70-83 + SEQ ID NO : 93 | 72,9% |
| 15genes = SEQ ID NO : 70-83 + SEQ ID NO : 94 | 72,9% |
| 15genes = SEQ ID NO : 70-83 + SEQ ID NO : 95 | 75,0% |
| 15genes = SEQ ID NO : 70-83 + SEQ ID NO : 96 | 75,0% |
| 15genes = SEQ ID NO : 70-83 + SEQ ID NO : 97 | 72,9% |
| 15genes = SEQ ID NO : 70-83 + SEQ ID NO : 88 | 77,1% |
| 16genes = SEQ ID NO : 70-83 + SEQ ID NO : 88 + SEQ ID NO : 84 | 77,1% |
| 16genes = SEQ ID NO : 70-83 + SEQ ID NO : 88 + SEQ ID NO : 85 | 81,3% |
| 16genes = SEQ ID NO : 70-83 + SEQ ID NO : 88 + SEQ ID NO : 86 | 77,1% |
| 16genes = SEQ ID NO : 70-83 + SEQ ID NO : 88 + SEQ ID NO : 87 | 79,2% |
| 16genes = SEQ ID NO : 70-83 + SEQ NO : 88 + SEQ ID NO : 89 | 77,1% |
| 16genes = SEQ ID NO : 70-83 + SEQ ID NO : 88 + SEQ ID NO : 90 | 79,2% |
| 16genes = SEQ ID NO : 70-83 + SEQ ID NO : 88 + SEQ ID NO : 91 | 79,2% |
| 16genes = SEQ ID NO : 70-83 + SEQ ID NO : 88 + SEQ ID NO : 92 | 79,2% |
| 16genes = SEQ ID NO : 70-83 + SEQ ID NO : 88 + SEQ ID NO : 93 | 77,1% |
| 16genes = SEQ ID NO : 70-83 + SEQ ID NO : 88 + SEQ ID NO : 94 | 79,2% |
| 16genes = SEQ ID NO : 70-83 + SEQ ID NO : 88 + SEQ ID NO : 95 | 79,2% |
| 16genes = SEQ ID NO : 70-83 + SEQ ID NO : 88 + SEQ ID NO : 96 | 79,2% |
| 16genes = SEQ ID NO : 70-83 + SEQ ID NO : 88 + SEQ ID NO : 97 | 77,1% |
| 16genes = SEQ ID NO : 70-83 + SEQ ID NO : 85 + SEQ ID NO : 88 | 81,3% |
| 17genes = SEQ ID NO : 70-83 + SEQ ID NO : 85 + SEQ ID NO : 88 + SEQ ID NO : 84 | 81,3% |
| 17genes = SEQ ID NO : 70-83 + SEQ ID NO : 85 + SEQ ID NO : 88 + SEQ ID NO : 86 | 81,3% |
| 17genes = SEQ ID NO : 70-83 + SEQ ID NO : 85 + SEQ ID NO : 88 + SEQ ID NO : 87 | 83,3% |
| 17genes = SEQ ID NO : 70-83 + SEQ ID NO : 85 + SEQ ID NO : 88 + SEQ ID NO : 89 | 81,3% |
| 17genes = SEQ ID NO : 70-83 + SEQ ID NO : 85 + SEQ ID NO : 88 + SEQ ID NO : 90 | 81,3% |
| 17genes = SEQ ID NO : 70-83 + SEQ ID NO : 85 + SEQ ID NO : 88 + SEQ ID NO : 91 | 83,3% |
| 17genes = SEQ ID NO : 70-83 + SEQ ID NO : 85 + SEQ ID NO : 88 + SEQ ID NO : 92 | 83,3% |
| 17genes = SEQ ID NO : 70-83 + SEQ ID NO : 85 + SEQ m NO : 88 **+** SEQ ID NO : 93 | 81,3% |
| 17genes = SEQ YID NO : 70-83 + SEQ ID NO : 85 + SEQ ID NO : 88 + SEQ ID NO : 94 | 83,3% |
| 17genes = SEQ ID NO : 70-83 + SEQ ID NO : 85 + SEQ ID NO : 88 + SEQ ID NO : 95 | 83,3% |
| 17genes = SEQ ID NO : 70-83 + SEQ ID NO : 85 + SEQ ID NO : 88 + SEQ ID NO : 96 | 83,3% |
| 17genes = SEQ ID NO : 70-83 + SEQ ID NO : 85 + SEQ ID NO : 88 + SEQ ID NO : 97 | 81,3% |
| 17genes = SEQ ID NO : 70-83 + SEQ ID NO : 85 + SEQ ID NO : 87-88 | 83,3% |
| 18genes = SEQ ID NO : 70-83 + SEQ ID NO : 85 + SEQ ID NO : 87-88 + SEQ ID NO : 84 | 85,4% |
| 18genes = SEQ ID NO : 70-83 + SEQ ID NO : 85 + SEQ ID NO : 87-88+ SEQ ID NO : 86 | 85,4% |
| 18genes = SEQ ID NO : 70-83 + SEQ ID NO : 85 + SEQ ID NO : 87-88+ SEQ ID NO : 89 | 83,3% |
| 18genes = SEQ ID NO : 70-83 + SEQ ID NO : 85 + SEQ ID NO : 87-88+ SEQ ID NO : 90 | 83,3% |
| 18genes = SEQ ID NO : 70-83 + SEQ ID NO : 85 + SEQ ID NO : 87-88+ SEQ ID NO : 91 | 85,4% |
| 18genes = SEQ ID NO : 70-83 + SEQ ID NO : 85 + SEQ ID NO : 87-88+ SEQ ID NO : 92 | 85,4% |
| 18genes = SEQ ID NO : 70-83 + SEQ ID NO : 85 + SEQ ID NO : 87-88+ SEQ ID NO : 93 | 85,4% |
| 18genes = SEQ ID NO : 70-83 + SEQ ID NO : 85 + SEQ ID NO : 87-88+ SEQ ID NO : 94 | 85,4% |
| 18genes = SEQ ID NO : 70-83 + SEQ ID NO : 85 + SEQ ID NO : 87-88+ SEQ ID NO: 95 | 85,4% |
| 18genes = SEQ ID NO : 70-83 + SEQ ID NO : 85 + SEQ ID NO : 87-88+ SEQ ID NO : 96 | 85,4% |
| 18genes = SEQ ID NO : 70-83 + SEQ ID NO : 85 + SEQ ID NO : 87-88+ SEQ ID NO : 97 | 83,3% |
| 18genes = SEQ ID NO : 70-83 + SEQ ID NO : 84-85 + SEQ ID NO: 87-88 | 85,4% |
| 19genes = SEQ ID NO : 70-83 + SEQ ID NO : 84-85 + SEQ ID NO: 87-88 + SEQ ID NO : 86 | 87,5% |
| 19genes = SEQ ID NO : 70-83 + SEQ ID NO : 84-85 + SEQ ID NO: 87-88 + SEQ ID NO : 90 | 85,4% |
| 19genes = SEQ ID NO : 70-83 + SEQ ID NO : 84-85 + SEQ ID NO: 87-88 + SEQ ID NO : 91 | 85,4% |
| 19genes = SEQ ID NO : 70-83 + SEQ ID NO : 84-85 + SEQ ID NO: 87-88 + SEQ ID NO : 92 | 87,5% |
| 19genes = SEQ ID NO : 70-83 + SEQ ID NO : 84-85 + SEQ ID NO: 87-88 + SEQ ID NO : 92 | 87,5% |
| 19genes = SEQ ID NO : 70-83 + SEQ ID NO : 84-85 + SEQ ID NO: 87-88 + SEQ ID NO : 93 | 87,5% |
| 19genes = SEQ ID NO : 70-83 + SEQ ID NO : 84-85 + SEQ ID NO: 87-88 + SEQ ID NO : 94 | 87,5% |
| 19genes = SEQ ID NO : 70-83 + SEQ ID NO : 84-85 + SEQ ID NO: 87-88 + SEQ ID NO : 95 | 87,5% |
| 19genes = SEQ ID NO : 70-83 + SEQ ID NO : 84-85 + SEQ ID NO: 87-88 + SEQ ID NO : 96 | 87,5% |
| 19genes = SEQ ID NO : 70-83 + SEQ ID NO : 84-85 + SEQ ID NO: 87-88 + SEQ ID NO : 97 | 85,4% |
| 19genes = SEQ ID NO : 70-88 | 87,5% |
| 20genes = SEQ ID NO : 70-88 + SEQ ID NO : 89 | 87,5% |
| 20genes = SEQ ID NO : 70-88 + SEQ ID NO : 90 | 87,5% |
| 20genes = SEQ ID NO : 70-88 + SEQ ID NO : 91 | 89,6% |
| 20genes = SEQ ID NO : 70-88 + SEQ ID NO : 92 | 89,6% |
| 20genes = SEQ ID NO : 70-88 + SEQ ID NO : 93 | 87,5% |
| 20genes = SEQ ID NO : 70-88 + SEQ ID NO : 94 | 89,6% |
| 20genes = SEQ ID NO : 70-88 + SEQ ID NO : 95 | 89,6% |
| 20genes = SEQ ID NO : 70-88 + SEQ ID NO : 96 | 89,6% |
| 20genes = SEQ ID NO : 70-88 + SEQ ID NO : 97 | 87,5% |
| 20genes = SEQ ID NO : 70-88 + SEQ ID NO : 91 | 89,6% |
| 21genes = SEQ ID NO : 70-88 + SEQ ID NO : 91 + SEQ ID NO : 89 | 89,6% |
| 21genes = SEQ ID NO : 70-88 + SEQ ID NO : 91 + SEQ ID NO : 90 | 89,6% |
| 21genes = SEQ ID NO : 70-88 + SEQ ID NO : 91 + SEQ ID NO : 92 | 91,7% |
| 21genes = SEQ ID NO : 70-88 + SEQ ID NO : 91 + SEQ ID NO : 93 | 89,6% |
| 21genes = SEQ ID NO : 70-88 + SEQ ID NO : 91 + SEQ ID NO : 94 | 91,7% |
| 21genes = SEQ ID NO : 70-88 + SEQ ID NO : 91 + SEQ ID NO : 95 | 91,7% |
| 21genes = SEQ ID NO : 70-88 + SEQ ID NO : 91 + SEQ ID NO : 96 | 91,7% |
| 21genes = SEQ ID NO : 70-88 + SEQ ID NO : 91 + SEQ ID NO : 97 | 89,6% |
| 21gens = SEQ ID NO : 70-88 + SEQ ID NO : 91 + SEQ ID NO : 92 | 91,7% |
| 22genes = SEQ ID NO : 70-88 + SEQ ID NO : 91-92 + SEQ ID NO : 89 | 91,7% |
| 22genes = SEQ ID NO : 70-88 + SEQ ID NO : 91-92 + SEQ ID NO : 90 | 91,7% |
| 22genes = SEQ ID NO : 70-88 + SEQ ID NO : 91-92 + SEQ ID NO : 93 | 91,7% |
| 22genes = SEQ ID NO : 70-88 + SEQ ID NO : 91-92 + SEQ ID NO : 94 | 93,8% |
| 22genes = SEQ ID NO : 70-88 + SEQ ID NO : 91-92 + SEQ ID NO : 95 | 93,8% |
| 22genes = SEQ ID NO : 70-88 + SEQ ID NO : 91-92 + SEQ ID NO : 96 | 93,8% |
| 22genes = SEQ ID NO : 70-88 + SEQ ID NO : 91-92 + SEQ ID NO : 97 | 91,7% |
| 22genes = SEQ ID NO : 70-88 + SEQ ID NO : 91-92 + SEQ ID NO: 94 | 93,8% |
| 23genes = SEQ ID NO : 70-88 + SEQ ID NO : 91-92 + SEQ ID NO : 94 + SEQ ID NO : 89 | 93,8% |
| 23genes = SEQ ID NO : 70-88 + SEQ ID NO : 91-92 + SEQ ID NO : 94 + SEQ ID NO : 90 | 93,8% |
| 23genes = SEQ ID NO : 70-88 + SEQ ID NO : 91-92 + SEQ ID NO : 94 + SEQ ID NO : 93 | 93,8% |
| 23genes = SEQ ID NO : 70-88 + SEQ ID NO : 91-92 + SEQ ID NO : 94 + SEQ ID NO : 95 | 95,8% |
| 23genes = SEQ ID NO : 70-88 + SEQ ID NO : 91-92 + SEQ ID NO : 94 + SEQ ID NO : 96 | 95,8% |
| 23genes = SEQ ID NO : 70-88 + SEQ ID NO : 91-92 + SEQ ID NO : 94 + SEQ ID NO : 97 | 93,8% |
| 23genes = SEQ ID NO : 70-88 + SEQ ID NO : 91-92 + SEQ ID NO : 94-95 | 95,8% |
| 24genes = SEQ ID NO : 70-88 + SEQ ID NO : 91-92 + SEQ ID NO : 94-95 + SEQ ID NO : 89 | 95,8% |
| 24genes = SEQ ID NO : 70-88 + SEQ ID NO : 91-92 + SEQ ID NO : 94-95 + SEQ ID NO : 90 | 95,8% |
| 24genes = SEQ ID NO : 70-88 + SEQ ID NO : 91-92 + SEQ ID NO : 94-95 + SEQ ID NO: 93 | 95,8% |
| 24genes = SEQ ID NO : 70-88 + SEQ ID NO : 91-92 + SEQ ID NO : 94-95 + SEQ ID NO : 96 | 97,9% |
| 24genes = SEQ ID NO : 70-88 + SEQ ID NO : 91-92 + SEQ m NO : 94-95 + SEQ ID NO : 97 | 95, 8% |
| 25genes = SEQ ID NO : 70-88 + SEQ ID NO : 91-92 + SEQ ID NO : 94-96 + SEQ ID NO : 89 | 97,9% |
| 25genes = SEQ ID NO : 70-88 + SEQ ID NO : 91-92 + SEQ ID NO : 94-96 + SEQ ID NO : 90 | 97,9% |
| 25genes = SEQ ID NO : 70-88 + SEQ ID NO : 91-92 + SEQ ID NO : 94-96 + SEQ ID NO : 93 | 97,9% |
| 25genes = SEQ ID NO : 70-88 + SEQ ID NO : 91-92 + SEQ ID NO : 94-96 + SEQ ID NO : 97 | 97,9% |
| 25genes == SEQ ID NO : 70-89 + SEQ ID NO : 91-92 + SEQ ID NO : 94-96 | 97,9% |
| 25genes = SEQ ID NO : 70-88 + SEQ ID NO : 90-92 + SEQ ID NO : 94-96 | 97,9% |
| 25genes = SEQ ID NO : 70-88 + SEQ ID NO : 91-96 | 97,9% |
| 25genes = SEQ ID NO : 70-88 + SEQ ID NO : 91-92 + SEQ ID NO : 94-97 | 97,9% |
| 26 genes = SEQ ID NO : 70-92 + SEQ ID NO : 94-96 | 100,0% |
| 26 genes = SEQ ID NO : 70-89 + SEQ ID NO : 91-96 | 97,9% |
| 26 genes = SEQ ID NO : 70-89 + SEQ ID NO : 91-92 + SEQ ID NO : 94-97 | 97,9% |
| 26 genes = SEQ ID NO : 70-92 + SEQ ID NO : 94-96 | 100,0% |
| 26 genes = SEQ ID NO : 70-88 + SEQ ID NO : 90-96 | 97,9% |
| 26 genes = SEQ ID NO : 70-88 + SEQ ID NO : 90-92 + SEQ ID NO : 94-97 | 97,9% |
| 26 genes = SEQ ID NO : 70-89 + SEQ ID NO : 91-96 | 97,9% |
| 26 genes = SEQ ID NO : 70-88 + SEQ ID NO : 90-96 | 97,9% |
| 26 genes = SEQ ID NO : 70-88 + SEQ ID NO : 91-97 | 97,9% |
| 26 genes = SEQ ID NO : 70-89 + SEQ ID NO : 91-97 | 97,9% |
| 26 genes = SEQ ID NO : 70-88 + SEQ ID NO : 90-97 | 97,9% |
| 26 genes = SEQ ID NO : 70-89 + SEQ ID NO : 91-96 | 97,9% |
| 27genes SEQ ID NO: 70-96 | 100,0% |
| 27 genes SEQ ID NO: 70-89 and SEQ ID NO: 91-97 | 97,9% |
| 28 genes SEQ ID NO: 70-97 | 100% |

The invention also relates to a method, preferably in vitro, for identifying patient afflicted by a lung cancer having of the survival rate of at most about 20% at 30 months, among a population of patients afflicted by lung cancer having an estimated survival rate of at least about 30% at 30 months based on the diagnosis of said lung cancer according to histopathological criteria,
said method allowing the identification of at least 66% of patient of those afflicted by a lung cancer having of the survival rate of at most about 20% at 30 months,
said method comprising
* a step of measuring, in a biological sample of said patients, the expression of
   - at least 13 genes chosen among a set of 28 genes comprising or consisting of the nucleic acid sequences SEQ ID NO 70 to 97,
   - or fragments of said genes
   - or complementary sequences of said genes
   said at least 13 genes being such that
   - 12 genes comprise or consist of the nucleic acid sequences SEQ ID NO: 70 to 81, and
   - at least one gene belongs to a subset of 16 genes comprising or consisting of the nucleic acid sequences SEQ ID NO: 82-97, and
* a step of identifying biological samples expressing said at least 13 genes.

Advantageously, the invention relates to the method as defined above, wherein said at least 13 genes being such that
- 12 genes comprise or consist of the nucleic acid sequences SEQ ID NO: 70 to 81, and
- at least one gene belongs to a subset of 16 genes comprising or consisting of the nucleic acid sequences SEQ ID NO: 82-97, said at least one gene comprising or consisting of the nucleic acid sequences SEQ ID NO: 82,
said method allowing the identification of at least 70% of patients of those afflicted by a lung cancer having of the survival rate of at most about 20% at 30 months.

In one advantageous embodiment, the invention relates to a method, according to the above definition, said method comprising a step of measuring, in a biological sample of said patients, the expression of at least 18 genes chosen among a set of 28 genes comprising or consisting of the nucleic acid sequences SEQ ID NO 70 to 97, said at least 18 genes being such that
- 12 genes comprise or consist of the nucleic acid sequences SEQ ID NO: 70 to 81, and
- at least 6 genes belongs to a subset of 16 genes comprising or consisting of the nucleic acid sequences SEQ ID NO: 82-97, said at least 6 genes comprising or consisting of the nucleic acid sequences SEQ ID NO:82-85 and 87-88,
said method allowing the identification of at least 83% of patients of those having a survival rate of at most about 20% at 30 months.

In one advantageous embodiment, the invention relates to a method, according to the above definition, said method comprising a step of measuring, in a biological sample of said patients, the expression of at least 21 genes chosen among a set of 28 genes comprising or consisting of the nucleic acid sequences SEQ ID NO 70 to 97,
said at least 21 genes being such that
- 12 genes comprise or consist of the nucleic acid sequences SEQ ID NO: 70 to 81, and
- at least 9 gene belongs to a subset of 16 genes comprising or consisting of the nucleic acid sequences SEQ ID NO: 82-97, said at least 9 genes preferably comprising or consisting of the nucleic acid sequences SEQ ID NO: 82-88 and 91-92,
said method allowing identifying at least 89% of patients having a survival rate of at most about 20% at 30 months.

In one advantageous embodiment, the invention relates to a method, according to the above definition,, said method comprising a step of measuring, in a biological sample of said patients, the expression of at least 26 genes chosen among a set of 28 genes comprising or consisting of the nucleic acid sequences SEQ ID NO 70 to 97,
said at least 26 genes being such that
- 12 genes comprise or consist of the nucleic acid sequences SEQ ID NO: 70 to 81, and
- at least 14 gene belongs to a subset of 16 genes comprising or consisting of the nucleic acid sequences SEQ ID NO: 82-97, said at least 14 genes preferably comprising or consisting of the nucleic acid sequences SEQ ID NO: 82-92 and 93-96,
said method allowing identifying at least 97% of patients of those having a survival rate of at most about 20% at 30 months.

In one advantageous embodiment, the invention relates to a method, according to the above definition,, said method comprising a step of measuring, in a biological sample of said patients, the expression of at least 27 genes chosen among a set of 28 genes comprising or consisting of the nucleic acid sequences SEQ ID NO 70 to 97,
said at least 26 genes being such that
- 12 genes comprise or consist of the nucleic acid sequences SEQ ID NO: 70 to 81, and
- at least 15 gene belongs to a subset of 16 genes comprising or consisting of the nucleic acid sequences SEQ ID NO: 82-97,
said method allowing identifying at least 97% of patients of those having a survival rate of at most about 20% at 30 months.

In one advantageous embodiment, the invention relates to a method, according to the above definition, said method comprising a step of measuring, in a biological sample of said patients, the expression of 28 genes chosen comprising or consisting of the nucleic acid sequences SEQ ID NO 70 to 97,
said method allowing the identification of 100% of patient afflicted by a lung cancer having of the survival rate of at most about 20% at 30 months
said method comprising
* a step of measuring, in a biological sample of said patients, the expression of 28 comprising or consisting of the nucleic acid sequences SEQ ID NO 70 to 97, and
* a step of identifying biological samples expressing said 28 genes.

In another advantageous embodiment, the invention relates to a method as previously defined, wherein the step of measuring is carried out by using nucleic acid molecules consisting of from 15 to 100 nucleotides molecules being complementary to said at least 13 genes, or said at least 18 genes, or said at least 21 genes, or said at least 26 genes, or said 28 genes.

The skilled person can easily carry out the above method by choosing the appropriate means allowing the detection of the genes as defined above.

The above method applies mutatis mutandis using the proteins coded by the at least 13 genes as defined above.

The invention is illustrated by the following 65 figures and the two examples.

### Legend to the Figures

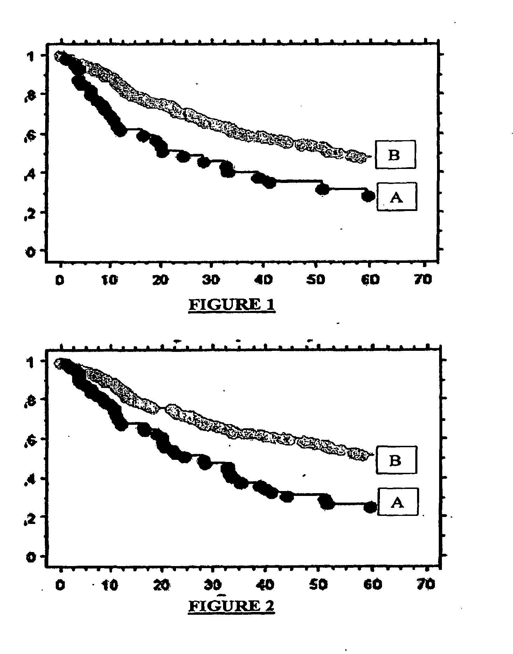

**Figure 1** represents the cumulative survival rate (Kaplan Mayer curve) over 60 months of patients expressing (A) or not (B) the gene SEQ ID NO: 4 (gene 1161). Y-axis represents cumulative survival in %, X-axis represents time in months.
**Figure 2** represents the cumulative survival rate (Kaplan Mayer curve) over 60 months of patients expressing (A) or not (B) the gene SEQ ID NO: 4 (gene 1161) and or the gene SEQ ID NO: 6 (gene 391). Y- axis represents cumulative survival in %, X-axis represents time in months.

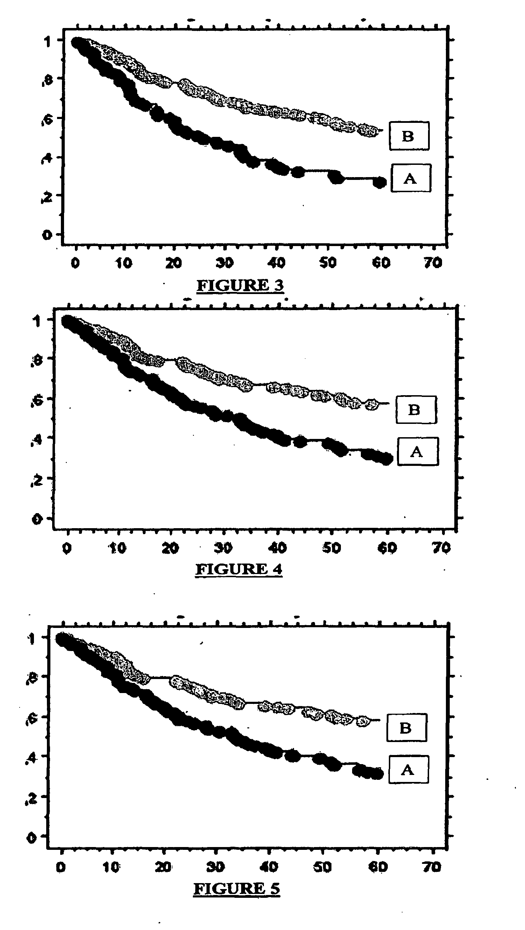

**Figure 3** represents the cumulative survival rate (Kaplan Mayer curve) over 60 months of patients expressing (A) or not (B) the gene SEQ ID NO: 4 (gene 1161) and/or the gene SEQ ID NO: 6 (gene 391) and/or the gene SEQ ID NO: 2 (gene 35).
Y- axis represents cumulative survival in %, X-axis represents time in months.
**Figure 4** represents the cumulative survival rate (Kaplan Mayer curve) over 60 months of patients expressing (A) or not (B) at least one of the following genes: SEQ ID NO: 4 (gene 1161), SEQ ID NO: 6 (gene 391), SEQ ID NO: 2 (gene 35) and SEQ ID NO: 1(gene 442). Y- axis represents cumulative survival in %, X-axis represents time in months.

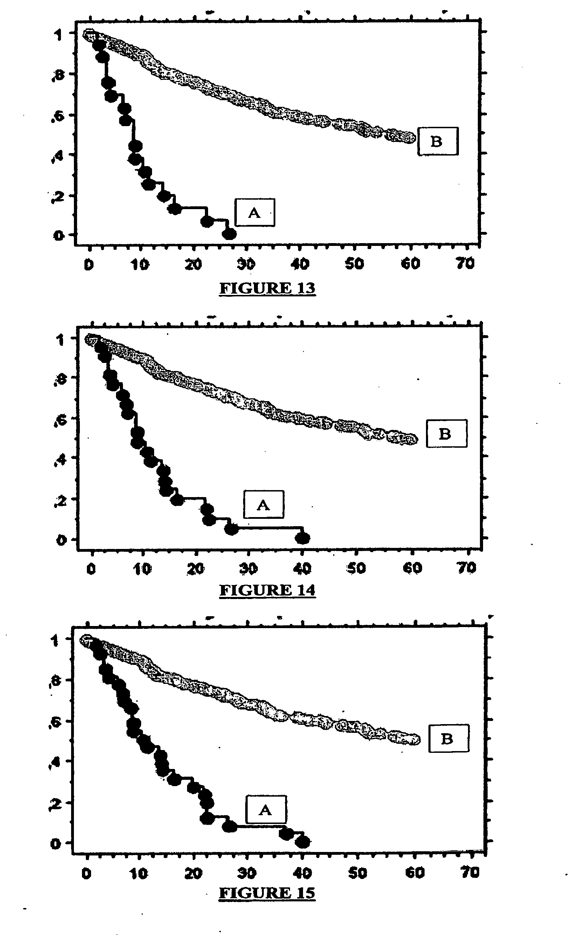

**Figure 5** represents the cumulative survival rate (Kaplan Mayer curve) over 60 months of patients expressing (A) or not (B) at least one of the following genes: SEQ ID NO: 4 (gene 1161), SEQ ID NO: 6 (gene 391), SEQ ID NO: 2 (gene 35), SEQ ID NO: 1(gene 442) and SEQ ID NO 5 (gene 102). Y- axis represents cumulative survival in %, X-axis represents time in months.

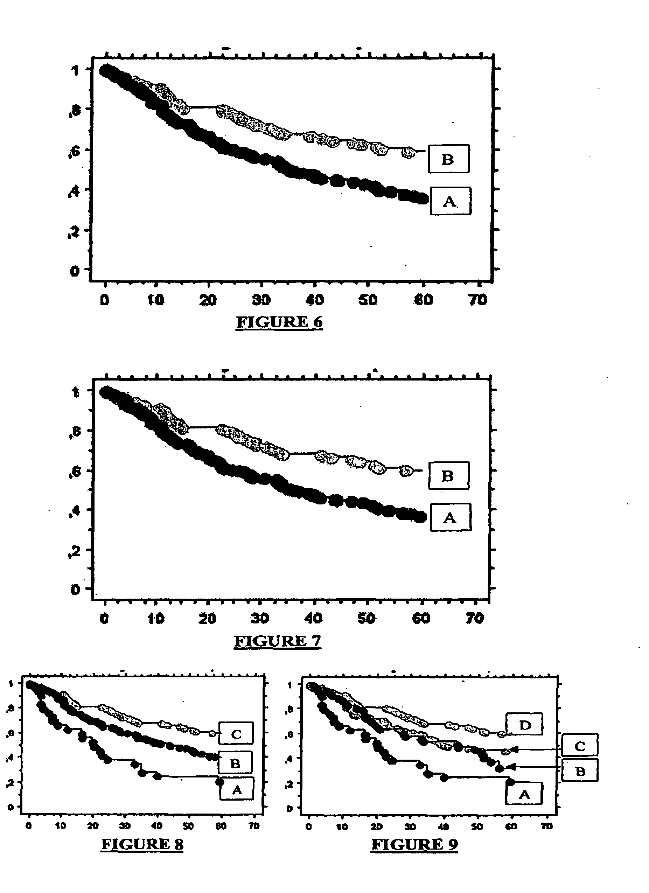

**Figure 6** represents the cumulative survival rate (Kaplan Mayer curve) over 60 months of patients expressing (A) or not (B) at least one of the following genes: SEQ ID NO: 4 (gene 1161), SEQ ID NO: 6 (gene 391), SEQ ID NO: 2 (gene 35), SEQ ID NO: 1(gene 442), SEQ ID NO 5 (gene 102) and SEQ ID NO:7 (gene 390). Y- axis represents cumulative survival in %, X-axis represents time in months.
**Figure 7** represents the cumulative survival rate (Kaplan Mayer curve) over 60 months of patients expressing (A) or not (B) at least one of the genes: SEQ ID NO: 1 to 7. Y- axis represents cumulative survival in %, X-axis represents time in months.
**Figure 8** represents the cumulative survival rate (Kaplan Mayer curve) over 60 months of patients expressing one or two (B), or three or more (A) or no (C) genes: SEQ ID NO: 1 to 7. Y- axis represents cumulative survival in %, X-axis represents time in months.
**Figure 9** represents the cumulative survival rate (Kaplan Mayer curve) over 60 months of patients expressing one (C) or two (B), or three or more (A) or no (D) genes: SEQ ID NO: 1 to 7. Y- axis represents cumulative survival in %, X-axis represents time in months.

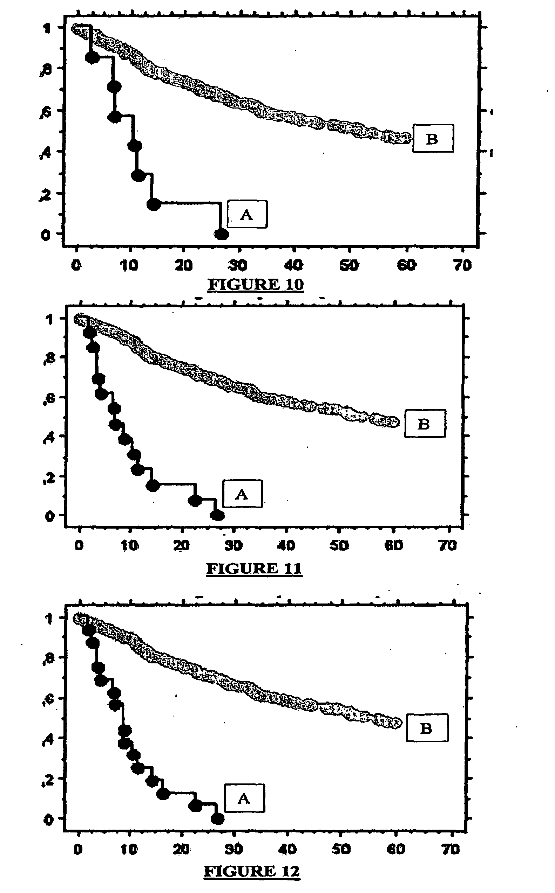

**Figure 10** represents the cumulative survival rate (Kaplan Mayer curve) over 60 months of patients expressing (A) or not (B) the gene SEQ ID NO: 16 (gene 125). Y-axis represents cumulative survival in %, X-axis represents time in months.
**Figure 11** represents the cumulative survival rate (Kaplan Mayer curve) over 60 months of patients expressing (A) or not (B) at least one of the following genes: SEQ ID NO: 16 (gene 125) and SEQ ID NO: 22 (gene 117). Y- axis represents cumulative survival in %, X-axis represents time in months.
**Figure 12** represents the cumulative survival rate (Kaplan Mayer curve) over 60 months of patients expressing (A) or not (B) at least one of the following genes: SEQ ID NO: 16 (gene 125), SEQ ID NO: 22 (gene 117) and SEQ ID NO:19 (766). Y- axis represents cumulative survival in %, X-axis represents time in months.
**Figure 13** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766) and SEQ ID NO: 17(gene 144). Y- axis represents cumulative survival in %, X-axis represents time in months.
**Figure 14** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144) and SEQ ID NO: 12 (gene 108). Y- axis represents cumulative survival in %, X-axis represents time in months.
**Figure 15** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108) and SEQ ID NO: 8 (gene 222). Y-axis represents cumulative survival in %, X-axis represents time in months.

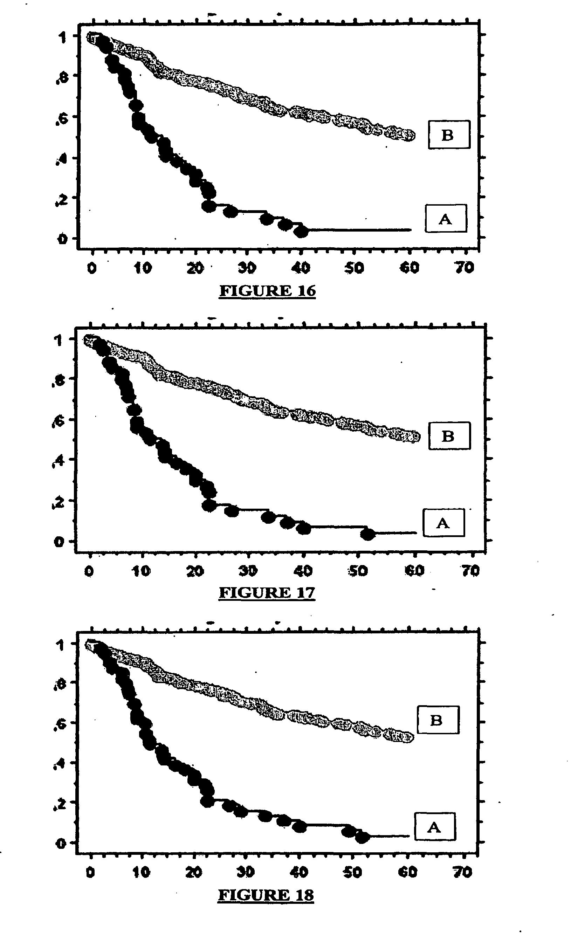

**Figure 16** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222) and SEQ ID NO: 17 (gene. 72). Y- axis represents cumulative survival in %, X-axis represents time in months.
**Figure 17** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72) and SEQ ID NO: 10 (gene 1165). Y- axis represents cumulative survival in %, X-axis represents time in months.
**Figure 18** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO: 19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165) and SEQ ID NO: 21 (gene 487). Y-axis represents cumulative survival in %, X-axis represents time in months.

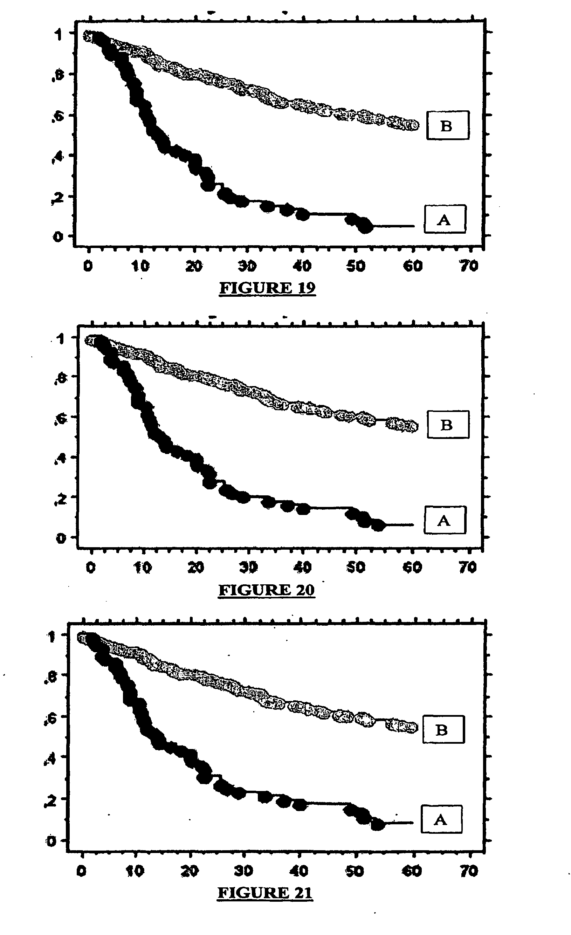

**Figure 19** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487) and SEQ ID NO: 9(gene 1261). Y- axis represents cumulative survival in %, X-axis represents time in months.
**Figure 20** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9(gene 1261) and SEQ ID N NO: 13 (gene 205). Y- axis represents cumulative survival in %, X-axis represents time in months.
**Figure 21** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9(gene 1261), SEQ ID N NO: 13 (gene 205) and SEQ ID NO: 18 (gene 437). Y-axis represents cumulative survival in %, X-axis represents time in months.

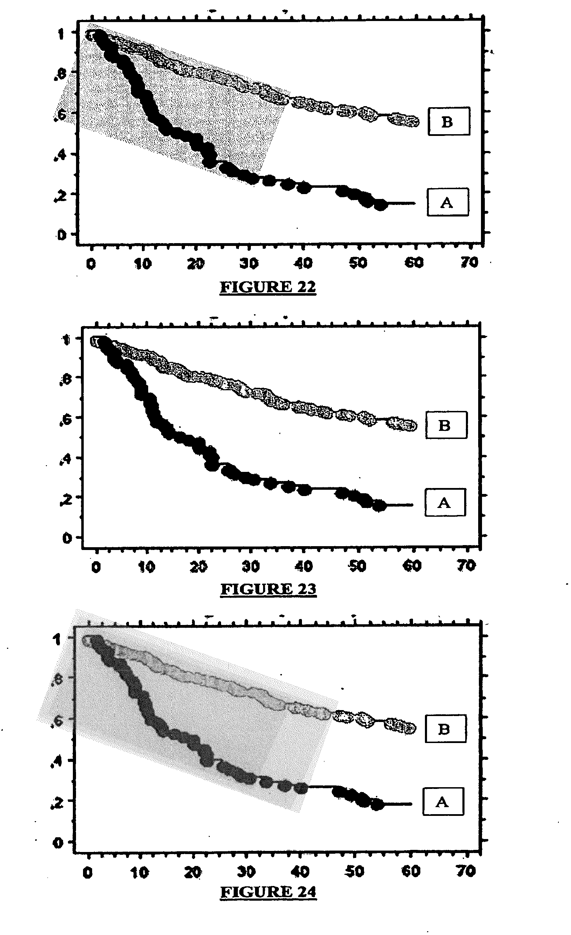

**Figure 22** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9(gene 1261), SEQ ID N NO: 13 (gene 205), SEQ ID NO: 18 (gene 437) and SEQ ID NO:15 (gene 1328). Y- axis represents cumulative survival in %, X-axis represents time in months.
**Figure 23** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO: 19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9(gene 1261), SEQ ID N NO: 13 (gene 205), SEQ ID NO: 18 (gene 437), SEQ ID NO:15 (gene 1328) and SEQ ID NO: 14 (gene 1188). Y- axis represents cumulative survival in %, X-axis represents time in months.
**Figure 24** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9(gene 1261), SEQ ID N NO: 13 (gene 205), SEQ ID NO: 18 (gene 437), SEQ ID NO: 15 (gene 1328), SEQ ID NO: 14 (gene 1188) and SEQ ID NO: 20 (gene 436). Y- axis represents cumulative survival in %, X-axis represents time in months.

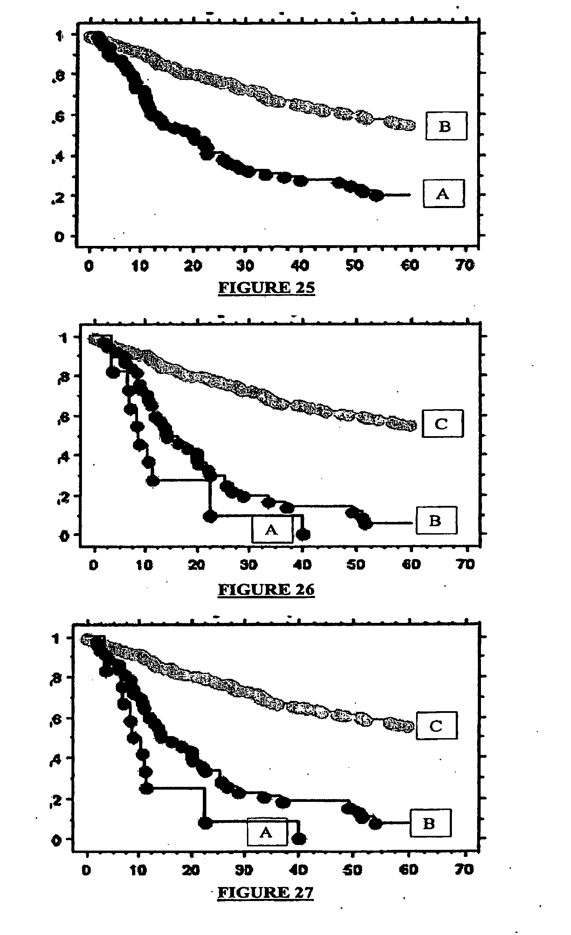

**Figure 25** represents the cumulative survival rate (Kaplan Mayer curve) over 60 months of patients expressing (A) or not (B) at least one of the following genes: SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9(gene 1261), SEQ ID N NO: 13 (gene 205), SEQ ID NO: 18 (gene 437), SEQ ID NO: 15 (gene 1328), SEQ ID NO: 14 (gene 1188), SEQ ID NO: 20 (gene 436) and SEQ ID NO: 23 (gene 135). Y- axis represents cumulative survival in %, X-axis represents time in months.
**Figure 26** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487) and SEQ ID NO: 9 (gene 1261). Y- axis represents cumulative survival in %, X-axis represents time in months.
**Figure 27** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO: 19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261) and SEQ ID N NO: 13 (gene 205). Y- axis represents cumulative survival in %, X-axis represents time in months.

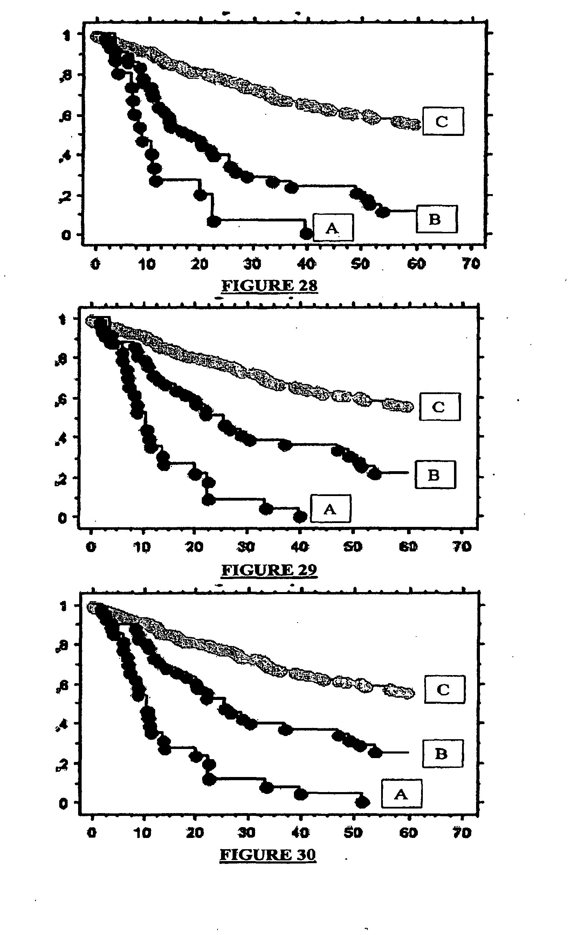

**Figure 28** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205) and SEQ ID NO: 18 (gene 437). Y- axis represents cumulative survival in %, X-axis represents time in months.
**Figure 29** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO: 19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205), SEQ ID NO: 18 (gene 437) and SEQ ID NO:15 (gene 1328). Y- axis represents cumulative survival in %, X-axis represents time in months.
**Figure 30** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO: 19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205), SEQ ID NO: 18 (gene 437), SEQ ID NO:15 (gene 1328) and SEQ ID NO: 14 (gene 1188). Y- axis represents cumulative survival in %, X-axis represents time in months.

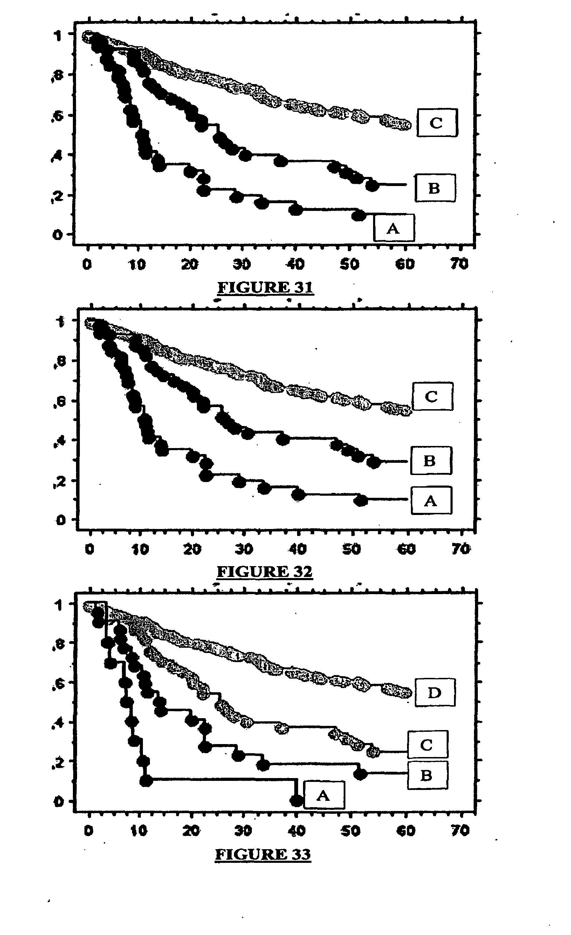

**Figure 31** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO: 19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205), SEQ ID NO: 18 (gene 437), SEQ ID NO:15 (gene 1328), SEQ ID NO: 14 (gene 1188) and SEQ ID NO: 20 (gene 436). Y- axis represents cumulative survival in %, X-axis represents time in months.
**Figure 32** represents the cumulative survival rate (Kaplan Mayer curve) over 60 months of patients expressing one (B), or two or more (A) or no (C) genes chosen among SEQ ID NO: 16 (gene 125), SEQ ID NO: 22 (gene 117), SEQ ID NO: 19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205), SEQ ID NO: 18 (gene 437), SEQ ID NO:15 (gene 1328), SEQ ID NO: 14 (gene 1188), SEQ ID NO: 20 (gene 436) and SEQ ID NO: 23 (gene 135). Y- axis represents cumulative survival in %, X-axis represents time in months.
**Figure 33** represents the cumulative survival rate (Kaplan Mayer curve) over 60 months of patients expressing one (D), or two (B) or three or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205), SEQ ID NO: 18 (gene 437), SEQ ID NO:15 (gene 1328), SEQ ID NO: 14 (gene 1188) and SEQ ID NO: 20 (gene 436). Y- axis represents cumulative survival in %, X-axis represents time in months.

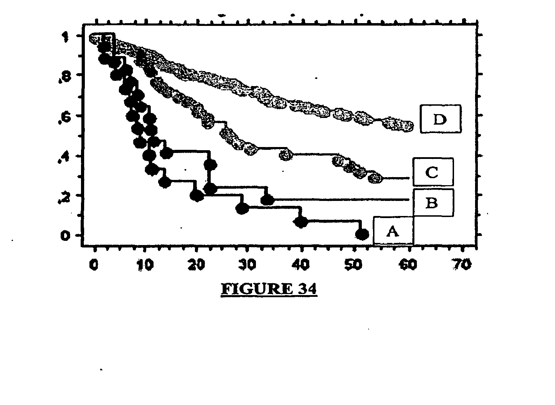

**Figure 34** represents the cumulative survival rate (Kaplan Mayer curve) over 60 months of patients expressing one (C), or two (B) or three or more (A) or no (C) genes chosen among SEQ ID NO: 16 (gene 125), SEQ ID NO: 22 (gene 117), SEQ ID NO:19 (gene 766), SEQ ID NO: 17(gene 144), SEQ ID NO: 12 (gene 108), SEQ ID NO: 8 (gene 222), SEQ ID NO: 17 (gene 72), SEQ ID NO: 10 (gene 1165), SEQ ID NO: 21 (gene 487), SEQ ID NO: 9 (gene 1261), SEQ ID N NO: 13 (gene 205), SEQ ID NO: 18 (gene 437), SEQ ID NO:15 (gene 1328), SEQ ID NO: 14 (gene 1188), SEQ ID NO: 20 (gene 436) and SEQ ID NO: 23 (gene 135). Y- axis represents cumulative survival in %, X-axis represents time in months.

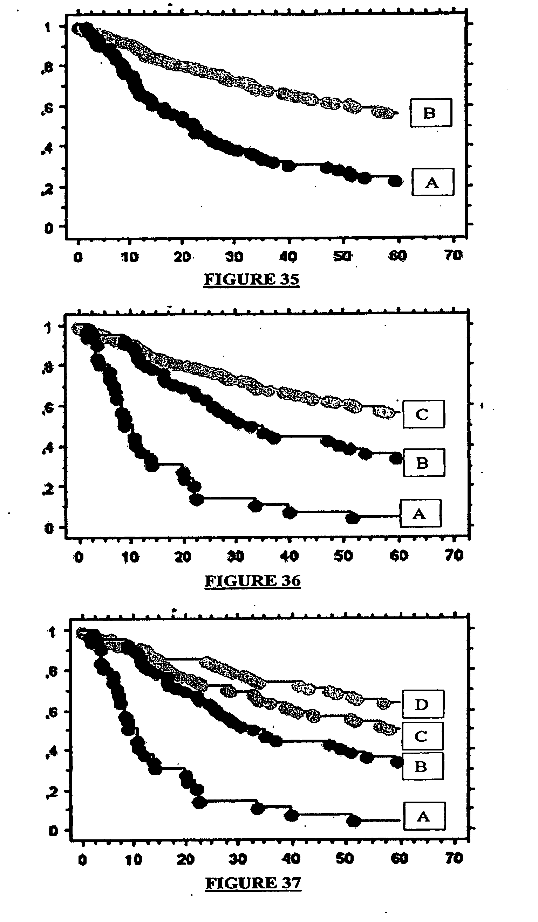

**Figure 35** represents the cumulative survival rate (Kaplan Mayer curve) over 60 months of patients expressing no (B), or at least one gene SEQ ID NO:1-23. Y- axis represents cumulative survival in %, X-axis represents time in months.
**Figure 36** represents the cumulative survival rate (Kaplan Mayer curve) over 60 months of patients expressing no (C),
   or none of the genes SEQ ID NO: 8-23 and at least 3 genes SEQ ID NO: 1-7 are expressed expressed, or at least 1 gene SEQ ID NO: 8-23 is expressed and from none to 2 genes SEQ ID NO: 1-7 is expressed, or at least 2 genes SEQ ID NO: 8-23 are expressed and no gene SEQ ID NO: 1-7 is expressed (B),
   or one gene SEQ ID NO: 8-23 is expressed and at least 3 genes SEQ ID NO: 1-7 are expressed, or at least 2 genes SEQ ID NO: 8-23 are expressed and at least 1 gene SEQ ID NO: 1-7 is expressed (A).
   Y- axis represents cumulative survival in %, X-axis represents time in months.
**Figure 37** represents the cumulative survival rate (Kaplan Mayer curve) over 60 months of patients expressing no (D),
   or if none of the genes SEQ ID NO: 8-23 is expressed and one or two genes SEQ ID NO: 1-7 is expressed (C)
   or none of the genes SEQ ID NO: 8-23 and at least 3 genes SEQ ID NO: 1-7 are expressed, or at least 1 gene SEQ ID NO: 8-23 is expressed and from none to 2 genes SEQ ID NO: 1-7 is expressed, or at least 2 genes SEQ ID NO: 8-23 are expressed and no gene SEQ ID NO: 1-7 is expressed (B),
   or one gene SEQ ID NO: 8-23 is expressed and at least 3 genes SEQ ID NO: 1-7 are expressed, or at least 2 genes SEQ ID NO: 8-23 are expressed and at least 1 gene SEQ ID NO: 1-7 is expressed (A).
   Y- axis represents cumulative survival in %, X-axis represents time in months.

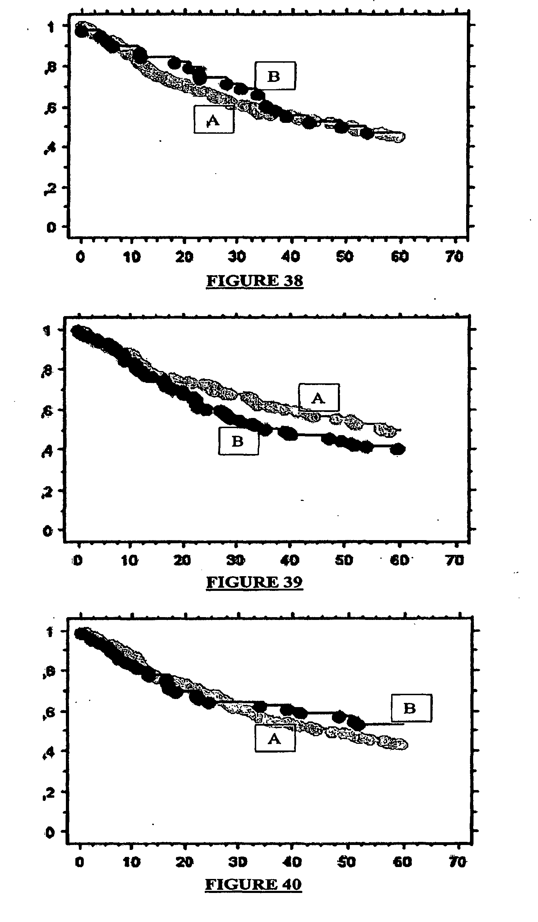

**Figure 38** represents the cumulative survival rate (Kaplan Mayer curve) over 60 months of patients expressing no genes SEQ ID NO: 1-23 (A) or expressing LDHC gene (B).
**Figure 39** represents the cumulative survival rate (Kaplan Mayer curve) over 60 months of patients expressing no genes SEQ ID NO: 1-23 (A) or expressing MAGEA5 gene (B).
**Figure 40** represents the cumulative survival rate (Kaplan Mayer curve) over 60 months of patients expressing no genes SEQ ID NO: 1-23 (A) or expressing MAGEB18 gene (B).
**Figures 41-65** **refer to the at least 13 genes chosen among 28 genes conmprising or consisting of SEQ ID NO: 70-97.**
**Figure 41** represents the global survival probability over 5 years of 300 lung cancer patients.
**Figure 42** represents the global survival probability over 5 years of 300 lung cancer patients by using the invention.
**Figure 43** represents the global survival probability over 5 years of patients with T1N0 (early stages) or advanced stages (T+N+) of lung cancer according to the TNM classification.
**Figure 44** represents the global survival probability over 5 years of patients with T1N0 (early stages) of lung cancer by using the invention.
**Figure 45** represents the global survival probability over 5 years of patients with advanced stages (T+N+) of lung cancer by using the invention.
**Figure 46** represents the respective global survival probabilities over 5 years of patients with BAS, SQC and LCNE lung cancer according to the histopathological classification.

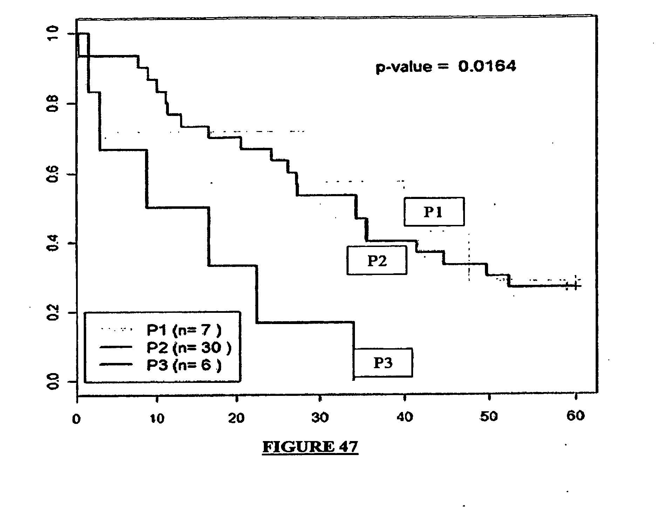

**Figure 47** represents the global survival probability over 5 years of patients with BAS lung cancer by using the invention.

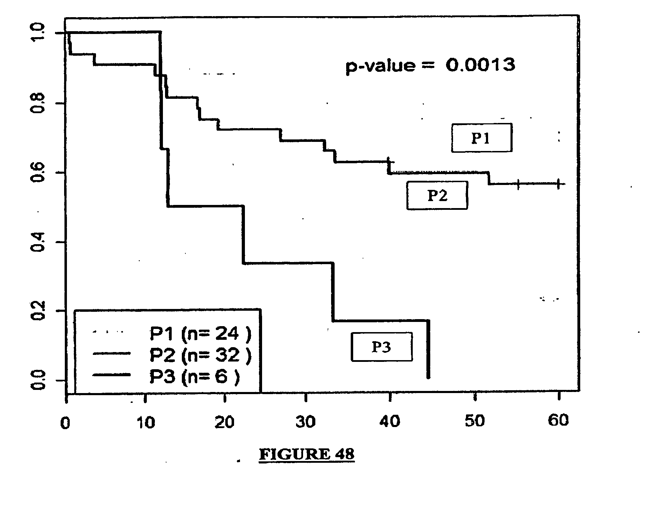

**Figure 48** represents the global survival probability over 5 years of patients with SQC lung cancer by using the invention.

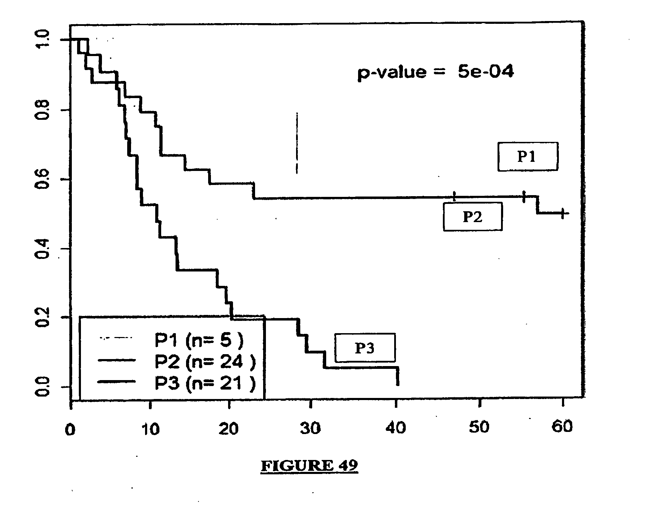

**Figure 49** represents the global survival probability over 5 years of patients with LCNE lung cancer by using the invention.

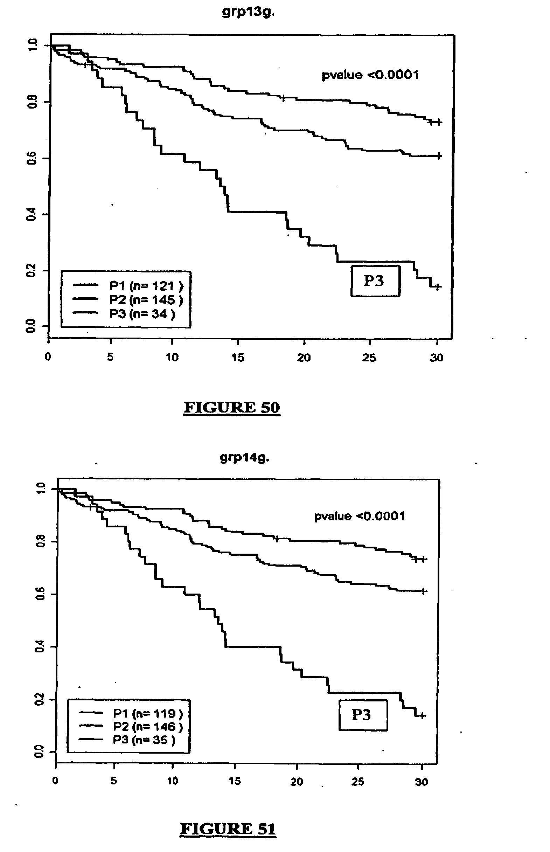

**Figure 50** represents the global survival probability over 30 months of 300 lung cancer patients by using 13 genes of invention.
**Figure 51** represents the global survival probability over 30 months of 300 lung cancer patients by using 14 genes of invention

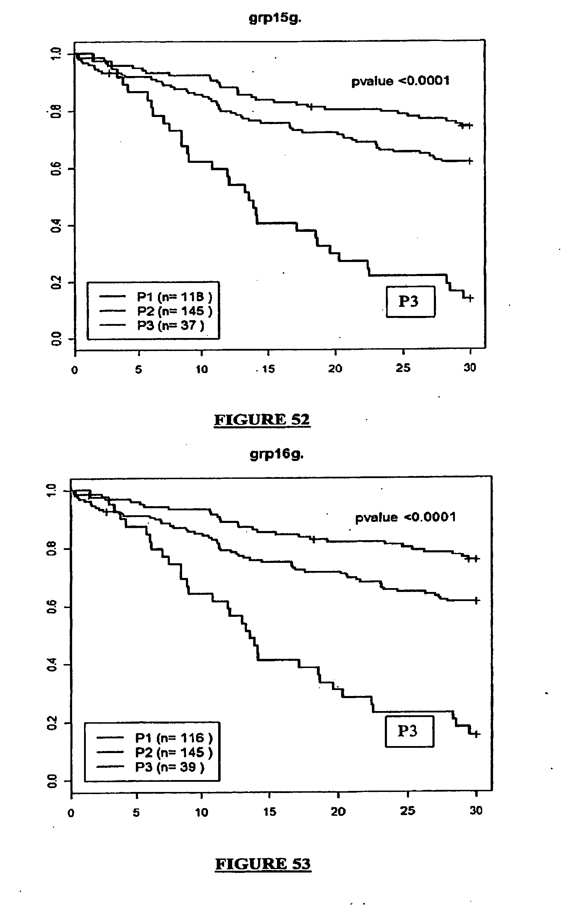

**Figure 52** represents the global survival probability over 30 months of 300 lung cancer patients by using 15 genes of invention
**Figure 53** represents the global survival probability over 30 months of 300 lung cancer patients by using 16 genes of invention

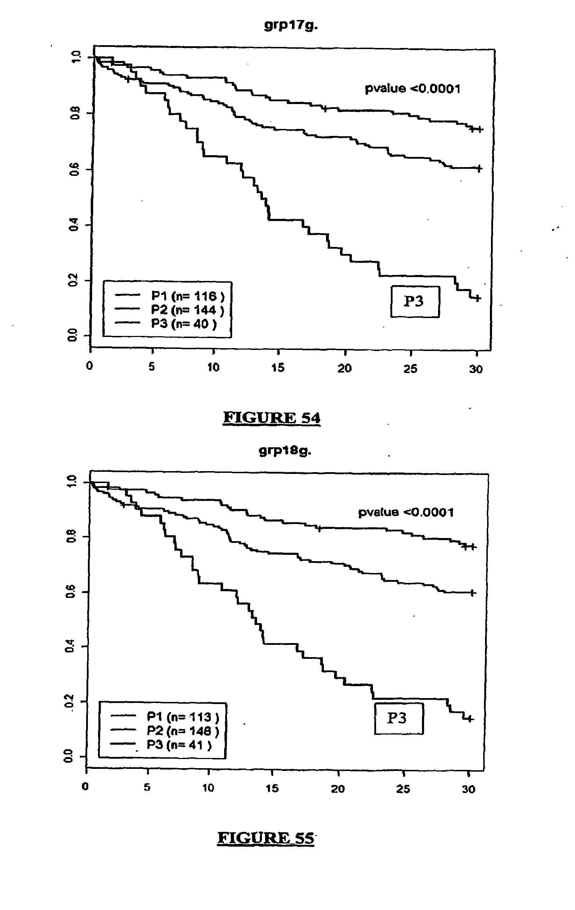

**Figure 54** represents the global survival probability over 30 months of 300 lung cancer patients by using 17 genes of invention
**Figure 55** represents the global survival probability over 30 months of 300 lung cancer patients by using 18 genes of invention

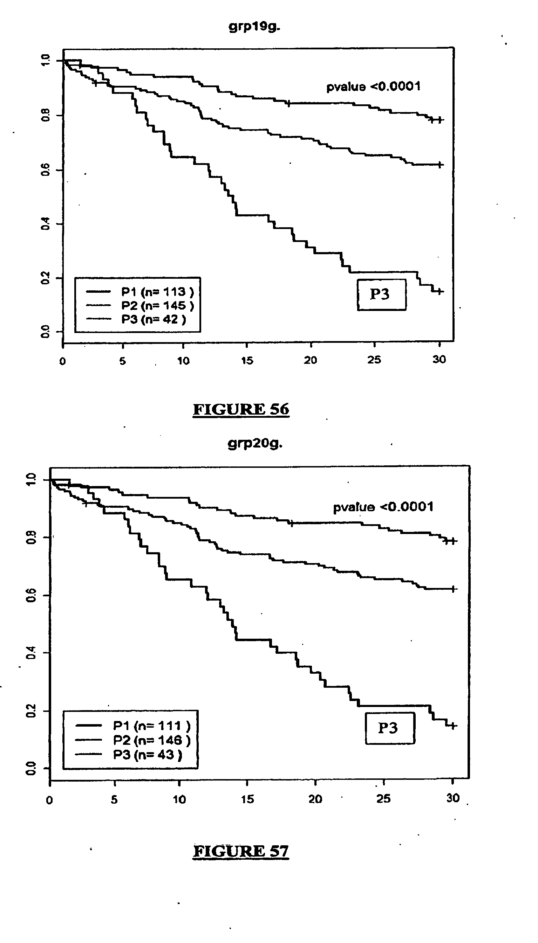

**Figure 56** represents the global survival probability over 30 months of 300 lung cancer patients by using 19 genes of invention
**Figure 57** represents the global survival probability over 30 months of 300 lung cancer patients by using 20 genes of invention

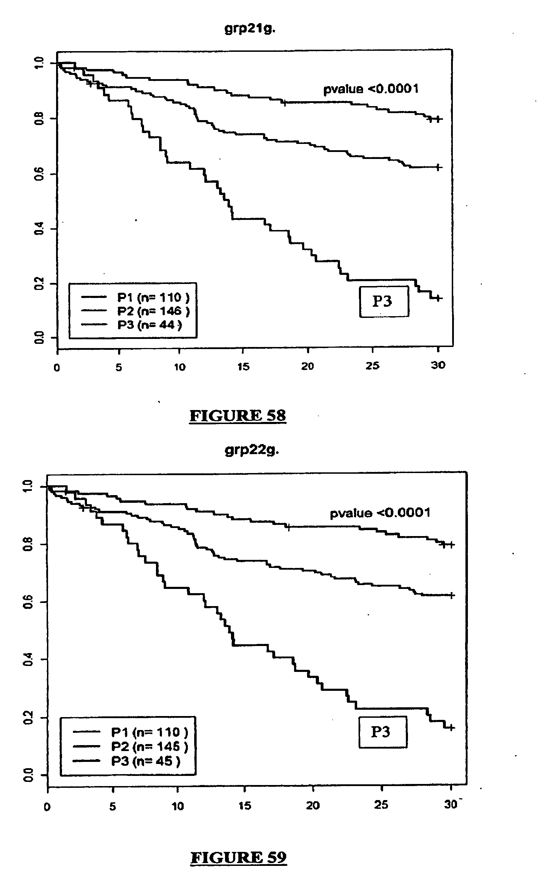

**Figure 58** represents the global survival probability over 30 months of 300 lung cancer patients by using 21 genes of invention
**Figure 59** represents the global survival probability over 30 months of 300 lung cancer patients by using 22 genes of invention

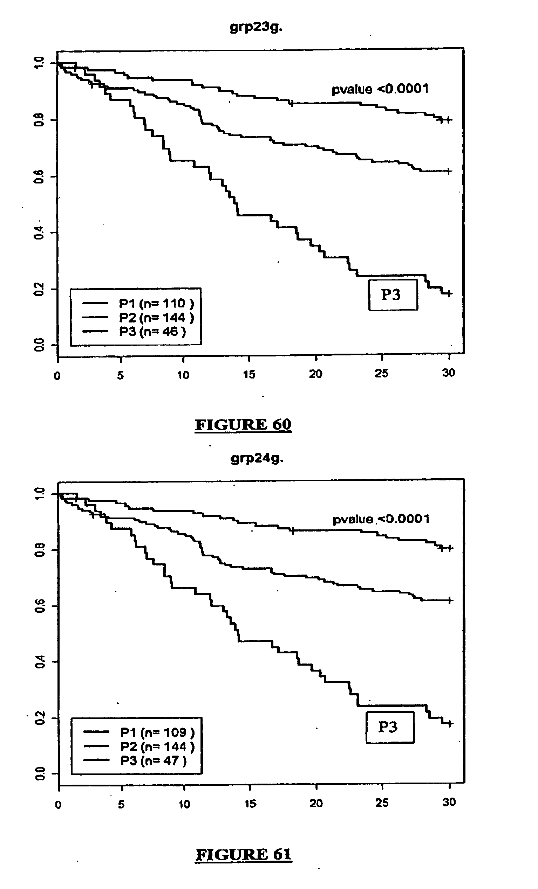

**Figure 60** represents the global survival probability over 30 months of 300 lung cancer patients by using 23 genes of invention
**Figure 61** represents the global survival probability over 30 months of 300 lung cancer patients by using 24 genes of invention

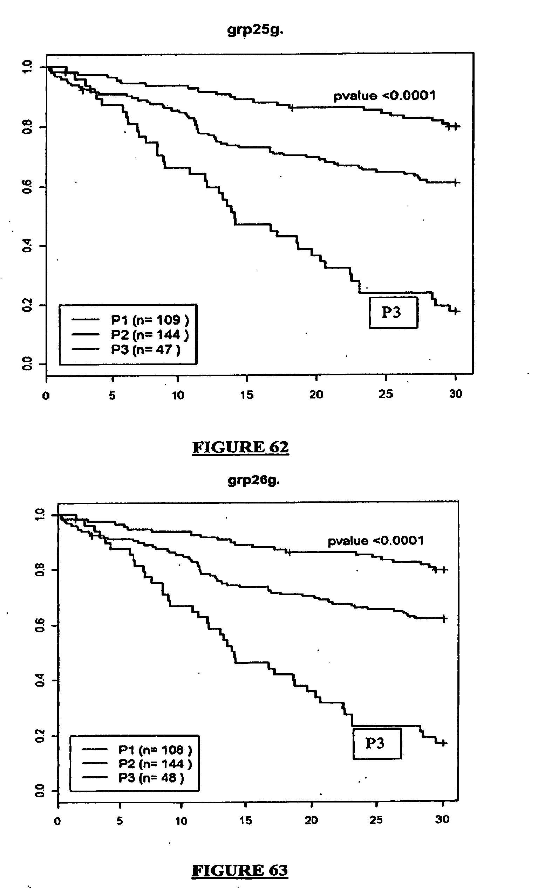

**Figure 62** represents the global survival probability over 30 months of 300 lung cancer patients by using 25 genes of invention
**Figure 63** represents the global survival probability over 30 months of 300 lung cancer patients by using 26 genes of invention

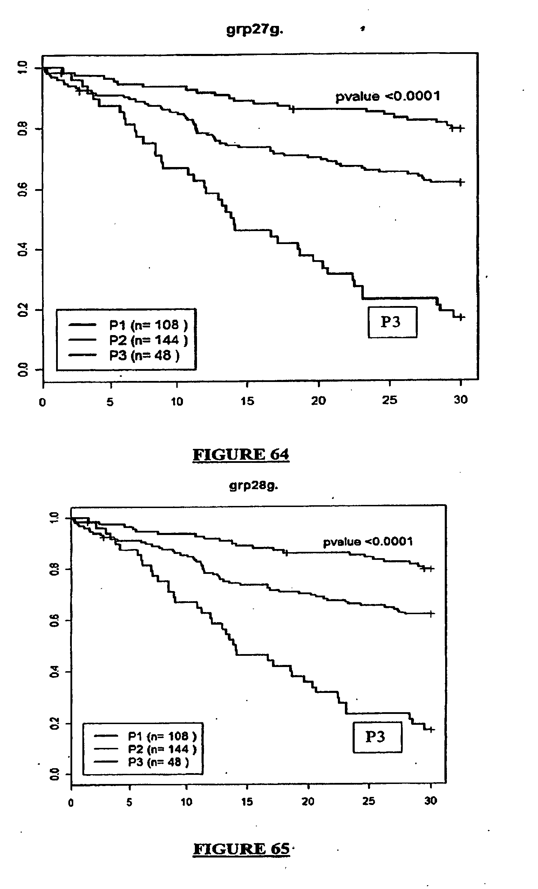

**Figure 64** represents the global survival probability over 30 months of 300 lung cancer patients by using 27 genes of invention
**Figure 65** represents the global survival probability over 30 months of 300 lung cancer patients by using 28 genes of invention

### EXAMPLES:

### EXAMPLE 1

The present disclosure describes a group of **23 genes,** which can be used to establish the survival prognosis of lung tumour patients. All these genes are actively repressed and silent in normal adult somatic cells, since their expression is strictly restricted to placenta or male germinal cells. The inventors have demonstrated that the aberrant expression in malignant cells of at least one of these genes is associated with significantly poorer prognosis for lung cancer patients. Moreover, the detection of the expression of several combinations of these genes allows predicting prognosis in lung tumour patients with higher significance and accuracy than with individual genes.

The invention has led to the identification of 23 genes, whose aberrant expression was found associated with poor prognosis in lung tumour patients.

According to their expression and prognosis value in lung cancer patients, these 23 genes were divided into two groups
- A group of 7 genes, whose aberrant expression in lung cancer is relatively frequent (>7% of cases of our series). The expression of each individual one of these seven genes is associated with a significantly reduced survival probability (global or disease free survival over five years significantly reduced, logrank test p<0,07) of all lung cancer patients (without considering histological subtypes).
- A group of 16 genes, whose aberrant expression in lung cancer is relatively rare (<7% of cases of our series). The expression of each individual one of these seven genes is associated with a significantly reduced survival probability (global or disease free survival over five years significantly reduced, logrank test p<0,07) of all lung cancer patients (without considering histological subtypes).

The lists of these genes and their individual association with survival rates in patients from a series of 271 lung tumours are shown in the following **table 1.**

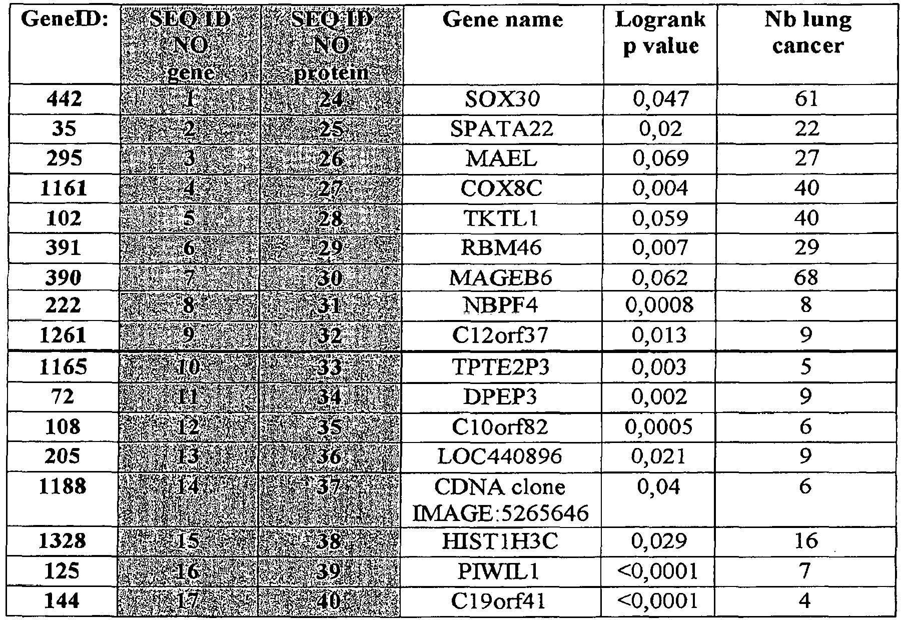

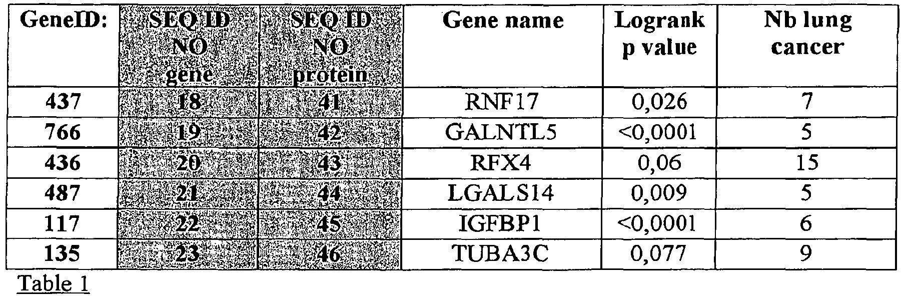

The correspondence between the gene ID number and the corresponding SEQ ID is represented as follows:
Gene Num ID : 442 corresponds to SEQ ID NO: 1, Gene Num ID : 35 corresponds to SEQ ID NO: 2, Gene Num ID : 295 corresponds to SEQ ID NO: 3, Gene Num ID : 1161 corresponds to SEQ ID NO: 4, Gene Num ID : 102 corresponds to SEQ ID NO: 5, Gene Num ID : 391 corresponds to SEQ ID NO: 6, Gene Num ID : 390 corresponds to SEQ ID NO: 7, Gene Num ID : 222 corresponds to SEQ ID NO: 8, Gene Num ID : 1261 corresponds to SEQ ID NO: 9, Gene Num ID : 1165 corresponds to SEQ ID NO: 10, Gene Num ID : 72 corresponds to SEQ ID NO: 11, Gene Num ID : 108 corresponds to SEQ ID NO: 12, Gene Num ID : 205 corresponds to SEQ ID NO: 13, Gene Num ID : 1188 corresponds to SEQ ID NO: 14, Gene Num ID : 1328 corresponds to SEQ ID NO: 15, Gene Num ID : 125 corresponds to SEQ ID NO: 16, Gene Num ID : 144 corresponds to SEQ ID NO: 17, Gene Num ID : 437 corresponds to SEQ ID NO: 18, Gene Num ID : 766 corresponds to SEQ ID NO: 19, Gene Num ID : 436 corresponds to SEQ ID NO: 20, Gene Num ID : 487 corresponds to SEQ ID NO: 21, Gene Num ID : 117 corresponds to SEQ ID NO: 22 and Gene Num ID : 135 corresponds to SEQ ID NO: 23.

Logrank p value corresponds to the significance of difference in cumulative global survival probabilities over 5 years between patients expressing the gene and those not expressing the gene. Nb lung cancer corresponds to the number of lung cancer patients expressing the gene (/271).

Each of these genes can be used individually to establish a prognosis in lung cancer patients: any patient expressing any one of the 23 genes (of the group of 7 or the group of 16) has significantly lower chances of survival compared to the patients not expressing the gene (see **table 2a).**

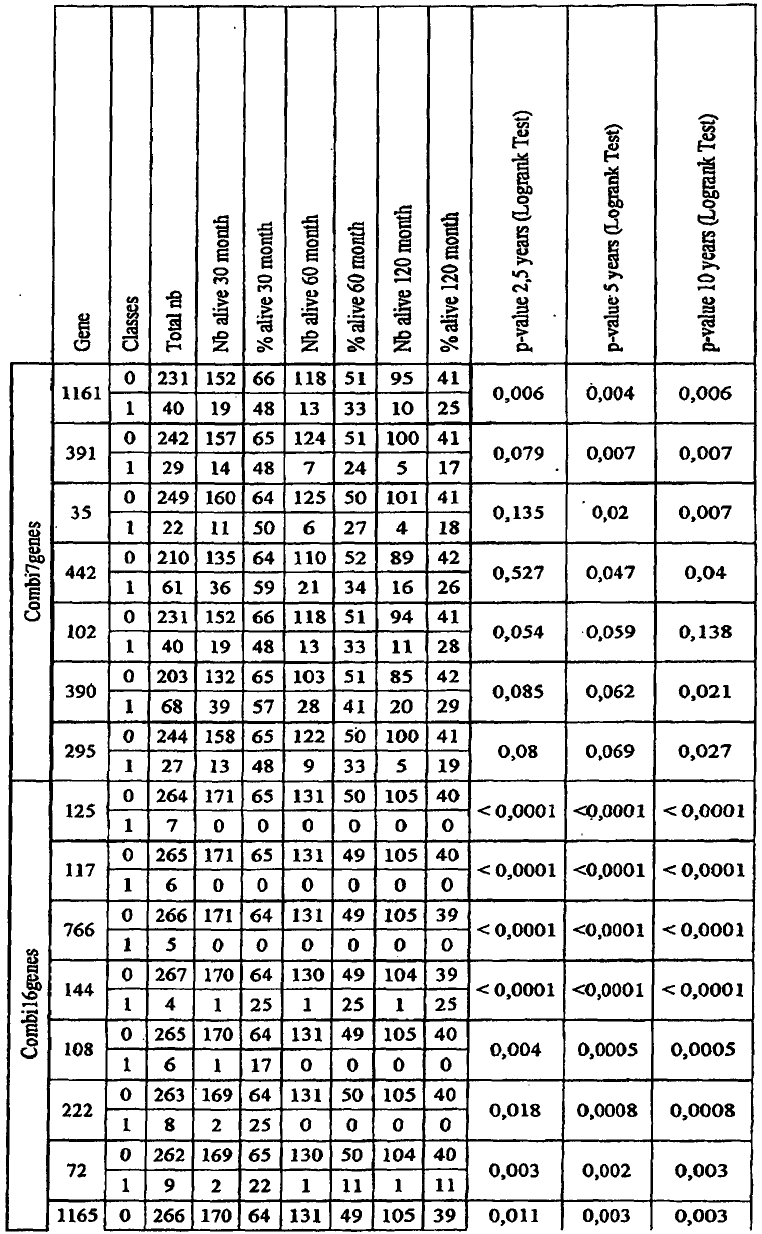

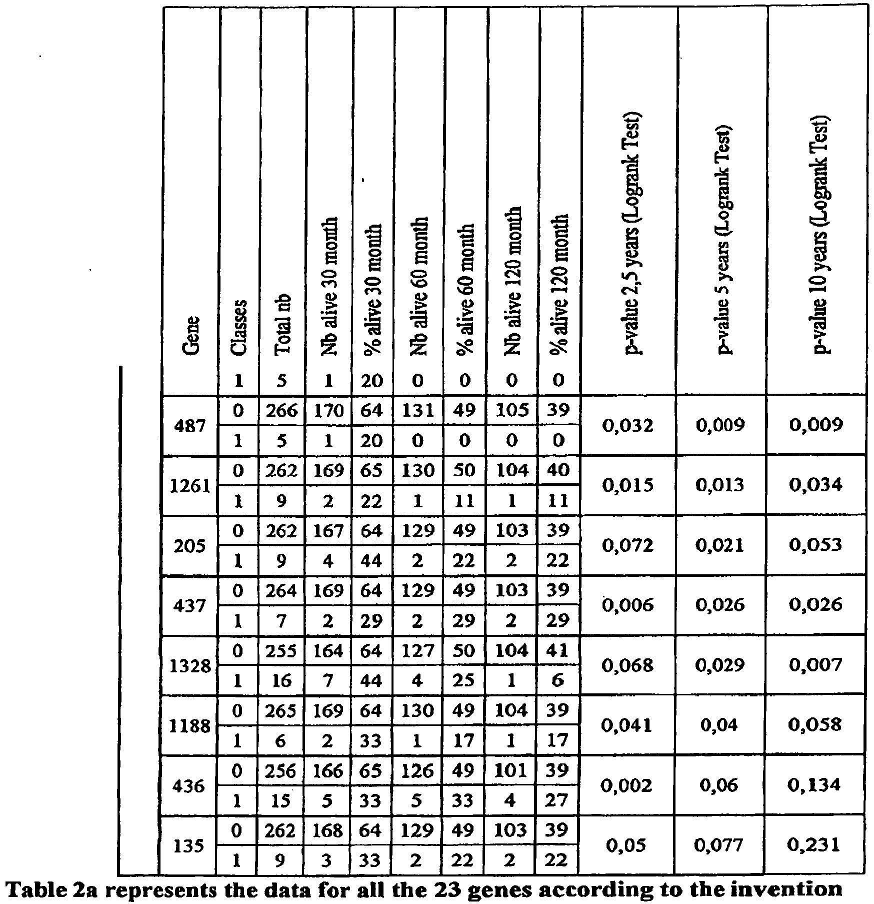

- Gene = gene identifier(s) of individual genes or combinations of genes
- Classes = Lung Kc patient not expressing (=0) or expressing (=1) the gene; In the case of combinations of several genes, some patients could express one gene only or at least one gene of the combination (=1), 2 genes only or 2 genes or more of the combination (=2) etc...
- Total Nb = total number of patients of this class (considering the whole Brambilla study with 271 cases, including all histological types)
- % or nb alive 30, 60, 120 month = % or number of patients of this class alive after 30, 60, 120 month
- p-value corresponding to the significance of the difference in cumulative global survival probabilities between the different classes of patients over 2,5, 5 and 10 years (Logrank Test, considering survival curves of all the classes of patients).

The inventors have also shown that using combinations of these genes allows a more accurate prognosis. Examples of these combinations and their use to establish a prognosis are shown below.

In order to establish a prognosis in lung cancer patients, the aberrant expression of these genes can be used as follows:
- Combinations of all or several of the genes of the group of 7 genes and/or of the group of 16 genes (examples of combinations, see tables 2b and 2c)
- Any one gene of the group of 7 genes (preferably) or of the group of 16 genes (see table 2a)

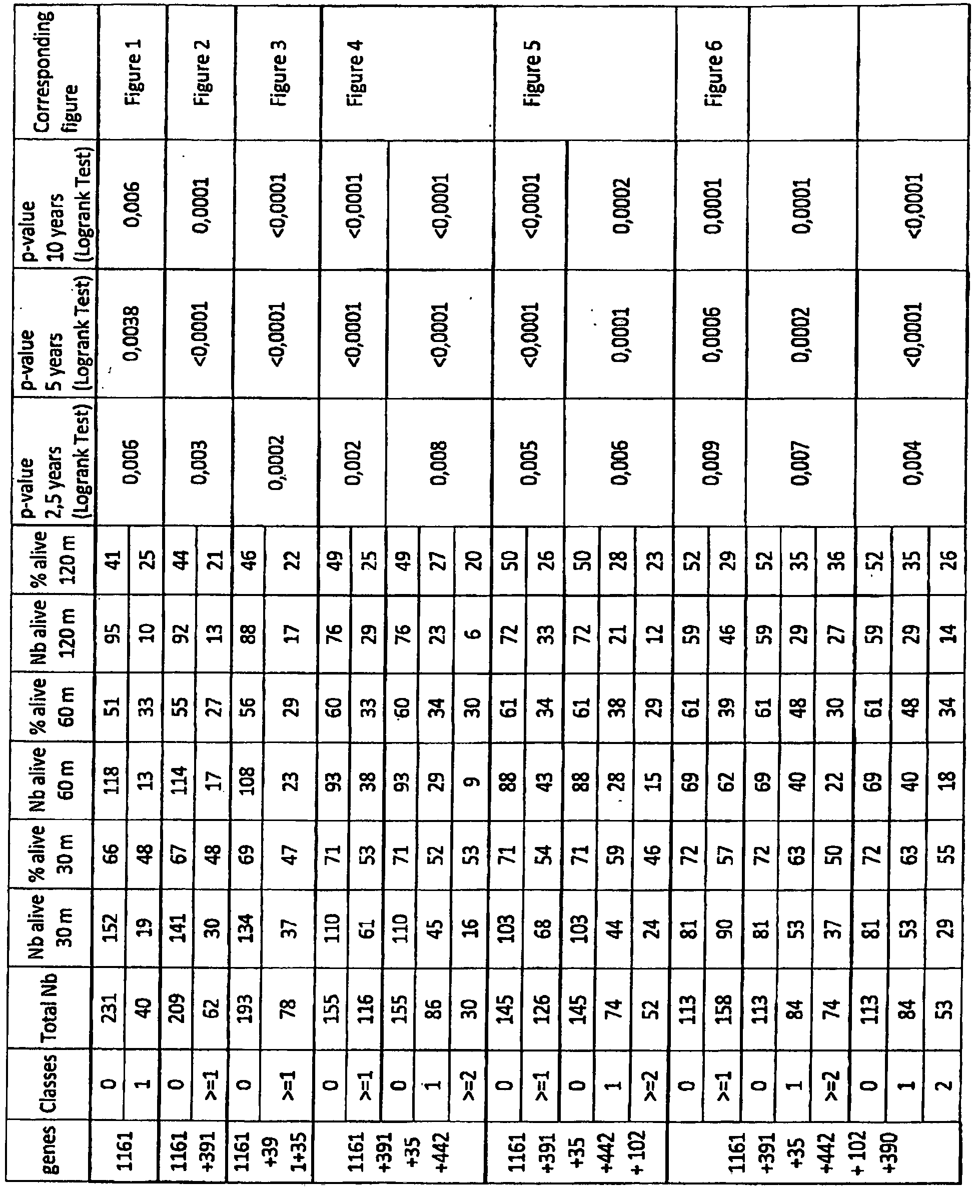

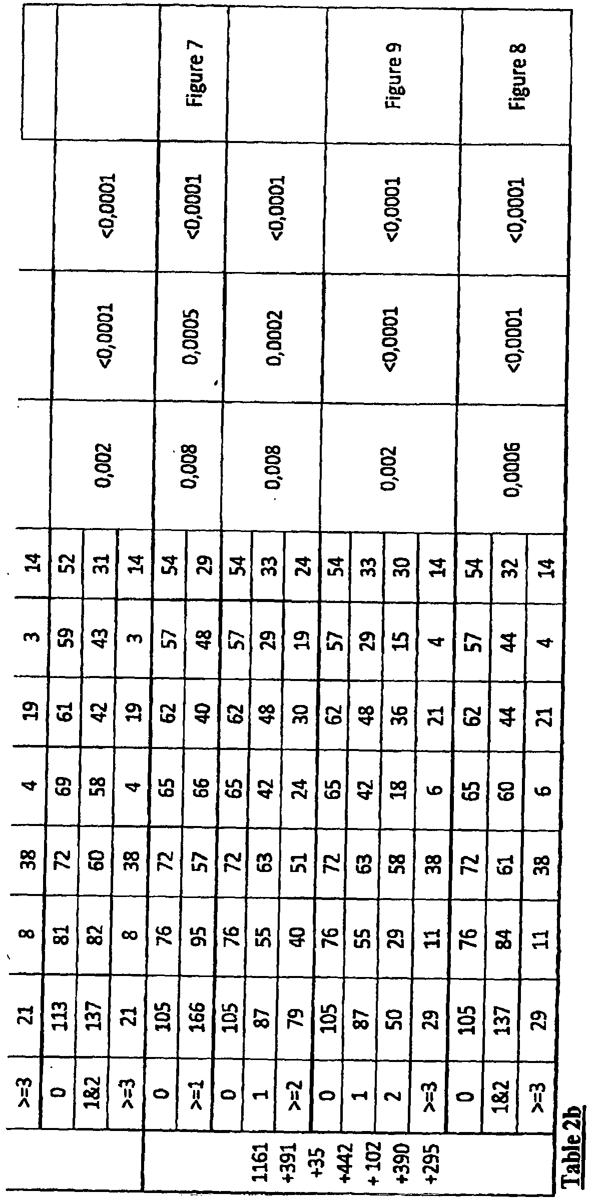

**Table 2c**

| Genes | Classes | Total Nb | Nb alive 30 m | % alive 30 m | Nb alive 60 m | % alive 60 m | Nb alive 120 m | % alive 120 m | p-value 2,5 years (Logrank Test) | p-value 5 years (Logrank Test) | p-value 10 years (Logrank Test) | Correspo nding figure |
|---|---|---|---|---|---|---|---|---|---|---|---|---|
| 125 | 0 | 264 | 171 | 65 | 131 | 50 | 105 | 40 | <0,0001 | <0,0001 | <0,0001 | Figure 10 |
| | 1 | 7 | 0 | 0 | 0 | 0 | 0 | 0 | | | | |
| 125 | 0 | 258 | 171 | 66 | 131 | 51 | 105 | 41 | <0,0001 | <0,0001 | <0,0001 | Figure 11 |
| +117 | >=1 | 13 | 0 | 0 | 0 | 0 | 0 | 0 | | | | |
| 125 | 0 | 255 | 171 | 67 | 131 | 51 | 105 | 41 | <0,0001 | <0,0001 | <0,0001 | Figure 12 |
| +117 | >=1 | 16 | 0 | 0 | 0 | 0 | 0 | 0 | | | | |
| +766 | | | | | | | | | | | | |
| 125 | 0 | 254 | 170 | 67 | 130 | 51 | 104 | 41 | <0,0001 | <0,0001 | <0,0001 | Figure 13 |
| +117 | >=1 | 17 | 1 | 6 | 1 | 6 | 0 | 0 | | | | |
| +766 | | | | | | | | | | | | |
| +144 | | | | | | | | | | | | |
| 125 | 0 | 249 | 169 | 68 | 130 | 52 | 104 | 42 | <0,0001 | <0,0001 | <0,0001 | Figure 14 |
| +117 | >=1 | 22 | 2 | 9 | 1 | 5 | 0 | 0 | | | | |
| +766 | | | | | | | | | | | | |
| +144 | | | | | | | | | | | | |
| +108 | | | | | | | | | | | | |
| 125 | 0 | 244 | 168 | 69 | 130 | 53 | 104 | 43 | <0,0001 | <0,0001 | <0,0001 | Figure 15 |
| +117 | >=1 | 27 | 3 | 11 | 1 | 4 | 0 | 0 | | | | |
| +766 | | | | | | | | | | | | |
| +144 | | | | | | | | | | | | |
| +108 | | | | | | | | | | | | |
| +222 | | | | | | | | | | | | |
| 125 | 0 | 238 | 166 | 70 | 129 | 54 | 103 | 43 | <0,0001 | <0,0001 | <0,0001 | Figure 16 |
| +117 | >=1 | 33 | 5 | 15 | 2 | 6 | 2 | 6 | | | | |
| +766 | | | | | | | | | | | | |
| +144 | | | | | | | | | | | | |
| +108 | | | | | | | | | | | | |
| +222 | | | | | | | | | | | | |
| +72 | | | | | | | | | | | | |
| 125 | 0 | 236 | 165 | 70 | 129 | 55 | 103 | 44 | <0,0001 | <0,0001 | <0,0001 | Figure 17 |
| +117 | >=1 | 35 | 6 | 17 | 2 | 6 | 2 | 6 | | | | |
| +766 | | | | | | | | | | | | |
| +144 | | | | | | | | | | | | |
| +108 | | | | | | | | | | | | |
| +222 | | | | | | | | | | | | |
| +72 | | | | | | | | | | | | |
| +1165 | | | | | | | | | | | | |
| 125 | 0 | 231 | 164 | 71 | 129 | 56 | 103 | 45 | <0,0001 | <0,0001 | <0,0001 | Figure 18 |
| +117 | >=1 | 40 | 7 | 18 | 2 | 5 | 2 | 5 | | | | |
| +766 | | | | | | | | | | | | |
| +144 | | | | | | | | | | | | |
| +108 | | | | | | | | | | | | |
| +222 | | | | | | | | | | | | |
| +72 | | | | | | | | | | | | |
| +1165 | | | | | | | | | | | | |
| +487 | | | | | | | | | | | | |
| 125 | 0 | 222 | 162 | 73 | 128 | 58 | 102 | 46 | <0,0001 | <0,0001 | <0,0001 | Figure 19 |
| +117 +766 | >=1 | 49 | 9 | 18 | 3 | 6 | 3 | 6 | | | | |
| +144 | 0 | 222 | 162 | 73 | 128 | 58 | 102 | 46 | <0,0001 | <0,0001 | <0,0001 | Figure 26 |
| +108 | 1 | 38 | 8 | 21 | 3 | 8 | 3 | 8 | | | | |
| +222 | >=2 | 11 | 1 | 9 | 0 | 0 | 0 | 0 | | | | |
| +72 | | | | | | | | | | | | |
| +1165 | | | | | | | | | | | | |
| +487 | | | | | | | | | | | | |
| +1261 | | | | | | | | | | | | |
| 125 | 0 | 216 | 159 | 74 | 126 | 58 | 100 | 46 | <0,0001 | <0,0001 | <0,0001 | Figure 20 |
| +117 | >=1 | 55 | 12 | 22 | 5 | 9 | 5 | 9 | | | | |
| +766 +144 | 0 | 216 | 159 | 74 | 126 | 58 | 100 | 46 | <0,0001 | <0,0001 | <0,0001 | Figure 27 |
| +108 | 1 | 43 | 11 | 26 | 5 | 12 | 5 | 12 | | | | |
| +222 | >=2 | 12 | 1 | 8 | 0 | 0 | 0 | 0 | | | | |
| +72 | | | | | | | | | | | | |
| +1165 | | | | | | | | | | | | |
| +487 | | | | | | | | | | | | |
| +1261 | | | | | | | | | | | | |
| +205 | | | | | | | | | | | | |
| 125 | 0 | 214 | 157 | 73 | 124 | 58 | 98 | 46 | <0,0001 | <0,0001 | <0,0001 | Figure 21 |
| +117 | >=1 | 57 | 14 | 25 | 7 | 12 | 7 | 12 | | | | |
| +766 +144 | 0 | 214 | 157 | 73 | 124 | 58 | 98 | 46 | <0,0001 | <0,0001 | <0,0001 | Figure 28 |
| +108 | 1 | 42 | 13 | 31 | 7 | 17 | 7 | 17 | | | | |
| +222 | >=2 | 15 | 1 | 7 | 0 | 0 | 0 | 0 | | | | |
| +72 | | | | | | | | | | | | |
| +1165 | | | | | | | | | | | | |
| +487 | | | | | | | | | | | | |
| +1261 | | | | | | | | | | | | |
| +205 | | | | | | | | | | | | |
| +437 | | | | | | | | | | | | |
| 125 | 0 | 206 | 151 | 73 | 120 | 58 | 97 | 47 | <0,0001 | <0,0001 | <0,0001 | Figure 22 |
| +117 | >=1 | 65 | 20 | 31 | 11 | 17 | 8 | 12 | | | | |
| +766 +144 | 0 | 206 | 151 | 73 | 120 | 58 | 97 | 47 | <0,0001 | <0,0001 | <0,0001 | Figure 29 |
| +108 | 1 | 42 | 18 | 43 | 11 | 26 | 8 | 19 | | | | |
| +222 | >=2 | 23 | 2 | 9 | 0 | 0 | 0 | 0 | | | | |
| +72 | | | | | | | | | | | | |
| +1165 | | | | | | | | | | | | |
| +487 | | | | | | | | | | | | |
| +1261 | | | | | | | | | | | | |
| +205 | | | | | | | | | | | | |
| +437 | | | | | | | | | | | | |
| +1328 | | | | | | | | | | | | |
| 125 | 0 | 204 | 150 | 74 | 119 | 58 | 96 | 47 | <0,0001 | <0,0001 | <0,0001 | Figure 23 |
| +117 +766 | >=1 | 67 | 21 | 31 | 12 | 18 | 9 | 13 | | | | |
| +144 | 0 | 204 | 150 | 74 | 119 | 58 | 96 | 47 | <0,0001 | <0,0001 | <0,0001 | Figure 30 |
| +108 | 1 | 41 | 18 | 44 | 12 | 29 | 9 | 22 | | | | |
| +222 | >=2 | 26 | 3 | 12 | 0 | 0 | 0 | 0 | | | | |
| +72 | | | | | | | | | | | | |
| +1165 | | | | | | | | | | | | |
| +487 | | | | | | | | | | | | |
| +1261 | | | | | | | | | | | | |
| +205 | | | | | | | | | | | | |
| +437 | | | | | | | | | | | | |
| +1328 | | | | | | | | | | | | |
| +1188 | | | | | | | | | | | | |
| 125 | 0 | 201 | 148 | 74 | 117 | 58 | 94 | 47 | <0,0001 | <0,0001 | <0,0001 | Figure 24 |
| +117 | >=1 | 70 | 23 | 33 | 14 | 20 | 11 | 16 | | | | |
| +766 +144 | 0 | 201 | 148 | 74 | 117 | 58 | 94 | 47 | <0,0001 | <0,0001 | <0,0001 | Figure 31 |
| +108 | 1 | 38 | 17 | 45 | 11 | 29 | 9 | 24 | | | | |
| +222 | >=2 | 32 | 6 | 19 | 3 | 9 | 2 | 6 | | | | |
| +72 | 0 | 201 | 148 | 74 | 117 | 58 | 94 | 47 | <0,0001 | <0,0001 | <0,0001 | Figure 33 |
| +1165 +487 | 1 | 38 | 17 | 45 | 11 | 29 | 9 | 24 | | | | |
| +1261 | 2 | 22 | 5 | 23 | 3 | 14 | 2 | 9 | | | | |
| +205 | >=3 | 10 | 1 | 10 | 0 | 0 | 0 | 0 | | | | |
| +437 | | | | | | | | | | | | |
| +1328 | | | | | | | | | | | | |
| +1188 | | | | | | | | | | | | |
| +436 | | | | | | | | | | | | |
| 125 | 0 | 199 | 146 | 73 | 115 | 58 | 92 | 46 | <0,0001 | <0,0001 | <0,0001 | Figure 25 |
| +117 | >=1 | 72 | 25 | 35 | 16 | 22 | 13 | 18 | | | | |
| +766 +144 | 0 | 199 | 146 | 73 | 115 | 58 | 92 | 46 | <0,0001 | <0,0001 | <0,0001 | Figure 32 |
| +108 | 1 | 40 | 19 | 48 | 13 | 33 | 11 | 28 | | | | |
| +222 | >=2 | 32 | 6 | 19 | 3 | 9 | 2 | 6 | | | | |
| +72 | 0 | 199 | 146 | 73 | 115 | 58 | 92 | 46 | <0,0001 | <0,0001 | <0,0001 | Figure 34 |
| +1165 | 1 | 40 | 19 | 48 | 13 | 33 | 11 | 28 | | | | |
| +487 +1261 | 2 | 17 | 4 | 24 | 3 | 18 | 2 | 12 | | | | |
| +205 | >=3 | 15 | 2 | 13 | 0 | 0 | 0 | 0 | | | | |
| +437 | | | | | | | | | | | | |
| +1328 | | | | | | | | | | | | |
| +1188 | | | | | | | | | | | | |
| +436 | | | | | | | | | | | | |
| +135 | | | | | | | | | | | | |

### Detailed procedure and examples

### I-Methodological approach

The following procedure allowed identifying the genes according to the invention.

### Overview

The expression of the 497 testis- and placenta- specific genes was studied in a series of 271 lung cancer samples by extracting the corresponding expression data from genome-wide transcriptomic data (the latter were obtained by C. and E. Brambilla supported by the Ligue CIT program (Carte d'Identite des Tumeurs)).

For each gene, the patients were divided into two groups, those expressing the gene (calculated as described below), and those not expressing the gene (following the procedure for the determination of the ON/OFF gene expression status).

For each of the 497 genes of the list, the global and disease free survival probabilities were compared between the patients expressing the gene (ON) and those not expressing the gene (OFF). This was done considering the whole period of the study, as well as for 10 years (120 months), 5 years (60 months) and 2,5 years (30 months) of follow-up. This was performed considering all patients of the study (n=271), as well as each of the main histological subtypes of this population (ADK=adenocarcinoma; BAS= basaloid; LCNE=Large cell neuroendocrine; SQC=squamous cell tumour). The genes whose ON status allowed discriminating the patients with good or bad prognosis with a significance corresponding to a p value =<0,07 (Logrank p value, obtained when comparing cumulative global survival and/or disease free survival over 5 years between patients expressing or not expressing the gene) were selected as candidate prognosis markers.

For all these genes, the correlation between their expression (ON status) and prognosis was validated in at least one of the two following lung published cancer transcriptomic studies with survival clinical data using the same Affymetrix technology (website GEO: http://www.ncbi.nlm.nih.gov/geo, respectively GSE4576 and GSE8894).

These studies were selected as external populations of lung cancer patients in order to validate our survival data obtained by analysing the transcriptomic data of the Brambilla study.

### Detailed procedure

### 1- Establishment of a list of placenta and testis restricted genes and analysis of their aberrant expression in lung cancer patients

1a- A list of 497 human genes whose expression was restricted to placenta or male germ cells was established as mentioned in the international application WO/2009/121878.

These genes are never expressed in normal adult somatic tissues (adult somatic tissues comprise all tissues except germinal cells, foetal tissues and placenta).

1b- Expression data were extracted from a series of 112 normal adult somatic tissues randomly selected from a genome wide study of normal human tissues (GSE3526 on GEO, this study was chosen because it uses the same probes and measurement technology to detect gene expression as the Brambilla study: Affymetrix Human Genome U133 Plus 2.0 Array). The CEL files (raw data) from the control samples were downloaded from GEO. They were entered in the Genespring software and normalized (RMA algorithm) simultaneously with the CEL files from the Brambilla study.

1c- For each of the 497 genes/probes, the mean hybridization intensity signal value of the 112 control samples + 2sd was defined as the threshold for expression.

This threshold was used to distinguish between the cancer samples expressing the gene ON) and those not expressing the gene (OFF).

The measurement of expression of the genes using Affymetrix microarrays involves the hybridization of fluorescence labeled cDNAs from each tissue sample on microarrays containing gene-specific probes, the fluorescence intensity signal corresponding to each probe of the microarray is measured and changed into a raw value. The absolute value of the fluorescence intensity signal is highly variable and probe-dependent (different probes corresponding to the same gene can give different intensities of fluorescence). Therefore, on the basis of these absolute fluorescence intensity values it is generally not possible to determine whether a gene is expressed or not, and commonly people use this technique to assess variations of expression between samples (see below for more details).

In the invention, the definition of a precise threshold for expression was possible because the selected 497 genes are NOT expressed in any normal adult somatic tissue (according to the original criteria for their selection). Therefore the signal values obtained in the 112 normal adult somatic control samples give a high confidence set of values corresponding to the background noise signal, which allow further analyses.

A threshold signal value for expression could not have been defined for genes, which do not have a restricted expression pattern. Indeed in all these types experiments the background noise signal value is highly dependent on the sequence of the probe. For instance several probes representative of the same gene generally give different signal values (although these signal values should normally vary between samples in the same direction). In the case of non-restricted genes (most genes have a pattern of expression, which is not restricted to germinal cells or placenta), it is therefore impossible to use these signal values as indicators of the presence or absence of expression. However, one can compare expression levels between two groups of tissues (= expression in group of tissues A is significantly higher/lower than expression in group of tissues B). Therefore, in this particular study, since we have previously demonstrated that all the studied 497 genes are NOT expressed in normal adult somatic tissues, we were able to define a threshold differentiating expression (ON) and non-expression (OFF). This is a specific key feature of our approach.

1d- Based on this threshold, the expression of each of the 497 genes in each of the samples was defined as negative (OFF) or positive (ON) as follow. In each cancer sample, if the normalised signal value was above this threshold, the gene was considered as aberrantly expressed in this sample (gene ON), if it was under this threshold, it was considered as not expressed (gene OFF).

le- From the Brambilla study (271 cases of lung cancer), the Inventors found that 130 of the 497 genes were aberrantly expressed in at least 1% of these lung cancer cases.

### 2- Correlation between the expression of each individual gene (of the list of 130 genes) and the prognosis for survival in the lung cancer patients, and selection of 23 genes individually associated to the prognosis of all lung cancer cases (without considering histological subtypes; named after "global prognosis genes").

2a- As a first step, using each of the 130 genes individually, we compared the global survival over a period of five years between the groups of patients expressing the gene (yes) versus those not expressing the gene (no). A Logrank Mantel-Cox test was performed and a p value was calculated. This analysis was performed first with the whole population of lung cancer patients of the Brambilla study (n=271), second with each one of the following populations: ADK cases (n=91), BAS cases (n=46), LCNE cases (n=47), SQC cases (n=62).

2b- **A total of 23 genes were selected, whose individual expression was significantly associated with a poorer prognosis (as measured by the cumulative global survival and/or disease-free survival over five years; p<0,05) in the Brambilla lung cancer study, as well as in at least one of the external validation populations.**

The expression of any one of 23 genes in lung cancer is significantly associated with a poor prognosis when considering all histological subtypes.

2c- A detailed **quantitative evaluation of the prognosis** is given in **table 2a,** using each of the 23 genes associated with poor prognosis in all lung cancer types. The Kaplan Meyer survival curves obtained using each of these genes can be visualized by clicking on the link in the last column of the table.

2d- These 23 genes were then divided into two groups
- A group of 7 genes, whose aberrant expression in lung cancer is relatively frequent (>10% of cases of our series). The expression of each individual one of these seven genes is associated with a significantly reduced survival probability (global or disease free survival over five years significantly reduced, logrank test p<0,07) of all lung cancer patients (without considering histological subtypes).
- A group of 16 genes, whose aberrant expression in lung cancer is relatively rare (<10% of cases of our series). The expression of each individual one of these seven genes is associated with a significantly reduced survival probability (global or disease free survival over five years significantly reduced, logrank test p<0,07) of all lung cancer patients (without considering histological subtypes).

### 3- Association of several or all of 23 genes of the groups of 7 genes or 16 genes allows a more accurate prognosis in lung cancer patients than the use of each of them

3a- Different associations of these 23 genes were tested for the correlation between the expression of at least one gene of a given association of genes and the prognosis.

3b- The **7 genes more frequently expressed** were classified by increasing Logrank p value as follows: 1161; 391; 35; 442; 102; 390; 295. Following the same order, **subgroups of the 1rst of these genes, the 1rst + 2^{nd} of these genes, the 1rst + 2^{nd} + 3^{rd} of these genes, etc... and finally the seven genes,** were respectively tested for their prognosis prediction value. The distribution of patients according to the number of the genes of the group aberrantly expressed was studied, and relevant groups of patients were compared for their survival probability. The detailed quantitative evaluation of the prognosis using these subgroups of the seven genes is given in **table 2b.**

3c- Similarly, the **16 genes rarely expressed** were classified by increasing p values as follow:125; 117; 766; 144; 108; 222; 72; 1165; 487; 1261; 205; 437; 1328; 1188; 436; 135. Following the same order, **subgroups of the 1rst of these genes, the 1rst + 2^{nd} of these genes, the 1rst + 2^{nd} + 3^{rd} of these genes, etc... and finally the sixteen genes,** were respectively tested for their prognosis prediction value. The distribution of patients according to the number of the genes of the group aberrantly expressed was studied, and relevant groups of patients were compared for their survival probability. The detailed quantitative evaluation of the prognosis using these subgroups of the sixteen genes is given **table 2c.**

3d- Using the expression data of **all 23 genes,** the distribution of the 271 lung cancer patients according to the number of genes aberrantly expressed **from the group of 7 genes and from the group of 16 genes respectively,** was studied. Nine groups of patients were constituted according to these criteria, and the survival Kaplan Meyer curves were compared between these nine groups of patients. Finally the 271 lung cancer patients were classified into three/four prognosis subgroups: P1a, P1b, P2 and P3. The Pla, P1b, P2 and P3 definition is indicated in the Table 4 below.

### II- Example of the combination using all the genes of the group of 7 genes and of the group of 16 genes to establish the prognosis of lung cancer:

Number of lung cancer patients distributed among the different groups according to the number of genes expressed from the "group7genes" (combination of 7 genes) and the "group16genes" (combination of 16 genes) (Table 3).

The groups P1a, P1b, P2 and P3 are defined as follows:
P1A corresponds to patient samples in which no gene of the group of 7 genes or of 16 genes are expressed.
P2B corresponds to patient samples in which 1 or 2 genes of the group of 7 genes are expressed but no genes of the group of 16 genes are expressed.
P2 corresponds to patient samples in which
   - either 3 or more genes of the group of 7 genes are expressed, but no genes of the group of 16 genes are expressed,
   - or at least 1 gene of the group of 16 genes is expressed but no genes of the group of 7 genes is expressed,
   - or one gene of the group of 16 gene is expressed, and 1 or 2 genes of the group of 7 genes is expressed.
P3 corresponds to patient samples in which
   - either 2 or more genes of the group of 16 genes are expressed, and 1 or 2 genes of the group of 7 genes are expressed,
   - or at least 3 genes of the group of 7 genes is expressed and at least 1 gene of the group of 16 genes are expressed.

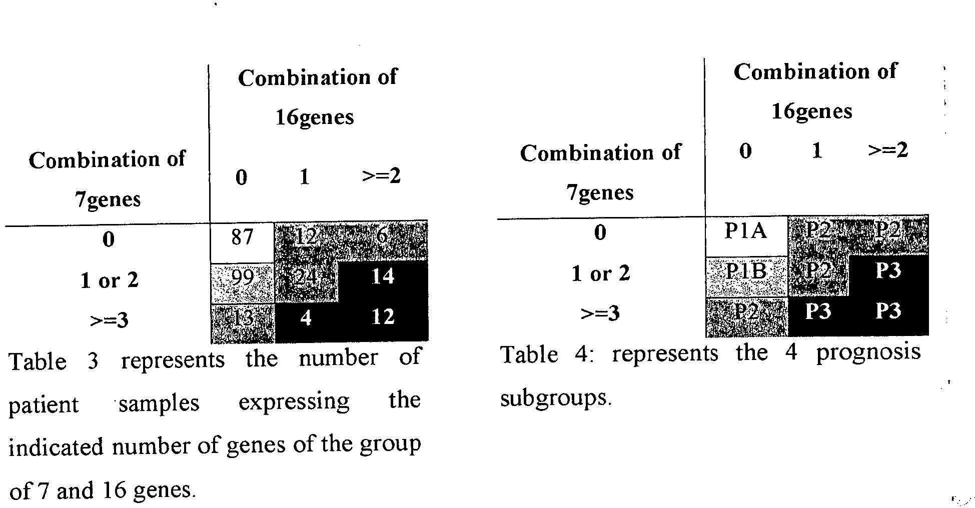

The following table 5 recapitulates the data of table 3 and table 4.

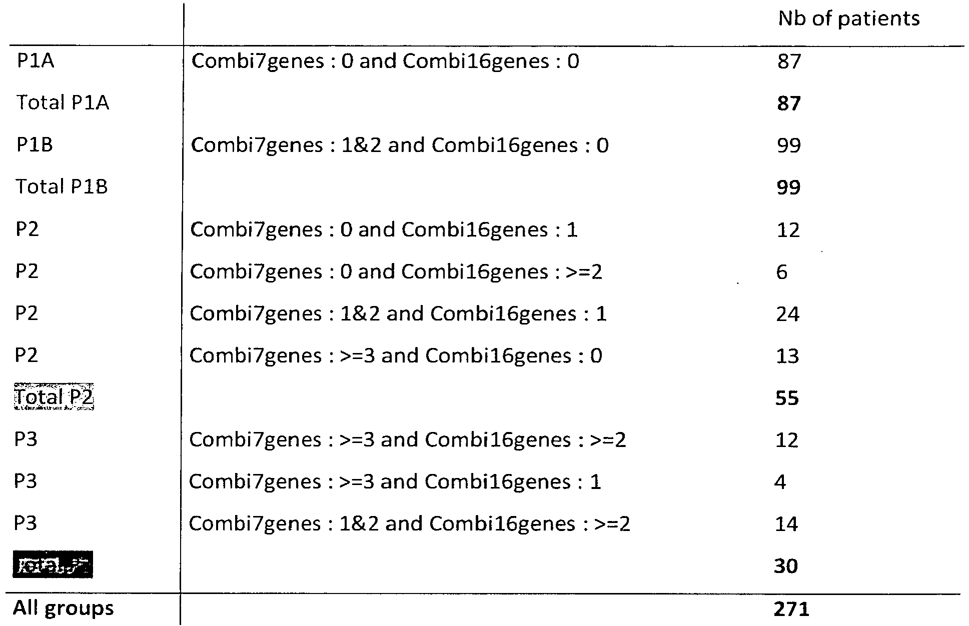

### III- Examples of CT genes whose aberrant expression in cancer is not correlated with prognosis

To enforce the specificity of the present prognosis method, the Inventors have evaluated the prognosis impact of 3 CT genes, identified as cancer marker.

The results are shown in figures 38-40.

These results demonstrate that 3 genes, well known as cancer markers, do not give any significant information about the survival rate of patients afflicted by lung tumors.

Therefore, the combination of 7 and 16 genes (i.e. 23 genes) according to the invention provides a very specific and useful method for prognosis lung tumors.

### EXAMPLE 2

### The ectopic activation of 28 tissue-restricted genes in lung tumors is a strong and independent predictor of poor prognosis

Having found that the "off-context" expression of normally silent genes systematically occurs in cancer, the Inventors next investigated whether these genes could represent useful biomarkers by considering one cancer type, lung cancer. Lung cancer is one of the most frequent cancers in humans and is the most frequent cause of mortality by cancer in men. In the context of a clinical research program, the Inventors constituted a cohort of 300 lung cancer cases (recruited in the Grenoble University Hospital, France), who received surgery, including 154 early clinical stage patients (T1N0) according to the TNM classification (tumor size, node positivity and metastasis). For each of these cases genome-wide transcriptomic analysis was performed on pretreatment diagnostic tumor samples, and pathological and clinical data recorded, including global and disease-free survival over a period of 5 to 10 years.

Applying the strategy described above, the Inventors could detect aberrant expressions of TSPS genes in all, including the 154 cases of early-stage T1N0, lung tumor samples of their series. Moreover, a series of nine paired tumor and corresponding non-tumoral lung samples confirmed that these genes are activated specifically in the tumors and not in the non-tumoral lung.

This screen identified 28 TSPS genes, whose aberrant expression was individually associated with a lower survival probability in the lung cancer patients of our series (log-rank test p-values < 0,05 and Hazard Ratios > 1,5). The Inventors then tested these 28 genes in combination as predictor of prognosis. Using the optimal and simplest combination, the Inventors assigned patients into two groups:
- none of the 28 genes expressed and
- at least one of the 28 genes expressed.

The Inventors then further refined this latter group by distinguishing tumors expressing one or two genes from tumors expressing three genes or .more. Finally tumors were stratified into three groups:
P1, expressing none of the 28 genes,
P2, expressing 1 or 2 and
P3 expressing 3 and more of the 28 genes.

Highly significant differences in overall survival probabilities between these three groups were found. Additionally, the prognostic power of this 28-gene classifier was independent of other parameters, including clinical stage (TNM classification) and histological subtype. In particular, this 28-gene group was a very efficient predictor for overall survival of early stage patients.

A multivariate analysis confirmed that the 28 genes combination of the invention was the strongest prognostic parameter associated with overall survival (p<0,0001).

A comparison of the clinical outcomes between P1 and P3 patients allowed the Inventors to confirm that the tumors classified "P3" presented a particularly aggressive phenotype.

Indeed most patients with these tumors quickly relapsed and/or developed metastases, which was generally followed by short-term fatal outcome.

The following tables summarize the results:
Tables A-C indicates the number of patients, their survival rate at 30 months according to the method disclosed in the invention.

**Table A : The table indicates the number of P3 patients and their survival rate at 30 months among the 300 pateinets having lung cancer, for the indicated group of genes.**

| | **grp13g** | | | **grp14g** | | | **grp15g** | | | **grp16g** | | |
|---|---|---|---|---|---|---|---|---|---|---|---|---|
| Pc_grp | Nb patients | % Survival | p-value | Nb patients | % Survival | p-value | Nb patients | % Survival | p-value | Nb patients | % Survival | p-value |
| P1 | 121 | 73,55 | < 0,0001 | 119 | 73,95 | < 0,0001 | 118 | 74,58 | < 0,0001 | 116 | 75,86 | < 0,0001 |
| P2 | 145 | 61,38 | | 146 | 61,64 | | 145 | 62,07 | | 145 | 61,38 | |
| **P3** | **34** | **14,71** | | **35** | **14,29** | | **37** | **13,51** | | **39** | **15,38** | |

| | **grp17g** | | | **grp18g** | | | **grp19g** | | | **grp20g** | | |
|---|---|---|---|---|---|---|---|---|---|---|---|---|
| Pc_grp | Nb patients | % Survival | p-value | Nb patients | % Survival | p-value | Nb patients | % Survival | p-value | Nb patients | % Survival | p-value |
| P1 | 116 | 75,86 | < 0,0001 | 113 | 77,88 | < 0,0001 | 113 | 77,88 | < 0,0001 | 111 | 78,38 | < 0,0001 |
| P2 | 144 | 61,81 | | 146 | 60,96 | | 145 | 61,38 | | 146 | 61,64 | |
| **P3** | **40** | **15,00** | | **41** | **14,63** | | **42** | **14,29** | | **43** | **13,95** | |

| | grp21g | | | **grp22g** | | | **grp23g** | | | **grp24g** | | |
|---|---|---|---|---|---|---|---|---|---|---|---|---|
| Pc_grp | Nb patients | % Survival | p-value | Nb patients | % Survival | p-value | Nb patients | % Survival | p-value | Nb patients | % Survival | p-value |
| P1 | 110 | 79,09 | < 0,0001 | 110 | 79,09 | < 0,0001 | 110 | 79,09 | < 0,0001 | 109 | 79,82 | < 0,0001 |
| P2 | 146 | 61,64 | | 145 | 61,38 | | 144 | 61,11 | | 144 | 61,11 | |
| **P3** | **44** | **13,64** | | **45** | **15,56** | | **46** | **17,39** | | **47** | **17,02** | |

| | **grp25g** | | | **grp26g** | | | **grp27g** | | | **grp28g** | | |
|---|---|---|---|---|---|---|---|---|---|---|---|---|
| Pc_grp | Nb patients | % Survival | p-value | Nb patients | % Survival | p-value | Nb patients | Survival | p-value | Nb patients | % Survival | p-value |
| P1 | 109 | 79,82 | < 0,0001 | 108 | 79,63 | < 0,0001 | 108 | 79,63 | < 0,0001 | 108 | 79,63 | < 0,0001 |
| P2 | 144 | 61,11 | | 144 | 61,81 | | 144 | 61,81 | | 144 | 61,81 | |
| **P3** | **47** | **17,02** | | **48** | **16,67** | | **48** | **16,67** | | **48** | **16,67** | |

**Table B: The table indicates the number of P3 patients and their survival rate at 30 months among the patients having T+N+lung cancer, for the indicated group of genes**

| | **grp15g** | | | **grp16g** | | | **grp17g** | | | **grp18g** | | |
|---|---|---|---|---|---|---|---|---|---|---|---|---|
| Pcgrp | Nb patients | % Survival | p-value | Nb patients | % Survival | p-value | Nb patients | % Survival | p-value | Nb patients | % Survival | p-value |
| **P1** | 37 | 54,05 | <0,0001 | 36 | 55,56 | <0,0001 | 35 | 57,14 | <0,0001 | 33 | 60,61 | <0,0001 |
| **P2** | 80 | 51,25 | | 81 | 50,62 | | 80 | 51,25 | | 81 | 50,62 | |
| **P3** | **29** | **10,34** | | **29** | **10,34** | | **31** | **9,68** | | **32** | **9,38** | |

| | **grp17g** | | | **grp18g** | | | **grp19g** | | | **grp20g** | | |
|---|---|---|---|---|---|---|---|---|---|---|---|---|
| Pcgrp | Nb patients | % Survival | p-value | Nb patients | % Survival | p-value | Nb patients | % Survival | p-value | Nb patients | % Survival | p-value |
| **P1** | 33 | 60,61 | <0,0001 | 30 | 66,67 | <0,0001 | 30 | 66,67 | <0,0001 | 30 | 66,67 | 0,0001 |
| **P2** | 81 | 50,62 | | 83 | 49,40 | | 82 | 50,00 | | 81 | 50,62 | |
| **P3** | 32 | **9,38** | | **33** | **9,09** | | **34** | **8,82** | | **35** | **8,57** | |

| | **grp21g** | | | **grp22g** | | | **grp23g** | | | **grp24g** | | |
|---|---|---|---|---|---|---|---|---|---|---|---|---|
| Pcgrp | Nb patients | % Survival | p-value | Nb patients | % Survival | p-value | Nb patients | % Survival | p-value | Nb patients | % Survival | p-value |
| **P1** | 29 | 68,97 | <0,0001 | 29 | 68,97 | <0,0001 | 29 | 68,97 | <0,0001 | 28 | 71,43 | <0,0001 |
| **P2** | 81 | 50,62 | | 81 | 50,62 | | 81 | 50,62 | | 81 | 50,62 | |
| **P3** | **36** | 8,33 | | **36** | **8,33** | | **36** | **8,33** | | 37 | **8,11** | |

| | **grp25g** | | | **grp26g** | | | **grp27g** | | | **grp28g** | | |
|---|---|---|---|---|---|---|---|---|---|---|---|---|
| Pcgrp | Nb patients | % Survival | p-value | Nb patients | % Survival | p-value | Nb patients | % Survival | p-value | Nb patients | % Survival | p-value |
| **P1** | 28 | 71,43 | <0,0001 | 28 | 71,43 | <0,0001 | 28 | 71,43 | 0,0001 | 28 | 71,43 | <0,0001 |
| **P2** | 81 | 50,62 | | 80 | 51,25 | | 80 | 51,25 | | 80 | 51,25 | |
| **P3** | **37** | **8,11** | | **38** | **7,89** | | **38** | **7,89** | | **38** | **7,89** | |

**Table C: The table indicates the number of P3 patients and their survival rate at 30 months among the patients having BAS lung cancer, for the indicated group of genes.**

| | | | | | | | **grp15g** | | | **grp16g** | | |
|---|---|---|---|---|---|---|---|---|---|---|---|---|
| | | | | | | | Nb patients | % Survival | p-value | Nb patients | % Survival | p-value |
| | | | | | | | 7 | 57,14286 | 0,145 | 7 | 57,14 | 0,145 |
| | | | | | | | 31 | 51,6129 | | 31 | 51,61 | |
| | | | | | | | 5 | 20 | | 5 | **20,00** | |

| | **grp17g** | | | **grp18g** | | | **grp19g** | | | **grp20g** | | |
|---|---|---|---|---|---|---|---|---|---|---|---|---|
| Pcgrp | Nb patients | % Survival | p-value | Nb patients | % Survival | p-value | Nb patients | % Survival | p-value | Nb patients | % Survival | p-value |
| P1 | 7 | 57,14 | 0,089 | 7 | 57,14 | 0,089 | 7 | 57,14 | 0,089 | 7 | 57,14 | 0,089 |
| P2 | 30 | 53,33 | | 30 | 53,33 | | 30 | 53,33 | | 30 | 53,33 | |
| **P3** | **6** | **16,67** | | **6** | **16,67** | | 6 | **16,67** | | **6** | **16,67** | |

| | **grp21g** | | | **grp22g** | | | **grp23g** | | | **grp24g** | | |
|---|---|---|---|---|---|---|---|---|---|---|---|---|
| Pcgrp | Nb patients | % Survival | p-value | Nb patients | % Survival | p-value | Nb patients | % Survival | p-value | Nb patients | % Survival | p-value |
| P1 | 7 | 57,14 | 0,089 | 7 | 57,14 | 0,089 | 7 | 57,14 | 0,089 | 7 | 57,14 | 0,089 |
| P2 | 30 | 53,33 | | 30 | 53,33 | | 30 | 53,33 | | 30 | 53,33 | |
| **P3** | **6** | **16,67** | | **6** | **16,67** | | **6** | **16,67** | | **6** | **16,67** | |

| | **grp25g** | | | **grp26g** | | | **grp27g** | | | **grp28g** | | |
|---|---|---|---|---|---|---|---|---|---|---|---|---|
| Pcgrp | Nb patients | % Survival | p-value | Nb patients | % Survival | p-value | Nb patients | % Survival | p-value | Nb patients | % Survival | p-value |
| P1 | 7 | 57,14 | 0,089 | 7 | 57,14 | 0,089 | 7 | 57,14 | 0,089 | 7 | 57,14286 | 0,089 |
| P2 | 30 | 53,33 | | 30 | 53,33 | | 30 | 53,33 | | 30 | 53,33333 | |
| **P3** | **6** | **16,67** | | **6** | **16,67** | | **6** | **16,67** | | **6** | **16,66667** | |

Figures 50-65 represent respectively the percentage of survival of patients over the time (in months) when using from at least 13 to 28 genes according to the invention. P3 population (and curves) are indicated.

The population is the population of 300 patients afflicted by lung cancer.

Figures 45 and 47 represent respectively the percentage of survival of patients over the time (in months) when using the 28 genes according to the invention. P3 population (and curves) are indicated, in e T+N+ polulation and BAS population.

## Claims

1. Use of
- at least 13 genes chosen among a set of 28 genes comprising or consisting of the nucleic acid sequences SEQ ID NO 70 to 97,
- or complementary sequences of said least 13 genes chosen among a set of 28 genes comprising or consisting of the nucleic acid sequences SEQ ID NO 70 to 97,
- or sequences having at least 80% homology with said genes,
- or proteins coded by said at least 13 genes chosen among a set of 28 genes comprising or consisting of said nucleic acid sequences,
said at least 13 genes being such that
- 12 genes comprise or consist of the nucleic acid sequences SEQ ID NO: 70 to 81, and
- at least one gene belongs to a subset of 16 genes comprising or consisting of the nucleic acid sequences SEQ ID NO: 82-97,
for carrying out a method for identifying at least 66% of patients having a survival rate of at most about 20% 30 months after the diagnosis of their lung cancer, among a population of patients afflicted by lung cancer having an estimated survival rate of at least 30% 30 months after the diagnosis of their lung cancer based on the diagnosis of said lung cancer according to histopathological criteria,
said method comprising a step of measuring, in a biological sample of said patients, the expression of said at least 13 genes, or said complementary sequences of said at least 13 genes, or said sequences having at least 80% homology with said genes or said proteins coded by said at least 13 genes.

2. Use according to claim 1, of
- at least 13 genes chosen among a set of 28 genes comprising or consisting of the nucleic acid sequences SEQ ID NO 70 to 97,
- or complementary sequences of said least 13 genes chosen among a set of 28 genes comprising or consisting of the nucleic acid sequences SEQ ID NO 70 to 97,
- or sequences having at least 80% homology with said genes,
said at least 13 genes being such that
- 12 genes comprise or consist of the nucleic acid sequences SEQ ID NO: 70 to 81, and
- at least one gene belongs to a subset of 16 genes comprising or consisting of the nucleic acid sequences SEQ ID NO: 82-97,
for carrying out a method for identifying at least 66% of patients having a survival rate of at most about 20% 30 months after the diagnosis of their lung cancer, among a population of patients afflicted by lung cancer having an estimated survival rate of at least 30% 30 months after the diagnosis of their lung cancer based on the diagnosis of said lung cancer according to histopathological criteria.

3. Use according to claim 2, wherein said at least one gene belongs to a subset of 16 genes comprising or consisting of the nucleic acid sequences SEQ ID NO: 82-97, is at least the gene comprising or consisting of the nucleic acid sequences SEQ ID NO: 82, for carrying out a method for identifying at least 70% of patients having a survival rate of at most about 20% 30 months after the diagnosis of their lung cancer.

4. Use according to claim 2 or 3, of at least 18 genes,
said at least 18 genes being such that
a. 12 genes comprise or consist of the nucleic acid sequences SEQ ID NO: 70 to 81, and
b. at least 6 genes belongs to a subset of 16 genes comprising or consisting of the nucleic acid sequences SEQ ID NO: 82-97, said at least 6 genes comprising or consisting of the nucleic acid sequences SEQ ID NO:82-85 and 87-88,
for carrying out a method for identifying at least 83% of patients having a survival rate of at most about 20% 30 months after the diagnosis of their lung cancer.

5. Use according to claim 2 or 3, of at least 21 genes,
said at least 21 genes being such that
- 12 genes comprise or consist of the nucleic acid sequences SEQ ID NO: 70 to 81, and
- at least 9 gene belongs to a subset of 16 genes comprising or consisting of the nucleic acid sequences SEQ ID NO: 82-97, said at least 9 genes preferably comprising or consisting of the nucleic acid sequences SEQ ID NO: 82-88 and 91-92,
for carrying out a method for identifying at least 89% of patients having a survival rate of at most about 20% 30 months after the diagnosis of their lung cancer.

6. Use according to anyone of claims 2 to 4, of at least 26 genes said at least 26 genes being such that
- 12 genes comprise or consist of the nucleic acid sequences SEQ ID NO: 70 to 81, and
- at least 14 gene belongs to a subset of 16 genes comprising or consisting of the nucleic acid sequences SEQ ID NO: 82-97, said at least 14 genes preferably comprising or consisting of the nucleic acid sequences SEQ ID NO: 82-92 and 93-96,
for carrying out a method for identifying at least 97% of patients having a survival rate of at most about 20% 30 months after the diagnosis of their lung cancer.

7. Use according to anyone of claims 2 to 5. of 28 genes comprising or consisting of the nucleic acid sequences SEQ ID NO: 70-97 for carrying out a method for identifying 100% of patients having a survival rate of at most about 20% 30 months after the diagnosis of their lung cancer.

8. *In vitro* method for identifying patients afflicted by a lung cancer having a survival rate of at most about 20% 30 months after the diagnosis of their lung cancer, among a population of patients afflicted by lung cancer having an estimated survival rate of at least 30% 30 months after the diagnosis of their lung cancer based on the diagnosis of said lung cancer according to histopathological criteria,
said method allowing the identification of at least 66% of patients afflicted by a lung cancer having a survival rate of at most about 20% 30 months after the diagnosis of their lung cancer,
said method comprising
* a step of measuring, in a biological sample of said patients, the expression of
- at least 13 genes chosen among a set of 28 genes comprising or consisting of the nucleic acid sequences SEQ ID NO 70 to 97,
- or complementary sequences of said genes
said at least 13 genes being such that
- 12 genes comprise or consist of the nucleic acid sequences SEQ ID NO: 70 to 81, and
- at least one gene belongs to a subset of 16 genes comprising or consisting of the nucleic acid sequences SEQ ID NO: 82-97, and
* a step of identifying biological samples expressing said at least 13 genes.

9. Method, according to claim 8, wherein said at least 13 genes being such that
- 12 genes comprise or consist of the nucleic acid sequences SEQ ID NO: 70 to 81, and
- at least one gene belongs to a subset of 16 genes comprising or consisting of the nucleic acid sequences SEQ ID NO: 82-97, said at least one gene comprising or consisting of the nucleic acid sequences SEQ ID NO: 82,
said method allowing the identification of at least 70% of patients afflicted by a lung cancer having a survival rate of at most about 20% 30 months after the diagnosis of their lung cancer.

10. Method, according to claim 8 or 9, said method comprising a step of measuring, in a biological sample of said patients, the expression of at least 18 genes chosen among a set of 28 genes comprising or consisting of the nucleic acid sequences SEQ ID NO 70 to 97,
said at least 18 genes being such that
- 12 genes comprise or consist of the nucleic acid sequences SEQ ID NO: 70 to 81, and
- at least 6 genes belongs to a subset of 16 genes comprising or consisting of the nucleic acid sequences SEQ ID NO: 82-97, said at least 6 genes comprising or consisting of the nucleic acid sequences SEQ ID NO:82-85 and 87-88,
said method allowing the identification of at least 83% of patients having a survival rate of at most about 20% 30 months after the diagnosis of their lung cancer.

11. Method, according to anyone of claims 8 to 10, said method comprising a step of measuring, in a biological sample of said patients, the expression of at least 21 genes chosen among a set of 28 genes comprising or consisting of the nucleic acid sequences SEQ ID NO 70 to 97,
said at least 21 genes being such that
- 12 genes comprise or consist of the nucleic acid sequences SEQ ID NO: 70 to 81, and
- at least 9 gene belongs to a subset of 16 genes comprising or consisting of the nucleic acid sequences SEQ ID NO: 82-97, said at least 9 genes preferably comprising or consisting of the nucleic acid sequences SEQ ID NO: 82-88 and 91-92,
said method allowing identifying at least 91% of patients having a survival rate of at most about 20% 30 months after the diagnosis of their lung cancer.

12. Method, according to anyone of claims 8 to 11, said method comprising a step of measuring, in a biological sample of said patients, the expression of at least 26 genes chosen among a set of 28 genes comprising or consisting of the nucleic acid sequences SEQ ID NO 70 to 97,
said at least 26 genes being such that
- 12 genes comprise or consist of the nucleic acid sequences SEQ ID NO: 70 to 81, and
- at least 14 gene belongs to a subset of 16 genes comprising or consisting of the nucleic acid sequences SEQ ID NO: 82-97, said at least 14 genes preferably comprising or consisting of the nucleic acid sequences SEQ ID NO: 82-92 and 93-96,
said method allowing identifying 100% of patients having a survival rate of at most about 20% 30 months after the diagnosis of their lung cancer.

13. Method, according to anyone of claims 8 to 12, said method comprising a step of measuring, in a biological sample of said patients, the expression of 28 genes chosen comprising or consisting of the nucleic acid sequences SEQ ID NO 70 to 97,
said method allowing identifying 100% of patients having a survival rate of at most about 20% 30 months after the diagnosis of their lung cancer,
said method comprising
* a step of measuring, in a biological sample of said patients, the expression of 28 genes comprising or consisting of the nucleic acid sequences SEQ ID NO 70 to 97, and
* a step of identifying biological samples expressing said 28 genes.

## Patentansprüche

1. Verwendung von
- mindestens 13 Genen, ausgewählt aus einer Gruppe von 28 Genen, umfassend oder bestehend aus den Nukleinsäuresequenzen SEQ ID NO: 70 bis 97,
- oder komplementären Sequenzen dieser mindestens 13 Gene, ausgewählt aus einer Gruppe von 28 Genen, umfassend oder bestehend aus den Nukleinsäuresequenzen SEQ ID NO: 70 bis 97,
- oder Sequenzen mit mindestens 80 % Homologie mit diesen Genen,
- oder Proteinen, die von den mindestens 13 Genen kodiert werden, ausgewählt aus einer Gruppe von 28 Genen, umfassend oder bestehend aus diesen Nukleinsäuresequenzen,
wobei die mindestens 13 Gene derart sind, dass
- 12 Gene die Nukleinsäuresequenzen SEQ ID NO: 70 bis 81 umfassen oder daraus bestehen und
- mindestens ein Gen zu einer Untergruppe aus 16 Genen gehört, umfassend oder bestehend aus den Nukleinsäuresequenzen SEQ ID NO: 82-97,
zum Durchführen eines Verfahrens zum Identifizieren von mindestens 66 % von Patienten mit einer Überlebensrate von höchstens etwa 20 % 30 Monate nach der Diagnose ihres Lungenkrebses aus einer Population von Patienten, die an Lungenkrebs leiden, mit einer geschätzten Überlebensrate von mindestens 30 % 30 Monate nach der Diagnose ihres Lungenkrebses, auf der Basis der Diagnose ihres Lungenkrebses gemäß histopathologischen Kriterien,
wobei das Verfahren einen Schritt zum Messen, in einer biologischen Probe dieser Patienten, der Expression der mindestens 13 Gene oder der komplementären Sequenzen der mindestens 13 Gene oder der Sequenzen mit mindestens 80 % Homologie mit den Genen oder den Proteinen, die von den mindestens 13 Genen kodiert werden, umfasst.

2. Verwendung nach Anspruch 1 von
- mindestens 13 Genen, ausgewählt aus einer Gruppe von 28 Genen, umfassend oder bestehend aus den Nukleinsäuresequenzen SEQ ID NO: 70 bis 97,
- oder komplementären Sequenzen dieser mindestens 13 Gene, ausgewählt aus einer Gruppe von 28 Genen, umfassend oder bestehend aus den Nukleinsäuresequenzen SEQ ID NO: 70 bis 97,
- oder Sequenzen mit mindestens 80% Homologie mit den Genen,
wobei die mindestens 13 Gene derart sind, dass
- 12 Gene die Nukleinsäuresequenzen SEQ ID NO: 70 bis 81 umfassen oder daraus bestehen und
- mindestens ein Gen zu einer Untergruppe aus 16 Genen gehört, umfassend oder bestehend aus den Nukleinsäuresequenzen SEQ ID NO: 82-97,
zum Durchführen eines Verfahrens zum Identifizieren von mindestens 66 % von Patienten mit einer Überlebensrate von höchstens etwa 20 % 30 Monate nach der Diagnose ihres Lungenkrebses aus einer Population von Patienten, die an Lungenkrebs leiden, mit einer geschätzten Überlebensrate von mindestens 30 % 30 Monate nach der Diagnose ihres Lungenkrebses, auf der Basis der Diagnose ihres Lungenkrebses gemäß histopathologischen Kriterien.

3. Verwendung nach Anspruch 2, wobei das mindestens eine Gen zu einer Untergruppe aus 16 Genen gehört, umfassend oder bestehend aus den Nukleinsäuresequenzen SEQ ID NO: 82-97, mindestens das Gen ist, das die Nukleinsäuresequenzen SEQ ID NO: 82 umfasst oder daraus besteht,
zum Durchführen eines Verfahrens zum Identifizieren von mindestens 70 % von Patienten mit einer Überlebensrate von mindestens etwa 20 % 30 Monate nach der Diagnose ihres Lungenkrebses.

4. Verwendung nach Anspruch 2 oder 3 von mindestens 18 Genen,
wobei die mindestens 18 Gene derart sind, dass
a. 12 Gene die Nukleinsäuresequenzen SEQ ID NO: 70 bis 81 umfassen oder daraus bestehen und
b. mindestens 6 Gene zu einer Untergruppe aus 16 Genen gehören, umfassend oder bestehend aus den Nukleinsäuresequenzen SEQ ID NO: 82-97, wobei die mindestens 6 Gene die Nukleinsäuresequenzen SEQ ID NO: 82-85 und 87-88 umfassen oder daraus bestehen,
zum Durchführen eines Verfahrens zum Identifizieren von mindestens 83 % von Patienten mit einer Überlebensrate von mindestens etwa 20 % 30 Monate nach der Diagnose ihres Lungenkrebses.

5. Verwendung nach Anspruch 2 oder 3 von mindestens 21 Genen,
wobei die mindestens 21 Gene derart sind, dass
- 12 Gene die Nukleinsäuresequenzen SEQ ID NO: 70 bis 81 umfassen oder daraus bestehen und
- mindestens 9 Gene zu einer Untergruppe aus 16 Genen gehören, umfassend oder bestehend aus den Nukleinsäuresequenzen SEQ ID NO: 82-97, wobei die mindestens 9 Gene bevorzugt die Nukleinsäuresequenzen SEQ ID NO: 82-88 und 91-92 umfassen oder daraus bestehen,
zum Durchführen eines Verfahrens zum Identifizieren von mindestens 89 % von Patienten mit einer Überlebensrate von mindestens etwa 20 % 30 Monate nach der Diagnose ihres Lungenkrebses.

6. Verwendung nach einem der Ansprüche 2 bis 4 von mindestens 26 Genen
wobei die mindestens 26 Gene derart sind, dass
- 12 Gene die Nukleinsäuresequenzen SEQ ID NO: 70 bis 81 umfassen oder daraus bestehen und
- mindestens 14 Gene zu einer Untergruppe aus 16 Genen gehören, umfassend oder bestehend aus den Nukleinsäuresequenzen SEQ ID NO: 82-97, wobei die mindestens 14 Gene bevorzugt die Nukleinsäuresequenzen SEQ ID NO: 82-92 und 93-96 umfassen oder daraus bestehen,
zum Durchführen eines Verfahrens zum Identifizieren von mindestens 97 % von Patienten mit einer Überlebensrate von mindestens etwa 20 % 30 Monate nach der Diagnose ihres Lungenkrebses.

7. Verwendung nach einem der Ansprüche 2 bis 5 von 28 Genen, umfassend oder bestehend aus den Nukleinsäuresequenzen SEQ ID NO: 70-97 zum Durchführen eines Verfahrens zum Identifizieren von 100 % von Patienten mit einer Überlebensrate von höchstens etwa 20 % 30 Monate nach der Diagnose ihres Lungenkrebses.

8. In-vitro-Verfahren zum Identifizieren von Patienten, die an Lungenkrebs leiden, mit der Überlebensrate von höchstens etwa 20 % 30 Monate nach der Diagnose ihres Lungenkrebses, aus einer Population von Patienten, die an Lungenkrebs leiden mit einer geschätzten Überlebensrate von mindestens 30 % 30 Monate nach der Diagnose ihres Lungenkrebses, auf der Basis der Diagnose des Lungenkrebs gemäß histopathologischen Kriterien,
wobei das Verfahren das Identifizieren von mindestens 66 % von Patienten ermöglicht, die an einem Lungenkrebs leiden, mit einer Überlebensrate von höchstens 20 % 30 Monate nach der Diagnose ihres Lungenkrebses,
wobei das Verfahren umfasst
* einen Schritt zum Messen, in einer biologischen Probe dieser Patienten, der Expression von
- mindestens 13 Genen, ausgewählt aus einer Gruppe von 28 Genen, umfassend oder bestehend aus den Nukleinsäuresequenzen SEQ ID NO: 70 bis 97,
- oder komplementären Sequenzen dieser Gene,
wobei die mindestens 13 Gene derart sind, dass
- 12 Gene die Nukleinsäuresequenzen SEQ ID NO: 70 bis 81 umfassen oder daraus bestehen und
- mindestens ein Gen zu einer Untergruppe aus 16 Genen gehört, umfassend oder bestehend aus den Nukleinsäuresequenzen SEQ ID NO: 82-97, und
* einen Schritt zum Identifizieren von biologischen Proben, die die mindestens 13 Gene exprimieren.

9. Verfahren nach Anspruch 8, wobei die mindestens 13 Gene derart sind, dass
- 12 Gene die Nukleinsäuresequenzen SEQ ID NO: 70 bis 81 umfassen oder daraus bestehen und
- mindestens ein Gen zu einer Untergruppe aus 16 Genen gehört, umfassend oder bestehend aus den Nukleinsäuresequenzen SEQ ID NO: 82-97, wobei das mindestens eine Gen die Nukleinsäuresequenzen SEQ ID NO: 82 umfasst oder daraus besteht,
wobei das Verfahren das Identifizieren von mindestens 70 % von Patienten ermöglicht, die an einem Lungenkrebs leiden, mit einer Überlebensrate von höchstens 20 % 30 Monate nach der Diagnose ihres Lungenkrebses.

10. Verfahren nach Anspruch 8 oder 9, wobei das Verfahren einen Schritt zum Messen, in einer biologischen Probe dieser Patienten, der Expression von mindestens 18 Genen umfasst, ausgewählt aus einer Gruppe von 28 Genen, die die Nukleinsäuresequenzen SEQ ID NO: 70 bis 97 umfassen oder daraus bestehen,
wobei die mindestens 18 Gene derart sind, dass
- 12 Gene die Nukleinsäuresequenzen SEQ ID NO: 70 bis 81 umfassen oder daraus bestehen und
- mindestens 6 Gene zu einer Untergruppe aus 16 Genen gehören, umfassend oder bestehend aus den Nukleinsäuresequenzen SEQ ID NO: 82-97, wobei die mindestens 6 Gene die Nukleinsäuresequenzen SEQ ID NO: 82-85 und 87-88 umfassen oder daraus bestehen,
wobei das Verfahren das Identifizieren von mindestens 83 % von Patienten mit einer Überlebensrate von höchstens 20 % 30 Monate nach der Diagnose ihres Lungenkrebses ermöglicht.

11. Verfahren nach einem der Ansprüche 8 bis 10, wobei das Verfahren einen Schritt zum Messen, in einer biologischen Probe dieser Patienten, der Expression von mindestens 21 Genen umfasst, ausgewählt aus einer Gruppe von 28 Genen, die die Nukleinsäuresequenzen SEQ ID NO: 70 bis 97 umfassen oder daraus,bestehen,
wobei die mindestens 21 Gene derart sind, dass
- 12 Gene die Nukleinsäuresequenzen SEQ ID NO: 70 bis 81 umfassen oder daraus bestehen und
- mindestens 9 Gene zu einer Untergruppe aus 16 Genen gehören, umfassend oder bestehend aus den Nukleinsäuresequenzen SEQ ID NO: 82-97, wobei die mindestens 9 Gene bevorzugt die Nukleinsäuresequenzen SEQ ID NO: 82-88 und 91-92 umfassen oder daraus bestehen,
wobei das Verfahren das Identifizieren von mindestens 91 % von Patienten mit einer Überlebensrate von höchstens 20 % 30 Monate nach der Diagnose ihres Lungenkrebses ermöglicht.

12. Verfahren nach einem der Ansprüche 8 bis 11, wobei das Verfahren einen Schritt zum Messen, in einer biologischen Probe dieser Patienten, der Expression von mindestens 26 Genen umfasst, ausgewählt aus einer Gruppe von 28 Genen, die die Nukleinsäuresequenzen SEQ ID NO: 70 bis 97 umfassen oder daraus bestehen,
wobei die mindestens 26 Gene derart sind, dass
- 12 Gene die Nukleinsäuresequenzen SEQ ID NO: 70 bis 81 umfassen oder daraus bestehen und
- mindestens 14 Gene zu einer Untergruppe aus 16 Genen gehören, umfassend oder bestehend aus den Nukleinsäuresequenzen SEQ ID NO: 82-97, wobei die mindestens 14 Gene bevorzugt die Nukleinsäuresequenzen SEQ ID NO: 82-92 und 93-96 umfassen oder daraus bestehen,
wobei das Verfahren das Identifizieren von 100 % von Patienten mit einer Überlebensrate von höchstens 20 % 30 Monate nach der Diagnose ihres Lungenkrebses ermöglicht.

13. Verfahren nach einem der Ansprüche 8 bis 12, wobei das Verfahren einen Schritt zum Messen, in einer biologischen Probe dieser Patienten, der Expression von 28 Genen umfasst, ausgewählt aus, umfassend oder bestehend aus den Nukleinsäuresequenzen SEQ ID NO: 70 bis 97,
wobei das Verfahren das Identifizieren von 100 % von Patienten mit einer Überlebensrate von höchstens 20 % 30 Monate nach der Diagnose ihres Lungenkrebses ermöglicht,
wobei das Verfahren umfasst
* einen Schritt zum Messen, in einer biologischen Probe dieser Patienten, der Expression von 28 Genen, umfassend oder bestehend aus den Nukleinsäuresequenzen SEQ ID NO 70 bis 97, und
* einen Schritt zum Identifizieren von biologischen Proben, die die 28 Gene exprimieren.

## Revendications

1. Utilisation de
- au moins 13 gènes choisi parmi un ensemble de 28 gènes comprenant ou consistant en les séquences d'acide nucléique SEQ ID NO 70 to 97,
- ou de séquences complémentaires desdits au moins 13 gènes choisis parmi un ensemble de 28 gènes comprenant ou consistant en les séquences d'acide nucléique SEQ ID NO 70 to 97,
- ou de séquences ayant au moins 80% d'homologie avec lesdits gènes,
- ou de protéines codées par lesdits au moins 13 gènes choisis parmi un ensemble de 28 gènes comprenant ou consistant en lesdites séquences d'acide nucléique,
lesdits au moins 13 gènes étant tels que
- 12 gènes comprennent ou consistent en les séquences d'acide nucléique SEQ ID NO 70 to 81, et
- au moins un gène appartient à un sous-ensemble de 16 gènes comprenant ou consistant en les séquences d'acides nucléiques SEQ ID NO : 82-97,
pour la mise en oeuvre d'une méthode pour identifier au moins 66% des patients ayant un taux de survie d'au plus environ 20% 30 mois après le diagnostic de leur cancer du poumon, parmi une population de patients atteints d'un cancer du poumon ayant un taux de survie estimé d'au moins 30% 30 mois après le diagnostic de leur cancer du poumon basé sur le diagnostic dudit cancer du poumon selon des critères histopathologiques,
ladite méthode comprenant une étape de mesure, dans un échantillon biologique desdits patients, de l'expression desdits au moins 13 gènes, ou desdites séquences complémentaires desdits au moins 13 gènes, ou desdites séquences ayant au moins 80% d'homologies avec lesdits gènes ou desdites protéines codées par lesdits au moins 13 gènes.

2. Utilisation selon la revendication 1, de
- au moins 13 gènes choisis parmi un ensemble de 28 gènes comprenant ou consistant en les séquences d'acide nucléique SEQ ID NO 70 à 97,
- ou de séquences complémentaires desdits au moins 13 gènes choisis parmi un ensemble de 28 gènes comprenant ou consistant en les séquences d'acide nucléique SEQ ID NO 70 à 97,
- ou de séquences ayant au moins 80% d'homologie avec lesdits gènes,
lesdits au moins 13 gènes étant tels que
- 12 gènes comprennent ou consistent en les séquences d'acide nucléique SEQ ID NO : 70 à 81, et
- au moins un gène appartient à un sous-ensemble de 16 gènes comprenant ou consistant en les séquences d'acide nucléique SEQ ID NO : 82-97,
pour la mise en oeuvre d'une méthode pour identifier au moins 66% des patients ayant un taux de survie d'au plus environ 20% 30 mois après le diagnostic de leur cancer du poumon, parmi une population de patients atteints d'un cancer du poumon ayant un taux de survie estimé d'au moins 30% 30 mois après le diagnostic de leur cancer du poumon basé sur le diagnostic dudit cancer du poumon selon des critères histopathologiques.

3. Utilisation selon la revendication 2, dans laquelle ledit au moins un gène appartient à un sous-ensemble de 16 gènes comprenant ou consistant en les séquences d'acide nucléique SEQ ID NO : 82-97, est au moins le gène comprenant ou consistant en les séquences d'acide nucléique SEQ ID NO : 82,
pour la mise en oeuvre d'une méthode pour identifier au moins 70% des patients ayant un taux de survie d'au plus environ 20% 30 mois après le diagnostic de leur cancer du poumon.

4. Utilisation selon la revendication 2 ou 3, d'au moins 18 gènes,
lesdits au moins 18 gènes étant tels que
a. 12 gènes comprennent ou consistent en les séquences d'acide nucléique SEQ ID NO : 70 à 81, et
b. au moins 6 gènes appartiennent à un sous-ensemble de 16 gènes comprenant ou consistant en les séquences d'acide nucléique SEQ ID NO : 82-87, lesdits au moins 6 gènes comprenant ou consistant en les séquences d'acide nucléique SEQ ID NO : 82-85 et 87-88,
pour la mise en oeuvre d'une méthode pour identifier au moins 83% des patients ayant un taux de survie d'au plus environ 20% 30 mois après le diagnostic de leur cancer du poumon.

5. Utilisation selon la revendication 2 ou 3, d'au moins 21 gènes,
lesdits au moins 21 gènes étant tels que
- 12 gènes comprennent ou consistent en les séquences d'acide nucléique SEQ ID NO : 70 à 81, et
- au moins 9 gènes appartiennent à un sous-ensemble de 16 gènes comprenant ou consistant en les séquences d'acide nucléique SEQ ID NO : 82-97, lesdits au moins 9 gènes comprenant ou consistant de préférence en les séquences d'acide nucléique SEQ ID NO : 82-88 et 91-92,
pour la mise en oeuvre d'une méthode pour identifier au moins 89% des patients ayant un taux de survie d'au plus environ 20% 30 mois après le diagnostic de leur cancer du poumon.

6. Utilisation selon l'une des revendications 2 à 4, d'au moins 26 gènes lesdits au moins 26 gènes étant tels que
- 12 gènes comprennent ou consistent en les séquences d'acide nucléique SEQ ID NO : 70 à 81, et
- au moins 14 gènes appartiennent à un sous-ensemble de 16 gènes comprenant ou consistant en les séquences d'acide nucléique SEQ ID NO : 82-97, lesdits au moins 14 gènes comprenant ou consistant de préférence en les séquences d'acide nucléique SEQ ID NO : 82-92 et 93-96,
pour la mise en oeuvre d'une méthode pour identifier au moins 97% des patients ayant un taux de survie d'au plus environ 20% 30 mois après le diagnostic de leur cancer du poumon.

7. Utilisation selon l'une des revendications 2 à 5, de 28 gènes comprenant ou consistant en les séquences d'acide nucléique SEQ ID NO : 70-97 pour la mise en oeuvre d'une méthode pour identifier 100% des patient ayant un taux de survie d'au plus environ 20% 30 mois après le diagnostic de leur cancer du poumon.

8. Méthode *in vitro* pour identifier des patients atteints d'un cancer du poumon ayant un taux de survie d'au plus environ 20% 30 mois après le diagnostic de leur cancer du poumon, parmi une population de patients atteints d'un cancer du poumon ayant un taux de survie estimé d'au moins 30% 30 mois après le diagnostic de leur cancer du poumon basé sur le diagnostic dudit cancer du poumon selon des critères histologiques,
ladite méthode permettant d'identifier au moins 66% des patients atteints d'un cancer du poumon ayant un taux de survie d'au plus environ 20% 30 mois après le diagnostic de leur cancer du poumon,
ladite méthode comprenant
* une étape de mesure, dans un échantillon biologique desdits patients, de l'expression de
- au moins 13 gènes choisis parmi un ensemble de 28 gènes comprenant ou consistant en les séquences d'acide nucléique SEQ ID NO 70 à 97,
- ou de séquences complémentaires desdits gènes
lesdits au moins 13 gènes étant tels que
- 12 gènes comprennent ou consistent en les séquences d'acide nucléiques SEQ ID NO: 70 à 81, et
- au moins un gène appartient à un sous-ensemble de 16 gènes comprenant ou consistant en les séquences d'acide nucléique SEQ ID NO : 82-97, et
* une étape d'identification des échantillons biologiques exprimant lesdits au moins 13 gènes.

9. Méthode, selon la revendication 8, dans laquelle lesdits au moins 13 gènes étant tels que
- 12 gènes comprennent ou consistent en les séquences d'acide nucléique SEQ ID NO: 70 à 81,
- au moins un gène appartient à un sous-ensemble de 16 gènes comprenant ou consistant en les séquences d'acide nucléique SEQ ID NO : 82-97, ledit au moins un gène comprenant ou consistant en les séquences d'acide nucléique SEQ ID NO : 82,
ladite méthode permettant d'identifier au moins 70% des patients atteints par un cancer du poumon ayant un taux de survie d'au plus environ 20% 30 mois après le diagnostic de leur cancer du poumon.

10. Méthode, selon la revendication 8 ou 9, ladite méthode comprenant une étape de mesure, dans un échantillon biologique desdits patients, de l'expression d'au moins 18 gènes choisis parmi un ensemble de 28 gènes comprenant ou consistant en les séquences d'acide nucléique SEQ ID NO 70 à 97,
lesdits au moins 18 gènes étant tels que
- 12 gènes comprennent ou consistent en les séquences d'acide nucléique SEQ ID NO : 70 à 81, et
- au moins 6 gènes appartiennent à un sous-ensemble de 16 gènes comprenant ou consistant en les séquences d'acide nucléique SEQ ID NO : 82-97, lesdits au moins 6 gènes comprenant ou consistant en les séquences d'acide nucléique SEQ ID NO : 82-85 et 87-88 ;
ladite méthode permettant d'identifier au moins 83% des patients ayant un taux de survie d'au plus environ 20% 30 mois après le diagnostic de leur cancer du poumon.

11. Méthode, selon l'une des revendications 8 à 10, ladite méthode comprenant une étape de mesure, dans un échantillon biologique desdits patients, de l'expression d'au moins 21 gènes choisis parmi un ensemble de 28 gènes comprenant ou consistant en les séquences d'acide nucléique SEQ ID NO 70 à 97,
lesdits au moins 21 gènes étant tels que
- 12 gènes comprennent ou consistent en les séquences d'acide nucléique SEQ ID NO: 70 à 81, et
- au moins 9 gènes appartiennent à un sous-ensemble de 16 gènes comprenant ou consistant en les séquences d'acide nucléique SEQ ID NO : 82-97, lesdits au moins 9 gènes comprenant ou consistant de préférence en les séquences d'acide nucléiques SEQ ID NO : 82-88 et 91-92,
ladite méthode permettant d'identifier au moins 91% des patients ayant un taux de survie d'au plus environ 20% 30 mois après le diagnostic de leur cancer du poumon.

12. Méthode, selon l'une des revendications 8 à 11, ladite méthode comprenant une étape de mesure, dans un échantillon biologique desdits patients, de l'expression d'au moins 26 gènes choisi parmi un ensemble de 28 gènes comprenant ou consistant en les séquences d'acide nucléique SEQ ID NO 70 à 97,
lesdits au moins 26 gènes étant tels que
- 12 gènes comprennent ou consistent en les séquences d'acide nucléique SEQ ID NO : 70 à 81, et
- au moins 14 gènes appartiennent à un sous-ensemble de 16 gènes comprenant ou consistant en les séquences d'acide nucléique SEQ ID NO : 82-97, lesdits au moins 14 gènes comprenant ou consistant de préférence en les séquences d'acide nucléique SEQ ID NO : 82-92 et 93-96,
ladite méthode permettant d'identifier 100% des patients ayant un taux de survie d'au plus environ 20% 30 mois après le diagnostic de leur cancer du poumon.

13. Méthode, selon l'une des revendications 8 à 12, ladite méthode comprenant une étape de mesure, dans un échantillon biologique desdits patients, de l'expression de 28 gènes choisis comprenant ou consistant en les séquences d'acide nucléique SEQ ID NO 70 à 97,
ladite méthode permettant d'identifier 100% des patients ayant un taux de survie d'au plus environ 20% 30 mois après le diagnostic de leur cancer du poumon,
ladite méthode comprenant
* une étape de mesure, dans un échantillon biologique desdits patients, de l'expression de 28 gènes comprenant ou consistant en les séquences d'acide nucléique SEQ ID NO 70 à 97, et
* une étape d'identification des échantillons biologiques exprimant lesdits 28 gènes.


### SEQUENCE LISTING

<110> University Joseph Fourier
<120> USE OF SPECIFIC GENES FOR THE PROGNOSIS OF LUNG CANCER AND THE CORRESPONDING PROGONOSIS METHOD
<130> WOB 09 CF UJF LUNG
<140> EP 10306142,0
   <141> 2010-10-20
<160> 122
<170> PatentIn version 3.5
<210> 1
   <211> 3280
   <212> DNA
   <213> Homo sapiens
<400> 1
   gggggaggga tgactaaaga caacggctgt aagagaactc cacaagagag tcagaacgaa 60
   aatgtgcaac aagtgcggcg gctcctacca tggcaggtga ttcgaggact cagcacgagg 120
   cgagagtagg gaccaggaag agccggaaaa cccgcctgtg attggccgtc cacgggtatc 180
   ggtcgttgtg attgggtgag gcccagacag acagctgcgt tttgaaccgc gtagggttct 240
   gggtagcaaa ggccttgcaa ggctcttaac cgaaaggggg agggggaagg tcgccaacaa 300
   acggctgagc tcacaatcct ggccggggcg tccccctccc ccatggagag agccagaccc 360
   gagccgccgc ctcagccgcg cccgttgcgt cccgctccgc ccccgctgcc ggtcgagggc 420
   acctcctttt gggcagcagc catggagccc cctccgtcgt ctcccacact gagcgcggca 480
   gccagtgcga ccttggcctc gtcgtgcggg gaggcagtgg cgtccggctt acagcccgcg 540
   gtgcggcggc tgctgcaggt gaagccagag caggtgttgc tgctaccaca gcctcaggcc 600
   cagaacgagg aagccgctgc ctcgtccgcg caggcgcggc tgttgcagtt caggcccgac 660
   ctgcggctcc tgcagccgcc gacagcgtca gacggcgcca cctccaggcc cgagttgcac 720
   ccggtgcagc ccctggcgct gcatgtcaag gccaagaagc agaagctggg gcccagcctg 780
   gatcagtcag tggggcctcg aggggccgtc gaaaccggtc ctagagcctc cagggtggtc 840
   aagttggaag gccccgggcc ggccctcggc tacttccgag gggacgagaa gggcaagctg 900
   gaggcggagg aggtcatgag agactcgatg caaggcgggg caggcaaaag cccggcagcc 960
   atccgagaag gtgtgatcaa aacggaggaa cccgagagac tcctcgagga ctgcaggctc 1020
   ggcgcggagc ccgcgtccaa tggcctggtt catggcagcg cggaggtcat cttggcccca 1080
   acgtccggtg cctttgggcc gcaccagcaa gaccttagga tccctttgac gctccacacg 1140
   gtcccccctg gggcccggat ccagtttcag ggagctccgc cttcagagct gataagattg 1200
   accaaggtcc ccctgacacc agtgcctact aaaatgcagt ccctactgga gccttctgta 1260
   aaaattgaaa ccaaagatgt cccgctcacc gtgttgccct cagatgcagg cataccagat 1320
   actcccttca gtaaggacag aaatggtcat gtgaagcgac ccatgaacgc atttatggtt 1380
   tgggcaagga tccaccgacc agcactagcc aaagctaacc cagcagccaa caatgcagaa 1440
   atcagtgtcc agcttgggtt agagtggaac aaacttagtg aagaacaaaa gaaaccctat 1500
   tacgatgaag cacaaaagat taaggaaaag cacagagagg aatttcctgg ttgggtttat 1560
   cagcctcgtc cagggaagcg aaaacgattc cctctaagtg tttccaatgt attttctggt 1620
   accacacaga atatcatctc tacaaatcct acaacagttt atccttaccg ctcacctacg 1680
   tactctgtgg taattcccag cctacagaat cccatcactc atccagttgg tgaaacctca 1740
   cctgctatcc agctgcccac acctgcagtc cagagcccaa gccctgtcac acttttccag 1800
   cccagcgtct ccagtgctgc tcaggtggct gtccaggatc caagtctacc tgtctatcca 1860
   gcactcccac cccaacgctt tactgggcct tcccaaacag acactcatca gctgcattct 1920
   gaagccactc acactgtgaa gcaacccact cctgtctctc tagagagcgc caacaggatt 1980
   tcaagtagtg caagtactgc ccatgccaga tttgcaactt cgaccatcca acctcctagg 2040
   gagtattcca gcgtttcccc ttgtcccaga agtgctccaa tcccccaggc ttctcccatt 2100
   ccacacccac atgtctacca gccccctccc cttggccatc cagccacact gttcgggaca 2160
   ccaccaagat tctcttttca tcacccttac ttcctacccg gacctcacta cttcccatca 2220
   agtacatgcc cttacagtcg gcctcccttt ggctatggaa attttccgag ttcaatgcca 2280
   gaatgcctta gttattatga agacaggtac ccaaaacatg agggtatctt ttcaacttta 2340
   aatagagact attcttttag agactactca agtgaatgca cacacagtga aaattctcgg 2400
   agttgtgaga acatgaatgg aacttcttac tataacagtc atagccacag tggggaagaa 2460
   aacttaaacc ctgtgcctca gctggacatt ggaaccttgg agaatgtctt cacagccccg 2520
   acatcaactc cttctagcat ccagcaagtc aatgtcaccg acagtgatga ggaggaagaa 2580
   gaaaaagtgc tcagggattt ataattttaa aacaaatatg cacagaaaat aaacatttct 2640
   taaaatatat tctgggtcag ttggtatgag aaaaaaaaaa gcctagaatt ctttgttgaa 2700
   agttttcagt cgtgatttga ggagttaaaa ccaaatgcaa tttatgtctt cataaaattt 2760
   tgattagtga aactagagtc tggatgtttc attgtaggaa tatttaagtt attaagtagt 2820
   ttaattttaa tggctgaaat ttgcatcaac atgtattatt attactttat cctggaacat 2880
   gcaaaatact gaagcctcac agttgtatgt gaggggaaag gggaaataaa tctagcatag 2940
   tgtgattttt attttatctc aggatacatt ttttaaatga ttttttgttt gctttttatg 3000
   taatacttat ggatgttgtc aatttttgat gtaacatttt gaaagtattt tgacaactcc 3060
   tagtgaactt ggacttggtt gctaaattta acttacacta ataaccaatt ataagttcca 3120
   aatgtgtttt aatggcacct gggtgattct tcagctaaat ttagtcattt ctgtttctaa 3180
   atatttttat cattttaaaa tatttttttt ccatttggca tacatcgttc tttgttgtaa 3240
   ttaaataaac atagataaaa ttgttaaaaa aaaaaaaaaa 3280
<210> 2
   <211> 1479
   <212> DNA
   <213> Homo sapiens
<400> 2
   aggcgaggga aactgagggc gaaagttgtg tgtcgtgttg gcaggagggc ctagaaggga      60
   aagactgtgc gttgatacca aactgccccc actccgtggg cgggtcagga gaggcctttg     120
   gaagagcgtc tcaactcgga ctggagcctc ttctctccca ccgcggtcta gtgggacaat     180
   gtcatattat aaatttggaa tgctgaatag aaaattatag attttgatat tgaaggaaat     240
   gaagcgaagc ctaaatgaaa attcagctcg aagtacagca ggctgtttgc ctgttccgtt     300
   gttcaatcag aaaaagagga acagacagcc attaacttct aatccactta aagatgattc     360
   aggtatcagt accccttctg acaattatga ttttcctcct ctacctacag attgggcctg     420
   ggaagctgtg aatccagagt tggctcctgt aatgaaaaca gtggacaccg ggcaaatacc     480
   acattcagtt tctcgtcctc tgagaagtca agattctgtc tttaactcta ttcaatcaaa     540
   tactggaaga agccagggtg gttggagcta cagagatggt aacaaaaata ccagcttgaa     600
   aacttggaat aaaaatgatt ttaagcctca atgtaaacga acaaacttag tggcaaatga     660
   tggaaaaaat tcttgtccag tgagttcggg agctcaacaa caaaaacaat taagaatacc     720
   tgaacctcct aacttatctc gcaacaaaga aaccgagcta ctcagacaaa cacattcatc     780
   aaaaatatct ggctgcacaa tgagagggct agacaaaaac agtgcactac agacacttaa     840
   gcccaatttt caacaaaatc aatataagaa acaaatgttg gatgatattc cagaagacaa     900
   caccctgaag gaaacctcat tgtatcagtt acagtttaag gaaaaagcta gttctttaag     960
   aattatttct gcagttattg aaagcatgaa gtattggcgt gaacatgcac agaaaactgt    1020
   acttcttttt gaagtattag ctgttcttga ttcagctgtt acacctggcc catattattc    1080
   gaagactttt cttatgaggg atgggaaaaa tactctgcct tgtgtctttt atgaaatcga    1140
   tcgtgaactt ccgagactga ttagaggccg agttcataga tgtgttggca actatgacca    1200
   gaaaaagaac attttccaat gtgtttctgt cagaccggcg tctgtttctg aacaaaaaac    1260
   tttccaggca tttgtcaaaa ttgcagatgt tgagatgcag tattatatta atgtgatgaa    1320
   tgaaacttaa gtagtgataa aaggaagttt agcataaatt atagcagttt tctgttattg    1380
   cttaatttac catctccata gttttatagc tactattgta tttcacttgt tgaattaaag    1440
   tatttgaatt cttttaaatg tggaaaaaaa aaaaaaaaa                           1479
<210> 3
   <211> 1731
   <212> DNA
   <213> Homo sapiens
<400> 3
   ttagggcggg agcccggcga gggcgccggt gctttgttct gtctgaggcc aggaagtttg      60
   accgcgctgc catgccgaac cgtaaggcca gccggaatgc ttactatttc ttcgtgcagg     120
   agaagatccc cgaactacgg cgacgaggcc tgcctgtggc tcgcgttgct gatgccatcc     180
   cttactgctc ctcagactgg gcgcttctga gggaggaaga aaaggagaaa tacgcagaaa     240
   tggctcgaga atggagggcc gctcagggaa aggaccctgg gccctcagag aagcagaaac     300
   ctgttttcac accactgagg aggccaggca tgcttgtacc aaagcagaat gtttcacctc     360
   cagatatgtc agctttgtct ttaaaaggtg atcaagctct ccttggaggc atttttattt     420
   ttttgaacat ttttagccat ggcgagctac ctcctcattg tgaacagcgc ttcctccctt     480
   gtgaaattgg ctgtgttaag tattctctcc aagaaggtat tatggcagat ttccacagtt     540
   ttataaatcc tggtgaaatt ccacgaggat ttcgatttca ttgtcaggct gcaagtgatt     600
   ctagtcacaa gattcctatt tcaaattttg aacgtgggca taaccaagca actgtgttac     660
   aaaaccttta tagatttatt catcccaacc cagggaactg gccacctatc tactgcaagt     720
   ctgatgatag aaccagagtc aactggtgtt tgaagcatat ggcaaaggca tcagaaatca     780
   ggcaagatct acaacttctc actgtagagg accttgtagt ggggatctac caacaaaaat     840
   ttctcaagga gccctctaag acttggattc gaagcctcct agatgtggcc atgtgggatt     900
   attctagcaa cacaaggtgc aagtggcatg aagaaaatga tattctcttc tgtgctttag     960
   ctgtttgcaa gaagattgcg tactgcatca gtaattctct ggccactctc tttggaatcc    1020
   agctcacaga ggctcatgta ccactacaag attatgaggc cagcaatagt gtgacaccca    1080
   aaatggttgt attggatgca gggcgttacc agaagctaag ggttgggagt tcaggattct    1140
   ctcatttcaa ctcttctaat gaggaacaaa gatcaaacac acccattggt gactacccat    1200
   ctagggcaaa aatttctggc caaaacagca gcgttcgggg aagaggaatt acccgcttac    1260
   tagagagcat ttccaattct tccagcaata tccacaaatt ctccaactgt gacacttcac    1320
   tctcacctta catgtcccaa aaagatggat acaaatcttt ctcttcctta tcttaatgat    1380
   ggtactcttt tcaatttctg aaaacagtaa caggcccaac ttccttctta ctacagtcat    1440
   attaaacaga tcacatcaat gacaaatgtc actactataa aaactactta atttgtaagg    1500
   aaattgtttc atagatttaa aaaaattgtg gttggagagc atcttggcat ttgtgctttt    1560
   tttcttgagg gattgttctg cttcctggct gtatgatggg tatatcatta aagtttggag    1620
   tcctatatga acaaaactga catttttaga gttgtacttt tgggaatgtt atagattgat    1680
   cattctttct cctgataata aaggtattga atatctgtta tgaaaggttc t              1731
<210> 4
   <211> 531
   <212> DNA
   <213> Homo sapiens
<400> 4
   acgagcactg gagcttgcgt tacttggcct cacctcacct gtgctgtcca cgcctggctt 60
   tgtctcacct gacgcgatat gcctctcctg cgtgggcgct gtcctgcccg ccgccactac 120
   cgccgcttgg ccctgctcgg cctgcagccc gctccccgct tcgcccactc ggggcccccg 180
   cgccagcggc ccctgtctgc cgcggaaatg gctgttggac ttgtggtgtt ttttacgacc 240
   ttcttaacac cagctgcata tgtgctaggc aacctgaagc agttcagaag gaattagatg 300
   gaagatgatg ttgaacagct gttaacgtcc aaaaaacttt cagaaaaagc tgtgtttttg 360
   ttaacgagca aaattgccta gttgagttga tgcaaccatt gtggtattca ctttcctcat 420
   gtttatgatg aatattttgc acttttttag tactgtgcat tatatagatg tatagtcaaa 480
   aatgttctgc ttaagtgtta aataaaacgg aaacacttat tcgtgcttgg t 531
<210> 5
   <211> 2652
   <212> DNA
   <213> Homo sapiens
<400> 5
   tcccttgact tgcttgcgga gggagcggcc ggcggaggga gcggcaggtg gagggagtgg 60
   cacgaggcat gcggagggag ctgcaccgac atcacataaa cgcactgggc agctcgcagg 120
   cgccattcgc tcttcagacg ccggagacgt aggagtgggt cttcagactc caaaggggtt 180
   ggactaatgg cggatgctga ggcgagggct gagttcccgg aggaggccag acctgacagg 240
   ggcaccttgc aggtgttgca agatatggcc agccgcttgc gaatccattc catcagggcc 300
   acatgctcca cgagctccgg ccaccctaca tcatgtagca gttcttctga gatcatgtct 360
   gtgctgttct tctacatcat gaggtacaag cagtcagatc cagagaatcc ggacaacgac 420
   cgatttgtcc tcgcaaagag actgtcgttt gtggatgtgg caacaggatg gctcggacaa 480
   ggactgggag ttgcatgtgg aatggcatat actggcaagt acttcgacag ggccagctac 540
   cgggtgttct gcctcatgag tgatggcgag tcctcagaag gctctgtctg ggaggcaatg 600
   gcctttgctt cctactacag tctggacaat cttgtggcaa tctttgatgt gaaccgcctg 660
   ggacacagtg gtgcattgcc cgccgagcac tgcataaaca tctatcagag gcgctgcgaa 720
   gcctttgggt ggaacactta tgtggtggac ggccgggacg tggaggcact gtgccaggta 780
   ttctggcagg cttctcaggt gaagcacaag cccactgctg tggtggccaa gaccttcaag 840
   ggccggggca ccccaagtat tgaggatgca gaaagttggc atgcaaagcc aatgccgaga 900
   gaaagagcag atgccattat caaattaatt gagagccaga tacagaccag caggaatctt 960
   gacccacagc cccccattga ggactcacct gaagtcaaca tcacagatgt aaggatgacc 1020
   tctccacctg attacagagt tggtgacaag atagctactc ggaaagcatg cggtctggct 1080
   ctggctaagc tgggctacgc gaacaacaga gtcgttgtgc tggatggtga caccaggtac 1140
   tctactttct ctgagatatt caacaaggag taccctgagc gcttcatcga gtgctttatg 1200
   gctgaacaaa acatggtgag cgtggctctg ggctgtgcct cccgtggacg gaccattgct 1260
   tttgctagca cctttgctgc ctttctgact cgagcatttg atcacatccg gataggaggc 1320
   ctcgctgaga gcaacatcaa cattattggt tcccactgtg gggtatctgt tggtgacgat 1380
   ggtgcttccc agatggccct ggaggatata gccatgttcc gaaccattcc caagtgcacg 1440
   atcttctacc caactgatgc cgtctccacg gagcatgctg ttgctctggc agccaatgcc 1500
   aaggggatgt gcttcattcg gaccacccga ccagaaacta tggttattta caccccacaa 1560
   gaacgctttg agatcggaca ggccaaggtc ctccgccact gtgtcagtga caaggtcaca 1620
   gttattggag ctggaattac tgtgtatgaa gccttagcag ctgctgatga gctttcgaaa 1680
   caagatattt ttatccgtgt catcgacctg tttaccatta aacctctgga tgtcgccacc 1740
   atcgtctcca gtgcaaaagc cacagagggc cggatcatta cagtggagga tcactacccg 1800
   caaggtggca tcggggaagc tgtctgcgca gccgtctcca tggatcctga cattcaggtt 1860
   cattcgctgg cagtgtcggg agtgccccag agtgggaagt ccgaggaatt gctggatatg 1920
   tatggaatta gtgccagaca tatcatagtg gccgtgaaat gcatgttgct gaactaaaat 1980
   agctgttagc tttggtcttt tggcctcttt accctgtgtt tatgtttgtt ccaaaaccat 2040
   catttaaatc tctactgtca cattttgttt cttaaaagca aagccagcta acaccttcat 2100
   tcatccctag ttcggaaatt caagctaact acttaccctt taaactgtca ctgcatatgc 2160
   aagtaccgct ctaatttttg gatcattaaa gggagttaca caacttttaa gtgaaaaaaa 2220
   taggtaacaa aacaaccacc tgatagtaag ttttctgata agactataga taagtggtag 2280
   aggtaatcaa ttcttccgaa gtgtttcctt cgtgaataac tggtagaggt aatagttttt 2340
   tcaatgtatt tccttcatga gtaaagaaaa tgtggattga agtatagatt ccagtagcct 2400
   agtttccaca gcacgataac accatgacgc ctactgctgt tcccaccttg ggattctgtg 2460
   tgctgccatc ccacctgcag ctgccctgga attcccttcg ctgtttgcct tcatctccct 2520
   ccacgtttga gaggctgtca ggcagcagcg aaagcttgtt aggatgtcct gtgctgcttg 2580
   tgatgagagc ctccacactg tactgttcaa gtcaatgtta ataaagcatt tcaaaaccag 2640
   ctgctttatt ca 2652
<210> 6
   <211> 2521
   <212> DNA
   <213> Homo sapiens
<400> 6
   aaacgagtgg agacacgagg accagcgcga gcggtcccgg tgggctaccc tccccctgcg 60
   acgacccccc ctcgctctga ccgactggtc ccctaaacgg tggcggcggt ttttggtcgt 120
   tgggccccgg gatttaggac caacatttga agacccgaag gggaactgca accatgaatg 180
   aagaaaatat agatggaaca aatggatgca gtaaagttcg aactggtatt cagaatgaag 240
   cagcattact tgctttgatg gaaaagactg gttacaacat ggttcaggaa aatggacaaa 300
   ggaaatttgg cggtcctcct ccaggttggg aaggtccacc tccacctaga ggctgtgaag 360
   tttttgtagg aaaaatacct cgtgatatgt atgaagatga gttagttcct gtatttgaaa 420
   gagctgggaa gatatatgaa tttcgactta tgatggaatt tagtggtgaa aatcgaggtt 480
   atgcttttgt gatgtacact acaaaagaag aagcccaatt agccatcaga attcttaata 540
   attatgaaat tcgaccaggg aagtttattg gtgtgtgtgt aagcctggat aattgtagat 600
   tatttattgg agctattccc aaggaaaaga agaaagaaga aattttagat gaaatgaaga 660
   aagttacaga aggagttgta gatgtcattg tttatccaag tgcaactgat aagaccaaaa 720
   atcgtggttt tgcatttgtg gaatatgaat ctcacagagc tgctgctatg gcaaggagga 780
   aactaattcc aggaacattc caactatggg gccacaccat tcaggtagat tgggctgacc 840
   cagagaaaga ggtggatgag gaaaccatgc agagagttaa agttctttat gtaagaaatt 900
   taatgatctc aactacagag gaaacaatta aagcagaatt caataaattt aagcctggtg 960
   cagttgaacg ggtaaagaaa cttagagatt atgcttttgt tcactttttc aaccgagaag 1020
   atgcagtggc tgccatgtct gttatgaatg gaaaatgcat tgatggagca agtattgagg 1080
   taacactagc taaaccagta aataaagaaa acacttggag acagcatctt aatggtcaga 1140
   ttagtccaaa ttctgaaaat ctgattgtgt ttgctaacaa agaagagagc cacccaaaaa 1200
   ctctaggcaa gctgccaact cttcctgctc gtctcaatgg tcagcatagc ccaagtccgc 1260
   ctgaagttga aagatgcact tacccttttt atcctggaac aaagcttact ccaattagta 1320
   tgtattcttt aaaatccaat cattttaatt ctgcagtaat gcatttggat tattactgca 1380
   acaaaaataa ctgggcacca ccagaatatt atttatattc aacaacaagt caagatggga 1440
   aagtactctt ggtgtataag atagttattc ctgctattgc aaatggatcc cagagttact 1500
   tcatgccaga caaactctgt actacgttag aagatgcaaa ggaactggca gcccagttta 1560
   cattacttca tttggactac aatttccatc gcagctcaat aaatagtctt tcccctgtta 1620
   gtgctaccct ctcttctggg actcccagcg tgcttcctta tacttcaagg ccttattctt 1680
   atccaggcta tcctttgtca ccaacaatat cacttgctaa tggcagccat gttggacagc 1740
   ggctatgtat ctccaatcag gcctccttct tctgaagaaa atactaacat tagtatgaaa 1800
   atttgtgtaa atttgtagta tgaaaacttg caaattaaaa tattgtttta ttttagaatc 1860
   gggtttgcat atttggtttt aaaaaggtat ttattccaaa gtactaaaca tcagctataa 1920
   ttcagaataa catggagttg tagaatttat aaaaatgcaa agtttaaaaa gttattcagt 1980
   ggtttctctt gataaaggta cagcaaacta ctattctttt taaacttcta ggattttctt 2040
   ctactttctg agtgggcaat agaacctagt catttatgtt tttttttttt tttgcataat 2100
   tttactaaat agtatttcac aaatattaaa gcacttgaag acaatggtta tagtagattt 2160
   gattaccaag gatcactatc tgtactggag attagaacaa ttatatgacc agaagcatct 2220
   aaccattatg taaaaagaaa tgatgagaca aaaagattaa gatacaaatt ttgtgcagta 2280
   ctaaagaaaa agcagtctac cattgtggtc cttgaaaata actatagata tttttgttat 2340
   ttgttagaca caaattataa ttttgttgtt aatgtattta agcattttat agttatgctt 2400
   tgtgtttttg atattctttg tattgttaat aacaagtgtt atgggttttt aatgttgaaa 2460
   tcatgtgtta atttttgtac ttgaattcaa attttttgac attaaatatg tgatgcttct 2520
   a 2521
<210> 7
   <211> 1949
   <212> DNA
   <213> Homo sapiens
<400> 7
   aataaagggg tctgagccgg tcgcctgagc ctgaaaagtg ctgtcacgtc agcggaagga 60
   ggcgtcccag atcttctcag ctgtcttggt gccagccttc ctagtcttcc tacccacact 120
   cctacctgct gtcacaggcc acagccatca tgcctcgggg tcacaagagt aagctccgta 180
   cctgtgagaa acgccaagag accaatggtc agccacaggg tctcacgggt ccccaggcca 240
   ctgcagagaa gcaggaagag tcccactctt cctcatcctc ttctcgcgct tgtctgggtg 300
   attgtcgtag gtcttctgat gcctccattc ctcaggagtc tcagggagtg tcacccactg 360
   ggtctcctga tgcagttgtt tcatattcaa aatccgatgt ggctgccaac ggccaagatg 420
   agaaaagtcc aagcacctcc cgtgatgcct ccgttcctca ggagtctcag ggagcttcac 480
   ccactggctc tcctgatgca ggtgtttcag gctcaaaata tgatgtggct gccaacggcc 540
   aagatgagaa aagtccaagc acttcccatg atgtctccgt tcctcaggag tctcagggag 600
   cttcacccac tggctcgcct gatgcaggtg tttcaggctc aaaatatgat gtggctgccg 660
   agggtgaaga tgaggaaagt gtaagcgcct cacagaaagc catcattttt aagcgcttaa 720
   gcaaagatgc tgtaaagaag aaggcgtgca cgttggcgca attcctgcag aagaagtttg 780
   agaagaaaga gtccattttg aaggcagaca tgctgaagtg tgtccgcaga gagtacaagc 840
   cctacttccc tcagatcctc aacagaacct cccaacattt ggtggtggcc tttggcgttg 900
   aattgaaaga aatggattcc agcggcgagt cctacaccct tgtcagcaag ctaggcctcc 960
   ccagtgaagg aattctgagt ggtgataatg cgctgccgaa gtcgggtctc ctgatgtcgc 1020
   tcctggttgt gatcttcatg aacggcaact gtgccactga agaggaggtc tgggagttcc 1080
   tgggtctgtt ggggatatat gatgggatcc tgcattcaat ctatggggat gctcggaaga 1140
   tcattactga agatttggtg caagataagt acgtggttta ccggcaggtg tgcaacagtg 1200
   atcctccatg ctatgagttc ctgtggggtc cacgagccta tgctgaaacc accaagatga 1260
   gagtcctgcg tgttttggcc gacagcagta acaccagtcc cggtttatac ccacatctgt 1320
   atgaagacgc tttgatagat gaggtagaga gagcattgag actgagagct taaggcaggg 1380
   ctggcactat ttccttggcc agggtacctt atggggccat atcctacaga tcctcccatt 1440
   tctagggagg tctgaagtag aattttcact ttatgttaga agagagtagt gagctttcta 1500
   agtagtgcag tatagtagag gctggaggga acaagatatg tatctttctt ttgttacaca 1560
   tgagtaactt gcagatttat gttttatctc tgtcagttat caacattgtt cctgttaagt 1620
   gaaggtttat tttgcttcag attatacaat tatcaataac atagctctca cattcatggc 1680
   tgtttaacca atctgaaagt tacggtttgg gaattaataa aacaaagtca tacaacacat 1740
   tttctttgta attgagaact agataacatg gtaacagaga attgattttc atatgaatct 1800
   taactccaca gtaaaatagt tgacatcata atatgaagag aaagaaaagg aaaaacagaa 1860
   atgtaaaagt tgtttaattc ttggtttgcc taattcgttt tcctatttct tttcatacaa 1920
   ataaaggata cctggattta tttaggtta 1949
<210> 8
   <211> 2499
   <212> DNA
   <213> Homo sapiens
<400> 8
   cttctaattc tgttattgca actgcagacc gttacctggt acgctggctg ctacctccct 60
   cactcttgtc agagtcggag ctacaggcag tgccttcagc tctgagctca ggcatcccgg 120
   tccctgtttt tgcggttaag gactctaaag tgttgtgtcg tgttcatcaa ctttttctca 180
   acttccctgg ctctacctct tctgccacaa acgtcagcat ggtggtatct gccgaccctt 240
   tgtccagcga gagggcagag atgaacatcc tagaaatcaa ccaggaattg cgctcgcagc 300
   tggcagagag caatcagcag ttccgagacc tcaaagagaa attccttata actcaagcta 360
   ctgcctactc cctggccaac cagctgaaga aatacaagtg tgaagagtac aaagacatca 420
   tagactctgt gctgagggat gaactgcagt ccatggagaa gctggcagag aagctcaggc 480
   aagctgagga gctcaggcag tataaagccc tggttcactc tcaggcaaaa gagctgaccc 540
   agttacggga gaagttacgg gaagggagag atgcctcccg ctggctgaac aagcatctga 600
   aaaccctcct cactcctgat gaccctgaca agtcccaggg tcaggacctc cgagagcagc 660
   tggctgaggg gcacaggctg gcagagcacc ttgttcacaa gctgagccca gaaaatgatg 720
   aagatgaaga tgaggatgaa gacgacaaag acgaggaggt tgagaaagta caggaatcac 780
   ctgcccccag agaggtgcag aagactgaag aaaaggaagt ccctcaggac tcactggagg 840
   aatgtgctgt cacttgttca aatagtcaca acccttctaa ctccaaccag cctcacagga 900
   gcaccaaaat cacatttaag gaacacgaag tcgactctgc tctggttgta gagagtgaac 960
   accctcatga tgaagaggag gaagctctaa acattccccc agaaaatcaa aatgaccatg 1020
   aggaggagga ggggaaagcg ccagtgcccc ccagacacca tgacaagtcc aactcttacc 1080
   ggcatcgtga agtctctttc ttggcattgg atgaacagaa agtttgctcc gctcaggatg 1140
   ttgccaggga ttactccaat cccaaatggg atgaaacctc acttggcttc ctcgaaaagc 1200
   aaagtgatct tgaagaggtg aaaggacaag aaacagttgc tcccaggctc agcaggggac 1260
   cgctgagagt ggacaagcat gaaatccccc aggagtcact ggatggatgt tgcttgactc 1320
   cttccatcct tcctgacctg actccctcct accaccctta ttggagcact ttgtactctt 1380
   ttgaagacaa gcaagtcagc ttggctcttg tagacaaaat taaaaaggat caagaggaga 1440
   tagaagacca aagcccacca tgccccaggc tcagccagga gctgccagag gtgaaggagc 1500
   aggaagtccc agaggactct gtgaatgaag tttacttgac tccctcagtt caccatgacg 1560
   tgtctgactg ccaccagcct tatagcagca ccttgtcctc attggaggat cagcttgcct 1620
   gctctgctct ggatgtagcc tcccccaccg aggcggcctg tccccaaggg acttggagtg 1680
   gagacttgag ccaccaccag tcagaggtgc aagtttcaca ggcacagctg gaaccaagca 1740
   ccctggtgcc cagttgtctg cgactacagc tggatcaagg gttccactgt gggaacggct 1800
   tggcccagcg gggcctttcc tccaccacct gcagcttctc agccaatgct gattctggga 1860
   accaatggcc cttccaagag ctggttttag agccctctct ggggatgaag aaccctcccc 1920
   agctggaaga tgatgcactt gaaggctcag caagcaacac acaagggcgt caagtcactg 1980
   gccggattcg tgcctccctt gtcctgatac tgaagaccat cagaagaaga ctcccgttca 2040
   gcaagtggag actggcattc agattcgctg gcccgcatgc tgagagcgca gagataccaa 2100
   atactgctgg aaggacgcaa aggatggcag gatgaaagaa tgtcacaaaa agcagctttt 2160
   ccacttgata aaaacaacta aaacagcaaa gcaagtttaa gtccaaacac aatactgcag 2220
   gggtccttca ctgaggattg aatttcagac acagaatact cttgatgact tcaagccact 2280
   atgctccttt gatttgagaa gccacattcc atccccctcc aattgtgatc aatacctagg 2340
   gagaccaatg cccagatgga caaatagcat tgaccggcgt tagccctgtt tctcaattcc 2400
   catcgtgtag agaacaggag tccgcagctg ctggcaggag acagcatgtc agccgggact 2460
   ctgccagggc agagtatgag caatgccatg ttcttgctg 2499
<210> 9
   <211> 1405
   <212> DNA
   <213> Homo sapiens
<400> 9
   atcttaagag gcgttccttt ttgcatagtt cccatgagca tgagagaaga agcaatgcac 60
   gctccggcag attcctagga accaaatacc tctgaggagc accagatttc agcttatggg 120
   atgctttgat tgctctgtgg ctgcatttag gagaaggaag ctgcagtcat gcgtcatcac 180
   tgccagcctc acatctcttg acagttaaag ccttagggtg gagcaaggga aaatttaaaa 240
   taacaaatga agcaaaagca agaggtgatg ttccaaagca gaggaaggct aagtttatat 300
   atacaaatgt caagtgtgta tagtgcaaaa ctaggaccag ttggtggaat ctgtggtcaa 360
   aaacaaaagc cttccttttt ttttttcaag gcccagtccc aagacgcaag accacttgcg 420
   ccagcagcgt gcatcagcaa gatagcaaaa gcaggacgag agctgcccgg aagacatcta 480
   cctggccaga agacacctac cctggccgga agacatgtac ccctgaagat agagaaagag 540
   gccatcgtgt actacgtagc agtcatgtca gactgggaca cttcctgttt acagaggact 600
   ataaaacccc tgtcctgtcc tcacttgggg ctgacgccat cttaggcctc agcccgcctg 660
   cagccaggcg ttcgttaaaa cagcatgttg ctccacaccg ccttgtattg tttgttggtc 720
   ccactctctg ggctcgaacc aatacaagca cctttcaagc agtatattct tcagtgtctt 780
   gatcctccaa ataactctct tctaattcct cctgaccaca aaaagcactt atactctagg 840
   atgactgatt ccagcccagt ggcctggcaa gggtgaatta caccttgcat atcacactct 900
   tgacatttgt gtgcgctagc ataagaatta taattgaaac agggatttaa gtatctcctc 960
   tctaggtgcc taccctcctt ggactcaggt caaatttatt aaaggaagtt ttgtttctag 1020
   ataggttgtt tgaaataaaa taacagaatg ttcaagtaac acagtgtacc tacagctttt 1080
   aacaaaattg aggacttggg tctcgaaaca atttcctttg attttcaggt attttatcta 1140
   taaaaaggga gataaagcat tagttcatag gacagttata tgtttaaatg tgataatgta 1200
   tattaaccac cttgcatgta ttcaaatgtg ttttgaaatc taacgtctac attttgatag 1260
   tttaactctt ctacataagt gacttacaac aggcattaaa tattgtttgg cattttcata 1320
   tatctgtaac tgtatcttaa tctacaatga gcttaatttt aagtgtagca taaaacagaa 1380
   ccttcaataa agtggtaata ttagg 1405
<210> 10
   <211> 3848
   <212> DNA
   <213> Homo sapiens
<400> 10
   agcgttgctg ctgccttgca gtttgatctc agactgctgt gctagcaatc agcgagactc 60
   cgtgggcgta ggaccctcca agccaggtga aagagcttat gatcctaagc acttccataa 120
   tagggtcagt agaatcatga tcgatgatca taatgtcccc actctacggg agatggtagc 180
   attctccaag gaagtgttgg agtggatggc tcaagattct gaaaacatcg tagtgattca 240
   ctgtaaagga ggcaaagaat agatatgttg gatattttgc acaagtgaaa catagctaca 300
   actggaatct ccctccaaga aaaacactgt ttataaaaag attcgttatt tattcgattc 360
   atggtgttgg aacaggcgat ggatatgatc taaaagtcca aatagtaatg aagaaaaaga 420
   ttgtcttttc ctgtacttcc ttaaagaatt gtcgggtatt tcatgacact gaaacagaca 480
   gggtaataac tgatgtgttc aactgtccac ctctgtatga tgatgtgaaa gtgcaagctt 540
   cctcttcaag agaagagggc agcacacctc gcagggctaa ctggaagggg gagccatcca 600
   ggagacctgt gctcaactga tgggtgggga gcaatagcga gaacgaggga gggacctgag 660
   agtggaagcc tttattggga tgtaaggtgt tacctgagca ggtttcctac ggggaggtct 720
   aactggtgga tttaatgcaa gcagtcatga gttccatgga gtcatgctgt gactgagagg 780
   tggtcattga tatatccaca tggtccatgc agagtacggg ggtctgtagg gaggttatat 840
   ctagctgtcc cataatgaag tagtcaccaa cagaaggttg tataaggcag atactgggat 900
   cagtcacatt gagaaacctg gaggaggtga actggaaact gtcaagggtg actgaaccct 960
   gcttctgata tcagaaagtc caatttatat ttgaaaggga tgctgaggca caaaaaaatt 1020
   gtaagaattc actacaaaaa tacttggcta tatataagca taggtcctta gtagattctg 1080
   tttagcacta tctaaaccag attcaaattt cagcatttaa attaaatatc tatcatggaa 1140
   aataaactat tccttgaaaa ttttggtaga aacagcaaga gaaagcaata gcattttctt 1200
   aagcctcctc ctctgtgtct tgagtgtgtt attatagaat gcagagtgct acctattgaa 1260
   tggttataat tatttgataa atatataaag gaataaagga aggaactttg atttctttgg 1320
   aatgatagtt cttggcatca attttacttt taaaatattt ttttttcttt ttaggatttt 1380
   cctaaatact atcacaacta cccttttttc ttctggttta acacatcttt aatacaaaat 1440
   aacgggtatg gatataatat caacccatag aaacaaccta atcttcaatg tctatgtata 1500
   agatgtaatg gcaagtcttt tgctggttgt cataagctta atttatagaa aacaaaaaat 1560
   ccttgagcca ccattgttca ttgccttact ccttttacgt tggctatttt aaaaatacag 1620
   ttgttcttga gacccccagt tgcagtatcc tcaaggtcca tgccatagga ctgtgttatg 1680
   agctcaaaag tattataatc agatcttaag tgtggaagta aattcctccc agagaagttc 1740
   aatatgaatc tgctcagtac cttcaacatg tcaggtcctc agtaggtgct gatttaccaa 1800
   tgacgaacca ccaccaaatt ttgtgctaaa gtaagggagg acctagggaa gcttcagcta 1860
   gctgaaaagc tgactgacac acttatatct aggagaagtt acaagacaca gtaagtatta 1920
   agaaatacag ctaaaaaatc attaaaattg gtagtctccc atttaaacat gggtttctaa 1980
   taactgaatt gggaaaactt tcttaaaaac tattaattgg aggctgggtg tggtggctca 2040
   tgcctgtaat cctagcactt tgggaggctg aggcgggcgg atcacctaag gttggaagtt 2100
   cgagactagc ctggccaaca tggtaaaact ccgtctctac taaaaataca aaaatcagcc 2160
   aggcgtggtg gcacatgcct gtaatcccag ctactcagga ggctgagcca gtagaatcgc 2220
   ttgaacccag gaggcagatt gcagtgagcc gagatcgcac cactacactc cagcctgggc 2280
   gacagagtga gactctgtct aaagaaaaaa gcaaaaaaac agaacaacta ttacttggat 2340
   ttggagatta ttgttcccag aaaaccttct gccatatttg gaaacttatt tctcagtcta 2400
   gaagttctcc actttaagta gcatttgttc tgtgctggtg aaaaactgag atttttttgt 2460
   attaaccata ctcttcaata caaaaggaga aaatattttt aaaatgcttc aggtcacagt 2520
   tgaggcagtt gctatgattg catgtggcat gaattggtag ttattgttac aaccagttct 2580
   agtcttttct tcaaatctga gctggatcta ataactcctt aagtccagca aggcaacagt 2640
   aaattaaacc tctggtctac acacttgcaa tacatacaca tttaatagat tttgatagag 2700
   tgaactttgg attggatgga aattttttaa aaatttgttt cttggatgca tacaaacaat 2760
   aagctttgac tcctaacatg agcaaagtcc ctcaattgtg agagctgggt ggagcttcat 2820
   ttgttgctgc tcctcaaatt gattcttggt aaaggataca gatttttcct ttgaaacacc 2880
   atgttcattt tggggaagca ataagttaga tcacctttat tttcactttt atataaattt 2940
   ctaaagattt ctgtaatatt taaatttata tactattggt aaagctgttt ttcttagttg 3000
   tgaaattgtt gtttagccaa aaatgccaac ttctgtcttt tagaacacta ggcataaatg 3060
   ggttaaccaa tttatgccta gtgttccatt attggaatgc taagcatgtg ggatttattt 3120
   atatcctact gctcaaggtc atcgccaagg gctgtttgca aaaattcaaa aaattgcaac 3180
   ctcaggcata aattaaaaga gatatagtat tttattattg ggttttgata catgtctaat 3240
   cagactgatt tctgtcacat atagaaattt agatactgta ttaaacctgg atgtcattaa 3300
   ttccataaaa agcaacgtta aaagaatcag tagcatgtgt tactgatgtg ttgctgaaga 3360
   ttaagatatt tttaagtctc accgaaaagg tagaaggagc caactgagac acaaaaaggg 3420
   gctgaggttc tattcatggt gagcaagtct ttttttttgt ttgtttcttc aagctctaac 3480
   aagggtgcct actacatggc ttttcagtta gccccaaaat aagatgtaac aatttttttt 3540
   tctattctta ggctttatct acaaagaaat gaattggata atcttcataa acaaaaaaca 3600
   tggaaaattt atcaaccaga atatgcagta gagatatatt ttaatgagaa atgacttaag 3660
   ttatgttgta actggtagct gattaagtat agttccctgc accccttctg ggaaagaatt 3720
   atgttctttc taaccctgcc acatagttat atgttctaaa tcttccttgc tggtacatct 3780
   atattgatat atgtatacac atgttcttta taaatctatt aaatatatac agaaaaaaaa 3840
   aaaaaaaa 3848
<210> 11
   <211> 1756
   <212> DNA
   <213> Homo sapiens
<400> 11
   gcttgggggc ggaaaagccg tggcgccccc ttgcgtggcg cgtcggtctc agagtcgcgt 60
   gacttcaacc ccctcttcgg gaggctgggt cgtcatgatc cggaccccat tgtcggcctc 120
   tgcccatcgc ctgctcctcc caggctcccg cggccgaccc ccgcgcaaca tgcagcccac 180
   gggccgcgag ggttcccgcg cgctcagccg gcggtatctg cggcgtctgc tgctcctgct 240
   actgctgctg ctgctgcggc agcccgtaac ccgcgcggag accacgccgg gcgcccccag 300
   agccctctcc acgctgggct cccccagcct cttcaccacg ccgggtgtcc ccagcgccct 360
   cactacccca ggcctcacta cgccaggcac ccccaaaacc ctggaccttc ggggtcgcgc 420
   gcaggccctg atgcggagtt tcccactcgt ggacggccac aatgacctgc cccaggtcct 480
   gagacagcgt tacaagaatg tgcttcagga tgttaacctg cgaaatttca gccatggtca 540
   gaccagcctg gacaggctta gagacggcct cgtgggtgcc cagttctggt cagcctccgt 600
   ctcatgccag tcccaggacc agactgccgt gcgcctcgcc ctggagcaga ttgacctcat 660
   tcaccgcatg tgtgcctcct actctgaact cgagcttgtg acctcagctg aaggtctgaa 720
   cagctctcaa aagctggcct gcctcattgg cgtggagggt ggtcactcac tggacagcag 780
   cctctctgtg ctgcgcagtt tctatgtgct gggggtgcgc tacctgacac ttaccttcac 840
   ctgcagtaca ccatgggcag agagttccac caagttcaga caccacatgt acaccaacgt 900
   cagcggattg acaagctttg gtgagaaagt agtagaggag ttgaaccgcc tgggcatgat 960
   gatagatttg tcctatgcat cggacacctt gataagaagg gtcctggaag tgtctcaggc 1020
   tcctgtgatc ttctcccact cagctgccag agctgtgtgt gacaatttgt tgaatgttcc 1080
   cgatgatatc ctgcagcttc tgaagaagaa cggtggcatc gtgatggtga cactgtccat 1140
   gggggtgctg cagtgcaacc tgcttgctaa cgtgtccact gtggcagatc actttgacca 1200
   catcagggca gtcattggat ctgagttcat cgggattggt ggaaattatg acgggactgg 1260
   ccggttccct caggggctgg aggatgtgtc cacataccca gtcctgatag aggagttgct 1320
   gagtcgtagc tggagcgagg aagagcttca aggtgtcctt cgtggaaacc tgctgcgggt 1380
   cttcagacaa gtggaaaagg tgagagagga gagcagggcg cagagccccg tggaggctga 1440
   gtttccatat gggcaactga gcacatcctg ccactcccac ctcgtgcctc agaatggaca 1500
   ccaggctact catctggagg tgaccaagca gccaaccaat cgggtcccct ggaggtcctc 1560
   aaatgcctcc ccataccttg ttccaggcct tgtggctgct gccaccatcc caaccttcac 1620
   ccagtggctc tgctgacaca gtcggtcccc gcagaggtca ctgtggcaaa gcctcacaaa 1680
   gccccctctc ctagttcatt cacaagcata tgctgagaat aaacatgtta cacatggaaa 1740
   aaaaaaaaaa aaaaaa 1756
<210> 12
   <211> 1009
   <212> DNA
   <213> Homo sapiens
<400> 12
   gggcagaggc caagtgggca ccggatagcg ccagccccgc ccagagagcg aaatcatgga 60
   gccttccaag accttcatga gaaacctgcc aatcacacca ggctatagcg gctttgtgcc 120
   attcctcagc tgccaaggaa tgtccaagga ggatgacatg aaccactgtg tgaaaacctt 180
   ccaggagaaa acacagcgct ataaagaaca gctgcgggaa ttgtgctgcg cagtggccac 240
   tgccccgaaa ctgaaacctg tcaactccga ggagacggtc ctgcaggccc tgcaccagta 300
   caatctgcag taccaccccc tgatcctgga atgcaaatat gtaaagaaac ctctccagga 360
   gcccccgatc cctggctggg caggctacct gccgagagcc aaggtcactg aatttggctg 420
   tggcacgaga tacactgtca tggccaaaaa ctgctacaag gacttcctgg agatcacgga 480
   gagggccaag aaggcacatc tgaaaccata tgaagagtga ggagaaatgt ctctttcctt 540
   cctactaccg ttttaaaaag gggatgaaat gtttgcagtg gcctttctgc ttagctgggc 600
   cagctccctg caactcacac ggacggttcc tctcctagat ggaagctgcc ctgcccttgg 660
   aaggcccctg agagaggacc ccaaaactcc gctgacatgt ggctgtgctc agaggccaag 720
   tataccatgc agtgggaaga tgtatctaga gccactgtcc tccgcaaagt atgcagaagg 780
   ctagaagcgc agagtctccc aaggaggtga actttaagtg gggcttccaa aacctgccat 840
   tctcatgttg gaatcacgcc cagtgagcaa taaagaaatt tagtaacaag aattttttaa 900
   ctgccgcctg catcctgagt ggttgacggt tgcatgtcat taatgataaa gaccgttttt 960
   tgtcatgtgg gaataaagag gctgcttctc cgcaaaaaaa aaaaaaaaa 1009
<210> 13
   <211> 3185
   <212> DNA
   <213> Homo sapiens
<400> 13
   aaaaaagttt gggattccca gtctacaaaa ccccacagct cctaggaatt ctctcaccac 60
   ccttgtgcct ttaggcttcg gtagattgca aatgacctgc tttctttcgg atcccgggct 120
   gctttcggac acctgtcgaa tagtaaatcc caagtaaggt acctgcggtc gtcggcagat 180
   ctgaattttc ttcttggaca cctaataccc acagtcctcc agagaccgag aggttgatgt 240
   cactcccaat atcggaggaa gtatacaccc ccgtgtgaga tggtccttaa taatattcca 300
   cggcggaggg ggtgatatga ctacatacat ggcagaaagt ggaaacctcc cagggatatt 360
   gttcccacga tcctggaggg aagaagatga tgttactttc aatatgacag aaggtggatg 420
   aagtggtgga ctgcccctcc acacctgtgg acacgccccc actgatattc cttctaattg 480
   cagcgtggga gaggaggata tgacacgcga tatcgcaggg agtagaaaca cccctgtgat 540
   actgttctta atattcaggg aggatgagga tgatattact cccaatacag acgggtgtac 600
   accctctgta caccgagggt gtacacctgt ctgtgaaaga gttcgtaatc tccagagggg 660
   gagatgatat tactcacaat atggtaaaga ggctgtgagt ccacggagga tcctcagagc      720
   cagcggggga agaggggctg gctctcagtc cccgcctcgc gggggtgact ccccccagtg      780
   cgatgggggt cctaagagcc agtgggggaa gaggggctgg ctctgagacc ccgcctcgtg 840
   gggggtgcct ccccgccctg tgatgggggt ccgaagagcc agaaggctta gaggggctgg      900
   ctctcagtcc ccgcctcgcg gggggtgcct cctccccctg cgatgggggt cgtaagagcc      960
   actgggggaa taggggcttg ctctcagtca ccgcctcgcg gggggtgcct cctttccttt 1020
   ctacatagac acagtgacag tctgatctct ctttcttttc cctacagatg gacacgcccc 1080
   cactgatatt gtttttaatg cagcgtggga ggggaggata tgacacgcga tatcgcaggt 1140
   agtagaaaca cccctttgat agtgttctta atattcaggg aggaagaaga tgatgttact 1200
   cccaatacag acggatgtac accctctgta caccgagggt gtacacccgt ctgtgaagga 1260
   gttcgtaatc tccagaggtg gagatgatat tactcacaat atggtaaaca ggctgtgagt 1320
   ccatcgcgga tcctcagagc caggtgggga agaggggctg gctgtcagtc ccctcctcgc 1380
   ggggggtgcc tcccccactg ctatggggat cccaagagcc agtgggggaa gaggtgctgg 1440
   ctctcagtct ccgcctcgcg aggtgcctct ccaccctgcg atcggggtcc gaagagccag 1500
   gggggaagag gggctggctc tcttcgtgga tgattctttt tccattctca ggcagttttc 1560
   tttttctttt ctttcttttt tttttttttt gagactgagt cttgctctgt tgcccatgct 1620
   ttgctcgatc tcgggtgact gcaaccactg cctcccaggt tcaagagatt ctcctgcctc 1680
   agcctcctga gtagctggga ctagaggcgt gtgtcaccac acccagctaa tttttgtatt 1740
   tttagtagag atggggtttc accatgtttg ccaggatggt ctctatctcc tgcccatgtg 1800
   atccacccac ctcagcctcc caaagtgctg ggattgcagg tgcgagccac cgggtccagc 1860
   ctctcaggcg attttcatac ctgcatactc tggtcactac tctgttaaac agtcaaggag 1920
   ggtaagtatt atcttcagat ttccagagct ctgtctctgt acagccctct cctcctcaat 1980
   attctgccct atgaattcta gccacattgg ccttcccagg ctcacagttc tgtcttctca 2040
   actcaggaag atctctgagt tccatctgca ttctttcttc ctgtgctgtg gcctggaaag 2100
   ttttctaagg tgttagggag gtcaattgtg gggctagcct catttgtttc tcatctcttg 2160
   aggatcactg ccctttgatg cttgattcca gtgattgatt ccctttgttg cttgagggcc 2220
   atagtttcat atattttgtc cagtagtttt gttgttttag gtcagaaagt aattttggtc 2280
   tctgttactc tatcttggcc agaagtgtaa gacctaagca tttacacatc aaaatactgc 2340
   acacataatt ttagtttaag ctacttttta aaaaatctcc ttcattttcc atttagcatt 2400
   ctatttaggg tattacattg gtttttttga aattctgtta ttggcagttt ctattgccta 2460
   tcaatcccat ttaaagatag tgcatagggt attctaaaat agctgttaag caaagagaaa 2520
   attgggcctg atagggtgag aatcacagct ctaataccta gagtgacctt ataatgtatt 2580
   gtccaaagga gatatttttg acagtgaaag agggtgttgt tagtaattat atcaggacca 2640
   tggcctaaac caggactatc ccaggaagcc tgggacatat ttgtacccca tctctattta 2700
   atgcctttat acaattcttt acttaattct accagccttt attgagcctg ctttctttgt 2760
   ctagctgagt gccacgtgct gacgtcacta agatcaatac agcaaactct gaaagatgga 2820
   cagagagaca ggagatggtc ctttataatg cagtgtgatc tgtgctgcaa tagagttttg 2880
   gaagctagaa gttcaaaacg aggtgttggc agcaccatgc tctctctgaa gatgctagga 2940
   agaatctgct ccatgccttt ccattcgctt ctggggtttc ctgcaagccc tgacattcct 3000
   tggcttgtag atgcatcacc ccagtttccg cccccatcat cacatggcct cctctctgtg 3060
   tgtgcctctg cgttccctct attcttcttc taaggacacc gacaccagtc atagtggatt 3120
   aagtgtccac ccctaaccaa ttacatctgc aacaacccta tttccaaata aagtcacatt 3180
   ctaag 3185
<210> 14
   <211> 2106
   <212> DNA
   <213> Homo sapiens
<400> 14
   agcggatcgt ctcttgccgc tgccatgaaa ggagtgcttt ttgcctccca ccatgattct 60
   gaggcctccc cagccatgta gaattagcca gcctgttttt gatgaagagc aactgtactg 120
   gagagaaagc ctgtattcat attcatcgtc atcaggcaaa tcgaggagct gacttgctta 180
   ggatcatctg ctggttgaat ccatggatgc agaaccccag attcggaggg ctgaatgtac 240
   tttcgatagg ttcctgagca gcagtgatta tagtcacagc agactggaca gtttggtgag 300
   caagttcagg ggctggctta gtgattcata gttctttctt cctcctcccc cagcagtttt 360
   gtttcagcag tgatttggtc aaatgtgaac cacaaaccag cagcaaaagc attttctagg 420
   aacttgttaa aactggaagg cccaaggttg cactcaggct tactgagtca gtcactccaa 480
   gtggacccca tccctaaacc ttgagactca ctgctcctct tttgatctgg gagagaaaca 540
   gaaaaaagaa ggaaaaaaaa tccccaatgt gagtatgctg gaaagtaaat ttgaatagct 600
   ttttatattg ggtacttact gtgggtaatt tacaaactat tggaaggaac cacaaattca 660
   aacacagcca aatttaaggt ctctttagca gaaatcaatc ttacatcatg gcagttctcc 720
   cattctccag tctttcactt gatagatcac ttcctcatta ggatgaccta cagcaattct 780
   acatgaactg ctctttacat ttaaacagtg ctttctgtcc taacatgtca aaatgcttga 840
   caagcaatca aaaggtagct cactaatttt ttcaactcat ggcagtaaat agcctgcatc 900
   taccatcact gctgtttact ggtgatttct tggcagggaa actttaaatg ccttagcaat 960
   gtctagaaag tcaatgacta aagaaaatat tcattttggc ttccataggg ctgtatttga 1020
   tgaactgctc actaacttct attattacgt ctcaagaata gataatgata ctcagcaagg 1080
   gcaaatccca aagaatagtc tatgctttcc ttgattgaat ctgatttttg agaaattaca 1140
   tggcccaatc tacatttaat atgatacaaa tgctacttaa ctccctgtta tttctggttg 1200
   catataattg atactacttg aagagtaaga agcagagtct cactacactg gggacccttt 1260
   gagagccaag tctgtgtctt agtcatcttt gatgccttag tgaccagtac catacctgga 1320
   atagtatatg tgatcagtaa ctcaaatgta tgtatttctt gaatgtatgg tgcatgaatg 1380
   agtcaagata cctttagcgt tggaagcaat ctgtcatgga gtaggaggag aaaaagtagt 1440
   gaaaaaactt ctaaattctc aaaacctagt gctgaactga atgctgataa taacttgaca 1500
   acgtcctttg gcattgcaga tgttgagata ccaacatcta atccgccaag aaagaatttc 1560
   agaattaaaa tttgggtgtg ttcttgcgct ggggatttct ctgacctacc ttctgataga 1620
   actttgaacc cgagtcaata gtcaatatac tagtttttta cttaaaactg taacatttta 1680
   atctaatttt ggggacgtga aataaaacta atgggaaact cttgaaattt ttatcactgg 1740
   aataacctaa ttttgaaaac actgatggtt agtttcttga aatattaata ttactacaag 1800
   tcataagtaa aagcattcta tcttaagtga gaaactataa agttggataa ttactatttg 1860
   agtttgtggc ttggtttgaa taaacacttg cttgttttaa gtaaaagttc agctgaagtg 1920
   acaatcaacc tttaatcttg taaagcttct gtgttagata ttttctatct ctaacatgcc 1980
   aaacatgcat attaaactga gtttttttgc atgcaaaaaa aaaaaaaaaa aaaaaaaaaa 2040
   aaaaaaaaaa aaaaaaaaaa aaaaaaaaaa aaaaaaaaaa aaaaaaaaaa aaaaaaaaaa 2100
   aaaaaa 2106
<210> 15
   <211> 459
   <212> DNA
   <213> Homo sapiens
<400> 15
   atggctcgta cgaagcaaac agctcgcaag tctaccggcg gcaaagctcc gcgcaagcag 60
   cttgctacta aagcagcccg taagagcgct ccggccaccg gtggcgtgaa gaaacctcat 120
   cgctaccgcc cgggcaccgt ggccttgcgc gaaatccgtc gctaccagaa gtccaccgag 180
   ctgctgatcc ggaagctgcc gttccagcgc ctggtgcgag aaatcgccca ggacttcaaa 240
   accgacctgc gtttccagag ctctgcggtg atggcgctgc aggaggcttg tgaggcctac 300
   ctggtgggac tcttcgaaga caccaatctg tgcgctattc acgctaaacg cgtcaccatc 360
   atgcccaaag atatccagct ggcacgtcgc atccgtgggg aaagggcata agtctgcccg 420
   tttcttcctc attgaaaagg ctcttttcag agccactca 459
<210> 16
   <211> 3591
   <212> DNA
   <213> Homo sapiens
<400> 16
   ttactgggcg tatggcgtac agacacgagg ccggcgcccg ggaggcggtg ttcatccgcc      60
   cgggaaaaga gcgcctgttg ctcgctgccc gcgtgtccct ggctctctcg ggaacccagc     120
   gccgaaggcg aggtgggcgc gggccgaagg aggtcctggg aggtcggcgg cgcggaggga     180
   tctccgcggg agccgttggg gctgttggcc tcgggctgag gtgcaaggac caggactagg     240
   gcgagggcag cggtccaaga aatagaaaac aatgactggg agagcccgag ccagagccag     300
   aggaagggcc cgcggtcagg agacagcgca gctggtgggc tccactgcca gtcagcaacc     360
   tggttatatt cagcctaggc ctcagccgcc accagcagag ggggaattat ttggccgtgg      420
   acggcagaga ggaacagcag gaggaacagc caagtcacaa ggactccaga tatctgctgg 480
   atttcaggag ttatcgttag cagagagagg aggtcgtcgt agagattttc atgatcttgg 540
   tgtgaataca aggcagaacc tagaccatgt taaagaatca aaaacaggtt cttcaggcat 600
   tatagtaagg ttaagcacta accatttccg gctgacatcc cgtccccagt gggccttata 660
   tcagtatcac attgactata acccactgat ggaagccaga agactccgtt cagctcttct 720
   ttttcaacac gaagatctaa ttggaaagtg tcatgctttt gatggaacga tattattttt 780
   acctaaaaga ctacagcaaa aggttactga agtttttagt aagacccgga atggagagga 840
   tgtgaggata acgatcactt taacaaatga acttccacct acatcaccaa cttgtttgca 900
   gttctataat attattttca ggaggctttt gaaaatcatg aatttgcaac aaattggacg 960
   aaattattat aacccaaatg acccaattga tattccaagt cacaggttgg tgatttggcc 1020
   tggcttcact acttccatcc ttcagtatga aaacagcatc atgctctgca ctgacgttag 1080
   ccataaagtc cttcgaagtg agactgtttt ggatttcatg ttcaactttt atcatcagac 1140
   agaagaacat aaatttcaag aacaagtttc caaagaacta ataggtttag ttgttcttac 1200
   caagtataac aataagacat acagagtgga tgatattgac tgggaccaga atcccaagag 1260
   cacctttaag aaagccgacg gctctgaagt cagcttctta gaatactaca ggaagcaata 1320
   caaccaagag atcaccgact tgaagcagcc tgtcttggtc agccagccca agagaaggcg 1380
   gggccctggg gggacactgc cagggcctgc catgctcatt cctgagctct gctatcttac 1440
   aggtctaact gataaaatgc gtaatgattt taacgtgatg aaagacttag ccgttcatac 1500
   aagactaact ccagagcaaa ggcagcgtga agtgggacga ctcattgatt acattcataa 1560
   aaacgataat gttcaaaggg agcttcgaga ctggggtttg agctttgatt ccaacttact 1620
   gtccttctca ggaagaattt tgcaaacaga aaagattcac caaggtggaa aaacatttga 1680
   ttacaatcca caatttgcag attggtccaa agaaacaaga ggtgcaccat taattagtgt 1740
   taagccacta gataactggc tgttgatcta tacgcgaaga aattatgaag cagccaattc 1800
   attgatacaa aatctattta aagttacacc agccatgggc atgcaaatga gaaaagcaat 1860
   aatgattgaa gtggatgaca gaactgaagc ctacttaaga gtcttacagc aaaaggtcac 1920
   agcagacacc cagatagttg tctgtctgtt gtcaagtaat cggaaggaca aatacgatgc 1980
   tattaaaaaa tacctgtgta cagattgccc taccccaagt cagtgtgtgg tggcccgaac 2040
   cttaggcaaa cagcaaactg tcatggccat tgctacaaag attgccctac agatgaactg 2100
   caagatggga ggagagctct ggagggtgga catccccctg aagctcgtga tgatcgttgg 2160
   catcgattgt taccatgaca tgacagctgg gcggaggtca atcgcaggat ttgttgccag 2220
   catcaatgaa gggatgaccc gctggttctc acgctgcata tttcaggata gaggacagga 2280
   gctggtagat gggctcaaag tctgcctgca agcggctctg agggcttgga atagctgcaa 2340
   tgagtacatg cccagccgga tcatcgtgta ccgcgatggc gtaggagacg gccagctgaa 2400
   aacactggtg aactacgaag tgccacagtt tttggattgt ctaaaatcca ttggtagagg 2460
   ttacaaccct agactaacgg taattgtggt gaagaaaaga gtgaacacca gattttttgc 2520
   tcagtctgga ggaagacttc agaatccact tcctggaaca gttattgatg tagaggttac 2580
   cagaccagaa tggtatgact tttttatcgt gagccaggct gtgagaagtg gtagtgtttc 2640
   tcccacacat tacaatgtca tctatgacaa cagcggcctg aagccagacc acatacagcg 2700
   cttgacctac aagctgtgcc acatctatta caactggcca ggtgtcattc gtgttcctgc 2760
   tccttgccag tacgcccaca agctggcttt tcttgttggc cagagtattc acagagagcc 2820
   aaatctgtca ctgtcaaacc gcctttacta cctctaacct gcagaagacg atgcagccgc 2880
   ttttcttttt gaaatgactt tgggattttt ttaagctttt atttactttt tttttaactg 2940
   ttatctttct ggatgaaact tgggaagggg attaggagat ctagcatttt atttctagca 3000
   ttgctattca ccggcttcct tattttatac gtaaaaatta agattttata ttttatcttc 3060
   ttgtttctca tagatatttt gtgagcattt ttttgtttat tttgaagaaa tgtggataag 3120
   atacttggta gtataaaaca gactctctga gagtatttga aatgtgtttg gagatttact 3180
   taaacgtact ttcaggagtg agcaagtcct acttataaac ctatattaac tttatttttg 3240
   agatacctgt tttgaattta aaggagataa gaggcgtaaa gtaggatgct cactacaacc 3300
   ataggtgggg tttcagctca tatcttaaag ataaaaggta ctattatata acctatacac 3360
   aagatacagg agaaaatatg cttgattttt atttggcagg ggggctaggt tgtatgggag 3420
   taaaaaaaac attgaaaatt tttaaattgt ccaaagaaac attttaagac tctttaacaa 3480
   aaaaggccat gagtaaatct ctatattaac attactattt attttgtttt ggaactggga 3540
   catgattcta tttgttataa aataaaattg atgtgattgt caccttattt g 3591
<210> 17
   <211> 819
   <212> DNA
   <213> Homo sapiens
<400> 17
   gtgatgctcc ctgggcctcc tgaccgcgcc ctcgcctggg aggcggggcg ggccgggttc 60
   tctctgtgac gtcacaaagg ccccgccatg cctctggctt tgacccttct gctgctctcg 120
   ggcttgggcg cccccggagg ctggggctgc ctgcagtgcg accccttggt gctggaggcc 180
   ctgggtcacc tgcgctccgc cctcatcccc agtcgcttcc agttggagca gctgcaggcg 240
   cgcgccgggg ccgtgctgat gggcatggag gggcctttct tccgggacta cgcgctgaac 300
   gtgtttgtgg ggaaagtgga gacaaatcaa ctggaccttg tggcgtcctt tgtcaagaac 360
   caaacgcagc acttaatggg taactctctg aaagatgagc ctctgctgga agagctggtg 420
   accctcaggg cgaatgtgat caaggaattc aagaaagttt taatttcata tgaattaaaa 480
   gcctgcaacc ccaaactttg ccgcttgcta aaagaagagg tgttggactg tttacattgc 540
   cagaggatca ctcccaagtg tatccacaaa aagtactgct ttgtcgaccg gcaaccccgc 600
   gtggccctgc agtaccagat ggacagcaaa tacccgagga accaggcgct gttgggcatc 660
   ctcatttctg tgtctctggc tgtctttgtc ttcgtggtca tcgtggtctc ggcttgtaca 720
   tacagacaaa accgaaaact cctgctgcag taggacggtg gtttgggggt aaggagaaag 780
   gaaaataaat ttaataaaat tggtgacaaa tccaaaaaa 819
<210> 18
   <211> 5120
   <212> DNA
   <213> Homo sapiens
<400> 18
   ggactcgcac tcggcggttg ttccagaaga aagagacagc gatggcggca gaggcttcga 60
   agactgggcc ttctaggtct tcctaccagc gaatggggag gaagagtcag ccctggggtg 120
   ccgctgaaat ccagtgcacc aggtgtggaa ggagggtatc cagatcatcc ggtcaccatt 180
   gtgaacttca atgtggacat gctttttgtg aactatgctt gttaatgact gaagaatgca 240
   ccacaattat atgccctgat tgtgaggttg ctacagctgt aaatactaga caacgctact 300
   acccaatggc tggatatatt aaggaagact ccataatgga aaaactgcag cctaagacga 360
   taaagaattg ttctcaggac tttaagaaga ctgctgatca gctaactact ggtttagaac 420
   gttcagcctc cacagacaag actcttttga actcatcagc tgtaatgttg gacactaata 480
   ctgcagaaga aattgatgaa gcattgaata cagcacacca tagtttcgaa cagttaagca 540
   ttgctggaaa agcacttgaa cacatgcaga agcaaacgat agaggaaaga gaaagagtta 600
   tagaagttgt ggagaaacag tttgaccaac ttttggcttt ttttgattcc aggaaaaaga 660
   acctgtgtga agaatttgca agaactactg atgattatct atcaaattta ataaaggcta 720
   aaagctacat tgaagagaaa aaaaataatt tgaatgcagc tatgaacata gcaagagcat 780
   tacaattatc gccttctcta agaacatact gtgacctgaa tcagattatc cggactttgc 840
   agttaacttc agatagtgaa ttagcacaag ttagttctcc acaactaagg aaccctccca 900
   ggttgagtgt gaattgcagt gagatcatct gtatgttcaa caatatggga aagattgaat 960
   ttagggactc aacaaaatgt tatccccaag aaaatgaaat tagacagaat gttcaaaaga 1020
   aatataataa caaaaaggaa ctttcttgtt acgatacata cccaccgcta gaaaagaaaa 1080
   aggttgacat gtctgtccta accagtgaag caccaccacc tcctttgcaa cctgagacaa 1140
   atgatgtaca tttagaagca aaaaacttcc agccacagaa agacgttgca acagcatccc 1200
   ctaaaaccat tgctgtgtta cctcagatgg gatctagccc tgatgtgata attgaagaaa 1260
   ttattgaaga caacgtggaa agttctgcag agctagtttt tgtaagccat gtaatagatc 1320
   cttgccattt ctacattcgg aagtattcac aaataaaaga cgccaaagta ctggagaaga 1380
   aggtgaatga attttgcaat aggagttcac accttgatcc ttcagacatt ttggaactag 1440
   gtgcaagaat atttgtcagc agtattaaaa atggaatgtg gtgtcgagga actatcacag 1500
   aattaattcc aatagagggt agaaatacca gaaaaccttg tagtccaacc agattatttg 1560
   tccatgaagt tgcactaata caaatattca tggtagattt tggaaattct gaagtcctga 1620
   ttgtcactgg agttgttgat acccatgtga gaccagaaca ctctgctaag caacatattg 1680
   cactaaatga tttatgtctg gttctaagga aatctgaacc atatactgaa gggctgctaa 1740
   aagacatcca gccattagca caaccatgct cattgaaaga cattgttcca cagaattcaa 1800
   atgaaggctg ggaagaggaa gctaaagtgg aatttttgaa aatggtaaat aacaaggctg 1860
   tttcaatgaa agtttttaga gaagaagatg gtgtgcttat tgtagatctg caaaaaccac 1920
   caccgaataa aataagcagt gatatgcctg tgtctcttag agatgcgcta gtttttatgg 1980
   aactagcaaa gtttaagtca caatcactaa gaagtcactt tgaaaaaaat actactttac 2040
   actatcatcc acctattttg cctaaagaaa tgacagatgt ttcagtaacg gtttgtcata 2100
   taaatagtcc tggagatttc tatcttcagt tgatagaggg cctggatatt ttatttctat 2160
   taaagacaat cgaggaattc tataaaagtg aagatggaga aaatctggaa atcctctgtc 2220
   cagttcaaga tcaagcctgt gtagctaaat ttgaagatgg aatttggtac cgagcaaaag 2280
   ttatcggatt gcctggacat caggaagttg aagttaaata tgtggacttt ggtaatactg 2340
   caaaaataac aatcaaagac gtgcgtaaaa taaaggatga gtttctgaat gccccagaga 2400
   aggcaattaa atgtaagttg gcctatattg aaccatataa aaggacaatg cagtggtcca 2460
   aagaagctaa agaaaaattt gaagaaaagg ctcaagataa atttatgaca tgttcagtta 2520
   tcaaaattct ggaagataat gtgctcttag ttgagctttt cgattctctt ggtgctcctg 2580
   aaatgactac tactagtatt aatgaccagc tagttaaaga gggcctagca tcttatgaaa 2640
   taggatacat cctcaaagat aattctcaaa agcatattga agtttgggat ccttctccag 2700
   aagaaattat ttcaaatgaa gtacacaact taaatcctgt gtctgcaaaa tctctaccta 2760
   atgagaattt tcagtcactt tataataagg aattgcctgt gcatatctgt aatgtaatat 2820
   ctcctgagaa gatttatgtt cagtggttgt taactgaaaa cttacttaat agtttagaag 2880
   aaaagatgat agctgcttat gaaaactcaa aatgggaacc tgttaaatgg gaaaatgata 2940
   tgcactgtgc tgttaagatc caagataaaa atcagtggcg aagaggccag atcatcagaa 3000
   tggttacaga cacattggta gaggtcttgc tgtatgatgt gggtgttgaa ctagtagtga 3060
   atgttgactg tttaagaaaa cttgaagaaa atctaaagac aatgggaaga ctctctttgg 3120
   aatgttctct ggttgacata agaccagctg gtgggagtga caagtggaca gcaacagctt 3180
   gtgactgtct ttcattgtac ctgactggag ctgtagcaac tataatctta caggtggata 3240
   gtgaggaaaa caacacaaca tggccattac ctgtgaaaat tttctgcaga gatgaaaaag 3300
   gagagcgtgt tgatgtttct aaatatttga ttaaaaaggg tttggctttg agagaaagga 3360
   gaattaataa cttagataac agccattcat tatctgagaa gtctctggaa gtccccctgg 3420
   aacaggaaga ttcagtagtt actaactgta ttaaaactaa ctttgaccct gacaagaaaa 3480
   ctgctgacat aatcagtgaa cagaaagtgt ctgaatttca ggagaaaatt ctagaaccaa 3540
   gaaccactag agggtataag ccaccagcta ttcctaacat gaacgtattt gaggcaacag 3600
   tcagctgtgt tggtgatgat ggaactatat ttgtagtacc taaactatca gaatttgagc 3660
   taataaaaat gacaaatgaa attcaaagta atttaaaatg ccttggtctt ttggagcctt 3720
   atttctggaa aaaaggagaa gcatgtgcag taagaggatc cgatactctg tggtatcgtg 3780
   gcaaggtgat ggaggttgta ggtggcgctg tcagagtaca atatttagat catggattca 3840
   ctgaaaagat tccgcagtgc catctttacc ctattttgct gtatcctgat ataccccagt 3900
   tttgtattcc ttgtcagctc cataatacca cacctgttgg gaatgtctgg caaccagatg 3960
   caatagaagt tcttcaacaa ctgctttcaa agagacaggt ggacattcac attatggagt 4020
   tacctaaaaa tccatgggag aaattgtcta ttcacctcta ttttgatgga atgtcacttt 4080
   cttattttat ggcatactat aaatactgta cttctgaaca tactgaggag atgttgaaag 4140
   aaaaaccaag atcagatcat gataaaaagt atgaagagga acaatgggaa ataaggtttg 4200
   aggaattgct ttcggctgaa acagacactc ctcttttacc accatatttg tcttcatctc 4260
   tgccttcccc aggagaactc tatgctgttc aagttaagca cgttgtctca cctaatgaag 4320
   tgtatatttg ccttgattct atagaaactt ctaaccagtc taaccagcat agtgacacag 4380
   atgatagtgg agtcagcggg gaatcagaat ccgagagcct tgatgaagca ctgcagaggg 4440
   ttaataagaa ggtagaggcg cttcctcctc tgacggattt tagaacagaa atgccttgcc 4500
   ttgcagaata tgatgatggc ttatggtata gagcgaagat tgttgccatt aaagaattta 4560
   atcctttatc tatcttagta caatttgttg attatggatc aactgcaaag ctgacattaa 4620
   acagactgtg ccaaattcct tctcatctta tgcggtatcc agctcgagcc ataaaggttc 4680
   tcttggcagg gtttaaacct cccttaaggg atctagggga gacaagaata ccatattgtc 4740
   ccaaatggag catggaggca ctgtgggcta tgatagactg tcttcaagga aaacaactct 4800
   atgctgtgtc catggctcca gcaccagaac agatagtgac attatatgac gatgaacagc 4860
   atccagttca tatgccgttg gtagaaatgg ggcttgcaga taaagatgaa taagtgccta 4920
   agtgtataca gtgagagcat ctatagaagc ctagaagaat tctgttatgt ttagactatg 4980
   tcttatcttt agactatttc aggcttaatt ttcctaactt gttcagcact agtgctttac 5040
   ctctcatttt taattgaact gttaggaatt gtgtggggaa aaaaagtaaa taaatgttcg 5100
   cttccaaaaa aaaaaaaaaa 5120
<210> 19
   <211> 1738
   <212> DNA
   <213> Homo sapiens
<400> 19
   gatgtcatca ggctttgtga gggggactgt actgcccttt gagtactact gtgtggcccc 60
   gcaacccagc acaaccaggt atctgcttgg aacccagcca ccataaagcc tgctagctaa 120
   aaaaaatttt acatctctca gttcattcgg cacagacccc tgcctcattc agctgtgact 180
   ctgcttggaa aattcatcag ttacaaagca gccaatgcaa ttatctcaag ggaaattgaa 240
   aaatggacct ttgaaaatgc tagatttaca atgagaaatg ccataattca aggtttattc 300
   tatgggtcct tgacatttgg gatctggaca gctctgttat tcatatattt gcaccataat 360
   catgtgagca gctggcagaa gaaaagccag gagcctctgt cagcttggtc ccctggaaaa 420
   aaagtgcatc agcaaattat ctatggctca gagcaaatac caaaacctca tgtaatagtc 480
   aaaaggactg atgaagataa agcaaagtct atgttaggta cagattttaa ccatacaaac 540
   ccagaacttc ataaagaact tttaaaatat ggatttaatg tgattatcag tagaagcttg 600
   ggcatcgaaa gagaagtgcc agataccagg agtaaaatgt gtcttcaaaa acattaccca 660
   gcccgcctcc cgactgccag cattgtcatt tgcttctata atgaagaatg taatgccttg 720
   tttcagacca tgtccagtgt cacgaacctc acgccacact attttcttga agaaattatt 780
   ttggtagatg acatgagcaa agttgatgat ttgaaagaaa aactagacta tcacctggaa 840
   acttttcggg gaaaggttaa aataataaga aacaaaaaga gagaggggct gattcgagca 900
   aggctgattg gagcttctca tgcttcaggg gatgttctgg tgttcctgga cagccactgt 960
   gaggtgaaca gagtatggct ggagcccctg ctgcatgcca ttgccaagga ccccaaaatg 1020
   gtggtgtgcc ccctgataga tgtcattgat gatagaactc tggagtataa gccctctcct 1080
   cttgtaaggg gaacttttga ttggaaccta caatttaaat gggataatgt tttctcttat 1140
   gagatggatg gaccagaagg atctactaaa ccaatccggt cacctgcaat gtctggagga 1200
   atttttgcta tacgtcggca ttattttaat gaaattggac agtatgacaa ggatatggat 1260
   ttttggggaa gagaaaattt ggaactttca ctaaggatct ggatgtgtgg aggccaactc 1320
   tttataatcc cctgctctcg agtaggacat atcagtaaga aacaaactgg aaaaccttct 1380
   acaatcatca gtgctatgac acataactac ctaagactgg tgcacgtttg gctggatgaa 1440
   tataaggagc agttttttct tcgaaagcct ggtctgaaat atgtcaccta cggaaatatt 1500
   cgcgagcgtg ttgagttaag gaaacgactg ggttgcaagt catttcagtg gtatttggat 1560
   aatgtcttcc cagagttgga ggcatctgtg aacagcctgt gaaaggaaaa caaatcactt 1620
   tcattaataa agggttaaaa gtctcctagt cattcaacat agtgtcacaa gagtgtaagt 1680
   ttggaacatc gtggaattac gtgaaatgca attaaaaaaa tatgaccaga cgtgaaaa 1738
<210> 20
   <211> 3623
   <212> DNA
   <213> Homo sapiens
<400> 20
   tctagcacag gggatcccca aacatcagga cttttggggg gcgcctgtgc tgtccatggg      60
   aagagcatgc attgtgggtt actggaggaa cccgacatgg attccacaga gagctggatt     120
   gaaagatgtc tcaacgaaag tgaaaacaaa cgttattcca gccacacatc tctggggaat     180
   gtttctaatg atgaaaatga ggaaaaagaa aataatagag catccaagcc ccactccact     240
   cctgctactc tgcaatggct ggaggagaac tatgagattg cagagggggt ctgcatccct     300
   cgcagtgccc tctatatgca ttacctggat ttctgcgaga agaatgatac ccaacctgtc     360
   aatgctgcca gctttggaaa gatcataagg cagcagtttc ctcagttaac caccagaaga     420
   ctcgggaccc gaggacagtc aaagtaccat tactatggca ttgcagtgaa agaaagctcc     480
   caatattatg atgtgatgta ttccaagaaa ggagctgcct gggtgagtga gacgggcaag     540
   aaagaagtga gcaaacagac agtggcatat tcaccccggt ccaaactcgg aacactgctg     600
   ccagaatttc ccaatgtcaa agatctaaat ctgccagcca gcctgcctga ggagaaggtt     660
   tctaccttta ttatgatgta cagaacacac tgtcagagaa tactggacac tgtaataaga     720
   gccaactttg atgaggttca aagtttcctt ctgcactttt ggcaaggaat gccgccccac     780
   atgctgcctg tgctgggctc ctccacggtg gtgaacattg tcggcgtgtg tgactccatc     840
   ctctacaaag ctatctccgg ggtgctgatg cccactgtgc tgcaggcatt acctgacagc     900
   ttaactcagg tgattcgaaa gtttgccaag caactggatg agtggctaaa agtggctctc     960
   cacgacctcc cagaaaactt gcgaaacatc aagttcgaat tgtcgagaag gttctcccaa    1020
   attctgagac ggcaaacatc actaaatcat ctctgccagg catctcgaac agtgatccac    1080
   agtgcagaca tcacgttcca aatgctggaa gactggagga acgtggacct gaacagcatc    1140
   accaagcaaa ccctttacac catggaagac tctcgcgatg agcaccggaa actcatcacc    1200
   caattatatc aggagtttga ccatctcttg gaggagcagt ctcccatcga gtcctacatt    1260
   gagtggctgg ataccatggt tgaccgctgt gttgtgaagg tggctgccaa gagacaaggg    1320
   tccttgaaga aagtggccca gcagttcctc ttgatgtggt cctgtttcgg cacaagggtg    1380
   atccgggaca tgaccttgca cagcgccccc agcttcgggt cttttcacct aattcactta    1440
   atgtttgatg actacgtgct ctacctgtta gaatctctgc actgtcagga gcgggccaat    1500
   gagctcatgc gagccatgaa gggagaagga agcactgcag aagtccgaga agagatcatc    1560
   ttgacagagg ctgccgcacc aaccccttca ccagtgccat cgttttctcc agcaaaatct    1620
   gccacatctg tggaagtgcc acctccctct tcccctgtta gcaatccttc ccctgagtac    1680
   actggcctca gcactacagg agcaatgcag tcttacacgt ggtctctaac atacacagtg    1740
   acgacggctg ctgggtcccc agctgagaac tcccaacagc tgccctgtat gaggaacact    1800
   catgtgcctt cttcctccgt cacacacagg ataccagttt atccccacag agaggaacat    1860
   ggatacacgg gaagctataa ctatgggagc tatggcaacc agcatcctca ccccatgcag 1920
   agccagtatc cggccctccc tcatgacaca gctatctctg ggccactcca ctatgcccct 1980
   taccacagga gctctgcaca gtaccctttt aatagcccca cttcccggat ggaaccttgt 2040
   ttgatgagca gtactcccag actgcatcct accccagtca ctccccgctg gccagaggtg 2100
   ccctcagcca acacgtgcta cacaagcccg tctgtgcatt ctgcgaggta cggaaactct 2160
   agtgacatgt atacacctct gacaacgcgc aggaattctg aatatgagca catgcaacac 2220
   tttcctggct ttgcttacat caacggagag gcctctacag gatgggctaa atgactgcta 2280
   tcataggcat ccatatttaa tattaataat aataattaat aataataata aacccaacac 2340
   ccatccccca gaagacttta tctctataca ttgtaactca tgggctattc ctaagtgccc 2400
   attttcctaa tgaacatgag gatgggatca atgtgggatg aataaacttt agttcagaaa 2460
   caggacttac taaaagtcag tgggactggg tttctgtagc caagccagac ttgactgttt 2520
   ctgtagagca ctatctcggg caggccattc tgtgcctttt ccctctgttc catgactttg 2580
   ctttgtgttg gcaaccactt ctagtaagct actgattttc ctgttgacaa aatctcttta 2640
   gtcttgaagg atggatactg gagacagaat ctggtttgtg ttcttggatg ggcacataat 2700
   ttaccaagag cattcacctt gccatctgtc ttgtcattgt actgtacaag gaacagccct 2760
   cagacgtgtt ctgcacatcc cttcttcctg gtggtaccat ccctatttcc tggagcacca 2820
   gggctaaatg gggagctatc tggaaactct agattttctg tcatacccac atctgtcaca 2880
   gtacctgcat tgtcttggaa tgtaagcact gtcttgaggg aaggaagagg tctgttctgt 2940
   attgccttaa gttgattgag gtttgtagga gactggttct tctacataca aggatttgtc 3000
   ttaagtttgc acaatggcta gtgtcagcaa aaggcaggag agggtttttg tttttttttt 3060
   aagttctatg agaatgtgga tttatggcat tgagtatcac actcagctct gctgtgttaa 3120
   ctttgtgaaa ctggatggaa caaactttaa cttaccaagc accaagtgtg aaagtgactt 3180
   tcacggttcc ttcataaaac tataataata tccgacactt tgatagaaaa aaattcaaag 3240
   ctgtgccttt gagcctatac tatactgtgt atgtgtggaa ataaaaatgt attgtacttt 3300
   tggagaattt tttgtaggca tttttctgtc agatttgtag taatttgtga ggtttgttag 3360
   agattaatat aggttttctt tctgtattat aaaatgcacc aagcaattat ggtggaccta 3420
   ttaccctatg ggtaagaaat aaatggaaat atgacatcgg atgtttcagc aactgttctg 3480
   taaataaaat ctttgatcac accactcagt gtgataattg tgtctacagc taaaatggaa 3540
   atagttttat ctgtacagtt gtgcaagata tgaatggttt cacactcaaa taaaaaatat 3600
   tgaaacgaaa aaaaaaaaaa aaa 3623
<210> 21
   <211> 1099
   <212> DNA
   <213> Homo sapiens
<400> 21
   gctgcattac agacacagac ctgcaaacat ctatggttgt gacagagttt ctttctgaca 60
   cctgagtctt tctcctgctg cacggaaagc ttgctgggag gggcttggaa tctggcatga 120
   agccaaaggg catctctgag ttgcagcatt taaatgatcc cactcagaga ttcacacaga 180
   agactggaca caattccgaa gagctgccca gaaggagaga acaatgtcat cactacccag 240
   tggcagacac ctttcacccc agctacacaa gagggggcag atgtgtgagg atcactgcag 300
   tccaggagtt cgatgtttca gtgagctgtg attgcaccac tgcatatcag cctgggtgac 360
   agagcaagac cctatctcaa aaatacagaa aaatcatcaa ccacttgcag tcgtcgtaga 420
   aatcaatcat tccctccagt tatgtccctg acccacaggc ttcatttgtg caagtactgg 480
   ggctgtgctg tcagtaatgt gtgccgcttc tgggaaggac gtccattgcc cttgatgatt 540
   gtggtaccat acacactgcc tgtttccttg cctgttggtt cgtgcgtgat aatcacaggg 600
   acaccgatcc tcacttttgt caaggaccca cagctggagg tgaatttcta cactgggatg 660
   gatgaggact cagatattgc tttccaattc cgactgcact ttggtcatcc tgcaatcatg 720
   aacagttgtg tgtttggcat atggagatat gaggagaaat gctactattt accctttgaa 780
   gatggcaaac catttgagct gtgcatctat gtgcgtcaca aggaatacaa ggtaatggta 840
   aatggccaac gcatttacaa ctttgcccat cgattcccgc cagcatctgt gaagatgctg 900
   caagtcttca gagatatctc cctgaccaga gtgcttatca gcgattgagg gagatgatca 960
   gactcctcat tgttgaggaa tccctctttc tacctgacca tgggattccc agagcctact 1020
   aacagaataa tccctcctca ccccttcccc tacacttgat cattaaaaca gcaccaaact 1080
   tcaaaaaaaa aaaaaaaaa 1099
<210> 22
   <211> 1660
   <212> DNA
   <213> Homo sapiens
<400> 22
   ggtgcactag caaaacaaac ttattttgaa cactcagctc ctagcgtgcg gcgctgccaa 60
   tcattaacct cctggtgcaa gtggcgcggc ctgtgccctt tataaggtgc gcgctgtgtc 120
   cagcgagcat cggccaccgc catcccatcc agcgagcatc tgccgccgcg ccgccgccac 180
   cctcccagag agcactggcc accgctccac catcacttgc ccagagtttg ggccaccgcc 240
   cgccgccacc agcccagaga gcatcggccc ctgtctgctg ctcgcgcctg gagatgtcag 300
   aggtccccgt tgctcgcgtc tggctggtac tgctcctgct gactgtccag gtcggcgtga 360
   cagccggcgc tccgtggcag tgcgcgccct gctccgccga gaagctcgcg ctctgcccgc 420
   cggtgtccgc ctcgtgctcg gaggtcaccc ggtccgccgg ctgcggctgt tgcccgatgt 480
   gcgccctgcc tctgggcgcc gcgtgcggcg tggcgactgc acgctgcgcc cggggactca 540
   gttgccgcgc gctgccgggg gagcagcaac ctctgcacgc cctcacccgc ggccaaggcg 600
   cctgcgtgca ggagtctgac gcctccgctc cccatgctgc agaggcaggg agccctgaaa 660
   gcccagagag cacggagata actgaggagg agctcctgga taatttccat ctgatggccc 720
   cttctgaaga ggatcattcc atcctttggg acgccatcag tacctatgat ggctcgaagg 780
   ctctccatgt caccaacatc aaaaaatgga aggagccctg ccgaatagaa ctctacagag 840
   tcgtagagag tttagccaag gcacaggaga catcaggaga agaaatttcc aaattttacc 900
   tgccaaactg caacaagaat ggattttatc acagcagaca gtgtgagaca tccatggatg 960
   gagaggcggg actctgctgg tgcgtctacc cttggaatgg gaagaggatc cctgggtctc 1020
   cagagatcag gggagacccc aactgccaga tatattttaa tgtacaaaac tgaaaccaga 1080
   tgaaataatg ttctgtcacg tgaaatattt aagtatatag tatatttata ctctagaaca 1140
   tgcacattta tatatatatg tatatgtata tatatatagt aactactttt tatactccat 1200
   acataacttg atatagaaag ctgtttattt attcactgta agtttatttt ttctacacag 1260
   taaaaacttg tactatgtta ataacttgtc ctatgtcaat ttgtatatca tgaaacactt 1320
   ctcatcatat tgtatgtaag taattgcatt tctgctcttc caaagctcct gcgtctgttt 1380
   ttaaagagca tggaaaaata ctgcctagaa aatgcaaaat gaaataagag agagtagttt 1440
   ttcagctagt ttgaaggagg acggttaact tgtatattcc accattcaca tttgatgtac 1500
   atgtgtaggg aaagttaaaa gtgttgatta cataatcaaa gctacctgtg gtgatgttgc 1560
   cacctgttaa aatgtacact ggatatgttg ttaaacacgt gtctataatg gaaacattta 1620
   caataaatat tctgcatgga aatactgtta aaaaaaaaaa 1660
<210> 23
   <211> 1504
   <212> DNA
   <213> Homo sapiens
<400> 23
   ggttgaggtc aagtagtagc gttgggctgc ggcagcggag gagctcaaca tgcgtgagtg 60
   tatctctatc cacgtggggc aggcaggagt ccagatcggc aatgcctgct gggaactgta 120
   ctgcctggaa catggaattc agcccgatgg tcagatgcca agtgataaaa ccattggtgg 180
   tggggacgac tccttcaaca cgttcttcag tgagactgga gctggcaagc acgtgcccag 240
   agcagtgttt gtggacctgg agcccactgt ggtcgatgaa gtgcgcacag gaacctatag 300
   gcagctcttc cacccagagc agctgatcac cgggaaggaa gatgcggcca ataattacgc 360
   cagaggccat tacaccatcg gcaaggagat cgtcgacctg gtcctggacc ggatccgcaa 420
   actggcggat ctgtgcacgg gactgcaggg cttcctcatc ttccacagtt ttgggggtgg 480
   cactggctct gggttcgcat ctctgctcat ggagcggctc tcagtggatt acggcaagaa 540
   gtccaagcta gaatttgcca tttacccagc cccccaggtc tccacggccg tggtggagcc 600
   ctacaactcc atcctgacca cccacacgac cctggaacat tctgactgtg ccttcatggt 660
   cgacaatgaa gccatctatg acatatgtcg gcgcaacctg gacatcgagc gtcccacgta 720
   caccaacctc aatcgcctga ttgggcagat cgtgtcctcc atcacggcct ccctgcgatt 780
   tgacggggcc ctgaatgtgg acttgacgga attccagacc aacctagtgc cgtacccccg 840
   catccacttc cccctggcca cctacgcccc ggtcatctca gccgagaagg cctaccacga 900
   gcagctgtcc gtggctgaga tcaccaatgc ctgcttcgag ccagccaatc agatggtcaa 960
   gtgtgaccct cgccacggca agtacatggc ctgctgcatg ttgtacaggg gggatgtggt 1020
   cccgaaagat gtcaacgcgg ccatcgccac catcaagacc aagcgcacca tccagtttgt 1080
   agattggtgc ccaactggat ttaaggtggg cattaactac cagcccccca cggtggtccc 1140
   tgggggagac ctggccaagg tgcagcgggc tgtgtgcatg ctgagcaaca ccacggccat 1200
   cgcggaggcc tgggctcgcc tggaccataa gttcgatctc atgtatgcca agcgggcctt 1260
   tgtgcactgg tacgtgggag aaggcatgga ggagggggag ttctctgagg cccgcgagga 1320
   cctggcagct ctggagaagg attatgaaga ggtgggcgtg gattccgtgg aagccgaggc 1380
   tgaagaaggt gaagaatact gaggggaggg tgtggtgggt tctccactcc actgccaccc 1440
   ccagcgtggc tgctttcaag ttctttgcaa ttaaaggttc tgtataaaaa aaaaaaaaaa 1500
   aaaa 1504
<210> 24
   <211> 753
   <212> PRT
   <213> Homo sapiens
<400> 24
<210> 25
   <211> 363
   <212> PRT
   <213> Homo sapiens
<400> 25
   Met Lys Arg Ser Leu Asn Glu Asn Ser Ala Arg Ser Thr Ala Gly Cys
   1 5 10 15
   Leu Pro Val Pro Leu Phe Asn Gln Lys Lys Arg Asn Arg Gln Pro Leu
   20 25 30
   Thr Ser Asn Pro Leu Lys Asp Asp Ser Gly Ile Ser Thr Pro Ser Asp
   35 40 45
   Asn Tyr Asp Phe Pro Pro Leu Pro Thr Asp Trp Ala Trp Glu Ala Val
   50 55 60
   Asn Pro Glu Leu Ala Pro Val Met Lys Thr Val Asp Thr Gly Gln Ile
   65 70 75 80
   Pro His Ser Val Ser Arg Pro Leu Arg Ser Gln Asp Ser Val Phe Asn
   85 90 95
   Ser Ile Gln Ser Asn Thr Gly Arg Ser Gln Gly Gly Trp Ser Tyr Arg
   100 105 110
   Asp Gly Asn Lys Asn Thr Ser Leu Lys Thr Trp Asn Lys Asn Asp Phe
   115 120 125
   Lys Pro Gln Cys Lys Arg Thr Asn Leu Val Ala Asn Asp Gly Lys Asn
   130 135 140
   Ser Cys Pro Val Ser Ser Gly Ala Gln Gln Gln Lys Gln Leu Arg Ile
   145 150 155 160
   Pro Glu Pro Pro Asn Leu Ser Arg Asn Lys Glu Thr Glu Leu Leu Arg
   165 170 175
   Gln Thr His Ser Ser Lys Ile Ser Gly Cys Thr Met Arg Gly Leu Asp
   180 185 190
   Lys Asn Ser Ala Leu Gln Thr Leu Lys Pro Asn Phe Gln Gln Asn Gln
   195 200 205
   Tyr Lys Lys Gln Met Leu Asp Asp Ile Pro Glu Asp Asn Thr Leu Lys
   210 215 220
   Glu Thr Ser Leu Tyr Gln Leu Gln Phe Lys Glu Lys Ala Ser Ser Leu
   225 230 235 240
   Arg Ile Ile Ser Ala Val Ile Glu Ser Met Lys Tyr Trp Arg Glu His
   245 250 255
   Ala Gln Lys Thr Val Leu Leu Phe Glu Val Leu Ala Val Leu Asp Ser
   260 265 270
   Ala Val Thr Pro Gly Pro Tyr Tyr Ser Lys Thr Phe Leu Met Arg Asp
   275 280 285
   Gly Lys Asn Thr Leu Pro Cys Val Phe Tyr Glu Ile Asp Arg Glu Leu
   290 295 300
   Pro Arg Leu Ile Arg Gly Arg Val His Arg Cys Val Gly Asn Tyr Asp
   305 310 315 320
   Gln Lys Lys Asn Ile Phe Gln Cys Val Ser Val Arg Pro Ala Ser Val
   325 330 335
   Ser Glu Gln Lys Thr Phe Gln Ala Phe Val Lys Ile Ala Asp Val Glu
   340 345 350
   Met Gln Tyr Tyr Ile Asn Val Met Asn Glu Thr
   355 360
<210> 26
   <211> 434
   <212> PRT
   <213> Homo sapiens
<400> 26
   Met Pro Asn Arg Lys Ala Ser Arg Asn Ala Tyr Tyr Phe Phe Val Gln
   1               5                   10                  15
   Glu Lys Ile Pro Glu Leu Arg Arg Arg Gly Leu Pro Val Ala Arg Val
               20                  25                  30
   Ala Asp Ala Ile Pro Tyr Cys Ser Ser Asp Trp Ala Leu Leu Arg Glu
           35                  40                  45
   Glu Glu Lys Glu Lys Tyr Ala Glu Met Ala Arg Glu Trp Arg Ala Ala
       50                  55                  60
   Gln Gly Lys Asp Pro Gly Pro Ser Glu Lys Gln Lys Pro Val Phe Thr
   65                  70                  75                  80
   Pro Leu Arg Arg Pro Gly Met Leu Val Pro Lys Gln Asn Val Ser Pro
                   85                  90                  95
   Pro Asp Met Ser Ala Leu Ser Leu Lys Gly Asp Gln Ala Leu Leu Gly
               100                 105                 110
   Gly Ile Phe Tyr Phe Leu Asn Ile Phe Ser His Gly Glu Leu Pro Pro
           115                 120                 125
   His Cys Glu Gln Arg Phe Leu Pro Cys Glu Ile Gly Cys Val Lys Tyr
       130                 135                 140
   Ser Leu Gln Glu Gly Ile Met Ala Asp Phe His Ser Phe Ile Asn Pro
   145                 150                 155                 160
   Gly Glu Ile Pro Arg Gly Phe Arg Phe His Cys Gln Ala Ala Ser Asp
                   165                 170                 175
   Ser Ser His Lys Ile Pro Ile Ser Asn Phe Glu Arg Gly His Asn Gln
               180                 185                 190
   Ala Thr Val Leu Gln Asn Leu Tyr Arg Phe Ile His Pro Asn Pro Gly
           195                 200                 205
   Asn Trp Pro Pro Ile Tyr Cys Lys Ser Asp Asp Arg Thr Arg Val Asn
   210 215 220
   Trp Cys Leu Lys His Met Ala Lys Ala Ser Glu Ile Arg Gln Asp Leu
   225 230 235 240
   Gln Leu Leu Thr Val Glu Asp Leu Val Val Gly Ile Tyr Gln Gln Lys
   245 250 255
   Phe Leu Lys Glu Pro Ser Lys Thr Trp Ile Arg Ser Leu Leu Asp Val
   260 265 270
   Ala Met Trp Asp Tyr Ser Ser Asn Thr Arg Cys Lys Trp His Glu Glu
   275 280 285
   Asn Asp Ile Leu Phe Cys Ala Leu Ala Val Cys Lys Lys Ile Ala Tyr
   290 295 300
   Cys Ile Ser Asn Ser Leu Ala Thr Leu Phe Gly Ile Gln Leu Thr Glu
   305 310 315 320
   Ala His Val Pro Leu Gln Asp Tyr Glu Ala Ser Asn Ser Val Thr Pro
   325 330 335
   Lys Met Val Val Leu Asp Ala Gly Arg Tyr Gln Lys Leu Arg Val Gly
   340 345 350
   Ser Ser Gly Phe Ser His Phe Asn Ser Ser Asn Glu Glu Gln Arg Ser
   355 360 365
   Asn Thr Pro Ile Gly Asp Tyr Pro Ser Arg Ala Lys Ile Ser Gly Gln
   370 375 380
   Asn Ser Ser Val Arg Gly Arg Gly Ile Thr Arg Leu Leu Glu Ser Ile
   385 390 395 400
   Ser Asn Ser Ser Ser Asn Ile His Lys Phe Ser Asn Cys Asp Thr Ser
   405 410 415
   Leu Ser Pro Tyr Met Ser Gln Lys Asp Gly Tyr Lys Ser Phe Ser Ser
   420 425 430
   Leu Ser
<210> 27
   <211> 72
   <212> PRT
   <213> Homo sapiens
<400> 27
   Met Pro Leu Leu Arg Gly Arg Cys Pro Ala Arg Arg His Tyr Arg Arg
   1 5 10 15
   Leu Ala Leu Leu Gly Leu Gln Pro Ala Pro Arg Phe Ala His Ser Gly
   20 25 30
   Pro Pro Arg Gln Arg Pro Leu Ser Ala Ala Glu Met Ala Val Gly Leu
   35 40 45
   Val Val Phe Phe Thr Thr Phe Leu Thr Pro Ala Ala Tyr Val Leu Gly
   50 55 60
   Asn Leu Lys Gln Phe Arg Arg Asn
   65 70
<210> 28
   <211> 596
   <212> PRT
   <213> Homo sapiens
<400> 28
   Met Ala Asp Ala Glu Ala Arg Ala Glu Phe Pro Glu Glu Ala Arg Pro
   1 5 10 15
   Asp Arg Gly Thr Leu Gln Val Leu Gln Asp Met Ala Ser Arg Leu Arg
   20 25 30
   Ile His Ser Ile Arg Ala Thr Cys Ser Thr Ser Ser Gly His Pro Thr
   35 40 45
   Ser Cys Ser Ser Ser Ser Glu Ile Met Ser Val Leu Phe Phe Tyr Ile
   50 55 60
   Met Arg Tyr Lys Gln Ser Asp Pro Glu Asn Pro Asp Asn Asp Arg Phe
   65 70 75 80
   Val Leu Ala Lys Arg Leu Ser Phe Val Asp Val Ala Thr Gly Trp Leu
   85 90 95
   Gly Gln Gly Leu Gly Val Ala Cys Gly Met Ala Tyr Thr Gly Lys Tyr
   100 105 110
   Phe Asp Arg Ala Ser Tyr Arg Val Phe Cys Leu Met Ser Asp Gly Glu
   115 120 125
   Ser Ser Glu Gly Ser Val Trp Glu Ala Met Ala Phe Ala Ser Tyr Tyr
   130 135 140
   Ser Leu Asp Asn Leu Val Ala Ile Phe Asp Val Asn Arg Leu Gly His
   145                 150                 155                 160
   Ser Gly Ala Leu Pro Ala Glu His Cys Ile Asn Ile Tyr Gln Arg Arg
                   165                 170                 175
   Cys Glu Ala Phe Gly Trp Asn Thr Tyr Val Val Asp Gly Arg Asp Val
               180                 185                 190
   Glu Ala Leu Cys Gln Val Phe Trp Gln Ala Ser Gln Val Lys His Lys
           195                 200                 205
   Pro Thr Ala Val Val Ala Lys Thr Phe Lys Gly Arg Gly Thr Pro Ser
       210                 215                 220
   Ile Glu Asp Ala Glu Ser Trp His Ala Lys Pro Met Pro Arg Glu Arg
   225                 230                 235                 240
   Ala Asp Ala Ile Ile Lys Leu Ile Glu Ser Gln Ile Gln Thr Ser Arg
                   245                 250                 255
   Asn Leu Asp Pro Gln Pro Pro Ile Glu Asp Ser Pro Glu Val Asn Ile
               260                 265                 270
   Thr Asp Val Arg Met Thr Ser Pro Pro Asp Tyr Arg Val Gly Asp Lys
           275                 280                 285
   Ile Ala Thr Arg Lys Ala Cys Gly Leu Ala Leu Ala Lys Leu Gly Tyr
       290                 295                 300
   Ala Asn Asn Arg Val Val Val Leu Asp Gly Asp Thr Arg Tyr Ser Thr
   305                 310                 315                 320
   Phe Ser Glu Ile Phe Asn Lys Glu Tyr Pro Glu Arg Phe Ile Glu Cys
                   325                 330                 335
   Phe Met Ala Glu Gln Asn Met Val Ser Val Ala Leu Gly Cys Ala Ser
               340                 345                 350
   Arg Gly Arg Thr Ile Ala Phe Ala Ser Thr Phe Ala Ala Phe Leu Thr
           355                 360                 365
   Arg Ala Phe Asp His Ile Arg Ile Gly Gly Leu Ala Glu Ser Asn Ile
       370                 375                 380
   Asn Ile Ile Gly Ser His Cys Gly Val Ser Val Gly Asp Asp Gly Ala
   385                 390                 395                 400
   Ser Gln Met Ala Leu Glu Asp Ile Ala Met Phe Arg Thr Ile Pro Lys
   405 410 415
   Cys Thr Ile Phe Tyr Pro Thr Asp Ala Val Ser Thr Glu His Ala Val
   420 425 430
   Ala Leu Ala Ala Asn Ala Lys Gly Met Cys Phe Ile Arg Thr Thr Arg
   435 440 445
   Pro Glu Thr Met Val Ile Tyr Thr Pro Gln Glu Arg Phe Glu Ile Gly
   450 455 460
   Gln Ala Lys Val Leu Arg His Cys Val Ser Asp Lys Val Thr Val Ile
   465 470 475 480
   Gly Ala Gly Ile Thr Val Tyr Glu Ala Leu Ala Ala Ala Asp Glu Leu
   485 490 495
   Ser Lys Gln Asp Ile Phe Ile Arg Val Ile Asp Leu Phe Thr Ile Lys
   500 505 510
   Pro Leu Asp Val Ala Thr Ile Val Ser Ser Ala Lys Ala Thr Glu Gly
   515 520 525
   Arg Ile Ile Thr Val Glu Asp His Tyr Pro Gln Gly Gly Ile Gly Glu
   530 535 540
   Ala Val Cys Ala Ala Val Ser Met Asp Pro Asp Ile Gln Val His Ser
   545 550 555 560
   Leu Ala Val Ser Gly Val Pro Gln Ser Gly Lys Ser Glu Glu Leu Leu
   565 570 575
   Asp Met Tyr Gly Ile Ser Ala Arg His Ile Ile Val Ala Val Lys Cys
   580 585 590
   Met Leu Leu Asn
   595
<210> 29
   <211> 533
   <212> PRT
   <213> Homo sapiens
<400> 29
   Met Asn Glu Glu Asn Ile Asp Gly Thr Asn Gly Cys Ser Lys Val Arg
   1 5 10 15
   Thr Gly Ile Gln Asn Glu Ala Ala Leu Leu Ala Leu Met Glu Lys Thr
   20 25 30
   Gly Tyr Asn Met Val Gln Glu Asn Gly Gln Arg Lys Phe Gly Gly Pro
   35 40 45
   Pro Pro Gly Trp Glu Gly Pro Pro Pro Pro Arg Gly Cys Glu Val Phe
   50 55 60
   Val Gly Lys Ile Pro Arg Asp Met Tyr Glu Asp Glu Leu Val Pro Val
   65 70 75 80
   Phe Glu Arg Ala Gly Lys Ile Tyr Glu Phe Arg Leu Met Met Glu Phe
   85 90 95
   Ser Gly Glu Asn Arg Gly Tyr Ala Phe Val Met Tyr Thr Thr Lys Glu
   100 105 110
   Glu Ala Gln Leu Ala Ile Arg Ile Leu Asn Asn Tyr Glu Ile Arg Pro
   115 120 125
   Gly Lys Phe Ile Gly Val Cys Val Ser Leu Asp Asn Cys Arg Leu Phe
   130 135 140
   Ile Gly Ala Ile Pro Lys Glu Lys Lys Lys Glu Glu Ile Leu Asp Glu
   145 150 155 160
   Met Lys Lys Val Thr Glu Gly Val Val Asp Val Ile Val Tyr Pro Ser
   165 170 175
   Ala Thr Asp Lys Thr Lys Asn Arg Gly Phe Ala Phe Val Glu Tyr Glu
   180 185 190
   Ser His Arg Ala Ala Ala Met Ala Arg Arg Lys Leu Ile Pro Gly Thr
   195 200 205
   Phe Gln Leu Trp Gly His Thr Ile Gln Val Asp Trp Ala Asp Pro Glu
   210 215 220
   Lys Glu Val Asp Glu Glu Thr Met Gln Arg Val Lys Val Leu Tyr Val
   225 230 235 240
   Arg Asn Leu Met Ile Ser Thr Thr Glu Glu Thr Ile Lys Ala Glu Phe
   245 250 255
   Asn Lys Phe Lys Pro Gly Ala Val Glu Arg Val Lys Lys Leu Arg Asp
   260 265 270
   Tyr Ala Phe Val His Phe Phe Asn Arg Glu Asp Ala Val Ala Ala Met
   275 280 285
   Ser Val Met Asn Gly Lys Cys Ile Asp Gly Ala Ser Ile Glu Val Thr
   290 295 300
   Leu Ala Lys Pro Val Asn Lys Glu Asn Thr Trp Arg Gln His Leu Asn
   305 310 315 320
   Gly Gln Ile Ser Pro Asn Ser Glu Asn Leu Ile Val Phe Ala Asn Lys
   325 330 335
   Glu Glu Ser His Pro Lys Thr Leu Gly Lys Leu Pro Thr Leu Pro Ala
   340 345 350
   Arg Leu Asn Gly Gln His Ser Pro Ser Pro Pro Glu Val Glu Arg Cys
   355 360 365
   Thr Tyr Pro Phe Tyr Pro Gly Thr Lys Leu Thr Pro Ile Ser Met Tyr
   370 375 380
   Ser Leu Lys Ser Asn His Phe Asn Ser Ala Val Met His Leu Asp Tyr
   385 390 395 400
   Tyr Cys Asn Lys Asn Asn Trp Ala Pro Pro Glu Tyr Tyr Leu Tyr Ser
   405 410 415
   Thr Thr Ser Gln Asp Gly Lys Val Leu Leu Val Tyr Lys Ile Val Ile
   420 425 430
   Pro Ala Ile Ala Asn Gly Ser Gln Ser Tyr Phe Met Pro Asp Lys Leu
   435 440 445
   Cys Thr Thr Leu Glu Asp Ala Lys Glu Leu Ala Ala Gln Phe Thr Leu
   450 455 460
   Leu His Leu Asp Tyr Asn Phe His Arg Ser Ser Ile Asn Ser Leu Ser
   465 470 475 480
   Pro Val Ser Ala Thr Leu Ser Ser Gly Thr Pro Ser Val Leu Pro Tyr
   485 490 495
   Thr Ser Arg Pro Tyr Ser Tyr Pro Gly Tyr Pro Leu Ser Pro Thr Ile
   500 505 510
   Ser Leu Ala Asn Gly Ser His Val Gly Gln Arg Leu Cys Ile Ser Asn
   515 520 525
   Gln Ala Ser Phe Phe
   530
<210> 30
   <211> 407
   <212> PRT
   <213> Homo sapiens
<400> 30
   Met Pro Arg Gly His Lys Ser Lys Leu Arg Thr Cys Glu Lys Arg Gln
   1 5 10 15
   Glu Thr Asn Gly Gln Pro Gln Gly Leu Thr Gly Pro Gln Ala Thr Ala
   20 25 30
   Glu Lys Gln Glu Glu Ser His Ser Ser Ser Ser Ser Ser Arg Ala Cys
   35 40 45
   Leu Gly Asp Cys Arg Arg Ser Ser Asp Ala Ser Ile Pro Gln Glu Ser
   50 55 60
   Gln Gly Val Ser Pro Thr Gly Ser Pro Asp Ala Val Val Ser Tyr Ser
   65 70 75 80
   Lys Ser Asp Val Ala Ala Asn Gly Gln Asp Glu Lys Ser Pro Ser Thr
   85 90 95
   Ser Arg Asp Ala Ser Val Pro Gln Glu Ser Gln Gly Ala Ser Pro Thr
   100 105 110
   Gly Ser Pro Asp Ala Gly Val Ser Gly Ser Lys Tyr Asp Val Ala Ala
   115 120 125
   Asn Gly Gln Asp Glu Lys Ser Pro Ser Thr Ser His Asp Val Ser Val
   130 135 140
   Pro Gln Glu Ser Gln Gly Ala Ser Pro Thr Gly Ser Pro Asp Ala Gly
   145 150 155 160
   Val Ser Gly Ser Lys Tyr Asp Val Ala Ala Glu Gly Glu Asp Glu Glu
   165 170 175
   Ser Val Ser Ala Ser Gln Lys Ala Ile Ile Phe Lys Arg Leu Ser Lys
   180 185 190
   Asp Ala Val Lys Lys Lys Ala Cys Thr Leu Ala Gln Phe Leu Gln Lys
   195 200 205
   Lys Phe Glu Lys Lys Glu Ser Ile Leu Lys Ala Asp Met Leu Lys Cys
   210 215 220
   Val Arg Arg Glu Tyr Lys Pro Tyr Phe Pro Gln Ile Leu Asn Arg Thr
   225 230 235 240
   Ser Gln His Leu Val Val Ala Phe Gly Val Glu Leu Lys Glu Met Asp
   245 250 255
   Ser Ser Gly Glu Ser Tyr Thr Leu Val Ser Lys Leu Gly Leu Pro Ser
   260 265 270
   Glu Gly Ile Leu Ser Gly Asp Asn Ala Leu Pro Lys Ser Gly Leu Leu
   275 280 285
   Met Ser Leu Leu Val Val Ile Phe Met Asn Gly Asn Cys Ala Thr Glu
   290 295 300
   Glu Glu Val Trp Glu Phe Leu Gly Leu Leu Gly Ile Tyr Asp Gly Ile
   305 310 315 320
   Leu His Ser Ile Tyr Gly Asp Ala Arg Lys Ile Ile Thr Glu Asp Leu
   325 330 335
   Val Gln Asp Lys Tyr Val Val Tyr Arg Gln Val Cys Asn Ser Asp Pro
   340 345 350
   Pro Cys Tyr Glu Phe Leu Trp Gly Pro Arg Ala Tyr Ala Glu Thr Thr
   355 360 365
   Lys Met Arg Val Leu Arg Val Leu Ala Asp Ser Ser Asn Thr Ser Pro
   370 375 380
   Gly Leu Tyr Pro His Leu Tyr Glu Asp Ala Leu Ile Asp Glu Val Glu
   385 390 395 400
   Arg Ala Leu Arg Leu Arg Ala
   405
<210> 31
   <211> 638
   <212> PRT
   <213> Homo sapiens
<400> 31
   Met Val Val Ser Ala Asp Pro Leu Ser Ser Glu Arg Ala Glu Met Asn
   1 5 10 15
   Ile Leu Glu Ile Asn Gln Glu Leu Arg Ser Gln Leu Ala Glu Ser Asn
   20 25 30
   Gln Gln Phe Arg Asp Leu Lys Glu Lys Phe Leu Ile Thr Gln Ala Thr
   35 40 45
   Ala Tyr Ser Leu Ala Asn Gln Leu Lys Lys Tyr Lys Cys Glu Glu Tyr
   50 55 60
   Lys Asp Ile Ile Asp Ser Val Leu Arg Asp Glu Leu Gln Ser Met Glu
   65 70 75 80
   Lys Leu Ala Glu Lys Leu Arg Gln Ala Glu Glu Leu Arg Gln Tyr Lys
   85 90 95
   Ala Leu Val His Ser Gln Ala Lys Glu Leu Thr Gln Leu Arg Glu Lys
   100 105 110
   Leu Arg Glu Gly Arg Asp Ala Ser Arg Trp Leu Asn Lys His Leu Lys
   115 120 125
   Thr Leu Leu Thr Pro Asp Asp Pro Asp Lys Ser Gln Gly Gln Asp Leu
   130 135 140
   Arg Glu Gln Leu Ala Glu Gly His Arg Leu Ala Glu His Leu Val His
   145 150 155 160
   Lys Leu Ser Pro Glu Asn Asp Glu Asp Glu Asp Glu Asp Glu Asp Asp
   165 170 175
   Lys Asp Glu Glu Val Glu Lys Val Gln Glu Ser Pro Ala Pro Arg Glu
   180 185 190
   Val Gln Lys Thr Glu Glu Lys Glu Val Pro Gln Asp Ser Leu Glu Glu
   195 200 205
   Cys Ala Val Thr Cys Ser Asn Ser His Asn Pro Ser Asn Ser Asn Gln
   210 215 220
   Pro His Arg Ser Thr Lys Ile Thr Phe Lys Glu His Glu Val Asp Ser
   225 230 235 240
   Ala Leu Val Val Glu Ser Glu His Pro His Asp Glu Glu Glu Glu Ala
   245 250 255
   Leu Asn Ile Pro Pro Glu Asn Gln Asn Asp His Glu Glu Glu Glu Gly
   260 265 270
   Lys Ala Pro Val Pro Pro Arg His His Asp Lys Ser Asn Ser Tyr Arg
   275 280 285
   His Arg Glu Val Ser Phe Leu Ala Leu Asp Glu Gln Lys Val Cys Ser
   290 295 300
   Ala Gln Asp Val Ala Arg Asp Tyr Ser Asn Pro Lys Trp Asp Glu Thr
   305 310 315 320
   Ser Leu Gly Phe Leu Glu Lys Gln Ser Asp Leu Glu Glu Val Lys Gly
   325 330 335
   Gln Glu Thr Val Ala Pro Arg Leu Ser Arg Gly Pro Leu Arg Val Asp
   340 345 350
   Lys His Glu Ile Pro Gln Glu Ser Leu Asp Gly Cys Cys Leu Thr Pro
   355 360 365
   Ser Ile Leu Pro Asp Leu Thr Pro Ser Tyr His Pro Tyr Trp Ser Thr
   370 375 380
   Leu Tyr Ser Phe Glu Asp Lys Gln Val Ser Leu Ala Leu Val Asp Lys
   385 390 395 400
   Ile Lys Lys Asp Gln Glu Glu Ile Glu Asp Gln Ser Pro Pro Cys Pro
   405 410 415
   Arg Leu Ser Gln Glu Leu Pro Glu Val Lys Glu Gln Glu Val Pro Glu
   420 425 430
   Asp Ser Val Asn Glu Val Tyr Leu Thr Pro Ser Val His His Asp Val
   435 440 445
   Ser Asp Cys His Gln Pro Tyr Ser Ser Thr Leu Ser Ser Leu Glu Asp
   450 455 460
   Gln Leu Ala Cys Ser Ala Leu Asp Val Ala Ser Pro Thr Glu Ala Ala
   465 470 475 480
   Cys Pro Gln Gly Thr Trp Ser Gly Asp Leu Ser His His Gln Ser Glu
   485 490 495
   Val Gln Val Ser Gln Ala Gln Leu Glu Pro Ser Thr Leu Val Pro Ser
   500 505 510
   Cys Leu Arg Leu Gln Leu Asp Gln Gly Phe His Cys Gly Asn Gly Leu
   515 520 525
   Ala Gln Arg Gly Leu Ser Ser Thr Thr Cys Ser Phe Ser Ala Asn Ala
   530 535 540
   Asp Ser Gly Asn Gln Trp Pro Phe Gln Glu Leu Val Leu Glu Pro Ser
   545 550 555 560
   Leu Gly Met Lys Asn Pro Pro Gln Leu Glu Asp Asp Ala Leu Glu Gly
   565 570 575
   Ser Ala Ser Asn Thr Gln Gly Arg Gln Val Thr Gly Arg Ile Arg Ala
   580 585 590
   Ser Leu Val Leu Ile Leu Lys Thr Ile Arg Arg Arg Leu Pro Phe Ser
   595 600 605
   Lys Trp Arg Leu Ala Phe Arg Phe Ala Gly Pro His Ala Glu Ser Ala
   610 615 620
   Glu Ile Pro Asn Thr Ala Gly Arg Thr Gln Arg Met Ala Gly
   625 630 635
<210> 32
   <211> 70
   <212> PRT
   <213> Homo sapiens
<400> 32
   Met Lys Gln Lys Gln Glu Val Met Phe Gln Ser Arg Gly Arg Leu Ser
   1 5 10 15
   Leu Tyr Ile Gln Met Ser Ser Val Tyr Ser Ala Lys Leu Gly Pro Val
   20 25 30
   Gly Gly Ile Cys Gly Gln Lys Gln Lys Pro Ser Phe Phe Phe Phe Lys
   35 40 45
   Ala Gln Ser Gln Asp Ala Arg Pro Leu Ala Pro Ala Ala Cys Ile Ser
   50 55 60
   Lys Ile Ala Lys Ala Gly
   65 70
<210> 33
   <211> 522
   <212> PRT
   <213> Homo sapiens
<400> 33
<210> 34
   <211> 513
   <212> PRT
   <213> Homo sapiens
<400> 34
   Met Ile Arg Thr Pro Leu Ser Ala Ser Ala His Arg Leu Leu Leu Pro
   1 5 10 15
   Gly Ser Arg Gly Arg Pro Pro Arg Asn Met Gln Pro Thr Gly Arg Glu
   20 25 30
   Gly Ser Arg Ala Leu Ser Arg Arg Tyr Leu Arg Arg Leu Leu Leu Leu
   35 40 45
   Leu Leu Leu Leu Leu Leu Arg Gln Pro Val Thr Arg Ala Glu Thr Thr
   50 55 60
   Pro Gly Ala Pro Arg Ala Leu Ser Thr Leu Gly Ser Pro Ser Leu Phe
   65 70 75 80
   Thr Thr Pro Gly Val Pro Ser Ala Leu Thr Thr Pro Gly Leu Thr Thr
   85 90 95
   Pro Gly Thr Pro Lys Thr Leu Asp Leu Arg Gly Arg Ala Gln Ala Leu
   100 105 110
   Met Arg Ser Phe Pro Leu Val Asp Gly His Asn Asp Leu Pro Gln Val
   115 120 125
   Leu Arg Gln Arg Tyr Lys Asn Val Leu Gln Asp Val Asn Leu Arg Asn
   130 135 140
   Phe Ser His Gly Gln Thr Ser Leu Asp Arg Leu Arg Asp Gly Leu Val
   145 150 155 160
   Gly Ala Gln Phe Trp Ser Ala Ser Val Ser Cys Gln Ser Gln Asp Gln
   165 170 175
   Thr Ala Val Arg Leu Ala Leu Glu Gln Ile Asp Leu Ile His Arg Met
   180 185 190
   Cys Ala Ser Tyr Ser Glu Leu Glu Leu Val Thr Ser Ala Glu Gly Leu
   195 200 205
   Asn Ser Ser Gln Lys Leu Ala Cys Leu Ile Gly Val Glu Gly Gly His
   210 215 220
   Ser Leu Asp Ser Ser Leu Ser Val Leu Arg Ser Phe Tyr Val Leu Gly
   225 230 235 240
   Val Arg Tyr Leu Thr Leu Thr Phe Thr Cys Ser Thr Pro Trp Ala Glu
   245 250 255
   Ser Ser Thr Lys Phe Arg His His Met Tyr Thr Asn Val Ser Gly Leu
   260 265 270
   Thr Ser Phe Gly Glu Lys Val Val Glu Glu Leu Asn Arg Leu Gly Met
   275 280 285
   Met Ile Asp Leu Ser Tyr Ala Ser Asp Thr Leu Ile Arg Arg Val Leu
   290 295 300
   Glu Val Ser Gln Ala Pro Val Ile Phe Ser His Ser Ala Ala Arg Ala
   305 310 315 320
   Val Cys Asp Asn Leu Leu Asn Val Pro Asp Asp Ile Leu Gln Leu Leu
   325 330 335
   Lys Lys Asn Gly Gly Ile Val Met Val Thr Leu Ser Met Gly Val Leu
   340 345 350
   Gln Cys Asn Leu Leu Ala Asn Val Ser Thr Val Ala Asp His Phe Asp
   355 360 365
   His Ile Arg Ala Val Ile Gly Ser Glu Phe Ile Gly Ile Gly Gly Asn
   370 375 380
   Tyr Asp Gly Thr Gly Arg Phe Pro Gln Gly Leu Glu Asp Val Ser Thr
   385 390 395 400
   Tyr Pro Val Leu Ile Glu Glu Leu Leu Ser Arg Ser Trp Ser Glu Glu
   405 410 415
   Glu Leu Gln Gly Val Leu Arg Gly Asn Leu Leu Arg Val Phe Arg Gln
   420 425 430
   Val Glu Lys Val Arg Glu Glu Ser Arg Ala Gln Ser Pro Val Glu Ala
   435 440 445
   Glu Phe Pro Tyr Gly Gln Leu Ser Thr Ser Cys His Ser His Leu Val
   450 455 460
   Pro Gln Asn Gly His Gln Ala Thr His Leu Glu Val Thr Lys Gln Pro
   465 470 475 480
   Thr Asn Arg Val Pro Trp Arg Ser Ser Asn Ala Ser Pro Tyr Leu Val
   485 490 495
   Pro Gly Leu Val Ala Ala Ala Thr Ile Pro Thr Phe Thr Gln Trp Leu
   500 505 510
   Cys
<210> 35
   <211> 154
   <212> PRT
   <213> Homo sapiens
<400> 35
   Met Glu Pro Ser Lys Thr Phe Met Arg Asn Leu Pro Ile Thr Pro Gly
   1               5                   10                  15
   Tyr Ser Gly Phe Val Pro Phe Leu Ser Cys Gln Gly Met Ser Lys Glu
               20                  25                  30
   Asp Asp Met Asn His Cys Val Lys Thr Phe Gln Glu Lys Thr Gln Arg
           35                  40                  45
   Tyr Lys Glu Gln Leu Arg Glu Leu Cys Cys Ala Val Ala Thr Ala Pro
       50                  55                  60
   Lys Leu Lys Pro Val Asn Ser Glu Glu Thr Val Leu Gln Ala Leu His
   65                  70                  75                  80
   Gln Tyr Asn Leu Gln Tyr His Pro Leu Ile Leu Glu Cys Lys Tyr Val
                   85                  90                  95
   Lys Lys Pro Leu Gln Glu Pro Pro Ile Pro Gly Trp Ala Gly Tyr Leu
               100                 105                 110
   Pro Arg Ala Lys Val Thr Glu Phe Gly Cys Gly Thr Arg Tyr Thr Val
           115                 120                 125
   Met Ala Lys Asn Cys Tyr Lys Asp Phe Leu Glu Ile Thr Glu Arg Ala
       130                 135                 140
   Lys Lys Ala His Leu Lys Pro Tyr Glu Glu
   145                 150
<210> 36
   <211> 120
   <212> PRT
   <213> Homo sapiens
<400> 36
   Met Gly Val Leu Arg Ala Arg Gly Glu Val Gly Leu Ala Leu Ser Pro
   1               5                   10                  15
   Arg Leu Val Gly Gly Ala Ser Pro Pro Cys Asp Gly Gly Pro Glu Ser
               20                  25                  30
   Arg Gly Arg Lys Arg Gly Cys Leu Leu Ser Pro Cys Leu Val Gly Val
           35                  40                  45
   Ala Ser Pro Ser Cys Asp Asp Gly Pro Lys Ser Gln Arg Gly Lys Arg
       50                  55                  60
   Gly Trp Leu Ser Val Pro Ala Ser Leu Gly Val Pro Pro Pro Pro Ala
   65 70 75 80
   Ile Gly Val Leu Arg Ser Arg Gly Gly Arg Gly Ala Gly Ser Gln Ser
   85 90 95
   Pro Pro Arg Gly Gly Cys Leu Pro Pro Cys Asp Gly Gly Pro Glu Ser
   100 105 110
   Arg Glu Arg Lys Arg Gly Cys Leu
   115 120
<210> 37
   <211> 76
   <212> PRT
   <213> Homo sapiens
<400> 37
   Met Thr Asp Val Glu Thr Thr Tyr Ala Asp Phe Ile Ala Ser Gly Arg
   1 5 10 15
   Thr Gly Arg Arg Asn Ala Ile His Asp Ile Leu Val Ser Ser Ala Ser
   20 25 30
   Gly Asn Ser Asn Glu Leu Ala Leu Lys Leu Ala Gly Leu Asp Ile Asn
   35 40 45
   Lys Thr Glu Gly Glu Glu Asp Ala Gln Arg Ser Ser Thr Glu Gln Ser
   50 55 60
   Gly Glu Ala Gln Gly Glu Ala Ala Lys Ser Glu Ser
   65 70 75
<210> 38
   <211> 70
   <212> PRT
   <213> Homo sapiens
<400> 38
   Met Ala Arg Thr Lys Gln Thr Ala Arg Lys Ser Thr Gly Gly Lys Ala
   1 5 10 15
   Pro Arg Lys Gln Leu Ala Thr Lys Ala Ala Arg Lys Ser Ala Pro Ala
   20 25 30
   Thr Gly Gly Val Lys Lys Pro His Arg Tyr Arg Pro Gly Thr Val Ala
   35 40 45
   Leu Arg Glu Ile Arg Arg Tyr Gln Lys Ser Thr Glu Leu Leu Ile Arg
   50 55 60
   Lys Leu Pro Phe Gln Arg
   65 70
<210> 39
   <211> 861
   <212> PRT
   <213> Homo sapiens
<400> 39
   Met Thr Gly Arg Ala Arg Ala Arg Ala Arg Gly Arg Ala Arg Gly Gln
   1 5 10 15
   Glu Thr Ala Gln Leu Val Gly Ser Thr Ala Ser Gln Gln Pro Gly Tyr
   20 25 30
   Ile Gln Pro Arg Pro Gln Pro Pro Pro Ala Glu Gly Glu Leu Phe Gly
   35 40 45
   Arg Gly Arg Gln Arg Gly Thr Ala Gly Gly Thr Ala Lys Ser Gln Gly
   50 55 60
   Leu Gln Ile Ser Ala Gly Phe Gln Glu Leu Ser Leu Ala Glu Arg Gly
   65 70 75 80
   Gly Arg Arg Arg Asp Phe His Asp Leu Gly Val Asn Thr Arg Gln Asn
   85 90 95
   Leu Asp His Val Lys Glu Ser Lys Thr Gly Ser Ser Gly Ile Ile Val
   100 105 110
   Arg Leu Ser Thr Asn His Phe Arg Leu Thr Ser Arg Pro Gln Trp Ala
   115 120 125
   Leu Tyr Gln Tyr His Ile Asp Tyr Asn Pro Leu Met Glu Ala Arg Arg
   130 135 140
   Leu Arg Ser Ala Leu Leu Phe Gln His Glu Asp Leu Ile Gly Lys Cys
   145 150 155 160
   His Ala Phe Asp Gly Thr Ile Leu Phe Leu Pro Lys Arg Leu Gln Gln
   165 170 175
   Lys Val Thr Glu Val Phe Ser Lys Thr Arg Asn Gly Glu Asp Val Arg
   180 185 190
   Ile Thr Ile Thr Leu Thr Asn Glu Leu Pro Pro Thr Ser Pro Thr Cys
   195 200 205
   Leu Gln Phe Tyr Asn Ile Ile Phe Arg Arg Leu Leu Lys Ile Met Asn
       210                 215                 220
   Leu Gln Gln Ile Gly Arg Asn Tyr Tyr Asn Pro Asn Asp Pro Ile Asp
   225                 230                 235                 240
   Ile Pro Ser His Arg Leu Val Ile Trp Pro Gly Phe Thr Thr Ser Ile
                   245                 250                 255
   Leu Gln Tyr Glu Asn Ser Ile Met Leu Cys Thr Asp Val Ser His Lys
               260                 265                 270
   Val Leu Arg Ser Glu Thr Val Leu Asp Phe Met Phe Asn Phe Tyr His
           275                 280                 285
   Gln Thr Glu Glu His Lys Phe Gln Glu Gln Val Ser Lys Glu Leu Ile
       290                 295                 300
   Gly Leu Val Val Leu Thr Lys Tyr Asn Asn Lys Thr Tyr Arg Val Asp
   305                 310                 315                 320
   Asp Ile Asp Trp Asp Gln Asn Pro Lys Ser Thr Phe Lys Lys Ala Asp
                   325                 330                 335
   Gly Ser Glu Val Ser Phe Leu Glu Tyr Tyr Arg Lys Gln Tyr Asn Gln
               340                 345                 350
   Glu Ile Thr Asp Leu Lys Gln Pro Val Leu Val Ser Gln Pro Lys Arg
           355                 360                 365
   Arg Arg Gly Pro Gly Gly Thr Leu Pro Gly Pro Ala Met Leu Ile Pro
       370                 375                 380
   Glu Leu Cys Tyr Leu Thr Gly Leu Thr Asp Lys Met Arg Asn Asp Phe
   385                 390                 395                 400
   Asn Val Met Lys Asp Leu Ala Val His Thr Arg Leu Thr Pro Glu Gln
                   405                 410                 415
   Arg Gln Arg Glu Val Gly Arg Leu Ile Asp Tyr Ile His Lys Asn Asp
               420                 425                 430
   Asn Val Gln Arg Glu Leu Arg Asp Trp Gly Leu Ser Phe Asp Ser Asn
           435                 440                 445
   Leu Leu Ser Phe Ser Gly Arg Ile Leu Gln Thr Glu Lys Ile His Gln
       450                 455                 460
   Gly Gly Lys Thr Phe Asp Tyr Asn Pro Gln Phe Ala Asp Trp Ser Lys
   465 470 475 480
   Glu Thr Arg Gly Ala Pro Leu Ile Ser Val Lys Pro Leu Asp Asn Trp
   485 490 495
   Leu Leu Ile Tyr Thr Arg Arg Asn Tyr Glu Ala Ala Asn Ser Leu Ile
   500 505 510
   Gln Asn Leu Phe Lys Val Thr Pro Ala Met Gly Met Gln Met Arg Lys
   515 520 525
   Ala Ile Met Ile Glu Val Asp Asp Arg Thr Glu Ala Tyr Leu Arg Val
   530 535 540
   Leu Gln Gln Lys Val Thr Ala Asp Thr Gln Ile Val Val Cys Leu Leu
   545 550 555 560
   Ser Ser Asn Arg Lys Asp Lys Tyr Asp Ala Ile Lys Lys Tyr Leu Cys
   565 570 575
   Thr Asp Cys Pro Thr Pro Ser Gln Cys Val Val Ala Arg Thr Leu Gly
   580 585 590
   Lys Gln Gln Thr Val Met Ala Ile Ala Thr Lys Ile Ala Leu Gln Met
   595 600 605
   Asn Cys Lys Met Gly Gly Glu Leu Trp Arg Val Asp Ile Pro Leu Lys
   610 615 620
   Leu Val Met Ile Val Gly Ile Asp Cys Tyr His Asp Met Thr Ala Gly
   625 630 635 640
   Arg Arg Ser Ile Ala Gly Phe Val Ala Ser Ile Asn Glu Gly Met Thr
   645 650 655
   Arg Trp Phe Ser Arg Cys Ile Phe Gln Asp Arg Gly Gln Glu Leu Val
   660 665 670
   Asp Gly Leu Lys Val Cys Leu Gln Ala Ala Leu Arg Ala Trp Asn Ser
   675 680 685
   Cys Asn Glu Tyr Met Pro Ser Arg Ile Ile Val Tyr Arg Asp Gly Val
   690 695 700
   Gly Asp Gly Gln Leu Lys Thr Leu Val Asn Tyr Glu Val Pro Gln Phe
   705 710 715 720
   Leu Asp Cys Leu Lys Ser Ile Gly Arg Gly Tyr Asn Pro Arg Leu Thr
   725 730 735
   Val Ile Val Val Lys Lys Arg Val Asn Thr Arg Phe Phe Ala Gln Ser
   740 745 750
   Gly Gly Arg Leu Gln Asn Pro Leu Pro Gly Thr Val Ile Asp Val Glu
   755 760 765
   Val Thr Arg Pro Glu Trp Tyr Asp Phe Phe Ile Val Ser Gln Ala Val
   770 775 780
   Arg Ser Gly Ser Val Ser Pro Thr His Tyr Asn Val Ile Tyr Asp Asn
   785 790 795 800
   Ser Gly Leu Lys Pro Asp His Ile Gln Arg Leu Thr Tyr Lys Leu Cys
   805 810 815
   His Ile Tyr Tyr Asn Trp Pro Gly Val Ile Arg Val Pro Ala Pro Cys
   820 825 830
   Gln Tyr Ala His Lys Leu Ala Phe Leu Val Gly Gln Ser Ile His Arg
   835 840 845
   Glu Pro Asn Leu Ser Leu Ser Asn Arg Leu Tyr Tyr Leu
   850 855 860
<210> 40
   <211> 221
   <212> PRT
   <213> Homo sapiens
<400> 40
   Met Pro Leu Ala Leu Thr Leu Leu Leu Leu Ser Gly Leu Gly Ala Pro
   1 5 10 15
   Gly Gly Trp Gly Cys Leu Gln Cys Asp Pro Leu Val Leu Glu Ala Leu
   20 25 30
   Gly His Leu Arg Ser Ala Leu Ile Pro Ser Arg Phe Gln Leu Glu Gln
   35 40 45
   Leu Gln Ala Arg Ala Gly Ala Val Leu Met Gly Met Glu Gly Pro Phe
   50 55 60
   Phe Arg Asp Tyr Ala Leu Asn Val Phe Val Gly Lys Val Glu Thr Asn
   65 70 75 80
   Gln Leu Asp Leu Val Ala Ser Phe Val Lys Asn Gln Thr Gln His Leu
   85 90 95
   Met Gly Asn Ser Leu Lys Asp Glu Pro Leu Leu Glu Glu Leu Val Thr
   100 105 110
   Leu Arg Ala Asn Val Ile Lys Glu Phe Lys Lys Val Leu Ile Ser Tyr
   115 120 125
   Glu Leu Lys Ala Cys Asn Pro Lys Leu Cys Arg Leu Leu Lys Glu Glu
   130 135 140
   Val Leu Asp Cys Leu His Cys Gln Arg Ile Thr Pro Lys Cys Ile His
   145 150 155 160
   Lys Lys Tyr Cys Phe Val Asp Arg Gln Pro Arg Val Ala Leu Gln Tyr
   165 170 175
   Gln Met Asp Ser Lys Tyr Pro Arg Asn Gln Ala Leu Leu Gly Ile Leu
   180 185 190
   Ile Ser Val Ser Leu Ala Val Phe Val Phe Val Val Ile Val Val Ser
   195 200 205
   Ala Cys Thr Tyr Arg Gln Asn Arg Lys Leu Leu Leu Gln
   210 215 220
<210> 41
   <211> 1623
   <212> PRT
   <213> Homo sapiens
<400> 41
   Met Ala Ala Glu Ala Ser Lys Thr Gly Pro Ser Arg Ser Ser Tyr Gln
   1 5 10 15
   Arg Met Gly Arg Lys Ser Gln Pro Trp Gly Ala Ala Glu Ile Gln Cys
   20 25 30
   Thr Arg Cys Gly Arg Arg Val Ser Arg Ser Ser Gly His His Cys Glu
   35 40 45
   Leu Gln Cys Gly His Ala Phe Cys Glu Leu Cys Leu Leu Met Thr Glu
   50 55 60
   Glu Cys Thr Thr Ile Ile Cys Pro Asp Cys Glu Val Ala Thr Ala Val
   65 70 75 80
   Asn Thr Arg Gln Arg Tyr Tyr Pro Met Ala Gly Tyr Ile Lys Glu Asp
   85 90 95
   Ser Ile Met Glu Lys Leu Gln Pro Lys Thr Ile Lys Asn Cys Ser Gln
   100 105 110
   Asp Phe Lys Lys Thr Ala Asp Gln Leu Thr Thr Gly Leu Glu Arg Ser
   115 120 125
   Ala Ser Thr Asp Lys Thr Leu Leu Asn Ser Ser Ala Val Met Leu Asp
   130 135 140
   Thr Asn Thr Ala Glu Glu Ile Asp Glu Ala Leu Asn Thr Ala His His
   145 150 155 160
   Ser Phe Glu Gln Leu Ser Ile Ala Gly Lys Ala Leu Glu His Met Gln
   165 170 175
   Lys Gln Thr Ile Glu Glu Arg Glu Arg Val Ile Glu Val Val Glu Lys
   180 185 190
   Gln Phe Asp Gln Leu Leu Ala Phe Phe Asp Ser Arg Lys Lys Asn Leu
   195 200 205
   Cys Glu Glu Phe Ala Arg Thr Thr Asp Asp Tyr Leu Ser Asn Leu Ile
   210 215 220
   Lys Ala Lys Ser Tyr Ile Glu Glu Lys Lys Asn Asn Leu Asn Ala Ala
   225 230 235 240
   Met Asn Ile Ala Arg Ala Leu Gln Leu Ser Pro Ser Leu Arg Thr Tyr
   245 250 255
   Cys Asp Leu Asn Gln Ile Ile Arg Thr Leu Gln Leu Thr Ser Asp Ser
   260 265 270
   Glu Leu Ala Gln Val Ser Ser Pro Gln Leu Arg Asn Pro Pro Arg Leu
   275 280 285
   Ser Val Asn Cys Ser Glu Ile Ile Cys Met Phe Asn Asn Met Gly Lys
   290 295 300
   Ile Glu Phe Arg Asp Ser Thr Lys Cys Tyr Pro Gln Glu Asn Glu Ile
   305 310 315 320
   Arg Gln Asn Val Gln Lys Lys Tyr Asn Asn Lys Lys Glu Leu Ser Cys
   325 330 335
   Tyr Asp Thr Tyr Pro Pro Leu Glu Lys Lys Lys Val Asp Met Ser Val
   340 345 350
   Leu Thr Ser Glu Ala Pro Pro Pro Pro Leu Gln Pro Glu Thr Asn Asp
   355 360 365
   Val His Leu Glu Ala Lys Asn Phe Gln Pro Gln Lys Asp Val Ala Thr
   370 375 380
   Ala Ser Pro Lys Thr Ile Ala Val Leu Pro Gln Met Gly Ser Ser Pro
   385 390 395 400
   Asp Val Ile Ile Glu Glu Ile Ile Glu Asp Asn Val Glu Ser Ser Ala
   405 410 415
   Glu Leu Val Phe Val Ser His Val Ile Asp Pro Cys His Phe Tyr Ile
   420 425 430
   Arg Lys Tyr Ser Gln Ile Lys Asp Ala Lys Val Leu Glu Lys Lys Val
   435 440 445
   Asn Glu Phe Cys Asn Arg Ser Ser His Leu Asp Pro Ser Asp Ile Leu
   450 455 460
   Glu Leu Gly Ala Arg Ile Phe Val Ser Ser Ile Lys Asn Gly Met Trp
   465 470 475 480
   Cys Arg Gly Thr Ile Thr Glu Leu Ile Pro Ile Glu Gly Arg Asn Thr
   485 490 495
   Arg Lys Pro Cys Ser Pro Thr Arg Leu Phe Val His Glu Val Ala Leu
   500 505 510
   Ile Gln Ile Phe Met Val Asp Phe Gly Asn Ser Glu Val Leu Ile Val
   515 520 525
   Thr Gly Val Val Asp Thr His Val Arg Pro Glu His Ser Ala Lys Gln
   530 535 540
   His Ile Ala Leu Asn Asp Leu Cys Leu Val Leu Arg Lys Ser Glu Pro
   545 550 555 560
   Tyr Thr Glu Gly Leu Leu Lys Asp Ile Gln Pro Leu Ala Gln Pro Cys
   565 570 575
   Ser Leu Lys Asp Ile Val Pro Gln Asn Ser Asn Glu Gly Trp Glu Glu
   580 585 590
   Glu Ala Lys Val Glu Phe Leu Lys Met Val Asn Asn Lys Ala Val Ser
   595 600 605
   Met Lys Val Phe Arg Glu Glu Asp Gly Val Leu Ile Val Asp Leu Gln
   610 615 620
   Lys Pro Pro Pro Asn Lys Ile Ser Ser Asp Met Pro Val Ser Leu Arg
   625 630 635 640
   Asp Ala Leu Val Phe Met Glu Leu Ala Lys Phe Lys Ser Gln Ser Leu
   645 650 655
   Arg Ser His Phe Glu Lys Asn Thr Thr Leu His Tyr His Pro Pro Ile
   660 665 670
   Leu Pro Lys Glu Met Thr Asp Val Ser Val Thr Val Cys His Ile Asn
   675 680 685
   Ser Pro Gly Asp Phe Tyr Leu Gln Leu Ile Glu Gly Leu Asp Ile Leu
   690 695 700
   Phe Leu Leu Lys Thr Ile Glu Glu Phe Tyr Lys Ser Glu Asp Gly Glu
   705 710 715 720
   Asn Leu Glu Ile Leu Cys Pro Val Gln Asp Gln Ala Cys Val Ala Lys
   725 730 735
   Phe Glu Asp Gly Ile Trp Tyr Arg Ala Lys Val Ile Gly Leu Pro Gly
   740 745 750
   His Gln Glu Val Glu Val Lys Tyr Val Asp Phe Gly Asn Thr Ala Lys
   755 760 765
   Ile Thr Ile Lys Asp Val Arg Lys Ile Lys Asp Glu Phe Leu Asn Ala
   770 775 780
   Pro Glu Lys Ala Ile Lys Cys Lys Leu Ala Tyr Ile Glu Pro Tyr Lys
   785 790 795 800
   Arg Thr Met Gln Trp Ser Lys Glu Ala Lys Glu Lys Phe Glu Glu Lys
   805 810 815
   Ala Gln Asp Lys Phe Met Thr Cys Ser Val Ile Lys Ile Leu Glu Asp
   820 825 830
   Asn Val Leu Leu Val Glu Leu Phe Asp Ser Leu Gly Ala Pro Glu Met
   835 840 845
   Thr Thr Thr Ser Ile Asn Asp Gln Leu Val Lys Glu Gly Leu Ala Ser
   850 855 860
   Tyr Glu Ile Gly Tyr Ile Leu Lys Asp Asn Ser Gln Lys His Ile Glu
   865 870 875 880
   Val Trp Asp Pro Ser Pro Glu Glu Ile Ile Ser Asn Glu Val His Asn
   885 890 895
   Leu Asn Pro Val Ser Ala Lys Ser Leu Pro Asn Glu Asn Phe Gln Ser
   900 905 910
   Leu Tyr Asn Lys Glu Leu Pro Val His Ile Cys Asn Val Ile Ser Pro
   915 920 925
   Glu Lys Ile Tyr Val Gln Trp Leu Leu Thr Glu Asn Leu Leu Asn Ser
   930 935 940
   Leu Glu Glu Lys Met Ile Ala Ala Tyr Glu Asn Ser Lys Trp Glu Pro
   945 950 955 960
   Val Lys Trp Glu Asn Asp Met His Cys Ala Val Lys Ile Gln Asp Lys
   965 970 975
   Asn Gln Trp Arg Arg Gly Gln Ile Ile Arg Met Val Thr Asp Thr Leu
   980 985 990
   Val Glu Val Leu Leu Tyr Asp Val Gly Val Glu Leu Val Val Asn Val
   995 1000 1005
   Asp Cys Leu Arg Lys Leu Glu Glu Asn Leu Lys Thr Met Gly Arg
   1010 1015 1020
   Leu Ser Leu Glu Cys Ser Leu Val Asp Ile Arg Pro Ala Gly Gly
   1025 1030 1035
   Ser Asp Lys Trp Thr Ala Thr Ala Cys Asp Cys Leu Ser Leu Tyr
   1040 1045 1050
   Leu Thr Gly Ala Val Ala Thr Ile Ile Leu Gln Val Asp Ser Glu
   1055 1060 1065
   Glu Asn Asn Thr Thr Trp Pro Leu Pro Val Lys Ile Phe Cys Arg
   1070 1075 1080
   Asp Glu Lys Gly Glu Arg Val Asp Val Ser Lys Tyr Leu Ile Lys
   1085 1090 1095
   Lys Gly Leu Ala Leu Arg Glu Arg Arg Ile Asn Asn Leu Asp Asn
   1100 1105 1110
   Ser His Ser Leu Ser Glu Lys Ser Leu Glu Val Pro Leu Glu Gln
   1115 1120 1125
   Glu Asp Ser Val Val Thr Asn Cys Ile Lys Thr Asn Phe Asp Pro
   1130 1135 1140
   Asp Lys Lys Thr Ala Asp Ile Ile Ser Glu Gln Lys Val Ser Glu
   1145 1150 1155
   Phe Gln Glu Lys Ile Leu Glu Pro Arg Thr Thr Arg Gly Tyr Lys
   1160 1165 1170
   Pro Pro Ala Ile Pro Asn Met Asn Val Phe Glu Ala Thr Val Ser
   1175 1180 1185
   Cys Val Gly Asp Asp Gly Thr Ile Phe Val Val Pro Lys Leu Ser
   1190 1195 1200
   Glu Phe Glu Leu Ile Lys Met Thr Asn Glu Ile Gln Ser Asn Leu
   1205 1210 1215
   Lys Cys Leu Gly Leu Leu Glu Pro Tyr Phe Trp Lys Lys Gly Glu
   1220 1225 1230
   Ala Cys Ala Val Arg Gly Ser Asp Thr Leu Trp Tyr Arg Gly Lys
   1235 1240 1245
   Val Met Glu Val Val Gly Gly Ala Val Arg Val Gln Tyr Leu Asp
   1250 1255 1260
   His Gly Phe Thr Glu Lys Ile Pro Gln Cys His Leu Tyr Pro Ile
   1265 1270 1275
   Leu Leu Tyr Pro Asp Ile Pro Gln Phe Cys Ile Pro Cys Gln Leu
   1280 1285 1290
   His Asn Thr Thr Pro Val Gly Asn Val Trp Gln Pro Asp Ala Ile
   1295 1300 1305
   Glu Val Leu Gln Gln Leu Leu Ser Lys Arg Gln Val Asp Ile His
   1310 1315 1320
   Ile Met Glu Leu Pro Lys Asn Pro Trp Glu Lys Leu Ser Ile His
   1325 1330 1335
   Leu Tyr Phe Asp Gly Met Ser Leu Ser Tyr Phe Met Ala Tyr Tyr
   1340 1345 1350
   Lys Tyr Cys Thr Ser Glu His Thr Glu Glu Met Leu Lys Glu Lys
   1355 1360 1365
   Pro Arg Ser Asp His Asp Lys Lys Tyr Glu Glu Glu Gln Trp Glu
   1370 1375 1380
   Ile Arg Phe Glu Glu Leu Leu Ser Ala Glu Thr Asp Thr Pro Leu
   1385 1390 1395
   Leu Pro Pro Tyr Leu Ser Ser Ser Leu Pro Ser Pro Gly Glu Leu
   1400 1405 1410
   Tyr Ala Val Gln Val Lys His Val Val Ser Pro Asn Glu Val Tyr
   1415 1420 1425
   Ile Cys Leu Asp Ser Ile Glu Thr Ser Asn Gln Ser Asn Gln His
   1430 1435 1440
   Ser Asp Thr Asp Asp Ser Gly Val Ser Gly Glu Ser Glu Ser Glu
   1445 1450 1455
   Ser Leu Asp Glu Ala Leu Gln Arg Val Asn Lys Lys Val Glu Ala
   1460 1465 1470
   Leu Pro Pro Leu Thr Asp Phe Arg Thr Glu Met Pro Cys Leu Ala
   1475 1480 1485
   Glu Tyr Asp Asp Gly Leu Trp Tyr Arg Ala Lys Ile Val Ala Ile
   1490 1495 1500
   Lys Glu Phe Asn Pro Leu Ser Ile Leu Val Gln Phe Val Asp Tyr
   1505 1510 1515
   Gly Ser Thr Ala Lys Leu Thr Leu Asn Arg Leu Cys Gln Ile Pro
   1520 1525 1530
   Ser His Leu Met Arg Tyr Pro Ala Arg Ala Ile Lys Val Leu Leu
   1535 1540 1545
   Ala Gly Phe Lys Pro Pro Leu Arg Asp Leu Gly Glu Thr Arg Ile
   1550 1555 1560
   Pro Tyr Cys Pro Lys Trp Ser Met Glu Ala Leu Trp Ala Met Ile
   1565 1570 1575
   Asp Cys Leu Gln Gly Lys Gln Leu Tyr Ala Val Ser Met Ala Pro
   1580 1585 1590
   Ala Pro Glu Gln Ile Val Thr Leu Tyr Asp Asp Glu Gln His Pro
   1595 1600 1605
   Val His Met Pro Leu Val Glu Met Gly Leu Ala Asp Lys Asp Glu
   1610 1615 1620
<210> 42
   <211> 443
   <212> PRT
   <213> Homo sapiens
<400> 42
   Met Arg Asn Ala Ile Ile Gln Gly Leu Phe Tyr Gly Ser Leu Thr Phe
   1               5                   10                  15
   Gly Ile Trp Thr Ala Leu Leu Phe Ile Tyr Leu His His Asn His Val
               20                  25                  30
   Ser Ser Trp Gln Lys Lys Ser Gln Glu Pro Leu Ser Ala Trp Ser Pro
           35                  40                  45
   Gly Lys Lys Val His Gln Gln Ile Ile Tyr Gly Ser Glu Gln Ile Pro
       50                  55                  60
   Lys Pro His Val Ile Val Lys Arg Thr Asp Glu Asp Lys Ala Lys Ser
   65                  70                  75                  80
   Met Leu Gly Thr Asp Phe Asn His Thr Asn Pro Glu Leu His Lys Glu
                   85                  90                  95
   Leu Leu Lys Tyr Gly Phe Asn Val Ile Ile Ser Arg Ser Leu Gly Ile
               100                 105                 110
   Glu Arg Glu Val Pro Asp Thr Arg Ser Lys Met Cys Leu Gln Lys His
           115                 120                 125
   Tyr Pro Ala Arg Leu Pro Thr Ala Ser Ile Val Ile Cys Phe Tyr Asn
       130                 135                 140
   Glu Glu Cys Asn Ala Leu Phe Gln Thr Met Ser Ser Val Thr Asn Leu
   145                 150                 155                 160
   Thr Pro His Tyr Phe Leu Glu Glu Ile Ile Leu Val Asp Asp Met Ser
                   165                 170                 175
   Lys Val Asp Asp Leu Lys Glu Lys Leu Asp Tyr His Leu Glu Thr Phe
               180                 185                 190
   Arg Gly Lys Val Lys Ile Ile Arg Asn Lys Lys Arg Glu Gly Leu Ile
           195                 200                 205
   Arg Ala Arg Leu Ile Gly Ala Ser His Ala Ser Gly Asp Val Leu Val
       210                 215                 220
   Phe Leu Asp Ser His Cys Glu Val Asn Arg Val Trp Leu Glu Pro Leu
   225 230 235 240
   Leu His Ala Ile Ala Lys Asp Pro Lys Met Val Val Cys Pro Leu Ile
   245 250 255
   Asp Val Ile Asp Asp Arg Thr Leu Glu Tyr Lys Pro Ser Pro Leu Val
   260 265 270
   Arg Gly Thr Phe Asp Trp Asn Leu Gln Phe Lys Trp Asp Asn Val Phe
   275 280 285
   Ser Tyr Glu Met Asp Gly Pro Glu Gly Ser Thr Lys Pro Ile Arg Ser
   290 295 300
   Pro Ala Met Ser Gly Gly Ile Phe Ala Ile Arg Arg His Tyr Phe Asn
   305 310 315 320
   Glu Ile Gly Gln Tyr Asp Lys Asp Met Asp Phe Trp Gly Arg Glu Asn
   325 330 335
   Leu Glu Leu Ser Leu Arg Ile Trp Met Cys Gly Gly Gln Leu Phe Ile
   340 345 350
   Ile Pro Cys Ser Arg Val Gly His Ile Ser Lys Lys Gln Thr Gly Lys
   355 360 365
   Pro Ser Thr Ile Ile Ser Ala Met Thr His Asn Tyr Leu Arg Leu Val
   370 375 380
   His Val Trp Leu Asp Glu Tyr Lys Glu Gln Phe Phe Leu Arg Lys Pro
   385 390 395 400
   Gly Leu Lys Tyr Val Thr Tyr Gly Asn Ile Arg Glu Arg Val Glu Leu
   405 410 415
   Arg Lys Arg Leu Gly Cys Lys Ser Phe Gln Trp Tyr Leu Asp Asn Val
   420 425 430
   Phe Pro Glu Leu Glu Ala Ser Val Asn Ser Leu
   435 440
<210> 43
   <211> 735
   <212> PRT
   <213> Homo sapiens
<400> 43
   Met His Cys Gly Leu Leu Glu Glu Pro Asp Met Asp Ser Thr Glu Ser
   1 5 10 15
   Trp Ile Glu Arg Cys Leu Asn Glu Ser Glu Asn Lys Arg Tyr Ser Ser
   20 25 30
   His Thr Ser Leu Gly Asn Val Ser Asn Asp Glu Asn Glu Glu Lys Glu
   35 40 45
   Asn Asn Arg Ala Ser Lys Pro His Ser Thr Pro Ala Thr Leu Gln Trp
   50 55 60
   Leu Glu Glu Asn Tyr Glu Ile Ala Glu Gly Val Cys Ile Pro Arg Ser
   65 70 75 80
   Ala Leu Tyr Met His Tyr Leu Asp Phe Cys Glu Lys Asn Asp Thr Gln
   85 90 95
   Pro Val Asn Ala Ala Ser Phe Gly Lys Ile Ile Arg Gln Gln Phe Pro
   100 105 110
   Gln Leu Thr Thr Arg Arg Leu Gly Thr Arg Gly Gln Ser Lys Tyr His
   115 120 125
   Tyr Tyr Gly Ile Ala Val Lys Glu Ser Ser Gln Tyr Tyr Asp Val Met
   130 135 140
   Tyr Ser Lys Lys Gly Ala Ala Trp Val Ser Glu Thr Gly Lys Lys Glu
   145 150 155 160
   Val Ser Lys Gln Thr Val Ala Tyr Ser Pro Arg Ser Lys Leu Gly Thr
   165 170 175
   Leu Leu Pro Glu Phe Pro Asn Val Lys Asp Leu Asn Leu Pro Ala Ser
   180 185 190
   Leu Pro Glu Glu Lys Val Ser Thr Phe Ile Met Met Tyr Arg Thr His
   195 200 205
   Cys Gln Arg Ile Leu Asp Thr Val Ile Arg Ala Asn Phe Asp Glu Val
   210 215 220
   Gln Ser Phe Leu Leu His Phe Trp Gln Gly Met Pro Pro His Met Leu
   225 230 235 240
   Pro Val Leu Gly Ser Ser Thr Val Val Asn Ile Val Gly Val Cys Asp
   245 250 255
   Ser Ile Leu Tyr Lys Ala Ile Ser Gly Val Leu Met Pro Thr Val Leu
   260 265 270
   Gln Ala Leu Pro Asp Ser Leu Thr Gln Val Ile Arg Lys Phe Ala Lys
   275 280 285
   Gln Leu Asp Glu Trp Leu Lys Val Ala Leu His Asp Leu Pro Glu Asn
   290 295 300
   Leu Arg Asn Ile Lys Phe Glu Leu Ser Arg Arg Phe Ser Gln Ile Leu
   305 310 315 320
   Arg Arg Gln Thr Ser Leu Asn His Leu Cys Gln Ala Ser Arg Thr Val
   325 330 335
   Ile His Ser Ala Asp Ile Thr Phe Gln Met Leu Glu Asp Trp Arg Asn
   340 345 350
   Val Asp Leu Asn Ser Ile Thr Lys Gln Thr Leu Tyr Thr Met Glu Asp
   355 360 365
   Ser Arg Asp Glu His Arg Lys Leu Ile Thr Gln Leu Tyr Gln Glu Phe
   370 375 380
   Asp His Leu Leu Glu Glu Gln Ser Pro Ile Glu Ser Tyr Ile Glu Trp
   385 390 395 400
   Leu Asp Thr Met Val Asp Arg Cys Val Val Lys Val Ala Ala Lys Arg
   405 410 415
   Gln Gly Ser Leu Lys Lys Val Ala Gln Gln Phe Leu Leu Met Trp Ser
   420 425 430
   Cys Phe Gly Thr Arg Val Ile Arg Asp Met Thr Leu His Ser Ala Pro
   435 440 445
   Ser Phe Gly Ser Phe His Leu Ile His Leu Met Phe Asp Asp Tyr Val
   450 455 460
   Leu Tyr Leu Leu Glu Ser Leu His Cys Gln Glu Arg Ala Asn Glu Leu
   465 470 475 480
   Met Arg Ala Met Lys Gly Glu Gly Ser Thr Ala Glu Val Arg Glu Glu
   485 490 495
   Ile Ile Leu Thr Glu Ala Ala Ala Pro Thr Pro Ser Pro Val Pro Ser
   500 505 510
   Phe Ser Pro Ala Lys Ser Ala Thr Ser Val Glu Val Pro Pro Pro Ser
   515 520 525
   Ser Pro Val Ser Asn Pro Ser Pro Glu Tyr Thr Gly Leu Ser Thr Thr
   530 535 540
   Gly Ala Met Gln Ser Tyr Thr Trp Ser Leu Thr Tyr Thr Val Thr Thr
   545 550 555 560
   Ala Ala Gly Ser Pro Ala Glu Asn Ser Gln Gln Leu Pro Cys Met Arg
   565 570 575
   Asn Thr His Val Pro Ser Ser Ser Val Thr His Arg Ile Pro Val Tyr
   580 585 590
   Pro His Arg Glu Glu His Gly Tyr Thr Gly Ser Tyr Asn Tyr Gly Ser
   595 600 605
   Tyr Gly Asn Gln His Pro His Pro Met Gln Ser Gln Tyr Pro Ala Leu
   610 615 620
   Pro His Asp Thr Ala Ile Ser Gly Pro Leu His Tyr Ala Pro Tyr His
   625 630 635 640
   Arg Ser Ser Ala Gln Tyr Pro Phe Asn Ser Pro Thr Ser Arg Met Glu
   645 650 655
   Pro Cys Leu Met Ser Ser Thr Pro Arg Leu His Pro Thr Pro Val Thr
   660 665 670
   Pro Arg Trp Pro Glu Val Pro Ser Ala Asn Thr Cys Tyr Thr Ser Pro
   675 680 685
   Ser Val His Ser Ala Arg Tyr Gly Asn Ser Ser Asp Met Tyr Thr Pro
   690 695 700
   Leu Thr Thr Arg Arg Asn Ser Glu Tyr Glu His Met Gln His Phe Pro
   705 710 715 720
   Gly Phe Ala Tyr Ile Asn Gly Glu Ala Ser Thr Gly Trp Ala Lys
   725 730 735
<210> 44
   <211> 168
   <212> PRT
   <213> Homo sapiens
<400> 44
   Met Ser Leu Thr His Arg Leu His Leu Cys Lys Tyr Trp Gly Cys Ala
   1 5 10 15
   Val Ser Asn Val Cys Arg Phe Trp Glu Gly Arg Pro Leu Pro Leu Met
   20 25 30
   Ile Val Val Pro Tyr Thr Leu Pro Val Ser Leu Pro Val Gly Ser Cys
   35 40 45
   Val Ile Ile Thr Gly Thr Pro Ile Leu Thr Phe Val Lys Asp Pro Gln
   50 55 60
   Leu Glu Val Asn Phe Tyr Thr Gly Met Asp Glu Asp Ser Asp Ile Ala
   65 70 75 80
   Phe Gln Phe Arg Leu His Phe Gly His Pro Ala Ile Met Asn Ser Cys
   85 90 95
   Val Phe Gly Ile Trp Arg Tyr Glu Glu Lys Cys Tyr Tyr Leu Pro Phe
   100 105 110
   Glu Asp Gly Lys Pro Phe Glu Leu Cys Ile Tyr Val Arg His Lys Glu
   115 120 125
   Tyr Lys Val Met Val Asn Gly Gln Arg Ile Tyr Asn Phe Ala His Arg
   130 135 140
   Phe Pro Pro Ala Ser Val Lys Met Leu Gln Val Phe Arg Asp Ile Ser
   145 150 155 160
   Leu Thr Arg Val Leu Ile Ser Asp
   165
<210> 45
   <211> 259
   <212> PRT
   <213> Homo sapiens
<400> 45
   Met Ser Glu Val Pro Val Ala Arg Val Trp Leu Val Leu Leu Leu Leu
   1 5 10 15
   Thr Val Gln Val Gly Val Thr Ala Gly Ala Pro Trp Gln Cys Ala Pro
   20 25 30
   Cys Ser Ala Glu Lys Leu Ala Leu Cys Pro Pro Val Ser Ala Ser Cys
   35 40 45
   Ser Glu Val Thr Arg Ser Ala Gly Cys Gly Cys Cys Pro Met Cys Ala
   50 55 60
   Leu Pro Leu Gly Ala Ala Cys Gly Val Ala Thr Ala Arg Cys Ala Arg
   65 70 75 80
   Gly Leu Ser Cys Arg Ala Leu Pro Gly Glu Gln Gln Pro Leu His Ala
   85 90 95
   Leu Thr Arg Gly Gln Gly Ala Cys Val Gln Glu Ser Asp Ala Ser Ala
   100 105 110
   Pro His Ala Ala Glu Ala Gly Ser Pro Glu Ser Pro Glu Ser Thr Glu
   115 120 125
   Ile Thr Glu Glu Glu Leu Leu Asp Asn Phe His Leu Met Ala Pro Ser
   130 135 140
   Glu Glu Asp His Ser Ile Leu Trp Asp Ala Ile Ser Thr Tyr Asp Gly
   145 150 155 160
   Ser Lys Ala Leu His Val Thr Asn Ile Lys Lys Trp Lys Glu Pro Cys
   165 170 175
   Arg Ile Glu Leu Tyr Arg Val Val Glu Ser Leu Ala Lys Ala Gln Glu
   180 185 190
   Thr Ser Gly Glu Glu Ile Ser Lys Phe Tyr Leu Pro Asn Cys Asn Lys
   195 200 205
   Asn Gly Phe Tyr His Ser Arg Gln Cys Glu Thr Ser Met Asp Gly Glu
   210 215 220
   Ala Gly Leu Cys Trp Cys Val Tyr Pro Trp Asn Gly Lys Arg Ile Pro
   225 230 235 240
   Gly Ser Pro Glu Ile Arg Gly Asp Pro Asn Cys Gln Ile Tyr Phe Asn
   245 250 255
   Val Gln Asn
<210> 46
   <211> 450
   <212> PRT
   <213> Homo sapiens
<400> 46
   Met Arg Glu Cys Ile Ser Ile His Val Gly Gln Ala Gly Val Gln Ile
   1 5 10 15
   Gly Asn Ala Cys Trp Glu Leu Tyr Cys Leu Glu His Gly Ile Gln Pro
   20 25 30
   Asp Gly Gln Met Pro Ser Asp Lys Thr Ile Gly Gly Gly Asp Asp Ser
   35 40 45
   Phe Asn Thr Phe Phe Ser Glu Thr Gly Ala Gly Lys His Val Pro Arg
   50 55 60
   Ala Val Phe Val Asp Leu Glu Pro Thr Val Val Asp Glu Val Arg Thr
   65 70 75 80
   Gly Thr Tyr Arg Gln Leu Phe His Pro Glu Gln Leu Ile Thr Gly Lys
   85 90 95
   Glu Asp Ala Ala Asn Asn Tyr Ala Arg Gly His Tyr Thr Ile Gly Lys
   100 105 110
   Glu Ile Val Asp Leu Val Leu Asp Arg Ile Arg Lys Leu Ala Asp Leu
   115 120 125
   Cys Thr Gly Leu Gln Gly Phe Leu Ile Phe His Ser Phe Gly Gly Gly
   130 135 140
   Thr Gly Ser Gly Phe Ala Ser Leu Leu Met Glu Arg Leu Ser Val Asp
   145 150 155 160
   Tyr Gly Lys Lys Ser Lys Leu Glu Phe Ala Ile Tyr Pro Ala Pro Gln
   165 170 175
   Val Ser Thr Ala Val Val Glu Pro Tyr Asn Ser Ile Leu Thr Thr His
   180 185 190
   Thr Thr Leu Glu His Ser Asp Cys Ala Phe Met Val Asp Asn Glu Ala
   195 200 205
   Ile Tyr Asp Ile Cys Arg Arg Asn Leu Asp Ile Glu Arg Pro Thr Tyr
   210 215 220
   Thr Asn Leu Asn Arg Leu Ile Gly Gln Ile Val Ser Ser Ile Thr Ala
   225 230 235 240
   Ser Leu Arg Phe Asp Gly Ala Leu Asn Val Asp Leu Thr Glu Phe Gln
   245 250 255
   Thr Asn Leu Val Pro Tyr Pro Arg Ile His Phe Pro Leu Ala Thr Tyr
   260 265 270
   Ala Pro Val Ile Ser Ala Glu Lys Ala Tyr His Glu Gln Leu Ser Val
   275 280 285
   Ala Glu Ile Thr Asn Ala Cys Phe Glu Pro Ala Asn Gln Met Val Lys
   290 295 300
   Cys Asp Pro Arg His Gly Lys Tyr Met Ala Cys Cys Met Leu Tyr Arg
   305 310 315 320
   Gly Asp Val Val Pro Lys Asp Val Asn Ala Ala Ile Ala Thr Ile Lys
   325 330 335
   Thr Lys Arg Thr Ile Gln Phe Val Asp Trp Cys Pro Thr Gly Phe Lys
   340 345 350
   Val Gly Ile Asn Tyr Gln Pro Pro Thr Val Val Pro Gly Gly Asp Leu
   355 360 365
   Ala Lys Val Gln Arg Ala Val Cys Met Leu Ser Asn Thr Thr Ala Ile
   370 375 380
   Ala Glu Ala Trp Ala Arg Leu Asp His Lys Phe Asp Leu Met Tyr Ala
   385 390 395 400
   Lys Arg Ala Phe Val His Trp Tyr Val Gly Glu Gly Met Glu Glu Gly
   405 410 415
   Glu Phe Ser Glu Ala Arg Glu Asp Leu Ala Ala Leu Glu Lys Asp Tyr
   420 425 430
   Glu Glu Val Gly Val Asp Ser Val Glu Ala Glu Ala Glu Glu Gly Glu
   435 440 445
   Glu Tyr
   450
<210> 47
   <211> 544
   <212> DNA
   <213> Artificial Sequence
<220>
   <223> probe derived from SOX30
<400> 47
   gaaagttttc agtcgtgatt tgaggagtta aaaccaaatg caatttatgt cttcataaaa 60
   ttttgattag tgaaactaga gtctggatgt ttcattgtag gaatatttaa gttattaagt 120
   agtttaattt taatggctga aatttgcatc aacatgtatt attattactt tatcctggaa 180
   catgcaaaat actgaagcct cacagttgta tgtgagggga aaggggaaat aaatctagca 240
   tagtgtgatt tttattttat ctcaggatac attttttaaa tgattttttg tttgcttttt 300
   atgtaatact tatggatgtt gtcaattttt gatgtaacat tttgaaagta ttttgacaac 360
   tcctagtgaa cttggacttg gttgctaaat ttaacttaca ctaataacca attataagtt 420
   ccaaatgtgt tttaatggca cctgggtgat tcttcagcta aatttagtca tttctgtttc 480
   taaatatttt tatcatttta aaatattttt tttccatttg gcatacatcg ttctttgttg 540
   taat 544
<210> 48
   <211> 289
   <212> DNA
   <213> Artificial Sequence
<220>
   <223> probe derived from SPATA22
<400> 48
   gatcgtgaac ttccgagact gattagaggc cgagttcata gatgtgttgg caactatgac 60
   cagaaaaaga acattttcca atgtgtttct gtcagaccgg cgtctgtttc tgaacaaaaa 120
   actttccagg catttgtcaa aattgcagat gttgagatgc agtattatat taatgtgatg 180
   aatgaaactt aagtagtgat aaaaggaagt ttagcataaa ttatagcagt tttctgttat 240
   tgcttaattt accatctcca tagttttata gctactattg tatttcact 289
<210> 49
   <211> 386
   <212> DNA
   <213> Artificial Sequence
<220>
   <223> probe derived from MAEL
<400> 49
   gaggccagca atagtgtgac acccaaaatg gttgtattgg atgcagggcg ttaccagaag 60
   ctaagggttg ggagttcagg attctctcat ttcaactctt ctaatgagga acaaagatca 120
   aacacaccca ttggtgacta cccatctagg gcaaaaattt ctggccaaaa cagcagcgtt 180
   cggggaagag gaattacccg cttactagag agcatttcca attcttccag caatatccac 240
   aaattctcca actgtgacac ttcactctca ccttacatgt cccaaaaaga tggatacaaa 300
   tctttctctt ccttatctta atgatggtac tcttttcaat ttctgaaaac agtaacaggc 360
   ccaacttcct tcttactaca gtcata 386
<210> 50
   <211> 313
   <212> DNA
   <213> Artificial Sequence
<220>
   <223> probe derived from COX8C
<220>
   <221> misc_feature
   <222> (47).. (47)
   <223> n is a, c, g, or t
<400> 50
   ccctgtctgc cgcggaaatg gctgttggac ttgtggtgtt ttttacnacc ttcttaacac 60
   cagctgcata tgtgctaggc aacctgaagc agttcagaag gaattagatg gaagatgatg 120
   ttgaacagct gttaacgtcc aaaaaacttt cagaaaaagc tgtgtttttg ttaacgagca 180
   aaattgccta gttgagttga tgcaaccatt gtggtattca ctttcctcat gtttatgatg 240
   aatattttgc acttttttag tactgtgcat tatatagatg tatagtcaaa aatgttctgc 300
   ttaagtgtta aat 313
<210> 51
   <211> 521
   <212> DNA
   <213> Artificial Sequence
<220>
   <223> probe derived from TKTL1
<400> 51
   ccttcattca tccctagttc ggaaattcaa gctaactact taccctttaa actgtcactg 60
   catatgcaag taccgctcta atttttggat cattaaaggg agttacacaa cttttaagtg 120
   aaaaaaatag gtaacaaaac aaccacctga tagtaagttt tctgataaga ctatagataa 180
   gtggtagagg taatcaattc ttccgaagtg tttccttcgt gaataactgg tagaggtaat 240
   agttttttca atgtatttcc ttcatgagta aagaaaatgt ggattgaagt atagattcca 300
   gtagcctagt ttccacagca cgataacacc atgacgccta ctgctgttcc caccttggga 360
   ttctgtgtgc tgccatccca cctgcagctg ccctggaatt cccttcgctg tttgccttca 420
   tctccctcca cgtttgagag gctgtcaggc agcagcgaaa gcttgttagg atgtcctgtg 480
   ctgcttgtga tgagagcctc cacactgtac tgttcaagtc a 521
<210> 52
   <211> 355
   <212> DNA
   <213> Artificial Sequence
<220>
   <223> probe derived from RBM46
<220>
   <221> misc_feature
   <222> (41)..(41)
   <223> n is a, c, g, or t
<220>
   <221> misc_feature
   <222> (56)..(56)
   <223> n is a, c, g, or t
<400> 52
   agcacttgaa gacaatggtt atagtagatt tgattaccaa ngatcactat ctgtanctgg 60
   agattagaac aattatatga ccagaagcat ctaaccatta tgtaaaaaga aatgatgaga 120
   caaaaagatt aagatacaaa ttttgtgcag tactaaagaa aaagcagtct accattgtgg 180
   tccttgaaaa taactataga tatttttgtt atttgttaga cacaaattat aattttgttg 240
   ttaatgtatt taagcatttt atagttatgc tttgtgtttt tgatattctt tgtattgtta 300
   ataacaagtg ttatgggttt ttaatgttga aatcatgtgt taatttttgt acttg 355
<210> 53
   <211> 279
   <212> DNA
   <213> Artificial Sequence
<220>
   <223> probe derived from MAGEB6
<220>
   <221> misc_feature
   <222> (138)..(138)
   <223> n is a, c, g, or t
<220>
   <221> misc_feature
   <222> (167)..(167)
   <223> n is a, c, g, or t
<400> 53
   tcacattcat ggctgtttaa ccaatctgaa agttacggtt tgggaattaa taaaacaaag 60
   tcatacaaca cattttcttt gtaattgaga actagataac atggtaacag agaattgatt 120
   ttcatatgaa tcttaacncc acagtaaaat agttgacatc ataatangaa gagaaagaaa 180
   aggaaaaaca gaaatgtaaa agttgtttaa ttcttggttt gcctaattcg ttttcctatt 240
   tcttttcata caaataaagg atacctggat ttatttagg 279
<210> 54
   <211> 497
   <212> DNA
   <213> Artificial Sequence
<220>
   <223> probe derived from NBPF4
<220>
   <221> misc_feature
   <222> (284)..(284)
   <223> n is a, c, g, or t
<220>
   <221> misc_feature
   <222> (393)..(393)
   <223> n is a, c, g, or t
<220>
   <221> misc_feature
   <222> (438)..(438)
   <223> n is a, c, g, or t
<400> 54
   gcagagaagg cccagtgtgt ccatccccag tgcggtgata ctaggatggt cacttggtta      60
   aggaggggtc taggagctct gtcccttgta aagacatctt atttgtaagt aatttggaaa     120
   gtggtttgaa atagtataaa tatcctgtat tctagtgatc ttcttcagaa cattttatca     180
   ccaattaatc accccgtctg tgtcagttat tatatttaag tttgtacatt gaaaattgtc     240
   tatctcaaaa tcttacctta tacttgcttt tgctggcatt cttngtaaaa aagatcattc     300
   cctgcccaaa ttttaacttt catccaaaat taattttaat ttctttttgc tggcattctg     360
   ttgtgaaaaa gaatattctc tgccccaatt atnactttca tccaaaatta attttagtcc     420
   atcagttaaa attttaantt ttaaatctgt ttaattaaaa catttcttgc ctctcactct     480
   ggactattgg atttttt                                                     497
<210> 55
   <211> 417
   <212> DNA
   <213> Artificial Sequence
<220>
   <223> probe derived from C12orf37
<400> 55
   ttcctcctga ccacaaaaag cacttatact ctaggatgac tgattccagc ccagtggcct      60
   ggcaagggtg aattacacct tgcatatcac actcttgaca tttgtgtgcg ctagcataag     120
   aattataatt gaaacaggga tttaagtatc tcctctctag gtgcctaccc tccttggact     180
   caggtcaaat ttattaaagg aagttttgtt tctagatagg ttgtttgaaa taaaataaca     240
   gaatgttcaa gtaacacagt gtacctacag cttttaacaa aattgaggac ttgggtctcg     300
   aaacaatttc ctttgatttt caggtatttt atctataaaa agggagataa agcattagtt     360
   cataggacag ttatatgttt aaatgtgata atgtatatta accaccttgc atgtatt        417
<210> 56
   <211> 364
   <212> DNA
   <213> Artificial Sequence
<220>
   <223> probe derived from TPTE2P2
<220>
   <221> misc_feature
   <222> (37)..(37)
   <223> n is a, c, g, or t
<220>
   <221> misc_feature
   <222> (90)..(90)
   <223> n is a, c, g, or t
<220>
   <221> misc_feature
   <222> (92)..(92)
   <223> n is a, c, g, or t
<220>
   <221> misc_feature
   <222> (102)..(102)
   <223> n is a, c, g, or t
<220>
   <221> misc_feature
   <222> (107)..(107)
   <223> n is a, c, g, or t
<220>
   <221> misc_feature
   <222> (120)..(120)
   <223> n is a, c, g, or t
<220>
   <221> misc_feature
   <222> (179)..(179)
   <223> n is a, c, g, or t
<220>
   <221> misc_feature
   <222> (214)..(214)
   <223> n is a, c, g, or t
<220>
   <221> misc_feature
   <222> (222)..(222)
   <223> n is a, c, g, or t
<220>
   <221> misc_feature
   <222> (277)..(277)
   <223> n is a, c, g, or t
<400> 56
   ctgaggttct attcatggtg agcaagtctt ttttttngtt tgtttcttca agctctaaca 60
   agggtgccta ctacatggct tttcagttan cnccaaaata anatgtnaca attttttttn 120
   ctattcttag gctttatcta caaagaaatg aattggataa tcttcataaa caaaaaacnt 180
   ggaaaattta tcaaccagaa tatgcagtag aganatattt tnatgagaaa tgacttaagt 240
   tatgttgtaa ctggtagctg attaagtata gttcccngca ccccttctgg gaaagaatta 300
   tgttctttct aaccctgcca catagttata tgttctaaat cttccttgct ggtacatcta 360
   tatt 364
<210> 57
   <211> 504
   <212> DNA
   <213> Artificial Sequence
<220>
   <223> probe derived from DPEP3
<400> 57
   gggcagtcat tggatctgag ttcatcggga ttggtggaaa ttatgacggg actggccggt 60
   tccctcaggg gctggaggat gtgtccacat acccagtcct gatagaggag ttgctgagtc 120
   gtagctggag cgaggaagag cttcaaggtg tccttcgtgg aaacctgctg cgggtcttca 180
   gacaagtgga aaaggtgaga gaggagagca gggcgcagag ccccgtggag gctgagtttc 240
   catatgggca actgagcaca tcctgccact cccacctcgt gcctcagaat ggacaccagg 300
   ctactcatct ggaggtgacc aagcagccaa ccaatcgggt cccctggagg tcctcaaatg 360
   cctccccata ccttgttcca ggccttgtgg ctgctgccac catcccaacc ttcacccagt 420
   ggctctgctg acacagtcgg tccccgcaga ggtcactgtg gcaaagcctc acaaagcccc 480
   ctctcctagt tcattcacaa gcat 504
<210> 58
   <211> 446
   <212> DNA
   <213> Artificial Sequence
<220>
   <223> probe derived from C10orf82
<400> 58
   aggtcactga atttggctgt ggcacgagat acactgtcat ggccaaaaac tgctacaagg 60
   acttcctgga gatcacggag agggccaaga aggcacatct gaaaccatat gaagaaatat 120
   atggagttag ctccacaaaa acttctgctc cgtctccaaa agttttgcag catgaagagc 180
   tgctgccaaa atatcccgat ttttctattc cagatggaag ctgccctgcc cttggaaggc 240
   ccctgagaga ggaccccaaa actccgctga catgtggctg tgctcagagg ccaagtatac 300
   catgcagtgg gaagatgtat ctagagccac tgtcctccgc aaagtatgca gaaggctaga 360
   agcgcagagt ctcccaagga ggtgaacttt aagtggggct tccaaaacct gccattctca 420
   tgttggaatc acgcccagtg agcaat 446
<210> 59
   <211> 262
   <212> DNA
   <213> Artificial Sequence
<220>
   <223> probe derived from LOC440896
<220>
   <221> misc_feature
   <222> (234)..(234)
   <223> n is a, c, g, or t
<400> 59
   agctgagtgc cacgtgctga cgtcactaag atcaatacag caaactctga aagatggaca 60
   gagagacagg agatggtcct ttataatgca gtgtgatctg tgctgcaata gagatggaag 120
   agtgttctga tcggctggaa aacaacgcac atgggaagct gtacagaaat gagtggggaa 180
   agttttggaa gctagaagtt caaaacgagg tgttggcagc accatgctct ctcngaagat 240
   gctaggaaga atctgctcca tg 262
<210> 60
   <211> 503
   <212> DNA
   <213> Artificial Sequence
<220>
   <223> probe derived from CDNA clone IMAGE:5265646
<220>
   <221> misc_feature
   <222> (189)..(196)
   <223> n is a, c, g, or t
<220>
   <221> misc_feature
   <222> (215)..(215)
   <223> n is a, c, g, or t
<400> 60
   gataccaaca tctaatccgc caagaaagaa tttcagaatt aaaatttggg tgtgttcttg 60
   cgctggggat ttctctgacc taccttctga tagaactttg aacccgagtc aatagtcaat 120
   atactagttt tttacttaaa actgtaacat tttaatctaa ttttggggac gtgaaataaa 180
   actaatggnn nnnnnnagaa atttttatca ctggnaataa cctaattttg aaaacactga 240
   tggttagttt cttgaaatat taatattact acaagtcata agtaaaagca ttctatctta 300
   agtgagaaac tacaaagttg gataattact atttgagttt gtggcttggt ttgaataaac 360
   acttgcttgt tttaagtaaa agttcagctg aagtgacaat caacctttaa tcttgtaaag 420
   cttctgtgtt agatattttc tatctctaac atgccaaaca tgcatattaa actgagtttt 480
   tttgcatgca atttctgtgc cca 503
<210> 61
   <211> 361
   <212> DNA
   <213> Artificial Sequence
<220>
   <223> probe derived from HIST1H3C
<400> 61
   tccgcgcaag cagcttgcta ctaaagcagc ccgtaagagc gctccggcca ccggtggcgt 60
   gaagaaacct catcgctacc gcccgggcac cgtggccttg cgcgaaatcc gtcgctacca 120
   gaagtccacc gagctgctga tccggaagct gccgttccag cgcctggtgc gagaaatcgc 180
   ccaggacttc aaaaccgacc tgcgtttcca gagctctgcg gtgatggcgc tgcaggaggc 240
   ttgcgaggcc tacctggtgg gactcttcga agacaccaat ctgtgcgcta ttcacgctaa 300
   acgcgtcacc atcatgccca aagatatcca gctggcacgt cgcatccgtg gggaaagggc 360
   a 361
<210> 62
   <211> 546
   <212> DNA
   <213> Artificial Sequence
<220>
   <223> probe derived from PIWIL1
<220>
   <221> misc_feature
   <222> (148)..(148)
   <223> n is a, c, g, or t
<400> 62
   attcaccggc ttccttattt tatatgtaaa aattaagatt ttatatttta tcttcttgtt 60
   tctcatagat attttgtgag catttttttg tttattttga agaaatgtgg ataagatact 120
   tggtagtata aaacagactc tctgagangt atttgaaatg tgtttggaga tttacttaaa 180
   cgtactttca ggagtgagca agtcctactt ataaacctat attaacttta tttttgagat 240
   acctgttttg aatttaaagg agataagagg cgtaaagtag gatgctcact acaaccatag 300
   gtggggtttc agctcatatc ttaaagataa aaggtactat tatataacct atacacaaga 360
   tacaggagaa aatatgcttg atttttattt ggcagggggg ctaggttgta tgggagtaaa 420
   aaaaacattg aaaattttta aattgtccaa agaaacattt taagactctt taacaaaaaa 480
   ggccatgagt aaatctctat attaacatta ctatttattt tgttttggaa ctgggacatg 540
   attcta 546
<210> 63
   <211> 521
   <212> DNA
   <213> Artificial Sequence
<220>
   <223> probe derived from C19orf41
<400> 63
   ggccgtgcgt gatgggcatg gaggggcctt tcttccggga ctacgcgctg aacgtgtttg 60
   tggggaaagt ggagacaaat caactggacc ttgtggcgtc ctttgtcaag aaccaaacgc 120
   agcacttaat gggtaactct ctgaaagatg agcctctgct ggaagagctg gtgaccctca 180
   gggcgaatgt gatcaaggaa ttcaagaaag ttttaatttc atatgaatta aaagcctgca 240
   accccaaact ttgccgcttg ctaaaagaag aggtgttgga ctgtttacat tgccagagga 300
   tcactcccaa gtgtatccac aaaaagtact gctttgtcga ccggcaaccc cgcgtggccc 360
   tgcagtacca gatggacagc aaatacccga ggaaccaggc gctgttgggc atcctcattt 420
   ctgtgtctct ggctgtcttt gtcttcgtgg tcatcgtggt ctcggcttgt acatacagac 480
   aaaaccgaaa actcctgctg cagtaggacg gtggtttggg g 521
<210> 64
   <211> 486
   <212> DNA
   <213> Artificial Sequence
<220>
   <223> probe derived from RNF17
<400> 64
   gaatttaatc ctttatctat cttagtacaa tttgttgatt atggatcaac tgcaaagctg 60
   acattaaaca gactgtgcca aattccttct catcttatgc ggtatccagc tcgagccata 120
   aaggttctct tggcagggtt taaacctccc ttaagggatc taggggagac aagaatacca 180
   tattgtccca aatggagcat ggaggcactg tgggctatga tagactgtct tcaaggaaaa 240
   caactctatg ctgtgtccat ggctccagca ccagaacaga tagtgacatt atatgacgat 300
   gaacagcatc cagttcatat gccgttggta gaaatggggc ttgcagataa agatgaataa 360
   gtgcctaagt gtatacagtg agagcatcta tagaagccta gaagaattct gttatgttta 420
   gactatgtct tatctttaga ctatttcagg cttaatttcc ctaacttgtt cagccctagt 480
   gcttta 486
<210> 65
   <211> 306
   <212> DNA
   <213> Artificial Sequence
<220>
   <223> probe derived from GALNTL5
<220>
   <221> misc_feature
   <222> (44)..(44)
   <223> n is a, c, g, or t
<400> 65
   ggaactttca ctaaggatct ggatgtgtgg aggccaactc tttnataatc ccctgctctc 60
   gagtaggaca tatcagtaag aaacaaactg gaaaaccttc tacaatcatc agtgctatga 120
   cacataacta cctaagactg gtgcacgttt ggctggatga atataaggag cagttttttc 180
   ttcgaaagcc tggtctgaaa tatgtcacct acggaaatat tcgcgagcgt gttgagttaa 240
   ggaaacgact gggttgcaag tcatttcagt ggtatttgga taatgtcttc ccagagttgg 300
   aggcat 306
<210> 66
   <211> 454
   <212> DNA
   <213> Artificial Sequence
<220>
   <223> probe derived from RFX4
<400> 66
   gatttatggc attgagtatc acactcagct ctgctgtgtt aactttgtga aactggatgg 60
<210> 67
   <211> 505
   <212> DNA
   <213> Artificial Sequence
<220>
   <223> probe derived from GALS14
<400> 67
   agaacaatgt catcactacc cgtaccatac acactgcctg tttccttgcc tgttggttcg 60
   tgcgtgataa tcacagggac accgatcctc acttttgtca aggacccaca gctggaggtg 120
   aatttctaca ctgggatgga tgaggactca gatattgctt tccaattccg actgcacttt 180
   ggtcatcctg caatcatgaa cagttgtgtg tttggcatat ggagatatga ggagaaatgc 240
   tactatttac cctttgaaga tggcaaacca tttgagctgt gcatctatgt gcgtcacaag 300
   gaatacaagg taatggtaaa tggccaacgc atttacaact ttgcccatcg attcccgcca 360
   gcatctgtga agatgctgca agtcttcaga gatatctccc tgaccagagt gcttatcagc 420
   gattgaggga gatgatcaga ctcctcattg ttgaggaatc cctctttcta cctgaccatg 480
   ggattcccag agcctactaa cagaa 505
<210> 68
   <211> 526
   <212> DNA
   <213> Artificial Sequence
<220>
   <223> probe derived from IGFBP1
<400> 68
   aataatgttc tgtcacgtga aatatttaag tatatagtat atttatactc tagaacatgc 60
   acatttatat atatatgtat atgtatatat atatagtaac tactttttat actccataca 120
   taacttgata tagaaagctg tttatttatt cactgtaagt ttattttttc tacacagtaa 180
   aaacttgtac tatgttaata acttgtccta tgtcaatttg tatatcatga aacacttctc 240
   atcatattgt atgtaagtaa ttgcatttct gctcttccaa agctcctgcg tctgttttta 300
   aagagcatgg aaaaatactg cctagaaaat gcaaaatgaa ataagagaga gtagtttttc 360
   agctagtttg aaggaggacg gttaacttgt atattccacc attcacattt gatgtacatg 420
   tgtagggaaa gttaaaagtg ttgattacat aatcaaagct acctgtggtg atgttgccac 480
   ctgttaaaat gtacactgga tatgttgtta aacacgtgtc gataat 526
<210> 69
   <211> 287
   <212> DNA
   <213> Artificial Sequence
<220>
   <223> probe derived from TUBA3C
<400> 69
   tctccggcag gtgggcatta actaccagcc ccccacggtg gtccctgggg gagacctggc 60
   caaggtgcag cgggctgtgt gcatgctgag caacaccacg gccatcgcgg aggcctgggc 120
   tcgcctggac cataagttcg atctcatgta tgccaagcgg gcctttgtgc actggtacgt 180
   gggagaaggc atggaggagg gggagttctc tgaggcccgc gaggacctgg cagctctgga 240
   gaaggattat gaagaggtgg gcgtggattc cgtggaagcc gaggctg 287
<210> 70
   <211> 1949
   <212> DNA
   <213> Homo sapiens
<400> 70
   aataaagggg tctgagccgg tcgcctgagc ctgaaaagtg ctgtcacgtc agcggaagga 60
   ggcgtcccag atcttctcag ctgtcttggt gccagccttc ctagtcttcc tacccacact 120
   cctacctgct gtcacaggcc acagccatca tgcctcgggg tcacaagagt aagctccgta 180
   cctgtgagaa acgccaagag accaatggtc agccacaggg tctcacgggt ccccaggcca 240
   ctgcagagaa gcaggaagag tcccactctt cctcatcctc ttctcgcgct tgtctgggtg 300
   attgtcgtag gtcttctgat gcctccattc ctcaggagtc tcagggagtg tcacccactg 360
   ggtctcctga tgcagttgtt tcatattcaa aatccgatgt ggctgccaac ggccaagatg 420
   agaaaagtcc aagcacctcc cgtgatgcct ccgttcctca ggagtctcag ggagcttcac 480
   ccactggctc tcctgatgca ggtgtttcag gctcaaaata tgatgtggct gccaacggcc 540
   aagatgagaa aagtccaagc acttcccatg atgtctccgt tcctcaggag tctcagggag 600
   cttcacccac tggctcgcct gatgcaggtg tttcaggctc aaaatatgat gtggctgccg 660
   agggtgaaga tgaggaaagt gtaagcgcct cacagaaagc catcattttt aagcgcttaa 720
   gcaaagatgc tgtaaagaag aaggcgtgca cgttggcgca attcctgcag aagaagtttg 780
   agaagaaaga gtccattttg aaggcagaca tgctgaagtg tgtccgcaga gagtacaagc 840
   cctacttccc tcagatcctc aacagaacct cccaacattt ggtggtggcc tttggcgttg 900
   aattgaaaga aatggattcc agcggcgagt cctacaccct tgtcagcaag ctaggcctcc 960
   ccagtgaagg aattctgagt ggtgataatg cgctgccgaa gtcgggtctc ctgatgtcgc 1020
   tcctggttgt gatcttcatg aacggcaact gtgccactga agaggaggtc tgggagttcc 1080
   tgggtctgtt ggggatatat gatgggatcc tgcattcaat ctatggggat gctcggaaga 1140
   tcattactga agatttggtg caagataagt acgtggttta ccggcaggtg tgcaacagtg 1200
   atcctccatg ctatgagttc ctgtggggtc cacgagccta tgctgaaacc accaagatga 1260
   gagtcctgcg tgttttggcc gacagcagta acaccagtcc cggtttatac ccacatctgt 1320
   atgaagacgc tttgatagat gaggtagaga gagcattgag actgagagct taaggcaggg 1380
   ctggcactat ttccttggcc agggtacctt atggggccat atcctacaga tcctcccatt 1440
   tctagggagg tctgaagtag aattttcact ttatgttaga agagagtagt gagctttcta 1500
   agtagtgcag tatagtagag gctggaggga acaagatatg tatctttctt ttgttacaca 1560
   tgagtaactt gcagatttat gttttatctc tgtcagttat caacattgtt cctgttaagt 1620
   gaaggtttat tttgcttcag attatacaat tatcaataac atagctctca cattcatggc 1680
   tgtttaacca atctgaaagt tacggtttgg gaattaataa aacaaagtca tacaacacat 1740
   tttctttgta attgagaact agataacatg gtaacagaga attgatttcc atatgaatct 1800
   taactccaca gtaaaatagt tgacatcata atatgaagag aaagaaaagg aaaaacagaa 1860
   atgtaaaagt tgtttaattc ttggtttgcc taattcgttt tcctatttct tttcatacaa 1920
   ataaaggata cctggattta tttaggtta 1949
<210> 71
   <211> 2145
   <212> DNA
   <213> Homo sapiens
<400> 71
   aaggcagggg gcggggcgtc tccgagcggc ggggccaagg gagggcacaa cagctgctac        60
   ctgaacagtt tctgacccaa cagttaccca gcgccggact cgctgcgccc cggcggctct       120
   agggaccccc ggcgcctaca cttagctccg cgcccgagag aatgttggac cgacgacaca       180
   agacctcaga cttgtgttat tctagcagct gaacacaccc caggctcttc tgaccggcag       240
   tggctctgga agcagtctgg tgtatagagt tatggattca ctaccagatt ctactgtatg       300
   ctcttgacaa ctatgaccac aatggtccac ccacaaatga attatcagga gtgaacccag       360
   aggcacgtat gaatgaaagt cctgatccga ctgacctggc gggagtcatc attgagctcg       420
   gccccaatga cagtccacag acaagtgaat ttaaaggagc aaccgaggag gcacctgcga       480
   aagaaagtgt gttagcacga ctttccaagt ttgaagttga agatgctgaa aatgttgctt       540
   catatgacag caagattaag aaaattgtgc attcaattgt atcatccttt gcatttggac       600
   tatttggagt tttcctggtc ttactggatg tcactctcat ccttgccgac ctaattttca       660
   ctgacagcaa actttatatt cctttggagt atcgttctat ttctctagct attgccttat       720
   tttttctcat ggatgttctt cttcgagtat ttgtagaaag gagacagcag tatttttctg 780
   acttatttaa cattttagat actgccatta ttgtgattct tctgctggtt gatgtcgttt 840
   acattttttt tgacattaag ttgcttagga atattcccag atggacacat ttacttcgac 900
   ttctacgact tattattctg ttaagaattt ttcatctgtt tcatcaaaaa agacaacttg 960
   aaaagctgat aagaaggcgg gtttcagaaa acaaaaggcg atacacaagg gatggatttg 1020
   acctagacct cacttacgtt acagaacgta ttattgctat gtcatttcca tcttctggaa 1080
   ggcagtcttt ctatagaaat ccaatcaagg aagttgtgcg gtttctagat aagaaacacc 1140
   gaaaccacta tcgagtctac aatctatgca gtgaaagagc ttacgatcct aagcacttcc 1200
   ataatagggt cgttagaatc atgattgatg atcataatgt ccccactcta catcagatgg 1260
   tggttttcac caaggaagta aatgagtgga tggctcaaga tcttgaaaac atcgtagcga 1320
   ttcactgtaa aggaggcaca gatagaacag gaactatggt ttgtgccttc cttattgcct 1380
   ctgaaatatg ttcaactgca aaggaaagcc tgtattattt tggagaaagg cgaacagata 1440
   aaacccacag cgaaaaattt cagggagtag aaactccttc tcagaagaga tatgttgcat 1500
   attttgcaca agtgaaacat ctctacaact ggaatctccc tccaagacgg atactcttta 1560
   taaaacactt cattatttat tcgattcctc gttatgtacg tgatctaaaa atccaaatag 1620
   aaatggagaa aaaggttgtc ttttccacta tttcattagg aaaatgttcg gtacttgata 1680
   acattacaac agacaaaata ttaattgatg tattcgacgg tctacctctg tatgatgatg 1740
   tgaaagtgca gtttttctat tcgaatcttc ctacatacta tgacaattgc tcattttact 1800
   tctggttgca cacatctttt attgaaaata acaggcttta tctaccaaaa aatgaattgg 1860
   ataatctaca taaacaaaaa gcacggagaa tttatccatc agattttgcc gtggagatac 1920
   tttttggcga gaaaatgact tccagtgatg ttgtagctgg atccgattaa gtatagctcc 1980
   cccttcccct tctgggaaag aattatgttc tttccaaccc tgccacatgt tcatatatcc 2040
   taaatctatc ctaaatgttc cttgaagtat ttatttatgt ttatatatgt ttatatatgt 2100
   tcttcataaa tctattacat atatatagat aaaaaaaaaa aaaaa 2145
<210> 72
   <211> 2462
   <212> DNA
   <213> Homo sapiens
<400> 72
   gctctgaccg actggtcccc taaacggtgg cggcggtttt tggtcgttgg gccccgggat 60
   ttaggaccaa catttgaaga cccgaagggg aactgcaacc atgaatgaag aaaatataga 120
   tggaacaaat ggatgcagta aagttcgaac tggtattcag aatgaagcag cattacttgc 180
   tttgatggaa aagactggtt acaacatggt tcaggaaaat ggacaaagga aatttggcgg 240
   tcctcctcca ggttgggaag gtccacctcc acctagaggc tgtgaagttt ttgtaggaaa 300
   aatacctcgt gatatgtatg aagatgagtt agttcctgta tttgaaagag ctgggaagat 360
   atatgaattt cgacttatga tggaatttag tggtgaaaat cgaggttatg cttttgtgat 420
   gtacactaca aaagaagaag cccaattagc catcagaatt cttaataatt atgaaattcg 480
   accagggaag tttattggtg tgtgtgtaag cctggataat tgtagattat ttattggagc 540
   tattcccaag gaaaagaaga aagaagaaat tttagatgaa atgaagaaag ttacagaagg 600
   agttgtagat gtcattgttt atccaagtgc aactgataag accaaaaatc gtggttttgc 660
   atttgtggaa tatgaatctc acagagctgc tgctatggca aggaggaaac taattccagg 720
   aacattccaa ctatggggcc acaccattca ggtagattgg gctgacccag agaaagaggt 780
   ggatgaggaa accatgcaga gagttaaagt tctttatgta agaaatttaa tgatctcaac 840
   tacagaggaa acaattaaag cagaattcaa taaatttaag cctggtgcag ttgaacgggt 900
   aaagaaactt agagattatg cttttgttca ctttttcaac cgagaagatg cagtggctgc 960
   catgtctgtt atgaatggaa aatgcattga tggagcaagt attgaggtaa cactagctaa 1020
   accagtaaat aaagaaaaca cttggagaca gcatcttaat ggtcagatta gtccaaattc 1080
   tgaaaatctg attgtgtttg ctaacaaaga agagagccac ccaaaaactc taggcaagct 1140
   gccaactctt cctgctcgtc tcaatggtca gcatagccca agtccgcctg aagttgaaag 1200
   atgcacttac cctttttatc ctggaacaaa gcttactcca attagtatgt attctttaaa 1260
   atccaatcat tttaattctg cagtaatgca tttggattat tactgcaaca aaaataactg 1320
   ggcaccacca gaatattatt tatattcaac aacaagtcaa gatgggaaag tactcttggt 1380
   gtataagata gttattcctg ctattgcaaa tggatcccag agttacttca tgccagacaa 1440
   actctgtact acgttagaag atgcaaagga actggcagcc cagtttacat tacttcattt 1500
   ggactacaat ttccatcgca gctcaataaa tagtctttcc cctgttagtg ctaccctctc 1560
   ttctgggact cccagcgtgc ttccttatac ttcaaggcct tattcttatc caggctatcc 1620
   tttgtcacca acaatatcac ttgctaatgg cagccatgtt ggacagcggc tatgtatctc 1680
   caatcaggcc tccttcttct gaagaaaata ctaacattag tatgaaaatt tgtgtaaatt 1740
   tgtagtatga aaacttgcaa attaaaatat tgttttattt tagaatcggg tttgcatatt 1800
   tggttttaaa aaggtattta ttccaaagta ctaaacatca gctataattc agaataacat 1860
   ggagttgtag aatttataaa aatgcaaagt ttaaaaagtt attcagtggt ttctcttgat 1920
   aaaggtacag caaactacta ttctttttaa acttctagga ttttcttcta ctttctgagt 1980
   gggcaataga acctagtcat ttatgttttt tttttttttg cataatttta ctaaatagta 2040
   tttcacaaat attaaagcac ttgaagacaa tggttatagt agatttgatt accaaggatc 2100
   actatctgta ctggagatta gaacaattat atgaccagaa gcatctaacc attatgtaaa 2160
   aagaaatgat gagacaaaaa gattaagata caaattttgt gcagtactaa agaaaaagca 2220
   gtctaccatt gtggtccttg aaaataacta tagatatttt tgttatttgt tagacacaaa 2280
   ttataatttt gttgttaatg tatttaagca ttttatagtt atgctttgtg tttttgatat 2340
   tctttgtatt gttaataaca agtgttatgg gtttttaatg ttgaaatcat gtgttaattt 2400
   ttgtacttga attcaaattt tttgacatta aatatgtgat gcttctaaaa aaaaaaaaaa 2460
   aa 2462
<210> 73
   <211> 459
   <212> DNA
   <213> Homo sapiens
<400> 73
   atggctcgta cgaagcaaac agctcgcaag tctaccggcg gcaaagctcc gcgcaagcag 60
   cttgctacta aagcagcccg taagagcgct ccggccaccg gtggcgtgaa gaaacctcat 120
   cgctaccgcc cgggcaccgt ggccttgcgc gaaatccgtc gctaccagaa gtccaccgag 180
   ctgctgatcc ggaagctgcc gttccagcgc ctggtgcgag aaatcgccca ggacttcaaa 240
   accgacctgc gtttccagag ctctgcggtg atggcgctgc aggaggcttg tgaggcctac 300
   ctggtgggac tcttcgaaga caccaatctg tgcgctattc acgctaaacg cgtcaccatc 360
   atgcccaaag atatccagct ggcacgtcgc atccgtgggg aaagggcata agtctgcccg 420
   tttcttcctc attgaaaagg ctcttttcag agccactca 459
<210> 74
   <211> 2591
   <212> DNA
   <213> Homo sapiens
<400> 74
   actctttctc tctcactctc tctcttttcc cacccttaag ccaagtacag ggatagttgt 60
   ctcatcattg gtggcttaaa atgatgtttt tgaacaagaa gacaccccat gggtactttt 120
   ggtgactagc actatctctg tttttttcct tttaaattcc tgagctattg tttagcagta 180
   caccctttta tctccattgc tactgaagct gaatgttact tgggtggaaa gcataactgc 240
   tttcttttct atgtccttaa accctttgat aatgttactg tttgagagtc cctgaagcca 300
   ggatattaga agagtctggc ttgtctgaac agctgaacta cgaaataatg gagtagggca 360
   ggtgggtggg ggagcagggc gttctgtcga taaacgagct cccttctttg cacacatagc 420
   cagttaatcg ggcattctga gatagtttgg atggggaggg ggagcttctg agaatcgcca 480
   gtgacagtta agtggcctat tgttgacgtc ctctgctgaa cgacttggtt ggattcagct 540
   tctgccttac ccccaccccc tgtggatttt ctgttagatt catcatttgc cattcaggca 600
   tcatctttca ctttctcctt ccaacatgac tgctttgtgt gctggcccct gctttactcc 660
   tgctattccc aaactataaa gggaactgtg tggggcttca gcaggactga gaaattgact 720
   ctgctgtctg ttgagaactc taataatgac tgcggtgacc ttcatgtcta gcagaaaacc 780
   cactgtctgt gctagcacaa tgatgaccac ctgaagacag agaaaaggga atcttgattg 840
   aattccttct catacaatat atagttattt cctttgttct cctctccatt ctcttctctt     900
   ccccttctcc ccatcgccac tggaggactg atctcaaatg cagctgtgac taaaagttaa     960
   tgccttttga ataataatac attgcatttt gcaggcttta ccaagccaat tcactcaagt 1020
   tgtctcatct ataccccttc aaaccctgtg agcctctagg tgctgtgctg tcctgaggcc 1080
   tgggccatgg tgcccaagga aagcccctga agctcaccag gaggaagaag catgcagggc 1140
   actcctggag gcgggacgcg ccctgggcca tcccccgtgg acaggcggac actcctggtc 1200
   ttcagcttta tcctggcagc agctttgggc caaatgaatt tcacagggga ccaggttctt 1260
   cgagtcctgg ccaaagatga gaagcagctt tcacttctcg gggatctgga gggcctgaaa 1320
   ccccagaagg tggacttctg gcgtggccca gccaggccca gcctccctgt ggatatgaga 1380
   gttcctttct ctgaactgaa agacatcaaa gcttatctgg agtctcatgg acttgcttac 1440
   agcatcatga taaaggacat ccaggtgctg ctggatgagg aaagacaggc catggcgaaa 1500
   tcccgccggc tggagcgcag caccaacagc ttcagttact catcatacca caccctggag 1560
   gagatatata gctggattga caactttgta atggagcatt ccgatattgt ctcaaaaatt 1620
   cagattggca acagctttga aaaccagtcc attcttgtcc tgaagttcag cactggaggt 1680
   tctcggcacc cagccatctg gattgacact ggaattcact cccgggagtg gatcacccat 1740
   gccaccggca tctggactgc caataagatt gtcagtgatt atggcaaaga ccgtgtcctg 1800
   acagacatac tgaatgccat ggacatcttc atagagctcg tcacaaaccc tgatgggttt 1860
   gcttttaccc acagcatgaa ccgcttatgg cggaagaaca agtccatcag acctggaatc 1920
   ttctgcatcg gcgtggatct caacaggaac tggaagtcgg gttttggagg aaatggttct 1980
   aacagcaacc cctgctcaga aacttatcac gggccctccc ctcagtcgga gccggaggtg 2040
   gctgccatag tgaacttcat cacagcccat ggcaacttca aggctctgat ctccatccac 2100
   agctactctc agatgcttat gtacccttac ggccgatcgc tggatcccgt ttcaaatcag 2160
   agggagttgt acgatcttgc caaggatgcg gtggaggcct tgtataaggt ccatgggatc 2220
   gagtacattt ttggcagcat cagcaccacc ctctatgtgg ccagtgggat caccgtcgac 2280
   tgggcctacg acagtggcat caagtacgcc ttcagctttg agctccggga cactgggcag 2340
   tatggcttcc tgctgccggc cacacagatc atccccacgg cccaggagac gtggatggcg 2400
   cttcggacca tcatggagca caccctgaat cacccctact agcagcacga ctgagggcag 2460
   gaggctccat ccttctcccc aaggtctgtg gctcctcccg aaacccaagt tatgcatccc 2520
   catccccatg ccctcatccc gacctcttag aaaataaata caagtttgaa caggcaaaaa 2580
   aaaaaaaaaa a 2591
<210> 75
   <211> 3623
   <212> DNA
   <213> Homo sapiens
<400> 75
   tctagcacag gggatcccca aacatcagga cttttggggg gcgcctgtgc tgtccatggg 60
   aagagcatgc attgtgggtt actggaggaa cccgacatgg attccacaga gagctggatt 120
   gaaagatgtc tcaacgaaag tgaaaacaaa cgttattcca gccacacatc tctggggaat 180
   gtttctaatg atgaaaatga ggaaaaagaa aataatagag catccaagcc ccactccact 240
   cctgctactc tgcaatggct ggaggagaac tatgagattg cagagggggt ctgcatccct 300
   cgcagtgccc tctatatgca ttacctggat ttctgcgaga agaatgatac ccaacctgtc 360
   aatgctgcca gctttggaaa gatcataagg cagcagtttc ctcagttaac caccagaaga 420
   ctcgggaccc gaggacagtc aaagtaccat tactatggca ttgcagtgaa agaaagctcc 480
   caatattatg atgtgatgta ttccaagaaa ggagctgcct gggtgagtga gacgggcaag 540
   aaagaagtga gcaaacagac agtggcatat tcaccccggt ccaaactcgg aacactgctg 600
   ccagaatttc ccaatgtcaa agatctaaat ctgccagcca gcctgcctga ggagaaggtt 660
   tctaccttta ttatgatgta cagaacacac tgtcagagaa tactggacac tgtaataaga 720
   gccaactttg atgaggttca aagtttcctt ctgcactttt ggcaaggaat gccgccccac 780
   atgctgcctg tgctgggctc ctccacggtg gtgaacattg tcggcgtgtg tgactccatc 840
   ctctacaaag ctatctccgg ggtgctgatg cccactgtgc tgcaggcatt acctgacagc 900
   ttaactcagg tgattcgaaa gtttgccaag caactggatg agtggctaaa agtggctctc 960
   cacgacctcc cagaaaactt gcgaaacatc aagttcgaat tgtcgagaag gttctcccaa 1020
   attctgagac ggcaaacatc actaaatcat ctctgccagg catctcgaac agtgatccac 1080
   agtgcagaca tcacgttcca aatgctggaa gactggagga acgtggacct gaacagcatc 1140
   accaagcaaa ccctttacac catggaagac tctcgcgatg agcaccggaa actcatcacc 1200
   caattatatc aggagtttga ccatctcttg gaggagcagt ctcccatcga gtcctacatt 1260
   gagtggctgg ataccatggt tgaccgctgt gttgtgaagg tggctgccaa gagacaaggg 1320
   tccttgaaga aagtggccca gcagttcctc ttgatgtggt cctgtttcgg cacaagggtg 1380
   atccgggaca tgaccttgca cagcgccccc agcttcgggt cttttcacct aattcactta 1440
   atgtttgatg actacgtgct ctacctgtta gaatctctgc actgtcagga gcgggccaat 1500
   gagctcatgc gagccatgaa gggagaagga agcactgcag aagtccgaga agagatcatc 1560
   ttgacagagg ctgccgcacc aaccccttca ccagtgccat cgttttctcc agcaaaatct 1620
   gccacatctg tggaagtgcc acctccctct tcccctgtta gcaatccttc ccctgagtac 1680
   actggcctca gcactacagg agcaatgcag tcttacacgt ggtctctaac atacacagtg 1740
   acgacggctg ctgggtcccc agctgagaac tcccaacagc tgccctgtat gaggaacact 1800
   catgtgcctt cttcctccgt cacacacagg ataccagttt atccccacag agaggaacat 1860
   ggatacacgg gaagctataa ctatgggagc tatggcaacc agcatcctca ccccatgcag 1920
   agccagtatc cggccctccc tcatgacaca gctatctctg ggccactcca ctatgcccct 1980
   taccacagga gctctgcaca gtaccctttt aatagcccca cttcccggat ggaaccttgt 2040
   ttgatgagca gtactcccag actgcatcct accccagtca ctccccgctg gccagaggtg 2100
   ccctcagcca acacgtgcta cacaagcccg tctgtgcatt ctgcgaggta cggaaactct 2160
   agtgacatgt atacacctct gacaacgcgc aggaattctg aatatgagca catgcaacac 2220
   tttcctggct ttgcttacat caacggagag gcctctacag gatgggctaa atgactgcta 2280
   tcataggcat ccatatttaa tattaataat aataattaat aataataata aacccaacac 2340
   ccatccccca gaagacttta tctctataca ttgtaactca tgggctattc ctaagtgccc 2400
   attttcctaa tgaacatgag gatgggatca atgtgggatg aataaacttt agttcagaaa 2460
   caggacttac taaaagtcag tgggactggg tttctgtagc caagccagac ttgactgttt 2520
   ctgtagagca ctatctcggg caggccattc tgtgcctttt ccctctgttc catgactttg 2580
   ctttgtgttg gcaaccactt ctagtaagct actgattttc ctgttgacaa aatctcttta 2640
   gtcttgaagg atggatactg gagacagaat ctggtttgtg ttcttggatg ggcacataat 2700
   ttaccaagag cattcacctt gccatctgtc ttgtcattgt actgtacaag gaacagccct 2760
   cagacgtgtt ctgcacatcc cttcttcctg gtggtaccat ccctatttcc tggagcacca 2820
   gggctaaatg gggagctatc tggaaactct agattttctg tcatacccac atctgtcaca 2880
   gtacctgcat tgtcttggaa tgtaagcact gtcttgaggg aaggaagagg tctgttctgt 2940
   attgccttaa gttgattgag gtttgtagga gactggttct tctacataca aggatttgtc 3000
   ttaagtttgc acaatggcta gtgtcagcaa aaggcaggag agggtttttg tttttttttt 3060
   aagttctatg agaatgtgga tttatggcat tgagtatcac actcagctct gctgtgttaa 3120
   ctttgtgaaa ctggatggaa caaactttaa cttaccaagc accaagtgtg aaagtgactt 3180
   tcacggttcc ttcataaaac tataataata tccgacactt tgatagaaaa aaattcaaag 3240
   ctgtgccttt gagcctatac tatactgtgt atgtgtggaa ataaaaatgt attgtacttt 3300
   tggagaattt tttgtaggca tttttctgtc agatttgtag taatttgtga ggtttgttag 3360
   agattaatat aggttttctt tctgtattat aaaatgcacc aagcaattat ggtggaccta 3420
   ttaccctatg ggtaagaaat aaatggaaat atgacatcgg atgtttcagc aactgttctg 3480
   taaataaaat ctttgatcac accactcagt gtgataattg tgtctacagc taaaatggaa 3540
   atagttttat ctgtacagtt gtgcaagata tgaatggttt cacactcaaa taaaaaatat 3600
   tgaaacgaaa aaaaaaaaaa aaa 3623
<210> 76
   <211> 1504
   <212> DNA
   <213> Homo sapiens
<400> 76
   ggttgaggtc aagtagtagc gttgggctgc ggcagcggag gagctcaaca tgcgtgagtg 60
   tatctctatc cacgtggggc aggcaggagt ccagatcggc aatgcctgct gggaactgta 120
   ctgcctggaa catggaattc agcccgatgg tcagatgcca agtgataaaa ccattggtgg 180
   tggggacgac tccttcaaca cgttcttcag tgagactgga gctggcaagc acgtgcccag 240
   agcagtgttt gtggacctgg agcccactgt ggtcgatgaa gtgcgcacag gaacctatag 300
   gcagctcttc cacccagagc agctgatcac cgggaaggaa gatgcggcca ataattacgc 360
   cagaggccat tacaccatcg gcaaggagat cgtcgacctg gtcctggacc ggatccgcaa 420
   actggcggat ctgtgcacgg gactgcaggg cttcctcatc ttccacagtt ttgggggtgg 480
   cactggctct gggttcgcat ctctgctcat ggagcggctc tcagtggatt acggcaagaa 540
   gtccaagcta gaatttgcca tttacccagc cccccaggtc tccacggccg tggtggagcc 600
   ctacaactcc atcctgacca cccacacgac cctggaacat tctgactgtg ccttcatggt 660
   cgacaatgaa gccatctatg acatatgtcg gcgcaacctg gacatcgagc gtcccacgta 720
   caccaacctc aatcgcctga ttgggcagat cgtgtcctcc atcacggcct ccctgcgatt 780
   tgacggggcc ctgaatgtgg acttgacgga attccagacc aacctagtgc cgtacccccg 840
   catccacttc cccctggcca cctacgcccc ggtcatctca gccgagaagg cctaccacga 900
   gcagctgtcc gtggctgaga tcaccaatgc ctgcttcgag ccagccaatc agatggtcaa 960
   gtgtgaccct cgccacggca agtacatggc ctgctgcatg ttgtacaggg gggatgtggt 1020
   cccgaaagat gtcaacgcgg ccatcgccac catcaagacc aagcgcacca tccagtttgt 1080
   agattggtgc ccaactggat ttaaggtggg cattaactac cagcccccca cggtggtccc 1140
   tgggggagac ctggccaagg tgcagcgggc tgtgtgcatg ctgagcaaca ccacggccat 1200
   cgcggaggcc tgggctcgcc tggaccataa gttcgatctc atgtatgcca agcgggcctt 1260
   tgtgcactgg tacgtgggag aaggcatgga ggagggggag ttctctgagg cccgcgagga 1320
   cctggcagct ctggagaagg attatgaaga ggtgggcgtg gattccgtgg aagccgaggc 1380
   tgaagaaggt gaagaatact gaggggaggg tgtggtgggt tctccactcc actgccaccc 1440
   ccagcgtggc tgctttcaag ttctttgcaa ttaaaggttc tgtataaaaa aaaaaaaaaa 1500
   aaaa 1504
<210> 77
   <211> 1746
   <212> DNA
   <213> Homo sapiens
<400> 77
<210> 78
   <211> 524
   <212> DNA
   <213> Homo sapiens
<400> 78
   tttttttttt caaatttttt aataaagcaa atggctggga aaaaaggtga gaaactgctc 60
   gcttggattt aagaactgct gctacttgct gtgtgacctg gactagtttt ttgtttgtta 120
   gttttatttt tatttttgtt tttttgctgg gctttacctt ggttttaatt ttctgaactt 180
   cattctgctc atctgaaaaa tgaaatacta atgtcttcat cttagtgtaa tagggatgat 240
   taggtaaggt caaatatatg gaagcatctt gtaaactgta aaggtatgta aaaatgtgag 300
   ggtttgtttt ttgtgtgtgt atgtgctctt aatgcccaag atgcagagta gaaattaggc 360
   agctgggatg ccaaacagcc aggatgcaac atattggaga gaagagccag atatctgagg 420
   aggaagtcac accatcattg ccatccatgt tgtgatagtc aacagaagga gtaaaaagag 480
   acatcagagg aatgaaaggg aggaatgagg attgggaagg aacg 524
<210> 79
   <211> 2837
   <212> DNA
   <213> Homo sapiens
<400> 79
   agtctcgcgg gaagctccgt tgtgggcgcc ccggctggtg gctgagctca ggccttcagg 60
   cagaggggag gcgagggcgg ggcggtcacg tgagagcact gccgcggtgg gttgtggggg 120
   tgctgcggcg ccgtttgctt tgccaaaccg acaaaagaga gatgatggcc aacgacgcca 180
   agcccgacgt gaagaccgtg caggtgctgc gggacacagc caaccgcctg cggatccatt 240
   ccatcagggc cacgtgtgcc tctggttctg gccagctcac gtcgtgctgc agtgcagcgg 300
   aggtcgtgtc tgtcctcttc ttccacacga tgaagtataa acagacagac ccagaacacc 360
   cggacaacga ccggttcatc ctctccaggg gacatgctgc tcctatcctc tatgctgctt 420
   gggtggaggt gggtgacatc agtgaatctg acttgctgaa cctgaggaaa cttcacagcg 480
   acttggagag acaccctacc ccccgattgc cgtttgttga cgtggcaaca gggtccctag 540
   gtcagggatt aggtactgca tgtggaatgg cttatactgg caagtacctt gacaaggcca 600
   gctaccgggt gttctgcctt atgggagatg gcgaatcctc agaaggctct gtgtgggagg 660
   cttttgcttt tgcctcccac tacaacttgg acaatctcgt ggcggtcttc gacgtgaacc     720
   gcttgggaca aagtggccct gcaccccttg agcatggcgc agacatctac cagaattgct     780
   gtgaagcctt tggatggaat acttacttag tggatggcca tgatgtggag gccttgtgcc 840
   aagcattttg gcaagcaagt caagtgaaga acaagcctac tgctatagtt gccaagacct 900
   tcaaaggtcg gggtattcca aatattgagg atgcagaaaa ttggcatgga aagccagtgc 960
   caaaagaaag agcagatgca attgtcaaat taattgagag tcagatacag accaatgaga 1020
   atctcatacc aaaatcgcct gtggaagact cacctcaaat aagcatcaca gatataaaaa 1080
   tgacctcccc acctgcttac aaagttggtg acaagatagc tactcagaaa acatatggtt 1140
   tggctctggc taaactgggc cgtgcaaatg aaagagttat tgttctgagt ggtgacacga 1200
   tgaactccac cttttctgag atattcagga aagaacaccc tgagcgtttc atagagtgta 1260
   ttattgctga acaaaacatg gtaagtgtgg cactaggctg tgctacacgt ggtcgaacca 1320
   ttgcttttgc tggtgctttt gctgcctttt ttactagagc attcgatcag ctccgaatgg 1380
   gagccatttc tcaagccaat atcaacctta ttggttccca ctgtggggta tccactggag 1440
   aagatggagt ctcccagatg gccctggagg atctagccat gttccgaagc attcccaatt 1500
   gtactgtttt ctatccaagt gatgccatct cgacagagca tgctatttat ctagccgcca 1560
   ataccaaggg aatgtgcttc attcgaacca gccaaccaga aactgcagtt atttataccc 1620
   cacaagaaaa ttttgagatt ggccaggcca aggtggtccg ccacggtgtc aatgataaag 1680
   tcacagtaat tggagctgga gttactctcc atgaagcctt agaagctgct gaccatcttt 1740
   ctcaacaagg tatttctgtc cgtgtcatcg acccatttac cattaaaccc ctggatgccg 1800
   ccaccatcat ctccagtgca aaagccacag gcggccgagt tatcacagtg gaggatcact 1860
   acagggaagg tggcattgga gaagctgttt gtgcagctgt ctccagggag cctgatatcc 1920
   ttgttcatca actggcagtg tcaggagtgc ctcaacgtgg gaaaactagt gaattgctgg 1980
   atatgtttgg aatcagtacc agacacatta tagcagccgt aacacttact ttaatgaagt 2040
   aaactaggct tatttctaaa aagtcaagtc tattggcttt ggcccaaaag cactggtatc 2100
   tttgtattaa attcatgttt attgtcacaa aaccattatt tatacctata cagttgtact 2160
   gtttctttta aagcaaagcc atttaacatc tttcttcatt cctaatttgg aaattaaagt 2220
   ttacctttct gttaatctat gtataaatgt tactctgagt tattaatgtg gattttaaaa 2280
   ttgtaagcaa tagaatagga aataaaacaa ctacctaata caaatatttc tgataagact 2340
   acaaatatct gactgagctg gggattaaag tagaggtaac tgtatcttaa atgagtatga 2400
   tttccttgta agttaaaaaa attgaaattt aattgtagac ttcaatagtc caagttttga 2460
   aggatgtttg agcttttgta taatgccatt tatacctgca gttttacaga taatgtttga 2520
   ctgcagttgc cttggaaatt cctccaaagt ttgccttcat ctctcctcta cagtttggag 2580
   gtgatggtgc agcagtggaa catctcttga tgcaccacac tacttgtgtt ctgtgaagtg 2640
   atgaaagtat aactggttct agtttgcaca ctacacacat agttttgtga agcttcagaa 2700
   atgtttttcc ttttccttgt ggccaaacca gtttgttaat ctgattatat tcatctgcta 2760
   atgatactaa agttaatgta ataaagcatt taaaaatcag aaaaaaaaaa aaaaaaaaaa 2820
   aaaaaaaaaa aaaaaaa 2837
<210> 80
   <211> 2345
   <212> DNA
   <213> Homo sapiens
<400> 80
   aaatgcaggg cgcagcagcc gctgcagtgg agccggtagg cctggccggc gggctgaaag 60
   gaagtgcgag ctgtccgccc agggccgggt atccgcccct gcaggctgtg gaggggatgt 120
   caggagactg gctggcctct tttcttggcc cccgactcct tccagtctga cactgaagac 180
   tttataagct tccccccgac caccctccac gggctccact ctccacgggc ctgggcttgc 240
   gccgcttcga gatcagcctg ggggtcgcgc cctcctggtc ttgtccacga agcgccgttc 300
   ttgggccgtt aggagctgct gggaagggct ctgataggcc cactcctctt ctccacccag 360
   gagatgagaa ggagggcagg cctttttaat ctgatcagaa tgttaaccca tctctccgcc 420
   ttgcggtaga acccctggat acattatttg ccctctcgaa aggcaggctc tgaatttgat 480
   tcaggtatat ttcttcatag ctaaccagca caatggaaaa ctcagggaaa gcaaataaaa 540
   aggatacaca tgacgggcca ccaaaagaaa ttaaactgcc taccagtgaa gcacttctag 600
   actatcaatg tcaaataaag gaagatgccg tggagcaatt catgtttcaa ataaagacac 660
   ttaggaaaaa gaaccaaaaa tatcatgaaa gaaatagccg cttaaaagaa gaacagattt 720
   ggcacatacg gcatctacta aaggaactga gtgaagagaa ggcagaggga ttgccagttg 780
   taacaagaga ggatgttgaa gaagcgatga aggaaaaatg gaagtttgaa agagaccagg 840
   aaaaaaactt gagagatatg cgcatgcaaa taagtaatgc tgagaaacta tttcttgaga 900
   aactcagtga aaaggaatat tgggaggagt acaagaatgt agggagtgaa cgacatgcta 960
   aactcattac ctccttacaa aatgacatca acacagttaa agagaatgca gagaaaatgt 1020
   cagaacacta taaaatcact ctggaagata ctagaaagaa aataatcaag gaaactttgt 1080
   tgcaactgga ccaaaagaag gaatgggcca cacagaatgc tgtaaagctc attgacaagg 1140
   gcagttatct agagatctgg gagaatgact ggctcaaaaa agaggttgca attcacagga 1200
   aggaagttga agaattaaaa aatgctattc atgaactgga agcagaaaat ttggtgctta 1260
   ttgatcaact atccaactgt agacttgtgg atctcaagat acccaggtat ccagtgctac 1320
   attcctgtcc cacctctaat cctcgtcatc tgctgctgct gcctttggaa tcatgtctaa 1380
   tctctgccag gcgttgctgg cgactatatc ttacccaagc tgctggacta gaagtgccac 1440
   ctgaagaaat gtctttggaa ttgccagaaa cacatataga agagaagtca gaattgcaac 1500
   ccacagaagt agaaagtaga gacttgatgt cctcatcaga tgagagcact atcttacatc 1560
   ttagtcatga aaatagcatc gaagatctcc agtatgtgaa gatagataaa gaggaaaact 1620
   caggcacaga gtttggggac actgatatga agtacttact atatgaggat gagaaggatt 1680
   tcaaggatta tgtaaacttg ggccccctgg gagtgaagct tatgagtgtg gagagcaaga 1740
   aaatgcccat tcattttcaa gagaaggaaa ttccagtcaa actctataaa gatgtcagga 1800
   gcccagaaag ccacatcaca tataagatga tgaagtcttt tctctaagac ggaaagctgc 1860
   aaaggaaaca caacttttcc ttataaatgt tctttgggaa ctgaagtata tccgttgccc 1920
   attttactta cactttggct catttttaaa ccagctgtta tttctaaagg tcatatttac 1980
   atttaaaatc aaaggtattc agctattcat ttacttgcat ggtatgagtg accaaaacgg 2040
   aagcacgctt tgtatttcta cactgaagta ttcagaagca tgacagtggg ttcaaggtag 2100
   tctctgaggt tccttttcac acacaaaaaa ttcactgatt aatctgtgat tccagtatga 2160
   aatagttcca ttagaaatgt ttctaagaaa aacttagaag tttgcatagc attgtctaca 2220
   catctttccc tctgaggatg ctcaatgtga tagacagcca gtctataatg caagccaatt 2280
   ctccgtagtt taaccctgtg tattagtctg ttctcatgct gctaataaag acataattga 2340
   aactg 2345
<210> 81
   <211> 1670
   <212> DNA
   <213> Homo sapiens
<400> 81
   gggtcgtcat gatccggacc ccattgtcgg cctctgccca tcgcctgctc ctcccaggct 60
   cccgcggccg acccccgcgc aacatgcagc ccacgggccg cgagggttcc cgcgcgctca 120
   gccggcggta tctgcggcgt ctgctgctcc tgctactgct gctgctgctg cggcagcccg 180
   taacccgcgc ggagaccacg ccgggcgccc ccagagccct ctccacgctg ggctccccca 240
   gcctcttcac cacgccgggt gtccccagcg ccctcactac cccaggcctc actacgccag 300
   gcacccccaa aaccctggac cttcggggtc gcgcgcaggc cctgatgcgg agtttcccac 360
   tcgtggacgg ccacaatgac ctgccccagg tcctgagaca gcgttacaag aatgtgcttc 420
   aggatgttaa cctgcgaaat ttcagccatg gtcagaccag cctggacagg cttagagacg 480
   gcctcgtggg tgcccagttc tggtcagcct ccgtctcatg ccagtcccag gaccagactg 540
   ccgtgcgcct cgccctggag cagattgacc tcattcaccg catgtgtgcc tcctactctg 600
   aactcgagct tgtgacctca gctgaaggtc tgaacagctc tcaaaagctg gcctgcctca 660
   ttggcgtgga gggtggtcac tcactggaca gcagcctctc tgtgctgcgc agtttctatg 720
   tgctgggggt gcgctacctg acacttacct tcacctgcag tacaccatgg gcagagagtt 780
   ccaccaagtt cagacaccac atgtacacca acgtcagcgg attgacaagc tttggtgaga 840
   aagtagtaga ggagttgaac cgcctgggca tgatgataga tttgtcctat gcatcggaca 900
   ccttgataag aagggtcctg gaagtgtctc aggctcctgt gatcttctcc cactcagctg 960
   ccagagctgt gtgtgacaat ttgttgaatg ttcccgatga tatcctgcag cttctgaaga 1020
   agaacggtgg catcgtgatg gtgacactgt ccatgggggt gctgcagtgc aacctgcttg 1080
   ctaacgtgtc cactgtggca gatcactttg accacatcag ggcagtcatt ggatctgagt 1140
   tcatcgggat tggtggaaat tatgacggga ctggccggtt ccctcagggg ctggaggatg 1200
   tgtccacata cccagtcctg atagaggagt tgctgagtcg tagctggagc gaggaagagc 1260
   ttcaaggtgt ccttcgtgga aacctgctgc gggtcttcag acaagtggaa aaggtgagag 1320
   aggagagcag ggcgcagagc cccgtggagg ctgagtttcc atatgggcaa ctgagcacat 1380
   cctgccactc ccacctcgtg cctcagaatg gacaccaggc tactcatctg gaggtgacca 1440
   agcagccaac caatcgggtc ccctggaggt cctcaaatgc ctccccatac cttgttccag 1500
   gccttgtggc tgctgccacc atcccaacct tcacccagtg gctctgctga cacagtcggt 1560
   ccccgcagag gtcactgtgg caaagcctca caaagccccc tctcctagtt cattcacaag 1620
   catatgctga gaataaacat gttacacatg gaaaaaaaaa aaaaaaaaaa 1670
<210> 82
   <211> 1009
   <212> DNA
   <213> Homo sapiens
<400> 82
   gggcagaggc caagtgggca ccggatagcg ccagccccgc ccagagagcg aaatcatgga 60
   gccttccaag accttcatga gaaacctgcc aatcacacca ggctatagcg gctttgtgcc 120
   attcctcagc tgccaaggaa tgtccaagga ggatgacatg aaccactgtg tgaaaacctt 180
   ccaggagaaa acacagcgct ataaagaaca gctgcgggaa ttgtgctgcg cagtggccac 240
   tgccccgaaa ctgaaacctg tcaactccga ggagacggtc ctgcaggccc tgcaccagta 300
   caatctgcag taccaccccc tgatcctgga atgcaaatat gtaaagaaac ctctccagga 360
   gcccccgatc cctggctggg caggctacct gccgagagcc aaggtcactg aatttggctg 420
   tggcacgaga tacactgtca tggccaaaaa ctgctacaag gacttcctgg agatcacgga 480
   gagggccaag aaggcacatc tgaaaccata tgaagagtga ggagaaatgt ctctttcctt 540
   cctactaccg ttttaaaaag gggatgaaat gtttgcagtg gcctttctgc ttagctgggc 600
   cagctccctg caactcacac ggacggttcc tctcctagat ggaagctgcc ctgcccttgg 660
   aaggcccctg agagaggacc ccaaaactcc gctgacatgt ggctgtgctc agaggccaag 720
   tataccatgc agtgggaaga tgtatctaga gccactgtcc tccgcaaagt atgcagaagg 780
   ctagaagcgc agagtctccc aaggaggtga actttaagtg gggcttccaa aacctgccat 840
   tctcatgttg gaatcacgcc cagtgagcaa taaagaaatt tagtaacaag aattttttaa 900
   ctgccgcctg catcctgagt ggttgacggt tgcatgtcat taatgataaa gaccgttttt 960
   tgtcatgtgg gaataaagag gctgcttctc cgcaaaaaaa aaaaaaaaa 1009
<210> 83
   <211> 1406
   <212> DNA
   <213> Homo sapiens
<400> 83
   atcttaagag gcgttccttt ttgcatagtt cccatgagca tgagagaaga agcaatgcac 60
   gctccggcag attcctagga accaaatacc tctgaggagc accagatttc agcttatggg 120
   atgctttgat tgctctgtgg ctgcatttag gagaaggaag ctgcagtcat gcgtcatcac 180
   tgccagcctc acatctcttg acagttaaag ccttagggtg gagcaaggga aaatttaaaa 240
   taacaaatga agcaaaagca agaggtgatg ttccaaagca gaggaaggct aagtttatat 300
   atacaaatgt caagtgtgta tagtgcaaaa ctaggaccag ttggtggaat ctgtggtcaa 360
   aaacaaaagc cttccttttt tttttttcaa ggcccagtcc caagacgcaa gaccacttgc 420
   gccagcagcg tgcatcagca agatagcaaa agcaggacga gagctgcccg gaagacatct 480
   acctggccag aagacaccta ccctggccgg aagacatgta cccctgaaga tagagaaaga 540
   ggccatcgtg tactacgtag cagtcatgtc agactgggac acttcctgtt tacagaggac 600
   tataaaaccc ctgtcctgtc ctcacttggg gctgacgcca tcttaggcct cagcccgcct 660
   gcagccaggc gttcgttaaa acagcatgtt gctccacacc gccttgtatt gtttgttggt 720
   cccactctct gggctcgaac caatacaagc acctttcaag cagtatattc ttcagtgtct 780
   tgatcctcca aataactctc ttctaattcc tcctgaccac aaaaagcact tatactctag 840
   gatgactgat tccagcccag tggcctggca agggtgaatt acaccttgca tatcacactc 900
   ttgacatttg tgtgcgctag cataagaatt ataattgaaa cagggattta agtatctcct 960
   ctctaggtgc ctaccctcct tggactcagg tcaaatttat taaaggaagt tttgtttcta 1020
   gataggttgt ttgaaataaa ataacagaat gttcaagtaa cacagtgtac ctacagcttt 1080
   taacaaaatt gaggacttgg gtctcgaaac aatttccttt gattttcagg tattttatct 1140
   ataaaaaggg agataaagca ttagttcata ggacagttat atgtttaaat gtgataatgt 1200
   atattaacca ccttgcatgt attcaaatgt gttttgaaat ctaacgtcta cattttgata 1260
   gtttaactgt tctacataag tgacttacaa caggcattaa atattgtttg gcattttcat 1320
   atatctgtaa ctgtatctta atctacaatg agcttaattt taagtgtagc ataaaacaga 1380
   accttcaata aagtggtaat attagg 1406
<210> 84
   <211> 3416
   <212> DNA
   <213> Homo sapiens
<400> 84
   ctccgcggga gccgttgggg ctgttggcct cgggctgagg tgcaaggacc aggactaggg 60
   cgagggcagc ggtccaagaa atagaaaaca atgactggga gagcccgagc cagagccaga 120
   ggaagggccc gcggtcagga gacagcgcag ctggtgggct ccactgccag tcagcaacct 180
   ggttatattc agcctaggcc tcagccgcca ccagcagagg gggaattatt tggccgtgga 240
   cggcagagag gaacagcagg aggaacagcc aagtcacaag gactccagat atctgctgga 300
   tttcaggagt tatcgttagc agagagagga ggtcgtcgta gagattttca tgatcttggt 360
   gtgaatacaa ggcagaacct agaccatgtt aaagaatcaa aaacaggttc ttcaggcatt 420
   atagtaaggt taagcactaa ccatttccgg ctgacatccc gtccccagtg ggccttatat 480
   cagtatcaca ttgactataa cccactgatg gaagccagaa gactccgttc agctcttctt 540
   tttcaacacg aagatctaat tggaaagtgt catgcttttg atggaacgat attattttta 600
   cctaaaagac tacagcaaaa ggttactgaa gtttttagta agacccggaa tggagaggat 660
   gtgaggataa cgatcacttt aacaaatgaa cttccaccta catcaccaac ttgtttgcag 720
   ttctataata ttattttcag gaggcttttg aaaatcatga atttgcaaca aattggacga 780
   aattattata acccaaatga cccaattgat attccaagtc acaggttggt gatttggcct 840
   ggcttcacta cttccatcct tcagtatgaa aacagcatca tgctctgcac tgacgttagc 900
   cataaagtcc ttcgaagtga gactgttttg gatttcatgt tcaactttta tcatcagaca 960
   gaagaacata aatttcaaga acaagtttcc aaagaactaa taggtttagt tgttcttacc 1020
   aagtataaca ataagacata cagagtggat gatattgact gggaccagaa tcccaagagc 1080
   acctttaaga aagccgacgg ctctgaagtc agcttcttag aatactacag gaagcaatac 1140
   aaccaagaga tcaccgactt gaagcagcct gtcttggtca gccagcccaa gagaaggcgg 1200
   ggccctgggg ggacactgcc agggcctgcc atgctcattc ctgagctctg ctatcttaca 1260
   ggtctaactg ataaaatgcg taatgatttt aacgtgatga aagacttagc cgttcataca 1320
   agactaactc cagagcaaag gcagcgtgaa gtgggacgac tcattgatta cattcataaa 1380
   aacgataatg ttcaaaggga gcttcgagac tggggtttga gctttgattc caacttactg 1440
   tccttctcag gaagaatttt gcaaacagaa aagattcacc aaggtggaaa aacatttgat 1500
   tacaatccac aatttgcaga ttggtccaaa gaaacaagag gtgcaccatt aattagtgtt 1560
   aagccactag ataactggct gttgatctat acgcgaagaa attatgaagc agccaattca 1620
   ttgatacaaa atctatttaa agttacacca gccatgggca tgcaaatgag aaaagcaata 1680
   atgattgaag tggatgacag aactgaagcc tacttaagag tcttacagca aaaggtcaca 1740
   gcagacaccc agatagttgt ctgtctgttg tcaagtaatc ggaaggacaa atacgatgct 1800
   attaaaaaat acctgtgtac agattgccct accccaagtc agtgtgtggt ggcccgaacc 1860
   ttaggcaaac agcaaactgt catggccatt gctacaaaga ttgccctaca gatgaactgc 1920
   aagatgggag gagagctctg gagggtggac atccccctga agctcgtgat gatcgttggc 1980
   atcgattgtt accatgacat gacagctggg cggaggtcaa tcgcaggatt tgttgccagc 2040
   atcaatgaag ggatgacccg ctggttctca cgctgcatat ttcaggatag aggacaggag 2100
   ctggtagatg ggctcaaagt ctgcctgcaa gcggctctga gggcttggaa tagctgcaat 2160
   gagtacatgc ccagccggat catcgtgtac cgcgatggcg taggagacgg ccagctgaaa 2220
   acactggtga actacgaagt gccacagttt ttggattgtc taaaatccat tggtagaggt 2280
   tacaacccta gactaacggt aattgtggtg aagaaaagag tgaacaccag attttttgct 2340
   cagtctggag gaagacttca gaatccactt cctggaacag ttattgatgt agaggttacc 2400
   agaccagaat ggtatgactt ttttatcgtg agccaggctg tgagaagtgg tagtgtttct 2460
   cccacacatt acaatgtcat ctatgacaac agcggcctga agccagacca catacagcgc 2520
   ttgacctaca agctgtgcca catctattac aactggccag gtgtcattcg tgttcctgct 2580
   ccttgccagt acgcccacaa gctggctttt cttgttggcc agagtattca cagagagcca 2640
   aatctgtcac tgtcaaaccg cctttactac ctctaacctg cagaagacga tgcagccgct 2700
   tttctttttg aaatgacttt gggatttttt taagctttta tttacttttt ttttaactgt 2760
   tatctttctg gatgaaactt gggaagggga ttaggagatc tagcatttta tttctagcat 2820
   tgctattcac cggcttcctt attttatacg taaaaattaa gattttatat tttatcttct 2880
   tgtttctcat agatattttg tgagcatttt tttgtttatt ttgaagaaat gtggataaga 2940
   tacttggtag tataaaacag actctctgag agtatttgaa atgtgtttgg agatttactt 3000
   aaacgtactt tcaggagtga gcaagtccta cttataaacc tatattaact ttatttttga 3060
   gatacctgtt ttgaatttaa aggagataag aggcgtaaag taggatgctc actacaacca 3120
   taggtggggt ttcagctcat atcttaaaga taaaaggtac tattatataa cctatacaca 3180
   agatacagga gaaaatatgc ttgattttta tttggcaggg gggctaggtt gtatgggagt 3240
   aaaaaaaaca ttgaaaattt ttaaattgtc caaagaaaca ttttaagact ctttaacaaa 3300
   aaaggccatg agtaaatctc tatattaaca ttactattta ttttgttttg gaactgggac 3360
   atgattctat ttgttataaa ataaaattga tgtgattgtc accttaaaaa aaaaaa 3416
<210> 85
   <211> 1112
   <212> DNA
   <213> Homo sapiens
<400> 85
   tagcacttca tagactgcta tcacgcattc atttttacta gtacctattg aacactaagt 60
   tccaggcact gggctaggca ctgggatggt gtggtgagca aaaactgacc agtcctgccg 120
   tggagtttgc tgggggagac acatgttact caaagaatca cactaaggat agcaatttat 180
   ctcaaagctg caagttcctg ccatctacat gtgcccagag tccccaaaat tgagtgtcaa 240
   acagaggctg ggtgacctcc tgtccaggat tgctctcatc gtatgaatcg aagttttcat 300
   cttaacgtgt ttttttctcc tgagaatagg ccaaccaatc aatggctcag acagataagc 360
   caacatgcat cccgccggag ctgccgaaga tgctgaagga gtttgccaaa gccgccatta 420
   gggtgcagcc gcaggacctc atccagtggg cagccgatta ttttgaggcc ctgtcccgtg 480
   gagagacgcc tccggtgaga gagcggtctg agcgagtcgc tttgtgtaac cgggcagagc 540
   taacacctga gctgttaaag atcctgcatt ctcaggttgc tggcagactg atcatccgtg 600
   cagaggagct ggcccagatg tggaaagtgg tgaatctccc aacagatctg tttaatagtg 660
   tgatgaatgt gggtcgcttc acggaggaga tcgagtggct gaagttttta gcccttgctt 720
   gcagcgctct gggagttact attaccaaaa ctctcaagat agtgtgtgag gtcttatcat 780
   gtgaccataa tggtgggtcg ccccggatcc cgttcagcac cttccagttt ctctacacgt 840
   atattgccaa agtggatggg gagatctctg catcacatgt cagcaggatg ctaaactaca 900
   tggaacagga agtaattggc cctgatggta taatcacagt gaatgacttt acccaaaacc 960
   ccagggttca gctggagtaa aagcacaatt ttggcaattt taaaggaaga tacagagatg 1020
   attgtacttc agaatgactg aaacccatat accacccaaa atccattttc ttgtacaact 1080
   ggtacacact aataaacaat taaaaaaaaa aa 1112
<210> 86
   <211> 2818
   <212> DNA
   <213> Homo sapiens
<400> 86
   gttacctggt acgctggctg ctacctccct cactcttgtc agagtcggag ctacaggcag 60
   tgccttcagc tctgagctca ggcatcccgg tccctgtttt tgcggttaag gactctaaag 120
   tgttgtgtcg tgttcatcaa ctttttctca acttccctgg ctctacctct tctgccacaa 180
   acgtcagcat ggtggtatct gccgaccctt tgtccagcga gagggcagag atgaacatcc 240
   tagaaatcaa ccaggaattg cgctcgcagc tggcagagag caatcagcag ttccgagacc 300
   tcaaagagaa attccttata actcaagcta ctgcctactc cctggccaac cagctgaaga 360
   aatacaagtg tgaagagtac aggaatcacc tgcccccaga gaggtgcaga agactgaaga 420
   aaaggaagtc cctcaggact cactggagga atgtgctgtc acttgttcaa atagtcacaa 480
   cccttctaac tccaaccagc ctcacaggag caccaaaatc acatttaagg aacacgaagt 540
   cgactctgct ctggttgtag agagtgaaca ccctcatgat gaagaggagg aagctctaaa 600
   cattccccca gaaaatcaaa atgaccatga ggaggaggag gggaaagcgc cagtgccccc 660
   cagacaccat gacaagtcca actcttaccg gcatcgtgaa gtctctttct tggcattgga 720
   tgaacagaaa gtttgctccg ctcaggatgt tgccagggat tactccaatc ccaaatggga 780
   tgaaacctca cttggcttcc tcgaaaagca aagtgatctt gaagaggtga aaggacaaga 840
   aacagttgct cccaggctca gcaggggacc gctgagagtg gacaagcatg aaatccccca 900
   ggagtcactg gatggatgtt gcttgactcc ttccatcctt cctgacctga ctccctccta 960
   ccacccttat tggagcactt tgtactcttt tgaagacaag caagtcagct tggctcttgt 1020
   agacaaaatt aaaaaggatc aagaggagat agaagaccaa agcccaccat gccccaggct 1080
   cagccaggag ctgccagagg tgaaggagca ggaagtccca gaggactctg tgaatgaagt 1140
   ttacttgact ccctcagttc accatgacgt gtctgactgc caccagcctt atagcagcac 1200
   cttgtcctca ttggaggatc agcttgcctg ctctgctctg gatgtagcct cccccaccga 1260
   ggcggcctgt ccccaaggga cttggagtgg agacttgagc caccaccagt cagaggtgca 1320
   agtttcacag gcacagctgg aaccaagcac cctggtgccc agttgtctgc gactacagct 1380
   ggatcaaggg ttccactgtg ggaacggctt ggcccagcgg ggcctttcct ccaccacctg    1440
   cagcttctca gccaatgctg attctgggaa ccaatggccc ttccaagagc tggttttaga    1500
   gccctctctg gggatgaaga accctcccca gctggaagat gatgcacttg aaggctcagc    1560
   aagcaacaca caagggcgtc aagtcactgg ccggattcgt gcctcccttg tcctgatact    1620
   gaagaccatc agaagaagac tcccgttcag caagtggaga ctggcattca gattcgctgg    1680
   cccgcatgct gagagcgcag agataccaaa tactgctgga aggacgcaaa ggatggcagg    1740
   atgaaagaat gtcacaaaaa gcagcttttc cacttgataa aaacaactaa aacagcaaag    1800
   caagtttaag tccaaacaca atactgcagg ggtccttcac tgaggattga atttcagaca    1860
   cagaatactc ttgatgactt caagccacta tgctcctttg atttgagaag ccacattcca    1920
   tccccctcca attgtgatca atacctaggg agaccaatgc ccagatggac aaatagcatt    1980
   gaccggcgtt agccctgttt ctcaattccc atcgtgtaga gaacaggagt ccgcagctgc    2040
   tggcaggaga cagcatgtca gccgggactc tgccagggca gagtatgagc aataccatgt    2100
   tcttgctgaa aacgcttagc ctgagtttca taggcggtaa ccctcagata actgcagaat    2160
   gtagaacatt gaacaggaca actgacatgg acttgtttgt ggaggacagg tcagctgtct    2220
   ggctcaatgg tctacattct gaagttatct gaaaatgtcg tcatgattaa attcagccta    2280
   aacattttgc caggaactct gcagagtcca tgctgtgagc ttcctacctc agcccatctg    2340
   caggcagaga aggcccagtg tgtccatccc cagtgcggtg atactaggat ggtcacttgg    2400
   ttaaggaggg gtctaggagc tctgtccctt gtaaagacat cttatttgta agtaatttgg    2460
   aaagtggttt gaaatagtat aaatatcctg tattctagtg atcttcttca gaacatttta    2520
   tcaccaatta atcaccccgt ctgtgtcagt tattatattt aagtttgtac attgaaaatt    2580
   gtctatctca aaatcttacc ttatacttgc ttttgctggc attctttgta aaaaagatca    2640
   ttccctgccc aaattttaac tttcatccaa aattaatttt aatttctttt tgctggcatt    2700
   ctgttgtgaa aaagaatatt ctctgcccca attataactt tcatccaaaa ttaattttag    2760
   tccatcagtt aaaattttaa attttaaatc tgtttaatta aaacatttct tgcctctc      2818
<210> 87
   <211> 3848
   <212> DNA
   <213> Homo sapiens
<400> 87
   agcgttgctg ctgccttgca gtttgatctc agactgctgt gctagcaatc agcgagactc      60
   cgtgggcgta ggaccctcca agccaggtga aagagcttat gatcctaagc acttccataa     120
   tagggtcagt agaatcatga tcgatgatca taatgtcccc actctacggg agatggtagc     180
   attctccaag gaagtgttgg agtggatggc tcaagattct gaaaacatcg tagtgattca     240
   ctgtaaagga ggcaaagaat agatatgttg gatattttgc acaagtgaaa catagctaca     300
   actggaatct ccctccaaga aaaacactgt ttataaaaag attcgttatt tattcgattc 360
   atggtgttgg aacaggcgat ggatatgatc taaaagtcca aatagtaatg aagaaaaaga 420
   ttgtcttttc ctgtacttcc ttaaagaatt gtcgggtatt tcatgacact gaaacagaca 480
   gggtaataac tgatgtgttc aactgtccac ctctgtatga tgatgtgaaa gtgcaagctt 540
   cctcttcaag agaagagggc agcacacctc gcagggctaa ctggaagggg gagccatcca 600
   ggagacctgt gctcaactga tgggtgggga gcaatagcga gaacgaggga gggacctgag 660
   agtggaagcc tttattggga tgtaaggtgt tacctgagca ggtttcctac ggggaggtct 720
   aactggtgga tttaatgcaa gcagtcatga gttccatgga gtcatgctgt gactgagagg 780
   tggtcattga tatatccaca tggtccatgc agagtacggg ggtctgtagg gaggttatat 840
   ctagctgtcc cataatgaag tagtcaccaa cagaaggttg tataaggcag atactgggat 900
   cagtcacatt gagaaacctg gaggaggtga actggaaact gtcaagggtg actgaaccct 960
   gcttctgata tcagaaagtc caatttatat ttgaaaggga tgctgaggca caaaaaaatt 1020
   gtaagaattc actacaaaaa tacttggcta tatataagca taggtcctta gtagattctg 1080
   tttagcacta tctaaaccag attcaaattt cagcatttaa attaaatatc tatcatggaa 1140
   aataaactat tccttgaaaa ttttggtaga aacagcaaga gaaagcaata gcattttctt 1200
   aagcctcctc ctctgtgtct tgagtgtgtt attatagaat gcagagtgct acctattgaa 1260
   tggttataat tatttgataa atatataaag gaataaagga aggaactttg atttctttgg 1320
   aatgatagtt cttggcatca attttacttt taaaatattt ttttttcttt ttaggatttt 1380
   cctaaatact atcacaacta cccttttttc ttctggttta acacatcttt aatacaaaat 1440
   aacgggtatg gatataatat caacccatag aaacaaccta atcttcaatg tctatgtata 1500
   agatgtaatg gcaagtcttt tgctggttgt cataagctta atttatagaa aacaaaaaat 1560
   ccttgagcca ccattgttca ttgccttact ccttttacgt tggctatttt aaaaatacag 1620
   ttgttcttga gacccccagt tgcagtatcc tcaaggtcca tgccatagga ctgtgttatg 1680
   agctcaaaag tattataatc agatcttaag tgtggaagta aattcctccc agagaagttc 1740
   aatatgaatc tgctcagtac cttcaacatg tcaggtcctc agtaggtgct gatttaccaa 1800
   tgacgaacca ccaccaaatt ttgtgctaaa gtaagggagg acctagggaa gcttcagcta 1860
   gctgaaaagc tgactgacac acttatatct aggagaagtt acaagacaca gtaagtatta 1920
   agaaatacag ctaaaaaatc attaaaattg gtagtctccc atttaaacat gggtttctaa 1980
   taactgaatt gggaaaactt tcttaaaaac tattaattgg aggctgggtg tggtggctca 2040
   tgcctgtaat cctagcactt tgggaggctg aggcgggcgg atcacctaag gttggaagtt 2100
   cgagactagc ctggccaaca tggtaaaact ccgtctctac taaaaataca aaaatcagcc 2160
   aggcgtggtg gcacatgcct gtaatcccag ctactcagga ggctgagcca gtagaatcgc 2220
   ttgaacccag gaggcagatt gcagtgagcc gagatcgcac cactacactc cagcctgggc 2280
   gacagagtga gactctgtct aaagaaaaaa gcaaaaaaac agaacaacta ttacttggat 2340
   ttggagatta ttgttcccag aaaaccttct gccatatttg gaaacttatt tctcagtcta 2400
   gaagttctcc actttaagta gcatttgttc tgtgctggtg aaaaactgag atttttttgt 2460
   attaaccata ctcttcaata caaaaggaga aaatattttt aaaatgcttc aggtcacagt 2520
   tgaggcagtt gctatgattg catgtggcat gaattggtag ttattgttac aaccagttct 2580
   agtcttttct tcaaatctga gctggatcta ataactcctt aagtccagca aggcaacagt 2640
   aaattaaacc tctggtctac acacttgcaa tacatacaca tttaatagat tttgatagag 2700
   tgaactttgg attggatgga aattttttaa aaatttgttt cttggatgca tacaaacaat 2760
   aagctttgac tcctaacatg agcaaagtcc ctcaattgtg agagctgggt ggagcttcat 2820
   ttgttgctgc tcctcaaatt gattcttggt aaaggataca gatttttcct ttgaaacacc 2880
   atgttcattt tggggaagca ataagttaga tcacctttat tttcactttt atataaattt 2940
   ctaaagattt ctgtaatatt taaatttata tactattggt aaagctgttt ttcttagttg 3000
   tgaaattgtt gtttagccaa aaatgccaac ttctgtcttt tagaacacta ggcataaatg 3060
   ggttaaccaa tttatgccta gtgttccatt attggaatgc taagcatgtg ggatttattt 3120
   atatcctact gctcaaggtc atcgccaagg gctgtttgca aaaattcaaa aaattgcaac 3180
   ctcaggcata aattaaaaga gatatagtat tttattattg ggttttgata catgtctaat 3240
   cagactgatt tctgtcacat atagaaattt agatactgta ttaaacctgg atgtcattaa 3300
   ttccataaaa agcaacgtta aaagaatcag tagcatgtgt tactgatgtg ttgctgaaga 3360
   ttaagatatt tttaagtctc accgaaaagg tagaaggagc caactgagac acaaaaaggg 3420
   gctgaggttc tattcatggt gagcaagtct ttttttttgt ttgtttcttc aagctctaac 3480
   aagggtgcct actacatggc ttttcagtta gccccaaaat aagatgtaac aatttttttt 3540
   tctattctta ggctttatct acaaagaaat gaattggata atcttcataa acaaaaaaca 3600
   tggaaaattt atcaaccaga atatgcagta gagatatatt ttaatgagaa atgacttaag 3660
   ttatgttgta actggtagct gattaagtat agttccctgc accccttctg ggaaagaatt 3720
   atgttctttc taaccctgcc acatagttat atgttctaaa tcttccttgc tggtacatct 3780
   atattgatat atgtatacac atgttcttta taaatctatt aaatatatac agaaaaaaaa 3840
   aaaaaaaa 3848
<210> 88
   <211> 1000
   <212> DNA
   <213> Homo sapiens
<400> 88
   agggagacgc ctagaagatg gaggcccaga ttcttgagac gtttctcttt ctgatctagc 60
   aggagggaca aagagctcct ccactccctc attccccaag aaggccccca gcctacccag 120
   tttccgtgac cattccgccc tgggaaagcg gcttcccaga cctccttatc tatttttctt      180
   gaatcatgag agatgaaatt gcaacaacag ttttctttgt cacaagattg gtgaaaaaac      240
   atgataaact aagtaaacag caaatagaag actttgcaga aaagctgatg acgatcttgt      300
   ttgaaacata cagaagtcac tggcactctg attgcccttc taaagggcaa gccttcaggt      360
   gcatcaggat aaacaacaat cagaataaag atcccattct agaaagggca tgtgtggaaa      420
   gtaatgtaga tttttctcac ctgggacttc cgaaggagat gaccatatgg gtagatccct      480
   ttgaagtatg ctgtaggtat ggtgagaaaa accatccatt tacagttgct tcttttaaag      540
   gcagatggga ggaatgggaa ctatatcaac aaatcagtta tgccgttagt agagcctcat      600
   cagacgtttc ctctggcact tcctgcgatg aagaaagttg tagcaaggaa cctcgtgtca      660
   ttcctaaagt cagcaatccg aagagtattt atcaggttga aaacttgaaa cagccctttc      720
   aatcttggtt acaaatcccc cgcaaaaaga atgtggtgga cggccgtgtt ggcctcctgg      780
   gaaacactta ccatggctcg cagaagcatc ctaagtgtta caggcctgct atgcaccggc      840
   tggacagaat tttataaccc acatctggga atgaatttgc agcacctggt agaagaaggc      900
   accttggaag gcactgcctt gggcttccat ggcaggaaga tgagaagaaa tcttcagggt      960
   gatttctgga gcctgaaaag aataaaaaac aaaaccaaaa                           1000
<210> 89
   <211> 2942
   <212> DNA
   <213> Homo sapiens
<400> 89
   agagcagcct cggaaccgag acgatgcgtg cgctccgcga ccgagccggg ctcctcctct       60
   gcgtgctgct gctggcggcg ctgctggagg cggcgctagg gctccccgtg aagaagccgc      120
   ggctccgcgg accacggcct gggagcctca cgaggctcgc agaggtctca gcctccccag      180
   atcctaggcc tctgaaggaa gaggaggagg caccactgct ccccagaacc cacctgcagg      240
   cagagccaca ccaacatgga tgctggactg tcactgagcc agcagccatg accccaggca      300
   acgccacccc tcccaggacc ccagaggtta ctccgttgcg gctggagctg cagaagctgc      360
   cgggattggc caacacaacc ttgagtaccc ctaaccctga tacccaggct tcagcctccc      420
   cagatcctag gcctctgagg gaagaggagg aggcacgact gctccccaga acccacctgc      480
   aggcagagct acaccaacat ggatgttgga ctgtcactga gccagcagcc ctgaccccag      540
   ggaatgccac gcctcccagg acccaggagg ttactccctt gctgctggag ctgcagaagc      600
   tgccagaatt ggtccacgca accttgagta cccctaaccc tgataaccag gtgaccatca      660
   aggtggtgga ggacccccag gccgaggtgt cgatagacct gttggctgag cccagcaatc      720
   ccccgcccca ggataccctt agctggctgc ccgccctctg gtccttcctc tggggagact      780
   acaaaggaga ggaaaaagac agggccccag gggagaaggg ggaggaaaag gaggaagacg      840
   aggactatcc ttcagaggat atcgagggtg aggatcaaga ggacaaagag gaagatgagg 900
   aagagcaggc gctctggttc aatggaacta cagacaactg ggaccagggc tggctggccc 960
   ccggggattg ggtcttcaag gattctgtca gctacgacta tgagcctcag aaggagtgga 1020
   gtccctggtc tccctgcagt gggaactgca gcactggcaa gcagcagagg actcggccct 1080
   gtggctatgg ctgcactgcc accgagaccc gtacctgtga cctgccctcc tgtcctggca 1140
   ctgaggacaa ggacaccttg ggcctcccca gtgaggagtg gaagctcctg gcccgcaatg 1200
   ctacggacat gcatgatcaa gatgtggaca gctgtgagaa gtggctgaac tgcaagagcg 1260
   acttcctaat caagtatctg agccagatgc tgcgggacct gcccagctgc ccgtgtgcct 1320
   acccactgga ggccatggac agccctgtga gcctacagga cgagcaccag ggccgcagct 1380
   tccggtggag ggatgccagt ggccctcgcg agcgcctgga catctaccag cccacggcgc 1440
   gcttctgcct gcgttccatg ctgtctgggg agagcagcac actggccgcc cagcactgct 1500
   gctatgacga ggacagccgg ctgctgaccc gtggcaaggg cgccggcatg cccaacctca 1560
   tcagcaccga cttctcacct aagctgcact tcaagttcga cacgacgccc tggatcctgt 1620
   gcaaggggga ctggagccgc ctccacgctg tgctccctcc caacaacggc cgagcctgca 1680
   ccgacaaccc cctggaggag gagtacctag cacagttgca ggaggccaag gagtactagt 1740
   gacggggttg ctgaacagac actgcaggga gagggcaggc ggctgctgct gttgcacggg 1800
   agaactttcc tcacccgccc ctgcccagac agggtgagga aagggctccc ccagtgaggt 1860
   tggtccgagg ctgtgtgccc tctgccagcg accccgaagc agatatctca gtggggttag 1920
   tgagaaggtt gaagggtatg tagggcccag ggtgggtgtc cctgggagcc ctggaaatgt 1980
   gcatatgtgc atgtgtctgc cggggcctcc ctctgctgcc tgctgggacc ctggccactc 2040
   atttttctcc tccttgggag ctgggctctt ctgccctggc tctgcacata agtgttagcc 2100
   agcagctcca gaaaaatccc gattcccggg atctgccacg agtcactcct actccaccct 2160
   gatggccagc agaggaaggg ccactcttct catgggcaca gccatccttt gccggggggg 2220
   catccagccc gggtggccac ccctccttat ctctgggtgg tgcacatgcc cttctttccc 2280
   cactccctgc cacgagccac tgcacaggag gctatctgta gccccaagct gcctttctgt 2340
   tggacaccaa ctttagtctt gggctgcaag ccagcccagc tgaggcgaag tggactccag 2400
   gcagggaatg ggttgcccaa ttctggtccc tttcctttgc tcagccccct ctgttctgct 2460
   gattgtaggg atgtgcaggg ctgggagttg gcactccccc cgagtgggga ggtgacagct 2520
   tgtcacagta gccaggcttg ggtgggttca gcactagctc gggacggtgt gtcacacgtc 2580
   tatagtaaac cagttctctg ggaggggaaa aaagccctga tttattgcat ttgggcagct 2640
   tctgtggtgt aaattctccc agcagtgtcc catgtcatgc tgccagcatc actgaatgca 2700
   ctgaactcag agttgggaag agatgcacat aatcgctctc ccggcacacc tcatgcctct 2760
   tccctgcctc cccattcccc tggctgcact tccttgcctt ctatggggtt gaaatattga 2820
   agtctcaact gtctctgttc acaagagcca ccaaaagtta ggggacttca gtcctagccc 2880
   ccagatggcc gccctgaagc tctctgggct cctcagcaat aaagcacttt attttcaaaa 2940
   aa 2942
<210> 90
   <211> 2570
   <212> DNA
   <213> Homo sapiens
<400> 90
   aatcggtata tttactgatt gtgtattggt gaattggtgc tggctctcag ttcccgcctc 60
   gcggcggggg gggcctgacc acccctgcga tgggtgtcct aagagccagg gggggaagag 120
   gggctggctg tcagtccccg cctcccgggg ggtgcctccc gcccctacga tggtggttcc 180
   aagagtctgg gggggaagag gggctggctt tcagtacccg cctcgcgggg ggtgcctccc 240
   ccaggtgcga tgggggtcct aagagccagg gggtgaagag gggctggctc tctgtcccct 300
   cgtcgcgggg gttgcatccc ccccctgtga tgggggtccc aagagccagg gggggaagaa 360
   gggctggctc tcagtccccg cctcactagc aggggagttg ctgccaaggc cctcaaacat 420
   gggggccatc ctttagaaac cctgtctagt tgtttagaga cataggccac cgacctcatc 480
   cagggcccca cagtttgggt taaaagtcca cctgccatct tttctctctc tgacacatac 540
   aatggaaaag gctttgtcag atcgggtaac cccagggctg aagctgccag aagtttttcc 600
   tttaactcat gaaagacttg ctgttgttag gatccccctt ccaaaggttc ccggtccccg 660
   acccctttgt gacctcatac aaaggcttgg cttatactgc aaagtttggg atccacagtc 720
   tacaaaaccc cacagctcct gagaattctc tcgcctgcct tcggccctta ggctctggta 780
   gattgcaaat aacatgcttt ctttctgttc ccgggtggct tcggacccct gtcggatcgg 840
   aaatcccaag taaggtacct gccctgggca gatttgagct ttcttcttgg acacctaata 900
   cccacagtcc tccaggctga ggtagattgc aaatgacctg ctttctttct gttcctgggt 960
   gccttcggac ccctgtcgga tagtaaatcc caagtaaggt acctgccgtc agcagatttg 1020
   agctttcttc ttggacacct aatacccaca gtcctccagg tgggtcctaa agttcatagg 1080
   atccgcgatg ggggtcccaa gccagggagg gacgaggggc tggctctcag tccccgcctc 1140
   gcgggggggt gcctcccccc ccccctgcga tgagggtcct aagagccagg ggggaagagg 1200
   ggatggctgt cagtccctcc ctcgtgggtg gtacctccgc cttctgcgat ggtggtccta 1260
   agagccaggg ggagacgagg ggctggctct cagtccccgc ttcgcgaggg gtgcctcccc 1320
   cccccgcgat gggggtccta agagtctggg gggaaagagg ggctggctct caatccccgc 1380
   ctcgcggggg tgcctccccc aggtgctatg tgggtcctaa gagccagggg tgaagagggg 1440
   ctagctctct gtcccctcgt cacgggggtt gcatcaccca ccctgcgatg gaggttccaa 1500
   gagccaaggg ggtaagaggg gctggctctc agtccccgcc tcgcggaggg tgcctccccc 1560
   accactgcga tgggggtccc aagagccagg gggggaagag ggtctggctc tccaccacca 1620
   caaaatgggg ggcctttatg ttcaggtttt gcccaagagt cagcttattt gcttcttgta 1680
   ctagcagggc agttgctgcc aaggccctca aacagggtgc catcctttag aaaccctgtc 1740
   tagttgttca gagacgtagg ccaccggcct catccagggc cccacagttt gggttaaaag 1800
   tccacctgcc atcttttctc tctctgacgc atacaatgga aaaggctttg tccgatcgga 1860
   tagccccagg gctgaagctg ccagaagttt ttcctttaac tcatgaaaga cttgctgttg 1920
   ttgggatccc ccttccaaag gttcccagtc cccgccccct ttgtgacatc atacaaagtc 1980
   ttggcttata ctgcaaagtt tgggatccac agtctacaaa accccacagc tcctgagaac 2040
   tctcttgcct gccttcggct cttaggctat agtagattcc aaataacctg ctttctttct 2100
   gttcccgggt ggcttcggac ccctgtcgga tcggaaatcc caagtaaggt acctgccgtc 2160
   ggcagatttg agctttcttc ttggacacct aatacccaca gtcctccagg ctccagtaga 2220
   ttgcaaatga cctgcttact ttctgttccc gggctgcgtt ctgacacctg tcggatagta 2280
   aatcccaagt aaggtaccag ccgtcggcag atttgagctt tcttcttgga cacctatacc 2340
   cacagtcctc cagtgtttta gacgcccagc tgcacaactt gattgcctta caaatgacct 2400
   gcttccagga tgcggaaatt cctaatttct tctgtgaccc ttctcaactc ccccatcttg 2460
   catgttgtga caccttcacc aataacataa tcatgtattt ccctgctgtc atatttggtt 2520
   ttcttcccat ctctgggacc cttttctctt actataaaat tgtttcctcc 2570
<210> 91
   <211> 1149
   <212> DNA
   <213> Homo sapiens
<400> 91
   ccgcagccat gaccccgcag cttctcctgg cccttgtcct ctgggccagc tgcccgccct 60
   gcagtggaag gaaagggccc ccagcagctc tgacactgcc ccgggtgcaa tgccgagcct 120
   ctcggtaccc gatcgccgtg gattgctcct ggaccctgcc gcctgctcca aactccacca 180
   gccccgtgtc cttcattgcc acgtacaggc tcggcatggc tgcccggggc cacagctggc 240
   cctgcctgca gcagacgcca acgtccacca gctgcaccat cacggatgtc cagctgttct 300
   ccatggctcc ctacgtgctc aatgtcaccg ccgtccaccc ctggggctcc agcagcagct 360
   tcgtgccttt cataacagag cacatcatca agcccgaccc tccagaaggc gtgcgcctaa 420
   gccccctcgc tgagcgccag ctacaggtgc agtgggagcc tcccgggtcc tggcccttcc 480
   cagagatctt ctcactgaag tactggatcc gttacaagcg tcagggagct gcgcgcttcc 540
   accgggtggg gcccattgaa gccacgtcct tcatcctcag ggctgtgcgg ccccgagcca 600
   ggtactacgt ccaagtggcg gctcaggacc tcacagacta cggggaactg agtgactgga 660
   gtctccccgc cactgccaca atgagcctgg gcaagtagca agggcttccc gctgcctcca 720
   gacagcacct gggtcctcgc caccctaagc cccgggacac ctgttggagg gcggatggga 780
   tctgcctagc ctgggctgga gtccttgctt tgctgctgct gagctgccgg gcaacctcag 840
   atgaccgact tttccctttg agcctcagtt tctctagctg agaaatggag atgtactact 900
   ctctccttta cctttacctt taccacagtg cagggctgac tgaactgtca ctgtgagata 960
   ttttttattg tttaattaga aaagaattgt tgttgggctg ggcgcagtgg atcgcacctg 1020
   taatcccagt cactgggaag ccgacgtggg agggtagctt gaggccagga gctcgaaacc 1080
   agtccgggcc acacagcaag accccatctc taaaaaatta atataaatat aaaataaaaa 1140
   aaaaaaaaa 1149
<210> 92
   <211> 1099
   <212> DNA
   <213> Homo sapiens
<400> 92
   gctgcattac agacacagac ctgcaaacat ctatggttgt gacagagttt ctttctgaca 60
   cctgagtctt tctcctgctg cacggaaagc ttgctgggag gggcttggaa tctggcatga 120
   agccaaaggg catctctgag ttgcagcatt taaatgatcc cactcagaga ttcacacaga 180
   agactggaca caattccgaa gagctgccca gaaggagaga acaatgtcat cactacccag 240
   tggcagacac ctttcacccc agctacacaa gagggggcag atgtgtgagg atcactgcag 300
   tccaggagtt cgatgtttca gtgagctgtg attgcaccac tgcatatcag cctgggtgac 360
   agagcaagac cctatctcaa aaatacagaa aaatcatcaa ccacttgcag tcgtcgtaga 420
   aatcaatcat tccctccagt tatgtccctg acccacaggc ttcatttgtg caagtactgg 480
   ggctgtgctg tcagtaatgt gtgccgcttc tgggaaggac gtccattgcc cttgatgatt 540
   gtggtaccat acacactgcc tgtttccttg cctgttggtt cgtgcgtgat aatcacaggg 600
   acaccgatcc tcacttttgt caaggaccca cagctggagg tgaatttcta cactgggatg 660
   gatgaggact cagatattgc tttccaattc cgactgcact ttggtcatcc tgcaatcatg 720
   aacagttgtg tgtttggcat atggagatat gaggagaaat gctactattt accctttgaa 780
   gatggcaaac catttgagct gtgcatctat gtgcgtcaca aggaatacaa ggtaatggta 840
   aatggccaac gcatttacaa ctttgcccat cgattcccgc cagcatctgt gaagatgctg 900
   caagtcttca gagatatctc cctgaccaga gtgcttatca gcgattgagg gagatgatca 960
   gactcctcat tgttgaggaa tccctctttc tacctgacca tgggattccc agagcctact 1020
   aacagaataa tccctcctca ccccttcccc tacacttgat cattaaaaca gcaccaaact 1080
   tcaaaaaaaa aaaaaaaaa 1099
<210> 93
   <211> 1021
   <212> DNA
   <213> Homo sapiens
<400> 93
   atgactggcc gcaggcggcc gggcgggctg taacccgccg ctgaactagc gcttctgtgt     60
   ccagaggctt cggcctggcc gccgtcgcct gtaagctacg aggaggagat ttacgacttg    120
   gccgggcgca gcaaaggcca gactctgcgc gaacaggcgc tgcgcaccaa ccggcaggca    180
   cctggcgggc accatcgcac ggtggcgcag aagcccttca atggccagcg ccagctgcag    240
   ccgcggccgc gcagtcgtcc cacctgagct tgggcgaatg tggattggga aagtcgacat    300
   taatcaactc attattcctc atagatttgt attctccaga gtatccaggt ccttctcaca    360
   gaattaaaaa gactgtacag ctggcagcct gttatcgatt acattgatag taaatttgag    420
   gactacctaa atgcagaatc atgcgtgaac agacatcaga tgcctgataa cagggtgcag    480
   tgttgttcat acttcattgc tccttcagga catggactta aaccattgga tattgagttt    540
   atgaagcgtt tgcatgaaaa agtgagtatc accccactta ttgccaaagc agacacactc    600
   acaccagagg aatgccaaca gtttaaaaaa cagttgaaaa tggtgaacat tgttatttta    660
   caattctaag aaatatgttg ataaggtaac ttttccggtc acacacaaca gactgatttt    720
   cctgtggaag acctcacagg agttctcttg aaccaggaga ctattttgag ctgaaatacc    780
   tggagccctc tcaagctatt tgggcctttc aatttgaaac ccaggtttgg atgattctaa    840
   gctctatggc agagactgta atcattttct cttcaccctc aggttgggga ctcaccctca    900
   gggttgggag tgactgctgt ctctccagcc cccaccaatt acaccatgtt ctggttgtta    960
   aaatccagtg tatgcatgaa atgtaataaa agtatcttcg cagcaaaaaa aaaaaaaaaa   1020
   a                                                                   1021
<210> 94
   <211> 551
   <212> DNA
   <213> Homo sapiens
<400> 94
   gagaggggta tacacaggga ggccaggcag cctggagtta gtcgaccgtt gcgagacgtt     60
   gagctgcggc agatgagtcc aaagccgaga gcctcgggac ctccggccaa ggccaaggag    120
   acaggaaaga ggaagtcctc ctctcagccg agccccagtg gcccgaagaa gaagactacc    180
   aaggtggccg agaagggaga agcagttcgt ggagggagac gcgggaagaa aggggctgcg    240
   acaaagatgg cggccgtgac ggcacctgag gcggagagcg ggccagcggc acccggcccc    300
   agcgaccagc ccagccagga gctccctcag cacgagctgc cgccggagga gccagtgagc    360
   gaggggaccc agcacgaccc cctgagtcag gagagcgagc tggaggaacc actgagtaag    420
   gggcgcccat ctactcccct atctccctga gcagcaacta agtttaggcc cagctgccag    480
   acctcagaga tctcaccagc agggtgcttc ccatgttgat gacaataaaa tgaatgtgtt    540
   gcaaaccgaa a 551
<210> 95
   <211> 4399
   <212> DNA
   <213> Homo sapiens
<400> 95
   caacctttag acctagggct tactataact ccagtatcca caaaggaggc tgagcattcg 60
   acaaccctga gaaaaactgc agttcctcca aaacaccctg aagtgactct tgcaactcca 120
   gaccatgtgc aggctcagca cacaaaccta actgaggtca cagtttaaac tttggatctg 180
   aaacttacca caattccaca acctactaca gagaatatat ttcctccaac catggagaac 240
   tcaaatcaac ttccagaacc acctacggag gttgtagctc aacttccacc tcgttatgag 300
   gtgacaattc caacacaagg tcaggatcaa gctcagcttt caacactggc cagtgtcaca 360
   cttcaacctt tggacctggg gtttatcatc actccagaat ccactacaga aattgaactt 420
   tctccaacca tgcaggagac cccaactcag cctcctaagg aatttgtacc ccaacctcca 480
   gtatatcaag aggtgagtgt tccaacaccg ggtcaggatc aagctcagca tccaatgtca 540
   cctagcgtta cagttcaacc tctggacctg gtggacttac cataactcca gaacccacta 600
   cagaggttga acattctaca cccctgaaaa agactacagt tcctccaaag caccctgaga 660
   tgacacttcc acatccacac caggttcaga ctctacattc aaacctgatt caagtcacag 720
   ttcaaccttt gggtctgaaa cttaccttaa ctctatggag gttgaatcct ctatggaggt 780
   tgaaccttct ccaaccatgc agaagacccc aactcggcct ccagagctac ctaaggagtt 840
   tgtagctcaa ccgcctgtgt attattatca gatacccatt ccaacaccaa gccaagatca 900
   agctctgccc ttctacagcc ccgatgacta cagctcctcc tccaaagcat cctgaagtga 960
   cacatccacc tccagacaag aaccaggctc agcatccaaa cctgactcaa ttcacagttc 1020
   aatctttgga cctggagctt accataacta cagaacctac tacagaggtt aaaacttctc 1080
   caaccatgga ggagacctca actcagcctt cagacctggg atttgccata gttccagaac 1140
   tcaccataga gactgaacat tctacaggcc tggacaagac tacagctcca catccagacc 1200
   aagttcagac tcagcattga aacctgactg aagtcacaca tttcaccttc tgaactagaa 1260
   cctactcaga attcactggt gcagtctgaa agttatgccc aaaataaggc tttaactgca 1320
   caggaggaac cgaaggcctc tacacgcacc aacatatgtg atctctatac ctgcagagat 1380
   gaaacactct catgtattga tctcagccca aagcagaggc tccaccaagt gcctgtacca 1440
   gagcccagca cctgcaatga caccttcacc atcctgtgag aattgtcttt cctcaattgt 1500
   tctgtgtcct gcctgacatg acagcctttt cgtggaggcc ttcctgggcc tcctttatct 1560
   caccaaaccg aactgacagc ggactttctg ctttcacctt tcttgtcaat tcttccttct 1620
   cctggttctc ctttactgtt aggccccttc tctggtcttt tacttgtgat tgctcttaac 1680
   ccttttctta tccactttcc tttagcccca tcacatcatt gcttaacagc ggctctcctc 1740
   ccattttcac ttcaccctct ttacagcagc ctgtccctct tcccatctca gtgatgatgc 1800
   tctaagtggt taagagttga ttctgtagcc aggctgcctg ggtttgaacc caggtctgtc 1860
   atttattagc ttggttaccc tgagcaagtt attcttctct gtgactcagt ttcctcatct 1920
   ttaaactggg gattatgcta gttaccacgc cataggattg ttgtgagatt taagtgagtg 1980
   catacatgta ttgcttacat tggtgcctag catatgtggg agtgttggct gctaacatga 2040
   ttactcagtc ctttagttat gtccagaacg catctttgtc cctggctttc tatctgtagc 2100
   agtcgttttc tgtcaaccct tggccaagta tgatactgtc ttcagaaatg aaaatgatag 2160
   gagggaagaa agagactagg catgaaaagg aggtatatat aatgaaatac tacaagataa 2220
   tgcagaccat cggtgctagg attcaccaga atctgtgatc cttgaggtgt ggagatcagg 2280
   gaaagctaca tcaataagct aaaacttact tgggacttaa agtgtagcta taatttgtta 2340
   aatagaaaac aaatgggagt acagtctagg caaagtcatg attacaggta tggttgaaat 2400
   ttggtagaca aggctgcagc tcagcctcca gagaacccca gggaggtgga ctcttcctca 2460
   acccaattag agggcccagc tcagacacca gagtgcactg aggagatgaa atattttgcc 2520
   cccagcaggg gaccccagct gagcctccag gtcctcctgt ggaggctgaa ccttccccca 2580
   gtcagcagga gcagccagct cagccttctg agttttctgg ggaggtggaa ttttctcaga 2640
   cccaggagac ccccaactct gcctccagag tcttctatag agagtgtagc tcaaactcca 2700
   ctgaatcatg aagtgacagt tcaaactcag ggtgaggatc aagctcatta taccttgccg 2760
   agcattacag ttaaacctgc agatgtagag attagcataa cttcagagcc taccacggac 2820
   actgactctt ctccagccca gcaggcggcc ccaaaccagc atccagagca ggtgtaacct 2880
   tctgcaaccc aacaggaggc cacaactgag cctccaggtc ctcatgtgaa tgctgaacat 2940
   tccccagtga gcaggagcag ccaggtctgc cttctgggtt ttctggagaa gttgagtcct 3000
   ctctagcctg caggagaccc cagcccagcc tccagaacat catcaagtaa cagttccacc 3060
   tcctggtcac catcaagttc aatactgaga tttgcccaat gtcactgtta agcctccaaa 3120
   tatgcagctc accatagcaa cacagcctac tgcagaggtg ggaactttgc cagtccatca 3180
   ggaggctaca gctcagctct cagggccagt taatgatgtg gaacattctg acatccagca 3240
   tggggccccg cctctgccta cagagtcatc ggaagagact ggacctttac cagttcaaca 3300
   ggagacttca gttgaatctc cagaacctac taaagatgag aacccctctc caatacagta 3360
   ggaggctgca ggtgagcatc cacagacccc tgagtaggtc gagtcttctc caacccagca 3420
   agatgcccca gctcagcctt cagagctccc taatgaagtt gtagctcaac ctccagagca 3480
   tcacagagta atagtttctc ctataagtca tgaggaagtt cagcctccaa catttcacca 3540
   tgtcattgtt aagcctgtgg atcacatggt taccatgact ccagagttca cctatcaggt 3600
   ggaagtttta actcaacaca gggccccagc tcagccttta atatcccctg agcagtttaa 3660
   acatttgaaa gaccagcaaa agattatcat tcagcagcta aatacccctg gaaatgatga 3720
   acttccgcca aatctatcaa gagcccatga ctccatctcc aactcagctc tcctcagaca 3780
   tttcatgctt atccaacgag tgtataaaag gcccaagaag acaaggttca gagagcttcc 3840
   ggatagctga acgcatggag gctgacagga cagtgaagga gaactcatcc acgcgctggg 3900
   cgagtggtgc accccaactc cacaggaacg gaagctcctc cagatcttgc cttgtgttat 3960
   ctttccatct ggctatttat ttgcatcctt tttaaatgta agtaagtgct tccataagtt 4020
   ccgtgagctc ctccagcaaa ttaatcaacc ccgaagaggg tgggtcatgg taaccccaac 4080
   ttgaagccag ctggtcagac attctggaag cccagactcg tgactggtgg gaaggaggga 4140
   gcagttctgt ggaactgatt cctcaacctg tggtttctga ggctatttcc aggtagatgg 4200
   tgtcacagtt gaattaactg gtggacaccc ggctgtgtcc actgcagaac taattgctta 4260
   cttggtgtgt gggaagaaac ccctacatat tttgtcacag aagtcttctg tattattatg 4320
   gtgtaagaga acaggaaaaa tgcatgttga ctgttttttc cacactccca gtccacaaaa 4380
   gttttctcca cttatgaac 4399
<210> 96
   <211> 1660
   <212> DNA
   <213> Homo sapiens
<400> 96
   ggtgcactag caaaacaaac ttattttgaa cactcagctc ctagcgtgcg gcgctgccaa 60
   tcattaacct cctggtgcaa gtggcgcggc ctgtgccctt tataaggtgc gcgctgtgtc 120
   cagcgagcat cggccaccgc catcccatcc agcgagcatc tgccgccgcg ccgccgccac 180
   cctcccagag agcactggcc accgctccac catcacttgc ccagagtttg ggccaccgcc 240
   cgccgccacc agcccagaga gcatcggccc ctgtctgctg ctcgcgcctg gagatgtcag 300
   aggtccccgt tgctcgcgtc tggctggtac tgctcctgct gactgtccag gtcggcgtga 360
   cagccggcgc tccgtggcag tgcgcgccct gctccgccga gaagctcgcg ctctgcccgc 420
   cggtgtccgc ctcgtgctcg gaggtcaccc ggtccgccgg ctgcggctgt tgcccgatgt 480
   gcgccctgcc tctgggcgcc gcgtgcggcg tggcgactgc acgctgcgcc cggggactca 540
   gttgccgcgc gctgccgggg gagcagcaac ctctgcacgc cctcacccgc ggccaaggcg 600
   cctgcgtgca ggagtctgac gcctccgctc cccatgctgc agaggcaggg agccctgaaa 660
   gcccagagag cacggagata actgaggagg agctcctgga taatttccat ctgatggccc 720
   cttctgaaga ggatcattcc atcctttggg acgccatcag tacctatgat ggctcgaagg 780
   ctctccatgt caccaacatc aaaaaatgga aggagccctg ccgaatagaa ctctacagag 840
   tcgtagagag tttagccaag gcacaggaga catcaggaga agaaatttcc aaattttacc 900
   tgccaaactg caacaagaat ggattttatc acagcagaca gtgtgagaca tccatggatg      960
   gagaggcggg actctgctgg tgcgtctacc cttggaatgg gaagaggatc cctgggtctc     1020
   cagagatcag gggagacccc aactgccaga tatattttaa tgtacaaaac tgaaaccaga     1080
   tgaaataatg ttctgtcacg tgaaatattt aagtatatag tatatttata ctctagaaca     1140
   tgcacattta tatatatatg tatatgtata tatatatagt aactactttt tatactccat     1200
   acataacttg atatagaaag ctgtttattt attcactgta agtttatttt ttctacacag     1260
   taaaaacttg tactatgtta ataacttgtc ctatgtcaat ttgtatatca tgaaacactt     1320
   ctcatcatat tgtatgtaag taattgcatt tctgctcttc caaagctcct gcgtctgttt     1380
   ttaaagagca tggaaaaata ctgcctagaa aatgcaaaat gaaataagag agagtagttt     1440
   ttcagctagt ttgaaggagg acggttaact tgtatattcc accattcaca tttgatgtac     1500
   atgtgtaggg aaagttaaaa gtgttgatta cataatcaaa gctacctgtg gtgatgttgc     1560
   cacctgttaa aatgtacact ggatatgttg ttaaacacgt gtctataatg gaaacattta     1620
   caataaatat tctgcatgga aatactgtta aaaaaaaaaa                            1660
<210> 97
   <211> 1953
   <212> DNA
   <213> Homo sapiens
<400> 97
   aggtgctcct gggtccgcgc gggtggcggg tgccgcgcac ttatccgttg gccagctgcg       60
   ttccgggatc agctaccaga cggtccctga gatgaccggg aaccgggctg ggggaggata      120
   gagccgagac tggaggatcg attggcacct ccgcccactt ttcccacaac gccttcccga      180
   acgataggtt ggaaaggctc ctggatccaa actcgctgag ggccaggcaa gaaaatgtcc      240
   tcaaatttat tgccaacact gaattctgga ggtaaagtaa aagatggctc aaccaaagag      300
   gacaggcctt ataagatctt tttcagagat ctctttcttg tcaaagaaaa tgaaatggca      360
   gcaaaggaaa cggaaaaatt tatgaaccgt aacatgaaag tctaccagaa aactactttt      420
   tcatccagaa tgaagagtca ttcatacctg agccaactag ctttctaccc taaaaggagt      480
   ggtaggtcat ttgaaaagtt tgggccaggt cctgctccga ttcctagatt aatagaaggt      540
   tccgacacaa aaaggactgt ccatgaattt attaatgacc agagagacag gtttctgctc      600
   gagtatgctt tgtcaaccaa aagaaacaca atcaaaaagt ttgaaaaaga catagcaatg      660
   agggaacggc aactaaaaaa agcagagaaa aagctccaag atgatgcact ggcctttgaa      720
   gagttccttc gagaaaatga ccagagatct gtagacgctc tgaaaatggc agcacaggaa      780
   acaataaaca aactccaaat gacagcagag ctgaagaaag caagcatgga ggtacaagca      840
   gtgaaaagtg aaatagcaaa aacagaattc ctcctcaggg agtatatgaa atatggtttt      900
   tttctgctgc aaatgtctcc aaaacattgg caaatccagc aagcactaaa aagagcacag      960
   gcatcaaaaa gtaaagcaaa tatcatcctt ccaaaaatat tagcaaaatt atcattacat    1020
   tcaagtaaca aggaaggcat ccttgaggag tccgggagga cagctgtcct ttcagaagat    1080
   gcttctcagg gaagagacag ccaaggaaag ccaagcagaa gcctgactcg cactccagag    1140
   aaaaagaaat caaacctggc tgaaagtttc ggttcagaag acagtttgga attcctttta    1200
   gatgatgaaa tggacgttga tttggagcca gcactttatt tcaaggaacc agaggagtta    1260
   cttcaagtcc tcagagagct ggaagagcag aatcttactt tgtttcaata ttcccaagat    1320
   gtagatgaaa atcttgaaga ggtaaacaaa agagaaaaag ttatacagga taaaacaaat    1380
   agcaacatag agtttctttt ggagcaagaa aaaatgctta aagctaactg tgtgagagaa    1440
   gaagagaaag cagcagaatt gcaattaaag tccaagctct ttagctttgg agaatttaat    1500
   tcagatgctc aggaaatact gatagactca cttagtaaaa agattactca agtatacaaa    1560
   gtctgcattg gagatgctga ggatgacggc ctcaacccaa ttcaaaagct ggtaaaagta    1620
   gaatctcgcc tggtagaact gtgtgacctc atcgaatcca ttcccaaaga aaatgtggag    1680
   gcaattgaga ggatgaaaca gaaagaatgg cggcaaaagt ttcgtgatga gaaaatgaaa    1740
   gaaaaacaaa gacaccaaca ggaaaggcta aaagctgctc tggaaaaagc agtagcacaa    1800
   ccaaagaaaa agttgggaag acaacttgtc tttcattcaa aacctccatc tggtaacaaa    1860
   cagcagctac ctttagtcaa tgaaacaaaa acaaaatcac aagaggaaga atattttttt    1920
   acttgaataa aagcagtaag acattttatt acc                                 1953
<210> 98
   <211> 407
   <212> PRT
   <213> Homo sapiens
<400> 98
   Met Pro Arg Gly His Lys Ser Lys Leu Arg Thr Cys Glu Lys Arg Gln
   1               5                   10                  15
   Glu Thr Asn Gly Gln Pro Gln Gly Leu Thr Gly Pro Gln Ala Thr Ala
               20                  25                  30
   Glu Lys Gln Glu Glu Ser His Ser Ser Ser Ser Ser Ser Arg Ala Cys
           35                  40                  45
   Leu Gly Asp Cys Arg Arg Ser Ser Asp Ala Ser Ile Pro Gln Glu Ser
       50                  55                  60
   Gln Gly Val Ser Pro Thr Gly Ser Pro Asp Ala Val Val Ser Tyr Ser
   65                  70                  75                  80
   Lys Ser Asp Val Ala Ala Asn Gly Gln Asp Glu Lys Ser Pro Ser Thr
                   85                  90                  95
   Ser Arg Asp Ala Ser Val Pro Gln Glu Ser Gln Gly Ala Ser Pro Thr
               100                 105                 110
   Gly Ser Pro Asp Ala Gly Val Ser Gly Ser Lys Tyr Asp Val Ala Ala
           115                 120                 125
   Asn Gly Gln Asp Glu Lys Ser Pro Ser Thr Ser His Asp Val Ser Val
       130                 135                 140
   Pro Gln Glu Ser Gln Gly Ala Ser Pro Thr Gly Ser Pro Asp Ala Gly
   145                 150                 155                 160
   Val Ser Gly Ser Lys Tyr Asp Val Ala Ala Glu Gly Glu Asp Glu Glu
                   165                 170                 175
   Ser Val Ser Ala Ser Gln Lys Ala Ile Ile Phe Lys Arg Leu Ser Lys
               180                 185                 190
   Asp Ala Val Lys Lys Lys Ala Cys Thr Leu Ala Gln Phe Leu Gln Lys
           195                 200                 205
   Lys Phe Glu Lys Lys Glu Ser Ile Leu Lys Ala Asp Met Leu Lys Cys
       210                 215                 220
   Val Arg Arg Glu Tyr Lys Pro Tyr Phe Pro Gln Ile Leu Asn Arg Thr
   225                 230                 235                 240
   Ser Gln His Leu Val Val Ala Phe Gly Val Glu Leu Lys Glu Met Asp
                   245                 250                 255
   Ser Ser Gly Glu Ser Tyr Thr Leu Val Ser Lys Leu Gly Leu Pro Ser
               260                 265                 270
   Glu Gly Ile Leu Ser Gly Asp Asn Ala Leu Pro Lys Ser Gly Leu Leu
           275                 280                 285
   Met Ser Leu Leu Val Val Ile Phe Met Asn Gly Asn Cys Ala Thr Glu
       290                 295                 300
   Glu Glu Val Trp Glu Phe Leu Gly Leu Leu Gly Ile Tyr Asp Gly Ile
   305                 310                 315                 320
   Leu His Ser Ile Tyr Gly Asp Ala Arg Lys Ile Ile Thr Glu Asp Leu
                   325                 330                 335
   Val Gln Asp Lys Tyr Val Val Tyr Arg Gln Val Cys Asn Ser Asp Pro
               340                 345                 350
   Pro Cys Tyr Glu Phe Leu Trp Gly Pro Arg Ala Tyr Ala Glu Thr Thr
   355 360 365
   Lys Met Arg Val Leu Arg Val Leu Ala Asp Ser Ser Asn Thr Ser Pro
   370 375 380
   Gly Leu Tyr Pro His Leu Tyr Glu Asp Ala Leu Ile Asp Glu Val Glu
   385 390 395 400
   Arg Ala Leu Arg Leu Arg Ala
   405
<210> 99
   <211> 533
   <212> PRT
   <213> Homo sapiens
<400> 99
   Met Asn Glu Ser Pro Asp Pro Thr Asp Leu Ala Gly Val Ile Ile Glu
   1 5 10 15
   Leu Gly Pro Asn Asp Ser Pro Gln Thr Ser Glu Phe Lys Gly Ala Thr
   20 25 30
   Glu Glu Ala Pro Ala Lys Glu Ser Val Leu Ala Arg Leu Ser Lys Phe
   35 40 45
   Glu Val Glu Asp Ala Glu Asn Val Ala Ser Tyr Asp Ser Lys Ile Lys
   50 55 60
   Lys Ile Val His Ser Ile Val Ser Ser Phe Ala Phe Gly Leu Phe Gly
   65 70 75 80
   Val Phe Leu Val Leu Leu Asp Val Thr Leu Ile Leu Ala Asp Leu Ile
   85 90 95
   Phe Thr Asp Ser Lys Leu Tyr Ile Pro Leu Glu Tyr Arg Ser Ile Ser
   100 105 110
   Leu Ala Ile Ala Leu Phe Phe Leu Met Asp Val Leu Leu Arg Val Phe
   115 120 125
   Val Glu Arg Arg Gln Gln Tyr Phe Ser Asp Leu Phe Asn Ile Leu Asp
   130 135 140
   Thr Ala Ile Ile Val Ile Leu Leu Leu Val Asp Val Val Tyr Ile Phe
   145 150 155 160
   Phe Asp Ile Lys Leu Leu Arg Asn Ile Pro Arg Trp Thr His Leu Leu
   165 170 175
   Arg Leu Leu Arg Leu Ile Ile Leu Leu Arg Ile Phe His Leu Phe His
               180                 185                 190
   Gln Lys Arg Gln Leu Glu Lys Leu Ile Arg Arg Arg Val Ser Glu Asn
           195                 200                 205
   Lys Arg Arg Tyr Thr Arg Asp Gly Phe Asp Leu Asp Leu Thr Tyr Val
       210                 215                 220
   Thr Glu Arg Ile Ile Ala Met Ser Phe Pro Ser Ser Gly Arg Gln Ser
   225                 230                 235                 240
   Phe Tyr Arg Asn Pro Ile Lys Glu Val Val Arg Phe Leu Asp Lys Lys
                   245                 250                 255
   His Arg Asn His Tyr Arg Val Tyr Asn Leu Cys Ser Glu Arg Ala Tyr
               260                 265                 270
   Asp Pro Lys His Phe His Asn Arg Val Val Arg Ile Met Ile Asp Asp
           275                 280                 285
   His Asn Val Pro Thr Leu His Gln Met Val Val Phe Thr Lys Glu Val
       290                 295                 300
   Asn Glu Trp Met Ala Gln Asp Leu Glu Asn Ile Val Ala Ile His Cys
   305                 310                 315                 320
   Lys Gly Gly Thr Asp Arg Thr Gly Thr Met Val Cys Ala Phe Leu Ile
                   325                 330                 335
   Ala Ser Glu Ile Cys Ser Thr Ala Lys Glu Ser Leu Tyr Tyr Phe Gly
               340                 345                 350
   Glu Arg Arg Thr Asp Lys Thr His Ser Glu Lys Phe Gln Gly Val Glu
           355                 360                 365
   Thr Pro Ser Gln Lys Arg Tyr Val Ala Tyr Phe Ala Gln Val Lys His
       370                 375                 380
   Leu Tyr Asn Trp Asn Leu Pro Pro Arg Arg Ile Leu Phe Ile Lys His
   385                 390                 395                 400
   Phe Ile Ile Tyr Ser Ile Pro Arg Tyr Val Arg Asp Leu Lys Ile Gln
                   405                 410                 415
   Ile Glu Met Glu Lys Lys Val Val Phe Ser Thr Ile Ser Leu Gly Lys
               420                 425                 430
   Cys Ser Val Leu Asp Asn Ile Thr Thr Asp Lys Ile Leu Ile Asp Val
   435 440 445
   Phe Asp Gly Leu Pro Leu Tyr Asp Asp Val Lys Val Gln Phe Phe Tyr
   450 455 460
   Ser Asn Leu Pro Thr Tyr Tyr Asp Asn Cys Ser Phe Tyr Phe Trp Leu
   465 470 475 480
   His Thr Ser Phe Ile Glu Asn Asn Arg Leu Tyr Leu Pro Lys Asn Glu
   485 490 495
   Leu Asp Asn Leu His Lys Gln Lys Ala Arg Arg Ile Tyr Pro Ser Asp
   500 505 510
   Phe Ala Val Glu Ile Leu Phe Gly Glu Lys Met Thr Ser Ser Asp Val
   515 520 525
   Val Ala Gly Ser Asp
   530
<210> 100
   <211> 533
   <212> PRT
   <213> Homo sapiens
<400> 100
   Met Asn Glu Glu Asn Ile Asp Gly Thr Asn Gly Cys Ser Lys Val Arg
   1 5 10 15
   Thr Gly Ile Gln Asn Glu Ala Ala Leu Leu Ala Leu Met Glu Lys Thr
   20 25 30
   Gly Tyr Asn Met Val Gln Glu Asn Gly Gln Arg Lys Phe Gly Gly Pro
   35 40 45
   Pro Pro Gly Trp Glu Gly Pro Pro Pro Pro Arg Gly Cys Glu Val Phe
   50 55 60
   Val Gly Lys Ile Pro Arg Asp Met Tyr Glu Asp Glu Leu Val Pro Val
   65 70 75 80
   Phe Glu Arg Ala Gly Lys Ile Tyr Glu Phe Arg Leu Met Met Glu Phe
   85 90 95
   Ser Gly Glu Asn Arg Gly Tyr Ala Phe Val Met Tyr Thr Thr Lys Glu
   100 105 110
   Glu Ala Gln Leu Ala Ile Arg Ile Leu Asn Asn Tyr Glu Ile Arg Pro
   115 120 125
   Gly Lys Phe Ile Gly Val Cys Val Ser Leu Asp Asn Cys Arg Leu Phe
   130 135 140
   Ile Gly Ala Ile Pro Lys Glu Lys Lys Lys Glu Glu Ile Leu Asp Glu
   145 150 155 160
   Met Lys Lys Val Thr Glu Gly Val Val Asp Val Ile Val Tyr Pro Ser
   165 170 175
   Ala Thr Asp Lys Thr Lys Asn Arg Gly Phe Ala Phe Val Glu Tyr Glu
   180 185 190
   Ser His Arg Ala Ala Ala Met Ala Arg Arg Lys Leu Ile Pro Gly Thr
   195 200 205
   Phe Gln Leu Trp Gly His Thr Ile Gln Val Asp Trp Ala Asp Pro Glu
   210 215 220
   Lys Glu Val Asp Glu Glu Thr Met Gln Arg Val Lys Val Leu Tyr Val
   225 230 235 240
   Arg Asn Leu Met Ile Ser Thr Thr Glu Glu Thr Ile Lys Ala Glu Phe
   245 250 255
   Asn Lys Phe Lys Pro Gly Ala Val Glu Arg Val Lys Lys Leu Arg Asp
   260 265 270
   Tyr Ala Phe Val His Phe Phe Asn Arg Glu Asp Ala Val Ala Ala Met
   275 280 285
   Ser Val Met Asn Gly Lys Cys Ile Asp Gly Ala Ser Ile Glu Val Thr
   290 295 300
   Leu Ala Lys Pro Val Asn Lys Glu Asn Thr Trp Arg Gln His Leu Asn
   305 310 315 320
   Gly Gln Ile Ser Pro Asn Ser Glu Asn Leu Ile Val Phe Ala Asn Lys
   325 330 335
   Glu Glu Ser His Pro Lys Thr Leu Gly Lys Leu Pro Thr Leu Pro Ala
   340 345 350
   Arg Leu Asn Gly Gln His Ser Pro Ser Pro Pro Glu Val Glu Arg Cys
   355 360 365
   Thr Tyr Pro Phe Tyr Pro Gly Thr Lys Leu Thr Pro Ile Ser Met Tyr
   370 375 380
   Ser Leu Lys Ser Asn His Phe Asn Ser Ala Val Met His Leu Asp Tyr
   385 390 395 400
   Tyr Cys Asn Lys Asn Asn Trp Ala Pro Pro Glu Tyr Tyr Leu Tyr Ser
   405 410 415
   Thr Thr Ser Gln Asp Gly Lys Val Leu Leu Val Tyr Lys Ile Val Ile
   420 425 430
   Pro Ala Ile Ala Asn Gly Ser Gln Ser Tyr Phe Met Pro Asp Lys Leu
   435 440 445
   Cys Thr Thr Leu Glu Asp Ala Lys Glu Leu Ala Ala Gln Phe Thr Leu
   450 455 460
   Leu His Leu Asp Tyr Asn Phe His Arg Ser Ser Ile Asn Ser Leu Ser
   465 470 475 480
   Pro Val Ser Ala Thr Leu Ser Ser Gly Thr Pro Ser Val Leu Pro Tyr
   485 490 495
   Thr Ser Arg Pro Tyr Ser Tyr Pro Gly Tyr Pro Leu Ser Pro Thr Ile
   500 505 510
   Ser Leu Ala Asn Gly Ser His Val Gly Gln Arg Leu Cys Ile Ser Asn
   515 520 525
   Gln Ala Ser Phe Phe
   530
<210> 101
   <211> 136
   <212> PRT
   <213> Homo sapiens
<400> 101
   Met Ala Arg Thr Lys Gln Thr Ala Arg Lys Ser Thr Gly Gly Lys Ala
   1 5 10 15
   Pro Arg Lys Gln Leu Ala Thr Lys Ala Ala Arg Lys Ser Ala Pro Ala
   20 25 30
   Thr Gly Gly Val Lys Lys Pro His Arg Tyr Arg Pro Gly Thr Val Ala
   35 40 45
   Leu Arg Glu Ile Arg Arg Tyr Gln Lys Ser Thr Glu Leu Leu Ile Arg
   50 55 60
   Lys Leu Pro Phe Gln Arg Leu Val Arg Glu Ile Ala Gln Asp Phe Lys
   65 70 75 80
   Thr Asp Leu Arg Phe Gln Ser Ser Ala Val Met Ala Leu Gln Glu Ala
   85 90 95
   Cys Glu Ala Tyr Leu Val Gly Leu Phe Glu Asp Thr Asn Leu Cys Ala
   100 105 110
   Ile His Ala Lys Arg Val Thr Ile Met Pro Lys Asp Ile Gln Leu Ala
   115 120 125
   Arg Arg Ile Arg Gly Glu Arg Ala
   130 135
<210> 102
   <211> 436
   <212> PRT
   <213> Homo sapiens
<400> 102
   Met Gln Gly Thr Pro Gly Gly Gly Thr Arg Pro Gly Pro Ser Pro Val
   1 5 10 15
   Asp Arg Arg Thr Leu Leu Val Phe Ser Phe Ile Leu Ala Ala Ala Leu
   20 25 30
   Gly Gln Met Asn Phe Thr Gly Asp Gln Val Leu Arg Val Leu Ala Lys
   35 40 45
   Asp Glu Lys Gln Leu Ser Leu Leu Gly Asp Leu Glu Gly Leu Lys Pro
   50 55 60
   Gln Lys Val Asp Phe Trp Arg Gly Pro Ala Arg Pro Ser Leu Pro Val
   65 70 75 80
   Asp Met Arg Val Pro Phe Ser Glu Leu Lys Asp Ile Lys Ala Tyr Leu
   85 90 95
   Glu Ser His Gly Leu Ala Tyr Ser Ile Met Ile Lys Asp Ile Gln Val
   100 105 110
   Leu Leu Asp Glu Glu Arg Gln Ala Met Ala Lys Ser Arg Arg Leu Glu
   115 120 125
   Arg Ser Thr Asn Ser Phe Ser Tyr Ser Ser Tyr His Thr Leu Glu Glu
   130 135 140
   Ile Tyr Ser Trp Ile Asp Asn Phe Val Met Glu His Ser Asp Ile Val
   145 150 155 160
   Ser Lys Ile Gln Ile Gly Asn Ser Phe Glu Asn Gln Ser Ile Leu Val
   165 170 175
   Leu Lys Phe Ser Thr Gly Gly Ser Arg His Pro Ala Ile Trp Ile Asp
   180 185 190
   Thr Gly Ile His Ser Arg Glu Trp Ile Thr His Ala Thr Gly Ile Trp
   195 200 205
   Thr Ala Asn Lys Ile Val Ser Asp Tyr Gly Lys Asp Arg Val Leu Thr
   210 215 220
   Asp Ile Leu Asn Ala Met Asp Ile Phe Ile Glu Leu Val Thr Asn Pro
   225 230 235 240
   Asp Gly Phe Ala Phe Thr His Ser Met Asn Arg Leu Trp Arg Lys Asn
   245 250 255
   Lys Ser Ile Arg Pro Gly Ile Phe Cys Ile Gly Val Asp Leu Asn Arg
   260 265 270
   Asn Trp Lys Ser Gly Phe Gly Gly Asn Gly Ser Asn Ser Asn Pro Cys
   275 280 285
   Ser Glu Thr Tyr His Gly Pro Ser Pro Gln Ser Glu Pro Glu Val Ala
   290 295 300
   Ala Ile Val Asn Phe Ile Thr Ala His Gly Asn Phe Lys Ala Leu Ile
   305 310 315 320
   Ser Ile His Ser Tyr Ser Gln Met Leu Met Tyr Pro Tyr Gly Arg Ser
   325 330 335
   Leu Asp Pro Val Ser Asn Gln Arg Glu Leu Tyr Asp Leu Ala Lys Asp
   340 345 350
   Ala Val Glu Ala Leu Tyr Lys Val His Gly Ile Glu Tyr Ile Phe Gly
   355 360 365
   Ser Ile Ser Thr Thr Leu Tyr Val Ala Ser Gly Ile Thr Val Asp Trp
   370 375 380
   Ala Tyr Asp Ser Gly Ile Lys Tyr Ala Phe Ser Phe Glu Leu Arg Asp
   385 390 395 400
   Thr Gly Gln Tyr Gly Phe Leu Leu Pro Ala Thr Gln Ile Ile Pro Thr
   405 410 415
   Ala Gln Glu Thr Trp Met Ala Leu Arg Thr Ile Met Glu His Thr Leu
   420 425 430
   Asn His Pro Tyr
   435
<210> 103
   <211> 735
   <212> PRT
   <213> Homo sapiens
<400> 103
   Met His Cys Gly Leu Leu Glu Glu Pro Asp Met Asp Ser Thr Glu Ser
   1 5 10 15
   Trp Ile Glu Arg Cys Leu Asn Glu Ser Glu Asn Lys Arg Tyr Ser Ser
   20 25 30
   His Thr Ser Leu Gly Asn Val Ser Asn Asp Glu Asn Glu Glu Lys Glu
   35 40 45
   Asn Asn Arg Ala Ser Lys Pro His Ser Thr Pro Ala Thr Leu Gln Trp
   50 55 60
   Leu Glu Glu Asn Tyr Glu Ile Ala Glu Gly Val Cys Ile Pro Arg Ser
   65 70 75 80
   Ala Leu Tyr Met His Tyr Leu Asp Phe Cys Glu Lys Asn Asp Thr Gln
   85 90 95
   Pro Val Asn Ala Ala Ser Phe Gly Lys Ile Ile Arg Gln Gln Phe Pro
   100 105 110
   Gln Leu Thr Thr Arg Arg Leu Gly Thr Arg Gly Gln Ser Lys Tyr His
   115 120 125
   Tyr Tyr Gly Ile Ala Val Lys Glu Ser Ser Gln Tyr Tyr Asp Val Met
   130 135 140
   Tyr Ser Lys Lys Gly Ala Ala Trp Val Ser Glu Thr Gly Lys Lys Glu
   145 150 155 160
   Val Ser Lys Gln Thr Val Ala Tyr Ser Pro Arg Ser Lys Leu Gly Thr
   165 170 175
   Leu Leu Pro Glu Phe Pro Asn Val Lys Asp Leu Asn Leu Pro Ala Ser
   180 185 190
   Leu Pro Glu Glu Lys Val Ser Thr Phe Ile Met Met Tyr Arg Thr His
   195 200 205
   Cys Gln Arg Ile Leu Asp Thr Val Ile Arg Ala Asn Phe Asp Glu Val
   210 215 220
   Gln Ser Phe Leu Leu His Phe Trp Gln Gly Met Pro Pro His Met Leu
   225 230 235 240
   Pro Val Leu Gly Ser Ser Thr Val Val Asn Ile Val Gly Val Cys Asp
   245 250 255
   Ser Ile Leu Tyr Lys Ala Ile Ser Gly Val Leu Met Pro Thr Val Leu
   260 265 270
   Gln Ala Leu Pro Asp Ser Leu Thr Gln Val Ile Arg Lys Phe Ala Lys
   275 280 285
   Gln Leu Asp Glu Trp Leu Lys Val Ala Leu His Asp Leu Pro Glu Asn
   290 295 300
   Leu Arg Asn Ile Lys Phe Glu Leu Ser Arg Arg Phe Ser Gln Ile Leu
   305 310 315 320
   Arg Arg Gln Thr Ser Leu Asn His Leu Cys Gln Ala Ser Arg Thr Val
   325 330 335
   Ile His Ser Ala Asp Ile Thr Phe Gln Met Leu Glu Asp Trp Arg Asn
   340 345 350
   Val Asp Leu Asn Ser Ile Thr Lys Gln Thr Leu Tyr Thr Met Glu Asp
   355 360 365
   Ser Arg Asp Glu His Arg Lys Leu Ile Thr Gln Leu Tyr Gln Glu Phe
   370 375 380
   Asp His Leu Leu Glu Glu Gln Ser Pro Ile Glu Ser Tyr Ile Glu Trp
   385 390 395 400
   Leu Asp Thr Met Val Asp Arg Cys Val Val Lys Val Ala Ala Lys Arg
   405 410 415
   Gln Gly Ser Leu Lys Lys Val Ala Gln Gln Phe Leu Leu Met Trp Ser
   420 425 430
   Cys Phe Gly Thr Arg Val Ile Arg Asp Met Thr Leu His Ser Ala Pro
   435 440 445
   Ser Phe Gly Ser Phe His Leu Ile His Leu Met Phe Asp Asp Tyr Val
   450 455 460
   Leu Tyr Leu Leu Glu Ser Leu His Cys Gln Glu Arg Ala Asn Glu Leu
   465 470 475 480
   Met Arg Ala Met Lys Gly Glu Gly Ser Thr Ala Glu Val Arg Glu Glu
   485 490 495
   Ile Ile Leu Thr Glu Ala Ala Ala Pro Thr Pro Ser Pro Val Pro Ser
   500 505 510
   Phe Ser Pro Ala Lys Ser Ala Thr Ser Val Glu Val Pro Pro Pro Ser
   515 520 525
   Ser Pro Val Ser Asn Pro Ser Pro Glu Tyr Thr Gly Leu Ser Thr Thr
   530 535 540
   Gly Ala Met Gln Ser Tyr Thr Trp Ser Leu Thr Tyr Thr Val Thr Thr
   545 550 555 560
   Ala Ala Gly Ser Pro Ala Glu Asn Ser Gln Gln Leu Pro Cys Met Arg
   565 570 575
   Asn Thr His Val Pro Ser Ser Ser Val Thr His Arg Ile Pro Val Tyr
   580 585 590
   Pro His Arg Glu Glu His Gly Tyr Thr Gly Ser Tyr Asn Tyr Gly Ser
   595 600 605
   Tyr Gly Asn Gln His Pro His Pro Met Gln Ser Gln Tyr Pro Ala Leu
   610 615 620
   Pro His Asp Thr Ala Ile Ser Gly Pro Leu His Tyr Ala Pro Tyr His
   625 630 635 640
   Arg Ser Ser Ala Gln Tyr Pro Phe Asn Ser Pro Thr Ser Arg Met Glu
   645 650 655
   Pro Cys Leu Met Ser Ser Thr Pro Arg Leu His Pro Thr Pro Val Thr
   660 665 670
   Pro Arg Trp Pro Glu Val Pro Ser Ala Asn Thr Cys Tyr Thr Ser Pro
   675 680 685
   Ser Val His Ser Ala Arg Tyr Gly Asn Ser Ser Asp Met Tyr Thr Pro
   690 695 700
   Leu Thr Thr Arg Arg Asn Ser Glu Tyr Glu His Met Gln His Phe Pro
   705 710 715 720
   Gly Phe Ala Tyr Ile Asn Gly Glu Ala Ser Thr Gly Trp Ala Lys
   725 730 735
<210> 104
   <211> 450
   <212> PRT
   <213> Homo sapiens
<400> 104
   Met Arg Glu Cys Ile Ser Ile His Val Gly Gln Ala Gly Val Gln Ile
   1               5                   10                  15
   Gly Asn Ala Cys Trp Glu Leu Tyr Cys Leu Glu His Gly Ile Gln Pro
               20                  25                  30
   Asp Gly Gln Met Pro Ser Asp Lys Thr Ile Gly Gly Gly Asp Asp Ser
           35                  40                  45
   Phe Asn Thr Phe Phe Ser Glu Thr Gly Ala Gly Lys His Val Pro Arg
       50                  55                  60
   Ala Val Phe Val Asp Leu Glu Pro Thr Val Val Asp Glu Val Arg Thr
   65                  70                  75                  80
   Gly Thr Tyr Arg Gln Leu Phe His Pro Glu Gln Leu Ile Thr Gly Lys
                   85                  90                  95
   Glu Asp Ala Ala Asn Asn Tyr Ala Arg Gly His Tyr Thr Ile Gly Lys
               100                 105                 110
   Glu Ile Val Asp Leu Val Leu Asp Arg Ile Arg Lys Leu Ala Asp Leu
           115                 120                 125
   Cys Thr Gly Leu Gln Gly Phe Leu Ile Phe His Ser Phe Gly Gly Gly
       130                 135                 140
   Thr Gly Ser Gly Phe Ala Ser Leu Leu Met Glu Arg Leu Ser Val Asp
   145                 150                 155                 160
   Tyr Gly Lys Lys Ser Lys Leu Glu Phe Ala Ile Tyr Pro Ala Pro Gln
                   165                 170                 175
   Val Ser Thr Ala Val Val Glu Pro Tyr Asn Ser Ile Leu Thr Thr His
               180                 185                 190
   Thr Thr Leu Glu His Ser Asp Cys Ala Phe Met Val Asp Asn Glu Ala
           195                 200                 205
   Ile Tyr Asp Ile Cys Arg Arg Asn Leu Asp Ile Glu Arg Pro Thr Tyr
   210 215 220
   Thr Asn Leu Asn Arg Leu Ile Gly Gln Ile Val Ser Ser Ile Thr Ala
   225 230 235 240
   Ser Leu Arg Phe Asp Gly Ala Leu Asn Val Asp Leu Thr Glu Phe Gln
   245 250 255
   Thr Asn Leu Val Pro Tyr Pro Arg Ile His Phe Pro Leu Ala Thr Tyr
   260 265 270
   Ala Pro Val Ile Ser Ala Glu Lys Ala Tyr His Glu Gln Leu Ser Val
   275 280 285
   Ala Glu Ile Thr Asn Ala Cys Phe Glu Pro Ala Asn Gln Met Val Lys
   290 295 300
   Cys Asp Pro Arg His Gly Lys Tyr Met Ala Cys Cys Met Leu Tyr Arg
   305 310 315 320
   Gly Asp Val Val Pro Lys Asp Val Asn Ala Ala Ile Ala Thr Ile Lys
   325 330 335
   Thr Lys Arg Thr Ile Gln Phe Val Asp Trp Cys Pro Thr Gly Phe Lys
   340 345 350
   Val Gly Ile Asn Tyr Gln Pro Pro Thr Val Val Pro Gly Gly Asp Leu
   355 360 365
   Ala Lys Val Gln Arg Ala Val Cys Met Leu Ser Asn Thr Thr Ala Ile
   370 375 380
   Ala Glu Ala Trp Ala Arg Leu Asp His Lys Phe Asp Leu Met Tyr Ala
   385 390 395 400
   Lys Arg Ala Phe Val His Trp Tyr Val Gly Glu Gly Met Glu Glu Gly
   405 410 415
   Glu Phe Ser Glu Ala Arg Glu Asp Leu Ala Ala Leu Glu Lys Asp Tyr
   420 425 430
   Glu Glu Val Gly Val Asp Ser Val Glu Ala Glu Ala Glu Glu Gly Glu
   435 440 445
   Glu Tyr
   450
<210> 105
   <211> 409
   <212> PRT
   <213> Homo sapiens
<400> 105
   Met Ser Leu His Ala Trp Glu Trp Glu Glu Asp Pro Ala Ser Ile Glu
   1               5                   10                  15
   Pro Ile Ser Ser Ile Thr Ser Phe Tyr Gln Ser Thr Ser Glu Cys Asp
               20                  25                  30
   Val Glu Glu His Leu Lys Ala Lys Ala Arg Ala Gln Glu Ser Asp Ser
           35                  40                  45
   Asp Arg Pro Cys Ser Ser Ile Glu Ser Ser Ser Glu Pro Ala Ser Thr
       50                  55                  60
   Phe Ser Ser Asp Val Pro His Val Val Pro Cys Lys Phe Thr Ile Ser
   65                  70                  75                  80
   Leu Ala Phe Pro Val Asn Met Gly Gln Lys Gly Lys Tyr Ala Ser Leu
                   85                  90                  95
   Ile Glu Lys Tyr Lys Lys His Pro Lys Thr Asp Ser Ser Val Thr Lys
               100                 105                 110
   Met Arg Arg Phe Tyr His Ile Glu Tyr Phe Leu Leu Pro Asp Asp Glu
           115                 120                 125
   Glu Pro Lys Lys Val Asp Ile Leu Leu Phe Pro Met Val Ala Lys Val
       130                 135                 140
   Phe Leu Glu Ser Gly Val Lys Thr Val Lys Pro Trp His Glu Gly Asp
   145                 150                 155                 160
   Lys Ala Trp Val Ser Trp Glu Gln Thr Phe Asn Ile Thr Val Thr Lys
                   165                 170                 175
   Glu Leu Leu Lys Lys Ile Asn Phe His Lys Ile Thr Leu Arg Leu Trp
               180                 185                 190
   Asn Thr Lys Asp Lys Met Ser Arg Lys Val Arg Tyr Tyr Arg Leu Lys
           195                 200                 205
   Thr Ala Gly Phe Thr Asp Asp Val Gly Ala Phe His Lys Ser Glu Val
       210                 215                 220
   Arg His Leu Val Leu Asn Gln Arg Lys Leu Ser Glu Gln Gly Ile Glu
   225                 230                 235                 240
   Asn Thr Asn Ile Val Arg Glu Glu Ser Asn Gln Glu His Pro Pro Gly
   245 250 255
   Lys Gln Glu Lys Thr Glu Lys His Pro Lys Ser Leu Gln Gly Ser His
   260 265 270
   Gln Ala Glu Pro Glu Thr Ser Ser Lys Asn Ser Glu Glu Tyr Glu Lys
   275 280 285
   Ser Leu Lys Met Asp Asp Ser Ser Thr Ile Gln Trp Ser Val Ser Arg
   290 295 300
   Thr Pro Thr Ile Ser Leu Ala Gly Ala Ser Met Met Glu Ile Lys Glu
   305 310 315 320
   Leu Ile Glu Ser Glu Ser Leu Ser Ser Leu Thr Asn Ile Leu Asp Arg
   325 330 335
   Gln Arg Ser Gln Ile Lys Gly Lys Asp Ser Glu Gly Arg Arg Lys Ile
   340 345 350
   Gln Arg Arg His Lys Lys Pro Leu Ala Glu Glu Glu Ala Asp Pro Thr
   355 360 365
   Leu Thr Gly Pro Arg Lys Gln Ser Ala Phe Ser Ile Gln Leu Ala Val
   370 375 380
   Met Pro Leu Leu Ala Gly Thr His Cys Leu Pro Cys Ser Gln Gln Leu
   385 390 395 400
   Leu Leu Val Leu Trp Pro Glu Arg Pro
   405
<210> 106
   <211> 110
   <212> PRT
   <213> Homo sapiens
<220>
   <221> misc_feature
   <222> (110)..(110)
   <223> Xaa can be any naturally occurring amino acid
<400> 106
   Glu His Ile His Thr Gln Lys Thr Asn Pro His Ile Phe Thr Tyr Leu
   1 5 10 15
   Tyr Ser Leu Gln Asp Ala Ser Ile Tyr Leu Thr Leu Pro Asn His Pro
   20 25 30
   Tyr Tyr Thr Lys Met Lys Thr Leu Val Phe His Phe Ser Asp Glu Gln
   35 40 45
   Asn Glu Val Gln Lys Ile Lys Thr Lys Val Lys Pro Ser Lys Lys Thr
   50 55 60
   Lys Ile Lys Ile Lys Leu Thr Asn Lys Lys Leu Val Gln Val Thr Gln
   65 70 75 80
   Gln Val Ala Ala Val Leu Lys Ser Lys Arg Ala Val Ser His Leu Phe
   85 90 95
   Ser Gln Pro Phe Ala Leu Leu Lys Asn Leu Lys Lys Lys Xaa
   100 105 110
<210> 107
   <211> 626
   <212> PRT
   <213> Homo sapiens
<400> 107
   Met Met Ala Asn Asp Ala Lys Pro Asp Val Lys Thr Val Gln Val Leu
   1 5 10 15
   Arg Asp Thr Ala Asn Arg Leu Arg Ile His Ser Ile Arg Ala Thr Cys
   20 25 30
   Ala Ser Gly Ser Gly Gln Leu Thr Ser Cys Cys Ser Ala Ala Glu Val
   35 40 45
   Val Ser Val Leu Phe Phe His Thr Met Lys Tyr Lys Gln Thr Asp Pro
   50 55 60
   Glu His Pro Asp Asn Asp Arg Phe Ile Leu Ser Arg Gly His Ala Ala
   65 70 75 80
   Pro Ile Leu Tyr Ala Ala Trp Val Glu Val Gly Asp Ile Ser Glu Ser
   85 90 95
   Asp Leu Leu Asn Leu Arg Lys Leu His Ser Asp Leu Glu Arg His Pro
   100 105 110
   Thr Pro Arg Leu Pro Phe Val Asp Val Ala Thr Gly Ser Leu Gly Gln
   115 120 125
   Gly Leu Gly Thr Ala Cys Gly Met Ala Tyr Thr Gly Lys Tyr Leu Asp
   130 135 140
   Lys Ala Ser Tyr Arg Val Phe Cys Leu Met Gly Asp Gly Glu Ser Ser
   145 150 155 160
   Glu Gly Ser Val Trp Glu Ala Phe Ala Phe Ala Ser His Tyr Asn Leu
                   165                 170                 175
   Asp Asn Leu Val Ala Val Phe Asp Val Asn Arg Leu Gly Gln Ser Gly
               180                 185                 190
   Pro Ala Pro Leu Glu His Gly Ala Asp Ile Tyr Gln Asn Cys Cys Glu
           195                 200                 205
   Ala Phe Gly Trp Asn Thr Tyr Leu Val Asp Gly His Asp Val Glu Ala
       210                 215                 220
   Leu Cys Gln Ala Phe Trp Gln Ala Ser Gln Val Lys Asn Lys Pro Thr
   225                 230                 235                 240
   Ala Ile Val Ala Lys Thr Phe Lys Gly Arg Gly Ile Pro Asn Ile Glu
                   245                 250                 255
   Asp Ala Glu Asn Trp His Gly Lys Pro Val Pro Lys Glu Arg Ala Asp
               260                 265                 270
   Ala Ile Val Lys Leu Ile Glu Ser Gln Ile Gln Thr Asn Glu Asn Leu
           275                 280                 285
   Ile Pro Lys Ser Pro Val Glu Asp Ser Pro Gln Ile Ser Ile Thr Asp
       290                 295                 300
   Ile Lys Met Thr Ser Pro Pro Ala Tyr Lys Val Gly Asp Lys Ile Ala
   305                 310                 315                 320
   Thr Gln Lys Thr Tyr Gly Leu Ala Leu Ala Lys Leu Gly Arg Ala Asn
                   325                 330                 335
   Glu Arg Val Ile Val Leu Ser Gly Asp Thr Met Asn Ser Thr Phe Ser
               340                 345                 350
   Glu Ile Phe Arg Lys Glu His Pro Glu Arg Phe Ile Glu Cys Ile Ile
           355                 360                 365
   Ala Glu Gln Asn Met Val Ser Val Ala Leu Gly Cys Ala Thr Arg Gly
       370                 375                 380
   Arg Thr Ile Ala Phe Ala Gly Ala Phe Ala Ala Phe Phe Thr Arg Ala
   385                 390                 395                 400
   Phe Asp Gln Leu Arg Met Gly Ala Ile Ser Gln Ala Asn Ile Asn Leu
                   405                 410                 415
   Ile Gly Ser His Cys Gly Val Ser Thr Gly Glu Asp Gly Val Ser Gln
   420 425 430
   Met Ala Leu Glu Asp Leu Ala Met Phe Arg Ser Ile Pro Asn Cys Thr
   435 440 445
   Val Phe Tyr Pro Ser Asp Ala Ile Ser Thr Glu His Ala Ile Tyr Leu
   450 455 460
   Ala Ala Asn Thr Lys Gly Met Cys Phe Ile Arg Thr Ser Gln Pro Glu
   465 470 475 480
   Thr Ala Val Ile Tyr Thr Pro Gln Glu Asn Phe Glu Ile Gly Gln Ala
   485 490 495
   Lys Val Val Arg His Gly Val Asn Asp Lys Val Thr Val Ile Gly Ala
   500 505 510
   Gly Val Thr Leu His Glu Ala Leu Glu Ala Ala Asp His Leu Ser Gln
   515 520 525
   Gln Gly Ile Ser Val Arg Val Ile Asp Pro Phe Thr Ile Lys Pro Leu
   530 535 540
   Asp Ala Ala Thr Ile Ile Ser Ser Ala Lys Ala Thr Gly Gly Arg Val
   545 550 555 560
   Ile Thr Val Glu Asp His Tyr Arg Glu Gly Gly Ile Gly Glu Ala Val
   565 570 575
   Cys Ala Ala Val Ser Arg Glu Pro Asp Ile Leu Val His Gln Leu Ala
   580 585 590
   Val Ser Gly Val Pro Gln Arg Gly Lys Thr Ser Glu Leu Leu Asp Met
   595 600 605
   Phe Gly Ile Ser Thr Arg His Ile Ile Ala Ala Val Thr Leu Thr Leu
   610 615 620
   Met Lys
   625
<210> 108
   <211> 444
   <212> PRT
   <213> Homo sapiens
<400> 108
   Met Glu Asn Ser Gly Lys Ala Asn Lys Lys Asp Thr His Asp Gly Pro
   1 5 10 15
   Pro Lys Glu Ile Lys Leu Pro Thr Ser Glu Ala Leu Leu Asp Tyr Gln
   20 25 30
   Cys Gln Ile Lys Glu Asp Ala Val Glu Gln Phe Met Phe Gln Ile Lys
   35 40 45
   Thr Leu Arg Lys Lys Asn Gln Lys Tyr His Glu Arg Asn Ser Arg Leu
   50 55 60
   Lys Glu Glu Gln Ile Trp His Ile Arg His Leu Leu Lys Glu Leu Ser
   65 70 75 80
   Glu Glu Lys Ala Glu Gly Leu Pro Val Val Thr Arg Glu Asp Val Glu
   85 90 95
   Glu Ala Met Lys Glu Lys Trp Lys Phe Glu Arg Asp Gln Glu Lys Asn
   100 105 110
   Leu Arg Asp Met Arg Met Gln Ile Ser Asn Ala Glu Lys Leu Phe Leu
   115 120 125
   Glu Lys Leu Ser Glu Lys Glu Tyr Trp Glu Glu Tyr Lys Asn Val Gly
   130 135 140
   Ser Glu Arg His Ala Lys Leu Ile Thr Ser Leu Gln Asn Asp Ile Asn
   145 150 155 160
   Thr Val Lys Glu Asn Ala Glu Lys Met Ser Glu His Tyr Lys Ile Thr
   165 170 175
   Leu Glu Asp Thr Arg Lys Lys Ile Ile Lys Glu Thr Leu Leu Gln Leu
   180 185 190
   Asp Gln Lys Lys Glu Trp Ala Thr Gln Asn Ala Val Lys Leu Ile Asp
   195 200 205
   Lys Gly Ser Tyr Leu Glu Ile Trp Glu Asn Asp Trp Leu Lys Lys Glu
   210 215 220
   Val Ala Ile His Arg Lys Glu Val Glu Glu Leu Lys Asn Ala Ile His
   225 230 235 240
   Glu Leu Glu Ala Glu Asn Leu Val Leu Ile Asp Gln Leu Ser Asn Cys
   245 250 255
   Arg Leu Val Asp Leu Lys Ile Pro Arg Tyr Pro Val Leu His Ser Cys
   260 265 270
   Pro Thr Ser Asn Pro Arg His Leu Leu Leu Leu Pro Leu Glu Ser Cys
   275 280 285
   Leu Ile Ser Ala Arg Arg Cys Trp Arg Leu Tyr Leu Thr Gln Ala Ala
   290 295 300
   Gly Leu Glu Val Pro Pro Glu Glu Met Ser Leu Glu Leu Pro Glu Thr
   305 310 315 320
   His Ile Glu Glu Lys Ser Glu Leu Gln Pro Thr Glu Val Glu Ser Arg
   325 330 335
   Asp Leu Met Ser Ser Ser Asp Glu Ser Thr Ile Leu His Leu Ser His
   340 345 350
   Glu Asn Ser Ile Glu Asp Leu Gln Tyr Val Lys Ile Asp Lys Glu Glu
   355 360 365
   Asn Ser Gly Thr Glu Phe Gly Asp Thr Asp Met Lys Tyr Leu Leu Tyr
   370 375 380
   Glu Asp Glu Lys Asp Phe Lys Asp Tyr Val Asn Leu Gly Pro Leu Gly
   385 390 395 400
   Val Lys Leu Met Ser Val Glu Ser Lys Lys Met Pro Ile His Phe Gln
   405 410 415
   Glu Lys Glu Ile Pro Val Lys Leu Tyr Lys Asp Val Arg Ser Pro Glu
   420 425 430
   Ser His Ile Thr Tyr Lys Met Met Lys Ser Phe Leu
   435 440
<210> 109
   <211> 513
   <212> PRT
   <213> Homo sapiens
<400> 109
   Met Ile Arg Thr Pro Leu Ser Ala Ser Ala His Arg Leu Leu Leu Pro
   1 5 10 15
   Gly Ser Arg Gly Arg Pro Pro Arg Asn Met Gln Pro Thr Gly Arg Glu
   20 25 30
   Gly Ser Arg Ala Leu Ser Arg Arg Tyr Leu Arg Arg Leu Leu Leu Leu
   35 40 45
   Leu Leu Leu Leu Leu Leu Arg Gln Pro Val Thr Arg Ala Glu Thr Thr
       50                  55                  60
   Pro Gly Ala Pro Arg Ala Leu Ser Thr Leu Gly Ser Pro Ser Leu Phe
   65                  70                  75                  80
   Thr Thr Pro Gly Val Pro Ser Ala Leu Thr Thr Pro Gly Leu Thr Thr
                   85                  90                  95
   Pro Gly Thr Pro Lys Thr Leu Asp Leu Arg Gly Arg Ala Gln Ala Leu
               100                 105                 110
   Met Arg Ser Phe Pro Leu Val Asp Gly His Asn Asp Leu Pro Gln Val
           115                 120                 125
   Leu Arg Gln Arg Tyr Lys Asn Val Leu Gln Asp Val Asn Leu Arg Asn
       130                 135                 140
   Phe Ser His Gly Gln Thr Ser Leu Asp Arg Leu Arg Asp Gly Leu Val
   145                 150                 155                 160
   Gly Ala Gln Phe Trp Ser Ala Ser Val Ser Cys Gln Ser Gln Asp Gln
                   165                 170                 175
   Thr Ala Val Arg Leu Ala Leu Glu Gln Ile Asp Leu Ile His Arg Met
               180                 185                 190
   Cys Ala Ser Tyr Ser Glu Leu Glu Leu Val Thr Ser Ala Glu Gly Leu
           195                 200                 205
   Asn Ser Ser Gln Lys Leu Ala Cys Leu Ile Gly Val Glu Gly Gly His
       210                 215                 220
   Ser Leu Asp Ser Ser Leu Ser Val Leu Arg Ser Phe Tyr Val Leu Gly
   225                 230                 235                 240
   Val Arg Tyr Leu Thr Leu Thr Phe Thr Cys Ser Thr Pro Trp Ala Glu
                   245                 250                 255
   Ser Ser Thr Lys Phe Arg His His Met Tyr Thr Asn Val Ser Gly Leu
               260                 265                 270
   Thr Ser Phe Gly Glu Lys Val Val Glu Glu Leu Asn Arg Leu Gly Met
           275                 280                 285
   Met Ile Asp Leu Ser Tyr Ala Ser Asp Thr Leu Ile Arg Arg Val Leu
       290                 295                 300
   Glu Val Ser Gln Ala Pro Val Ile Phe Ser His Ser Ala Ala Arg Ala
   305 310 315 320
   Val Cys Asp Asn Leu Leu Asn Val Pro Asp Asp Ile Leu Gln Leu Leu
   325 330 335
   Lys Lys Asn Gly Gly Ile Val Met Val Thr Leu Ser Met Gly Val Leu
   340 345 350
   Gln Cys Asn Leu Leu Ala Asn Val Ser Thr Val Ala Asp His Phe Asp
   355 360 365
   His Ile Arg Ala Val Ile Gly Ser Glu Phe Ile Gly Ile Gly Gly Asn
   370 375 380
   Tyr Asp Gly Thr Gly Arg Phe Pro Gln Gly Leu Glu Asp Val Ser Thr
   385 390 395 400
   Tyr Pro Val Leu Ile Glu Glu Leu Leu Ser Arg Ser Trp Ser Glu Glu
   405 410 415
   Glu Leu Gln Gly Val Leu Arg Gly Asn Leu Leu Arg Val Phe Arg Gln
   420 425 430
   Val Glu Lys Val Arg Glu Glu Ser Arg Ala Gln Ser Pro Val Glu Ala
   435 440 445
   Glu Phe Pro Tyr Gly Gln Leu Ser Thr Ser Cys His Ser His Leu Val
   450 455 460
   Pro Gln Asn Gly His Gln Ala Thr His Leu Glu Val Thr Lys Gln Pro
   465 470 475 480
   Thr Asn Arg Val Pro Trp Arg Ser Ser Asn Ala Ser Pro Tyr Leu Val
   485 490 495
   Pro Gly Leu Val Ala Ala Ala Thr Ile Pro Thr Phe Thr Gln Trp Leu
   500 505 510
   Cys
<210> 110
   <211> 154
   <212> PRT
   <213> Homo sapiens
<400> 110
   Met Glu Pro Ser Lys Thr Phe Met Arg Asn Leu Pro Ile Thr Pro Gly
   1 5 10 15
   Tyr Ser Gly Phe Val Pro Phe Leu Ser Cys Gln Gly Met Ser Lys Glu
   20 25 30
   Asp Asp Met Asn His Cys Val Lys Thr Phe Gln Glu Lys Thr Gln Arg
   35 40 45
   Tyr Lys Glu Gln Leu Arg Glu Leu Cys Cys Ala Val Ala Thr Ala Pro
   50 55 60
   Lys Leu Lys Pro Val Asn Ser Glu Glu Thr Val Leu Gln Ala Leu His
   65 70 75 80
   Gln Tyr Asn Leu Gln Tyr His Pro Leu Ile Leu Glu Cys Lys Tyr Val
   85 90 95
   Lys Lys Pro Leu Gln Glu Pro Pro Ile Pro Gly Trp Ala Gly Tyr Leu
   100 105 110
   Pro Arg Ala Lys Val Thr Glu Phe Gly Cys Gly Thr Arg Tyr Thr Val
   115 120 125
   Met Ala Lys Asn Cys Tyr Lys Asp Phe Leu Glu Ile Thr Glu Arg Ala
   130 135 140
   Lys Lys Ala His Leu Lys Pro Tyr Glu Glu
   145 150
<210> 111
   <211> 861
   <212> PRT
   <213> Homo sapiens
<400> 111
   Met Thr Gly Arg Ala Arg Ala Arg Ala Arg Gly Arg Ala Arg Gly Gln
   1 5 10 15
   Glu Thr Ala Gln Leu Val Gly Ser Thr Ala Ser Gln Gln Pro Gly Tyr
   20 25 30
   Ile Gln Pro Arg Pro Gln Pro Pro Pro Ala Glu Gly Glu Leu Phe Gly
   35 40 45
   Arg Gly Arg Gln Arg Gly Thr Ala Gly Gly Thr Ala Lys Ser Gln Gly
   50 55 60
   Leu Gln Ile Ser Ala Gly Phe Gln Glu Leu Ser Leu Ala Glu Arg Gly
   65 70 75 80
   Gly Arg Arg Arg Asp Phe His Asp Leu Gly Val Asn Thr Arg Gln Asn
   85 90 95
   Leu Asp His Val Lys Glu Ser Lys Thr Gly Ser Ser Gly Ile Ile Val
   100 105 110
   Arg Leu Ser Thr Asn His Phe Arg Leu Thr Ser Arg Pro Gln Trp Ala
   115 120 125
   Leu Tyr Gln Tyr His Ile Asp Tyr Asn Pro Leu Met Glu Ala Arg Arg
   130 135 140
   Leu Arg Ser Ala Leu Leu Phe Gln His Glu Asp Leu Ile Gly Lys Cys
   145 150 155 160
   His Ala Phe Asp Gly Thr Ile Leu Phe Leu Pro Lys Arg Leu Gln Gln
   165 170 175
   Lys Val Thr Glu Val Phe Ser Lys Thr Arg Asn Gly Glu Asp Val Arg
   180 185 190
   Ile Thr Ile Thr Leu Thr Asn Glu Leu Pro Pro Thr Ser Pro Thr Cys
   195 200 205
   Leu Gln Phe Tyr Asn Ile Ile Phe Arg Arg Leu Leu Lys Ile Met Asn
   210 215 220
   Leu Gln Gln Ile Gly Arg Asn Tyr Tyr Asn Pro Asn Asp Pro Ile Asp
   225 230 235 240
   Ile Pro Ser His Arg Leu Val Ile Trp Pro Gly Phe Thr Thr Ser Ile
   245 250 255
   Leu Gln Tyr Glu Asn Ser Ile Met Leu Cys Thr Asp Val Ser His Lys
   260 265 270
   Val Leu Arg Ser Glu Thr Val Leu Asp Phe Met Phe Asn Phe Tyr His
   275 280 285
   Gln Thr Glu Glu His Lys Phe Gln Glu Gln Val Ser Lys Glu Leu Ile
   290 295 300
   Gly Leu Val Val Leu Thr Lys Tyr Asn Asn Lys Thr Tyr Arg Val Asp
   305 310 315 320
   Asp Ile Asp Trp Asp Gln Asn Pro Lys Ser Thr Phe Lys Lys Ala Asp
   325 330 335
   Gly Ser Glu Val Ser Phe Leu Glu Tyr Tyr Arg Lys Gln Tyr Asn Gln
   340 345 350
   Glu Ile Thr Asp Leu Lys Gln Pro Val Leu Val Ser Gln Pro Lys Arg
           355                 360                     365
   Arg Arg Gly Pro Gly Gly Thr Leu Pro Gly Pro Ala Met Leu Ile Pro
       370                 375                 380
   Glu Leu Cys Tyr Leu Thr Gly Leu Thr Asp Lys Met Arg Asn Asp Phe
   385                 390                 395                 400
   Asn Val Met Lys Asp Leu Ala Val His Thr Arg Leu Thr Pro Glu Gln
                   405                 410                 415
   Arg Gln Arg Glu Val Gly Arg Leu Ile Asp Tyr Ile His Lys Asn Asp
               420                 425                 430
   Asn Val Gln Arg Glu Leu Arg Asp Trp Gly Leu Ser Phe Asp Ser Asn
           435                 440                 445
   Leu Leu Ser Phe Ser Gly Arg Ile Leu Gln Thr Glu Lys Ile His Gln
       450                 455                 460
   Gly Gly Lys Thr Phe Asp Tyr Asn Pro Gln Phe Ala Asp Trp Ser Lys
   465                 470                 475                 480
   Glu Thr Arg Gly Ala Pro Leu Ile Ser Val Lys Pro Leu Asp Asn Trp
                   485                 490                 495
   Leu Leu Ile Tyr Thr Arg Arg Asn Tyr Glu Ala Ala Asn Ser Leu Ile
               500                 505                 510
   Gln Asn Leu Phe Lys Val Thr Pro Ala Met Gly Met Gln Met Arg Lys
           515                 520                 525
   Ala Ile Met Ile Glu Val Asp Asp Arg Thr Glu Ala Tyr Leu Arg Val
       530                 535                 540
   Leu Gln Gln Lys Val Thr Ala Asp Thr Gln Ile Val Val Cys Leu Leu
   545                 550                 555                 560
   Ser Ser Asn Arg Lys Asp Lys Tyr Asp Ala Ile Lys Lys Tyr Leu Cys
                   565                 570                 575
   Thr Asp Cys Pro Thr Pro Ser Gln Cys Val Val Ala Arg Thr Leu Gly
               580                 585                 590
   Lys Gln Gln Thr Val Met Ala Ile Ala Thr Lys Ile Ala Leu Gln Met
           595                 600                 605
   Asn Cys Lys Met Gly Gly Glu Leu Trp Arg Val Asp Ile Pro Leu Lys
       610                 615                 620
   Leu Val Met Ile Val Gly Ile Asp Cys Tyr His Asp Met Thr Ala Gly
   625                 630                 635                 640
   Arg Arg Ser Ile Ala Gly Phe Val Ala Ser Ile Asn Glu Gly Met Thr
                   645                 650                 655
   Arg Trp Phe Ser Arg Cys Ile Phe Gln Asp Arg Gly Gln Glu Leu Val
               660                 665                 670
   Asp Gly Leu Lys Val Cys Leu Gln Ala Ala Leu Arg Ala Trp Asn Ser
           675                 680                 685
   Cys Asn Glu Tyr Met Pro Ser Arg Ile Ile Val Tyr Arg Asp Gly Val
       690                 695                 700
   Gly Asp Gly Gln Leu Lys Thr Leu Val Asn Tyr Glu Val Pro Gln Phe
   705                 710                 715                 720
   Leu Asp Cys Leu Lys Ser Ile Gly Arg Gly Tyr Asn Pro Arg Leu Thr
                   725                 730                 735
   Val Ile Val Val Lys Lys Arg Val Asn Thr Arg Phe Phe Ala Gln Ser
               740                 745                 750
   Gly Gly Arg Leu Gln Asn Pro Leu Pro Gly Thr Val Ile Asp Val Glu
           755                 760                 765
   Val Thr Arg Pro Glu Trp Tyr Asp Phe Phe Ile Val Ser Gln Ala Val
       770                 775                 780
   Arg Ser Gly Ser Val Ser Pro Thr His Tyr Asn Val Ile Tyr Asp Asn
   785                 790                 795                 800
   Ser Gly Leu Lys Pro Asp His Ile Gln Arg Leu Thr Tyr Lys Leu Cys
                   805                 810                 815
   His Ile Tyr Tyr Asn Trp Pro Gly Val Ile Arg Val Pro Ala Pro Cys
               820                 825                 830
   Gln Tyr Ala His Lys Leu Ala Phe Leu Val Gly Gln Ser Ile His Arg
           835                 840                 845
   Glu Pro Asn Leu Ser Leu Ser Asn Arg Leu Tyr Tyr Leu
       850                 855                 860
<210> 112
   <211> 212
   <212> PRT
   <213> Homo sapiens
<400> 112
   Met Ala Gln Thr Asp Lys Pro Thr Cys Ile Pro Pro Glu Leu Pro Lys
   1               5                   10                  15
   Met Leu Lys Glu Phe Ala Lys Ala Ala Ile Arg Val Gln Pro Gln Asp
               20                  25                  30
   Leu Ile Gln Trp Ala Ala Asp Tyr Phe Glu Ala Leu Ser Arg Gly Glu
           35                  40                  45
   Thr Pro Pro Val Arg Glu Arg Ser Glu Arg Val Ala Leu Cys Asn Arg
       50                  55                  60
   Ala Glu Leu Thr Pro Glu Leu Leu Lys Ile Leu His Ser Gln Val Ala
   65                  70                  75                  80
   Gly Arg Leu Ile Ile Arg Ala Glu Glu Leu Ala Gln Met Trp Lys Val
                   85                  90                  95
   Val Asn Leu Pro Thr Asp Leu Phe Asn Ser Val Met Asn Val Gly Arg
               100                 105                 110
   Phe Thr Glu Glu Ile Glu Trp Leu Lys Phe Leu Ala Leu Ala Cys Ser
           115                 120                 125
   Ala Leu Gly Val Thr Ile Thr Lys Thr Leu Lys Ile Val Cys Glu Val
       130                 135                 140
   Leu Ser Cys Asp His Asn Gly Gly Ser Pro Arg Ile Pro Phe Ser Thr
   145                 150                 155                 160
   Phe Gln Phe Leu Tyr Thr Tyr Ile Ala Lys Val Asp Gly Glu Ile Ser
                   165                 170                 175
   Ala Ser His Val Ser Arg Met Leu Asn Tyr Met Glu Gln Glu Val Ile
               180                 185                 190
   Gly Pro Asp Gly Ile Ile Thr Val Asn Asp Phe Thr Gln Asn Pro Arg
           195                 200                 205
   Val Gln Leu Glu
       210
<210> 113
   <211> 123
   <212> PRT
   <213> Homo sapiens
<400> 113
   Met Val Val Ser Ala Asp Pro Leu Ser Ser Glu Arg Ala Glu Met Asn
   1               5                   10                  15
   Ile Leu Glu Ile Asn Gln Glu Leu Arg Ser Gln Leu Ala Glu Ser Asn
               20                  25                  30
   Gln Gln Phe Arg Asp Leu Lys Glu Lys Phe Leu Ile Thr Gln Ala Thr
           35                  40                  45
   Ala Tyr Ser Leu Ala Asn Gln Leu Lys Lys Tyr Lys Cys Glu Glu Tyr
       50                  55                  60
   Arg Asn His Leu Pro Pro Glu Arg Cys Arg Arg Leu Lys Lys Arg Lys
   65                  70                  75                  80
   Ser Leu Arg Thr His Trp Arg Asn Val Leu Ser Leu Val Gln Ile Val
                   85                  90                  95
   Thr Thr Leu Leu Thr Pro Thr Ser Leu Thr Gly Ala Pro Lys Ser His
               100                 105                 110
   Leu Arg Asn Thr Lys Ser Thr Leu Leu Trp Leu
           115                 120
<210> 114
   <211> 86
   <212> PRT
   <213> Homo sapiens
<400> 114
   Ala Leu Leu Leu Pro Cys Ser Leu Ile Ser Asp Cys Cys Ala Ser Asn
   1               5                   10                  15
   Gln Arg Asp Ser Val Gly Val Gly Pro Ser Lys Pro Gly Glu Arg Ala
               20                  25                  30
   Tyr Asp Pro Lys His Phe His Asn Arg Val Ser Arg Ile Met Ile Asp
           35                  40                  45
   Asp His Asn Val Pro Thr Leu Arg Glu Met Val Ala Phe Ser Lys Glu
       50                  55                  60
   Val Leu Glu Trp Met Ala Gln Asp Ser Glu Asn Ile Val Val Ile His
   65                  70                  75                  80
   Cys Lys Gly Gly Lys Glu
                   85
<210> 115
   <211> 223
   <212> PRT
   <213> Homo sapiens
<400> 115
   Met Arg Asp Glu Ile Ala Thr Thr Val Phe Phe Val Thr Arg Leu Val
   1 5 10 15
   Lys Lys His Asp Lys Leu Ser Lys Gln Gln Ile Glu Asp Phe Ala Glu
   20 25 30
   Lys Leu Met Thr Ile Leu Phe Glu Thr Tyr Arg Ser His Trp His Ser
   35 40 45
   Asp Cys Pro Ser Lys Gly Gln Ala Phe Arg Cys Ile Arg Ile Asn Asn
   50 55 60
   Asn Gln Asn Lys Asp Pro Ile Leu Glu Arg Ala Cys Val Glu Ser Asn
   65 70 75 80
   Val Asp Phe Ser His Leu Gly Leu Pro Lys Glu Met Thr Ile Trp Val
   85 90 95
   Asp Pro Phe Glu Val Cys Cys Arg Tyr Gly Glu Lys Asn His Pro Phe
   100 105 110
   Thr Val Ala Ser Phe Lys Gly Arg Trp Glu Glu Trp Glu Leu Tyr Gln
   115 120 125
   Gln Ile Ser Tyr Ala Val Ser Arg Ala Ser Ser Asp Val Ser Ser Gly
   130 135 140
   Thr Ser Cys Asp Glu Glu Ser Cys Ser Lys Glu Pro Arg Val Ile Pro
   145 150 155 160
   Lys Val Ser Asn Pro Lys Ser Ile Tyr Gln Val Glu Asn Leu Lys Gln
   165 170 175
   Pro Phe Gln Ser Trp Leu Gln Ile Pro Arg Lys Lys Asn Val Val Asp
   180 185 190
   Gly Arg Val Gly Leu Leu Gly Asn Thr Tyr His Gly Ser Gln Lys His
   195 200 205
   Pro Lys Cys Tyr Arg Pro Ala Met His Arg Leu Asp Arg Ile Leu
   210 215 220
<210> 116
   <211> 571
   <212> PRT
   <213> Homo sapiens
<400> 116
   Met Arg Ala Leu Arg Asp Arg Ala Gly Leu Leu Leu Cys Val Leu Leu
   1               5                   10                  15
   Leu Ala Ala Leu Leu Glu Ala Ala Leu Gly Leu Pro Val Lys Lys Pro
               20                  25                  30
   Arg Leu Arg Gly Pro Arg Pro Gly Ser Leu Thr Arg Leu Ala Glu Val
           35                  40                  45
   Ser Ala Ser Pro Asp Pro Arg Pro Leu Lys Glu Glu Glu Glu Ala Pro
       50                  55                  60
   Leu Leu Pro Arg Thr His Leu Gln Ala Glu Pro His Gln His Gly Cys
   65                  70                  75                  80
   Trp Thr Val Thr Glu Pro Ala Ala Met Thr Pro Gly Asn Ala Thr Pro
                   85                  90                  95
   Pro Arg Thr Pro Glu Val Thr Pro Leu Arg Leu Glu Leu Gln Lys Leu
               100                 105                 110
   Pro Gly Leu Ala Asn Thr Thr Leu Ser Thr Pro Asn Pro Asp Thr Gln
           115                 120                 125
   Ala Ser Ala Ser Pro Asp Pro Arg Pro Leu Arg Glu Glu Glu Glu Ala
       130                 135                 140
   Arg Leu Leu Pro Arg Thr His Leu Gln Ala Glu Leu His Gln His Gly
   145                 150                 155                 160
   Cys Trp Thr Val Thr Glu Pro Ala Ala Leu Thr Pro Gly Asn Ala Thr
                   165                 170                 175
   Pro Pro Arg Thr Gln Glu Val Thr Pro Leu Leu Leu Glu Leu Gln Lys
               180                 185                 190
   Leu Pro Glu Leu Val His Ala Thr Leu Ser Thr Pro Asn Pro Asp Asn
           195                 200                 205
   Gln Val Thr Ile Lys Val Val Glu Asp Pro Gln Ala Glu Val Ser Ile
       210                 215                 220
   Asp Leu Leu Ala Glu Pro Ser Asn Pro Pro Pro Gln Asp Thr Leu Ser
   225                 230                 235                 240
   Trp Leu Pro Ala Leu Trp Ser Phe Leu Trp Gly Asp Tyr Lys Gly Glu
                   245                 250                 255
   Glu Lys Asp Arg Ala Pro Gly Glu Lys Gly Glu Glu Lys Glu Glu Asp
               260                 265                 270
   Glu Asp Tyr Pro Ser Glu Asp Ile Glu Gly Glu Asp Gln Glu Asp Lys
           275                 280                 285
   Glu Glu Asp Glu Glu Glu Gln Ala Leu Trp Phe Asn Gly Thr Thr Asp
       290                 295                 300
   Asn Trp Asp Gln Gly Trp Leu Ala Pro Gly Asp Trp Val Phe Lys Asp
   305                 310                 315                 320
   Ser Val Ser Tyr Asp Tyr Glu Pro Gln Lys Glu Trp Ser Pro Trp Ser
                   325                 330                 335
   Pro Cys Ser Gly Asn Cys Ser Thr Gly Lys Gln Gln Arg Thr Arg Pro
               340                 345                 350
   Cys Gly Tyr Gly Cys Thr Ala Thr Glu Thr Arg Thr Cys Asp Leu Pro
           355                 360                 365
   Ser Cys Pro Gly Thr Glu Asp Lys Asp Thr Leu Gly Leu Pro Ser Glu
       370                 375                 380
   Glu Trp Lys Leu Leu Ala Arg Asn Ala Thr Asp Met His Asp Gln Asp
   385                 390                 395                 400
   Val Asp Ser Cys Glu Lys Trp Leu Asn Cys Lys Ser Asp Phe Leu Ile
                   405                 410                 415
   Lys Tyr Leu Ser Gln Met Leu Arg Asp Leu Pro Ser Cys Pro Cys Ala
               420                 425                 430
   Tyr Pro Leu Glu Ala Met Asp Ser Pro Val Ser Leu Gln Asp Glu His
           435                 440                 445
   Gln Gly Arg Ser Phe Arg Trp Arg Asp Ala Ser Gly Pro Arg Glu Arg
       450                 455                 460
   Leu Asp Ile Tyr Gln Pro Thr Ala Arg Phe Cys Leu Arg Ser Met Leu
   465                 470                 475                 480
   Ser Gly Glu Ser Ser Thr Leu Ala Ala Gln His Cys Cys Tyr Asp Glu
                   485                 490                 495
   Asp Ser Arg Leu Leu Thr Arg Gly Lys Gly Ala Gly Met Pro Asn Leu
   500 505 510
   Ile Ser Thr Asp Phe Ser Pro Lys Leu His Phe Lys Phe Asp Thr Thr
   515 520 525
   Pro Trp Ile Leu Cys Lys Gly Asp Trp Ser Arg Leu His Ala Val Leu
   530 535 540
   Pro Pro Asn Asn Gly Arg Ala Cys Thr Asp Asn Pro Leu Glu Glu Glu
   545 550 555 560
   Tyr Leu Ala Gln Leu Gln Glu Ala Lys Glu Tyr
   565 570
<210> 117
   <211> 229
   <212> PRT
   <213> Homo sapiens
<400> 117
   Met Thr Pro Gln Leu Leu Leu Ala Leu Val Leu Trp Ala Ser Cys Pro
   1 5 10 15
   Pro Cys Ser Gly Arg Lys Gly Pro Pro Ala Ala Leu Thr Leu Pro Arg
   20 25 30
   Val Gln Cys Arg Ala Ser Arg Tyr Pro Ile Ala Val Asp Cys Ser Trp
   35 40 45
   Thr Leu Pro Pro Ala Pro Asn Ser Thr Ser Pro Val Ser Phe Ile Ala
   50 55 60
   Thr Tyr Arg Leu Gly Met Ala Ala Arg Gly His Ser Trp Pro Cys Leu
   65 70 75 80
   Gln Gln Thr Pro Thr Ser Thr Ser Cys Thr Ile Thr Asp Val Gln Leu
   85 90 95
   Phe Ser Met Ala Pro Tyr Val Leu Asn Val Thr Ala Val His Pro Trp
   100 105 110
   Gly Ser Ser Ser Ser Phe Val Pro Phe Ile Thr Glu His Ile Ile Lys
   115 120 125
   Pro Asp Pro Pro Glu Gly Val Arg Leu Ser Pro Leu Ala Glu Arg Gln
   130 135 140
   Leu Gln Val Gln Trp Glu Pro Pro Gly Ser Trp Pro Phe Pro Glu Ile
   145 150 155 160
   Phe Ser Leu Lys Tyr Trp Ile Arg Tyr Lys Arg Gln Gly Ala Ala Arg
   165 170 175
   Phe His Arg Val Gly Pro Ile Glu Ala Thr Ser Phe Ile Leu Arg Ala
   180 185 190
   Val Arg Pro Arg Ala Arg Tyr Tyr Val Gln Val Ala Ala Gln Asp Leu
   195 200 205
   Thr Asp Tyr Gly Glu Leu Ser Asp Trp Ser Leu Pro Ala Thr Ala Thr
   210 215 220
   Met Ser Leu Gly Lys
   225
<210> 118
   <211> 168
   <212> PRT
   <213> Homo sapiens
<400> 118
   Met Ser Leu Thr His Arg Leu His Leu Cys Lys Tyr Trp Gly Cys Ala
   1 5 10 15
   Val Ser Asn Val Cys Arg Phe Trp Glu Gly Arg Pro Leu Pro Leu Met
   20 25 30
   Ile Val Val Pro Tyr Thr Leu Pro Val Ser Leu Pro Val Gly Ser Cys
   35 40 45
   Val Ile Ile Thr Gly Thr Pro Ile Leu Thr Phe Val Lys Asp Pro Gln
   50 55 60
   Leu Glu Val Asn Phe Tyr Thr Gly Met Asp Glu Asp Ser Asp Ile Ala
   65 70 75 80
   Phe Gln Phe Arg Leu His Phe Gly His Pro Ala Ile Met Asn Ser Cys
   85 90 95
   Val Phe Gly Ile Trp Arg Tyr Glu Glu Lys Cys Tyr Tyr Leu Pro Phe
   100 105 110
   Glu Asp Gly Lys Pro Phe Glu Leu Cys Ile Tyr Val Arg His Lys Glu
   115 120 125
   Tyr Lys Val Met Val Asn Gly Gln Arg Ile Tyr Asn Phe Ala His Arg
   130 135 140
   Phe Pro Pro Ala Ser Val Lys Met Leu Gln Val Phe Arg Asp Ile Ser
   145 150 155 160
   Leu Thr Arg Val Leu Ile Ser Asp
   165
<210> 119
   <211> 125
   <212> PRT
   <213> Homo sapiens
<400> 119
   Met Ser Pro Lys Pro Arg Ala Ser Gly Pro Pro Ala Lys Ala Lys Glu
   1 5 10 15
   Thr Gly Lys Arg Lys Ser Ser Ser Gln Pro Ser Pro Ser Gly Pro Lys
   20 25 30
   Lys Lys Thr Thr Lys Val Ala Glu Lys Gly Glu Ala Val Arg Gly Gly
   35 40 45
   Arg Arg Gly Lys Lys Gly Ala Ala Thr Lys Met Ala Ala Val Thr Ala
   50 55 60
   Pro Glu Ala Glu Ser Gly Pro Ala Ala Pro Gly Pro Ser Asp Gln Pro
   65 70 75 80
   Ser Gln Glu Leu Pro Gln His Glu Leu Pro Pro Glu Glu Pro Val Ser
   85 90 95
   Glu Gly Thr Gln His Asp Pro Leu Ser Gln Glu Ser Glu Leu Glu Glu
   100 105 110
   Pro Leu Ser Lys Gly Arg Pro Ser Thr Pro Leu Ser Pro
   115 120 125
<210> 120
   <211> 123
   <212> PRT
   <213> Homo sapiens
<400> 120
   Met Lys Tyr Phe Ala Pro Ser Arg Gly Pro Gln Leu Ser Leu Gln Val
   1 5 10 15
   Leu Leu Trp Arg Leu Asn Leu Pro Pro Val Ser Arg Ser Ser Gln Leu
   20 25 30
   Ser Leu Leu Ser Phe Leu Gly Arg Trp Asn Phe Leu Arg Pro Arg Arg
   35 40 45
   Pro Pro Thr Leu Pro Pro Glu Ser Ser Ile Glu Ser Val Ala Gln Thr
   50 55 60
   Pro Leu Asn His Glu Val Thr Val Gln Thr Gln Gly Glu Asp Gln Ala
   65 70 75 80
   His Tyr Thr Leu Pro Ser Ile Thr Val Lys Pro Ala Asp Val Glu Ile
   85 90 95
   Ser Ile Thr Ser Glu Pro Thr Thr Asp Thr Asp Ser Ser Pro Ala Gln
   100 105 110
   Gln Ala Ala Pro Asn Gln His Pro Glu Gln Val
   115 120
<210> 121
   <211> 259
   <212> PRT
   <213> Homo sapiens
<400> 121
   Met Ser Glu Val Pro Val Ala Arg Val Trp Leu Val Leu Leu Leu Leu
   1 5 10 15
   Thr Val Gln Val Gly Val Thr Ala Gly Ala Pro Trp Gln Cys Ala Pro
   20 25 30
   Cys Ser Ala Glu Lys Leu Ala Leu Cys Pro Pro Val Ser Ala Ser Cys
   35 40 45
   Ser Glu Val Thr Arg Ser Ala Gly Cys Gly Cys Cys Pro Met Cys Ala
   50 55 60
   Leu Pro Leu Gly Ala Ala Cys Gly Val Ala Thr Ala Arg Cys Ala Arg
   65 70 75 80
   Gly Leu Ser Cys Arg Ala Leu Pro Gly Glu Gln Gln Pro Leu His Ala
   85 90 95
   Leu Thr Arg Gly Gln Gly Ala Cys Val Gln Glu Ser Asp Ala Ser Ala
   100 105 110
   Pro His Ala Ala Glu Ala Gly Ser Pro Glu Ser Pro Glu Ser Thr Glu
   115 120 125
   Ile Thr Glu Glu Glu Leu Leu Asp Asn Phe His Leu Met Ala Pro Ser
   130 135 140
   Glu Glu Asp His Ser Ile Leu Trp Asp Ala Ile Ser Thr Tyr Asp Gly
   145 150 155 160
   Ser Lys Ala Leu His Val Thr Asn Ile Lys Lys Trp Lys Glu Pro Cys
   165 170 175
   Arg Ile Glu Leu Tyr Arg Val Val Glu Ser Leu Ala Lys Ala Gln Glu
   180 185 190
   Thr Ser Gly Glu Glu Ile Ser Lys Phe Tyr Leu Pro Asn Cys Asn Lys
   195 200 205
   Asn Gly Phe Tyr His Ser Arg Gln Cys Glu Thr Ser Met Asp Gly Glu
   210 215 220
   Ala Gly Leu Cys Trp Cys Val Tyr Pro Trp Asn Gly Lys Arg Ile Pro
   225 230 235 240
   Gly Ser Pro Glu Ile Arg Gly Asp Pro Asn Cys Gln Ile Tyr Phe Asn
   245 250 255
   Val Gln Asn
<210> 122
   <211> 563
   <212> PRT
   <213> Homo sapiens
<400> 122
   Met Ser Ser Asn Leu Leu Pro Thr Leu Asn Ser Gly Gly Lys Val Lys
   1 5 10 15
   Asp Gly Ser Thr Lys Glu Asp Arg Pro Tyr Lys Ile Phe Phe Arg Asp
   20 25 30
   Leu Phe Leu Val Lys Glu Asn Glu Met Ala Ala Lys Glu Thr Glu Lys
   35 40 45
   Phe Met Asn Arg Asn Met Lys Val Tyr Gln Lys Thr Thr Phe Ser Ser
   50 55 60
   Arg Met Lys Ser His Ser Tyr Leu Ser Gln Leu Ala Phe Tyr Pro Lys
   65 70 75 80
   Arg Ser Gly Arg Ser Phe Glu Lys Phe Gly Pro Gly Pro Ala Pro Ile
   85 90 95
   Pro Arg Leu Ile Glu Gly Ser Asp Thr Lys Arg Thr Val His Glu Phe
   100 105 110
   Ile Asn Asp Gln Arg Asp Arg Phe Leu Leu Glu Tyr Ala Leu Ser Thr
   115 120 125
   Lys Arg Asn Thr Ile Lys Lys Phe Glu Lys Asp Ile Ala Met Arg Glu
   130 135 140
   Arg Gln Leu Lys Lys Ala Glu Lys Lys Leu Gln Asp Asp Ala Leu Ala
   145 150 155 160
   Phe Glu Glu Phe Leu Arg Glu Asn Asp Gln Arg Ser Val Asp Ala Leu
   165 170 175
   Lys Met Ala Ala Gln Glu Thr Ile Asn Lys Leu Gln Met Thr Ala Glu
   180 185 190
   Leu Lys Lys Ala Ser Met Glu Val Gln Ala Val Lys Ser Glu Ile Ala
   195 200 205
   Lys Thr Glu Phe Leu Leu Arg Glu Tyr Met Lys Tyr Gly Phe Phe Leu
   210 215 220
   Leu Gln Met Ser Pro Lys His Trp Gln Ile Gln Gln Ala Leu Lys Arg
   225 230 235 240
   Ala Gln Ala Ser Lys Ser Lys Ala Asn Ile Ile Leu Pro Lys Ile Leu
   245 250 255
   Ala Lys Leu Ser Leu His Ser Ser Asn Lys Glu Gly Ile Leu Glu Glu
   260 265 270
   Ser Gly Arg Thr Ala Val Leu Ser Glu Asp Ala Ser Gln Gly Arg Asp
   275 280 285
   Ser Gln Gly Lys Pro Ser Arg Ser Leu Thr Arg Thr Pro Glu Lys Lys
   290 295 300
   Lys Ser Asn Leu Ala Glu Ser Phe Gly Ser Glu Asp Ser Leu Glu Phe
   305 310 315 320
   Leu Leu Asp Asp Glu Met Asp Val Asp Leu Glu Pro Ala Leu Tyr Phe
   325 330 335
   Lys Glu Pro Glu Glu Leu Leu Gln Val Leu Arg Glu Leu Glu Glu Gln
   340 345 350
   Asn Leu Thr Leu Phe Gln Tyr Ser Gln Asp Val Asp Glu Asn Leu Glu
   355 360 365
   Glu Val Asn Lys Arg Glu Lys Val Ile Gln Asp Lys Thr Asn Ser Asn
   370 375 380
   Ile Glu Phe Leu Leu Glu Gln Glu Lys Met Leu Lys Ala Asn Cys Val
   385 390 395 400
   Arg Glu Glu Glu Lys Ala Ala Glu Leu Gln Leu Lys Ser Lys Leu Phe
   405 410 415
   Ser Phe Gly Glu Phe Asn Ser Asp Ala Gln Glu Ile Leu Ile Asp Ser
   420 425 430
   Leu Ser Lys Lys Ile Thr Gln Val Tyr Lys Val Cys Ile Gly Asp Ala
   435 440 445
   Glu Asp Asp Gly Leu Asn Pro Ile Gln Lys Leu Val Lys Val Glu Ser
   450 455 460
   Arg Leu Val Glu Leu Cys Asp Leu Ile Glu Ser Ile Pro Lys Glu Asn
   465 470 475 480
   Val Glu Ala Ile Glu Arg Met Lys Gln Lys Glu Trp Arg Gln Lys Phe
   485 490 495
   Arg Asp Glu Lys Met Lys Glu Lys Gln Arg His Gln Gln Glu Arg Leu
   500 505 510
   Lys Ala Ala Leu Glu Lys Ala Val Ala Gln Pro Lys Lys Lys Leu Gly
   515 520 525
   Arg Gln Leu Val Phe His Ser Lys Pro Pro Ser Gly Asn Lys Gln Gln
   530 535 540
   Leu Pro Leu Val Asn Glu Thr Lys Thr Lys Ser Gln Glu Glu Glu Tyr
   545 550 555 560
   Phe Phe Thr